(19) Europäisches Patentamt
European Patent Office
Office européen des brevets

(11) **EP 2 298 291 A2**

(12) **EUROPEAN PATENT APPLICATION**


(43) Date of publication:
**23.03.2011 Bulletin 2011/12**

(21) Application number: **10184342.3**

(22) Date of filing: **17.06.2005**

(51) Int Cl.:
***A61K 31/00*** *(2006.01)* ***A61K 31/416*** *(2006.01)*
***A61P 35/00*** *(2006.01)* ***A61P 43/00*** *(2006.01)*
***A61P 25/28*** *(2006.01)* ***A61P 25/16*** *(2006.01)*
***A61P 9/10*** *(2006.01)* ***A61P 35/04*** *(2006.01)*

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **18.06.2004 US 580868 P**

(62) Document number(s) of the earlier application(s) in accordance with Art. 76 EPC:
**05762859.6 / 1 763 345**



(71) Applicant: **Agennix USA Inc.**
**Princeton, NJ 08540 (US)**

(72) Inventors:
- **Caligiuri, Maureen, G.**
  **Reading, MA 01867 (US)**
- **Kley, Nicolai, A.**
  **Wellesley, MA 02481 (US)**
- **Murthi, Krishna, K.**
  **Andover, MA 01810 (US)**

(74) Representative: **Dörries, Hans Ulrich**
**df-mp**
**Fünf Höfe**
**Theatinerstrasse 16**
**80333 München (DE)**


Remarks:
This application was filed on 30-09-2010 as a divisional application to the application mentioned under INID code 62.

(54) **Kinase inhibitors for treating cancers**


(57) The invention pertains to inhibitors of various kinases (e. g. S/T kinases; Tyr kinases etc.) which inhibitors are previously known as cyclin dependent kinase inhibitors (CDKs). As described therein, the inhibitors of this invention are capable of inhibiting various wild-type and mutant form kinases, including drug resistant forms of mutant kinases. Thus the subject kinase inhibitors are useful in treating a wide range of diseases / conditions associated with abnormal functions / excessive activities of the target kinases, including mutant kinases. The invention further provides methods for treating cancers, tumors and patients which are resistant or refractrory to other therapeutic agents. Pharmaceutical compositions and packaged pharmaceuticals which instructions of these inhibitors, and methods of using these inhibitors are also provided.


Figure 1

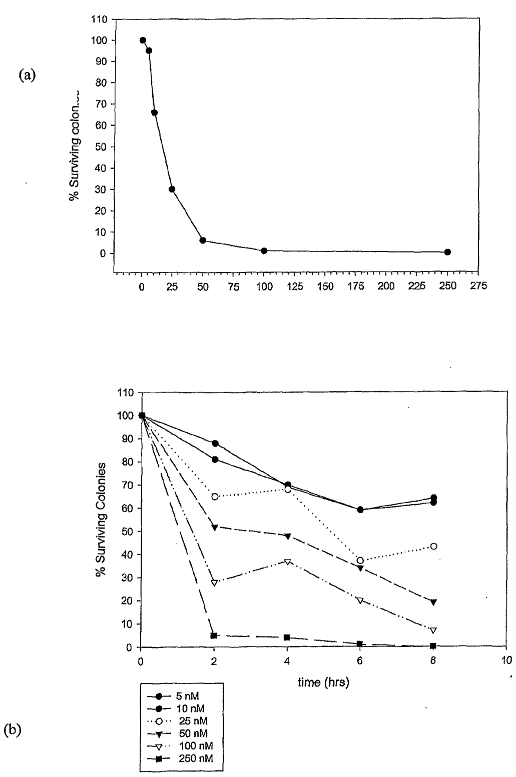


EP 2 298 291 A2

**Description**

## Background of the Invention

Nearly all aspects of cell life are controlled by the reversible phosphorylation of target proteins mediated by protein kinases. It is now well established that mutation or misregulation of members of this family of enzymes is a cause or consequence of many disease states. This finding has led pharmaceutical and biotechnology companies to develop kinase inhibitors which may have value for the treatment of a variety of diseases. Thus, kinases as a class have been and continue to be widely utilized as targets for therapeutic intervention (see for example, Broxterman and Georgopapadakou, Drug Resist Updat. 7(2): 79-87, 2004). Kinases are involved in a variety of cellular processes, including cell-cycle regulation (see, for example, Harbour et al., Cell 98: 859-869, 1999), cellular signaling (see, for example, Carpenter, Cell 37: 357-358, 1984), initiation of protein synthesis, cell proliferation, cell differentiation, apoptosis, and are essential for embryonic development (see, for example, Wagman et al., Curr Pharm Des. 10(10): 1105-37, 2004).

A number of these kinases or the pathways or processes they regulate are involved in disease onset or progression, such as cancer. Just to illustrate, Wolfel et al. (Science 269: 1281-1284, 1995) identified a mutated CDK4 as a tumor-specific antigen recognized by autologous cytolytic T lymphocytes in a human melanoma. The mutation presumably also contributes to malignant transformation in melanoma in addition to creating a tumor-specific antigen. In another well-known example, the oncogene originally called NEU was derived from rat neuro/glioblastoma cell lines (Yang-Feng et al., Cytogenet. Cell Genet. 40: 784, 1985). It encodes a tumor antigen, p185, which is serologically related to EGFR, the epidermal growth factor receptor. Coussens et al. (Science 230: 1132-1139, 1985) identified a potential cell surface receptor of the tyrosine kinase gene family and characterized it by cloning the gene. Its primary sequence is very similar to that of the human epidermal growth factor receptor. Because of the seemingly close relationship to the human EGF receptor, the authors called the gene HER2. Semba et al. (Proc. Nat. Acad. Sci. 82: 6497-6501, 1985) identified an ERBB-related gene, ERBB2, that is distinct from the ERBB gene, called ERBB 1 by these authors. Di Fiore et al. (Science 237: 178-182, 1987) later indicated that NEU and HER2 are both the same as ERBB2. Akiyama et al. (Science 232: 1644-1646, 1986) raised antibodies against a synthetic peptide corresponding to 14 amino acid residues at the COOH terminus of a protein deduced from the ERBB2 nucleotide sequence. With these antibodies, they precipitated the ERBB2 gene product from adenocarcinoma cells and demonstrated it to be a 185-kD glycoprotein with tyrosine kinase activity. Semba *et al.* (supra) observed about 30-fold amplification of ERBB2 in a human adenocarcinoma of the salivary gland. Fukushige et al. (Biochem. Biophys. Res. Commun. 134: 477-483, 1986) observed amplification and elevated expression of the ERBB2 gene in a gastric cancer cell line. Di Fiore *et al.* (supra) demonstrated that overexpression alone can convert the gene for a normal growth factor receptor, namely, ERBB2, into an oncogene. Van de Vijver et al. (New Eng. J. Med. 319: 1239-1245, 1988) found a correlation between overexpression of NEU protein and the large-cell, comedo growth type of ductal carcinoma. Science 244: 707-712, Slamon et al. (1989) described the role of HER2/NEU in breast and ovarian cancer, which together account for one-third of all cancers in women and approximately one-quarter of cancer-related deaths in females.

Indeed, a great number of drugs currently marketed or in development target kinases and hence the disease-related pathways or process such proteins regulate. (See Broxterman and Georgopapadakou, supra). These include Herceptin® (Trastuzumab, by Genentech, Inc., San Francisco, CA), which targets cancer cells that overexpress HER-2 or erb-B2 on the surface of cancer cells, including approximately 25 to 30 percent of breast cancers.

In another example, GLEEVEC® (imatinib mesylate) is a new drug approved by FDA to treat chronic myeloid leukemia, also known as CML. CML patients exhibit an overproduction of white blood cells and have a specific genetic abnormality known as the Philadelphia chromosome. GLEEVEC® is different from many other non-specific medications that are used to treat CML, in that it specifically inhibits the abnormal Bcr-Ab1 tyrosine kinase associated with the Philadelphia chromosome, thereby preventing the growth and reducing the number of abnormal white blood cells. Currently, GLEEVEC® is being tested in several clinical trials for other types of cancers, such as or primary and recurrent operable malignant GISTs (Gastro-Intestinal Stromal Tumors) expressing the Kit receptor tyrosine kinase, recurrent malignant gliomas, recurrent meningioma, advanced unresectable neuroendocrine tumor, acute myelogenous leukemia, and newly diagnosed prostate cancer.

Despite the substantial efforts and investment made by the biopharmaceutical industry to identify and develop new drug-candidates or drugs to treat disease, particularly those targeted to kinases, there still remains a need to provide new therapeutic opportunities to develop treatments for a variety of diseases, for example cancer, proliferative, degenerative and other diseases, including those listed in Table I.

This present invention provides such opportunities for new therapeutic uses of a variety of kinase inhibitors, where following the disclosure herein, such kinase inhibitors can be suitable for further pre-clinical or clinical research and development towards the treatment of a variety of diseases including cancer, proliferative, degenerative and other diseases. The further development of such new therapeutic opportunities provided by the present invention would result in one or more effective therapies, and marketed drugs, for particularly debilitating diseases including neurodegenerative

disorders such as Alzheimer's disease (AD), and including haematologial tumors such as Chronic Myelogenous Leukemia (CML) and Acute Lymphocyte Leukemia (ALL).

## Summary of the Invention

The present invention describes the novel uses of compounds that are known as potent inhibitors of the class of enzymes known as cyclin-dependent kinases (CDKs). Cyclin-dependent kinases play a key role in regulating the cell cycle machinery and are complexes consisting of two components: a catalytic subunit (the kinase) and a regulatory subunit (the cyclin). To date, nine kinase subunits (cyclin-dependent kinase 1-9) have been identified along with several regulatory subunits (cyclins A-H, K, N, and T). It was surprisingly observed that the molecules shown by the general formulae below, initially found to be CDK inhibitors, had not only a broad range of CDK inhibitory activity, but also an inhibitory activity to a variety of other kinases, and even to disease-associated mutants of such kinases.

Thus these kinase inhibitors can be effective in the treatment of diseases associated with these other, non-CDK kinases.

Thus, in a preferred embodiment, said treatment is the treatment of a disease or disorder wherein such treatment is essentially mediated and/or effected by inhibiting a kinase not being a cyclin-dependent kinase. Particularly preferred is the treatment of a disease or disorder listed in Table I.

Thus, the present invention provides new methods of treating cancer, proliferative, degenerative and other disorders or diseases by administering a therapeutically effective amount of at least one of the compounds disclosed herein or an isomeric, prodrug, tautomeric, pharmaceutically acceptable salt, N-oxide or stereoisomeric form thereof. The present invention further provides methods of treating cancer, proliferative, degenerative or other diseases by administering a therapeutically effective combination of at least one of these compounds and another anti-cancer or anti-proliferative agent.

The present invention further provides new methods of treating tumors or cancers that have become resistant or refractory to other therapies.

Furthermore, the present invention provides new methods of treating in individual with a disease condition associated with a mutant kinase.

In certain embodiments, the invention contemplates the treatment of diseases or patients using a compound, or an isomeric, prodrug, tautomeric, pharmaceutically acceptable salt, N-oxide, or stereoisomeric form thereof, having a structure of Formula I:

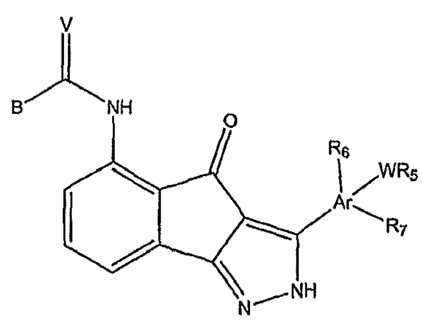

wherein

B represents $M_nR_8$;
Ar represents an aryl or heteroaryl ring;
V represents O, S, or N-CN;
W represents O, S, $S(O_2)$, C(=O), C(=S), $CH_2$, or NR";
R' represents, independently for each occurrence, H, lower alkyl, or a metal counterion;
R" represents, independently for each occurrence, H or lower alkyl;
$R_5$ represents H, $P(=O)(OR')_2$,or $M_nQ$;
$R_6$ represents H, OH, or MnQ;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
M, independently for each occurrence, represents a substituted or unsubstituted methylene group (including C(=O) and C(=S)), NR", O, S, S(O), or $S(O_2)$;

n represents an integer from 1-4 when present in B, from 0-6 when present in $R_5$ and from 1-3 when present in $R_6$; and
Q represents a substituted or unsubstituted: tertiary amino substituent, or nitrogen-containing heterocycle;
with the proviso that said compound is not compound **A37.**

In a preferred embodiment, only one of $R_5$ and $R_6$ represents H.
In certain embodiments, $R_8$ represents substituted or unsubstituted morpholino, piperazinyl, or cyclohexyl.
In certain embodiments, R" represents H.
In certain embodiments, $R_5$ represents $M_nQ$.
In certain embodiments, the occurrence of M attached to Q represents $CH_2$, $S(O_2)$, C(=S), or C(=O).
In certain embodiments, the occurrence of M attached to Q represents $CH_2$.
In certain embodiments, the occurrence of M attached to Q is C(=O).
In certain embodiments, the occurrence of M attached to Q represents substituted NR".
In certain embodiments, Q represents a substituted or unsubstituted nitrogen-containing heterocycle.
In certain embodiments, Q represents a substituted or unsubstituted tertiary amino group.
In certain embodiments, $R_8$ represents substituted or unsubstituted morpholino, piperazinyl, or cyclohexyl. In certain embodiments, R" represents H, while in certain embodiments at least one occurrence of M represents $CH_2$, substituted NR" or, when attached to Q, represents $CH_2$, $S(O_2)$, C(=S), or C(=O).
In certain embodiments, Q represents a substituted or unsubstituted nitrogen-containing heteroaryl ring. In certain other embodiments, Q represents a substituted or unsubstituted nitrogen-containing heterocycle. In certain embodiments, Q represents a substituted or unsubstituted tertiary amino group. In certain embodiments, Q represents a substituted or unsubstituted secondary amino group.
In certain embodiments, substituents include, independently for each occurrence, alkyl, oxo, acyl amino, hydroxyl, carbonyl, sulfonyl, ester, amide, $N(R")_2$, hydroxy alkyl, alkoxy alkyl, aryl, heterocyclyl, cycloalkyl, or oligo(ethylene glycol).
In certain embodiments, the subject compounds have a structure of Formula Ia:

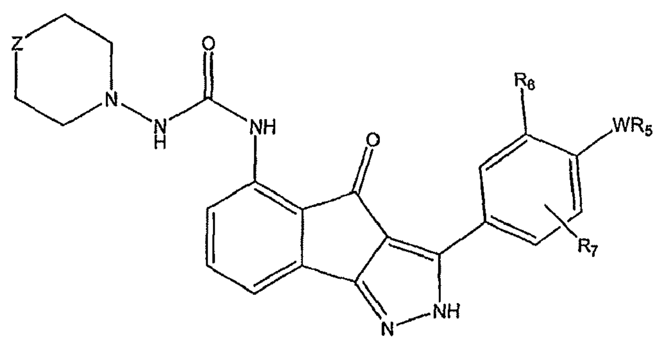

wherein

W and Z, independently, represent O or NR";
R' represents, independently for each occurrence, H, lower alkyl, or a metal counterion;
R" represents, independently for each occurrence, H or lower alkyl;
$R_5$ represents H, $P(=O)(OR')_2$,or $M_nQ$;
$R_6$ represents H, OH, or $M_nQ$;
$R_7$, independently for each occurrence, represents hydrogen, halogen, lower alkyl, or lower alkoxyl;
M, independently for each occurrence, represents a substituted or unsubstituted methylene group (including C(=S) and C(=O)), NR", O, S, S(O), $S(O_2)$;
n represents an integer from 1-5; and
Q represents a nitrogen-containing heteroaryl ring, a tertiary amino substituent, or a substituted or unsubstituted nitrogen-containing heterocycle;
with the proviso that said compound is not compound **A37.**

In a preferred embodiment, only one of $R_5$ and $R_6$ represents H.
In certain embodiments, the subject compounds with the structure of Formula I do not include compounds with the structure of Formula Ia.
In certain embodiments of Formula Ia, $R_6$ is H and $R_7$ represents a methyl or methoxy substituent ortho to W.

Preferably, $R_7$ is methyl.

In certain embodiments, Q in Formula Ia represents a tertiary amino substituent, *e.g.*, dialkyl amine. In certain embodiments, Q in Formula Ia represents a substituted or unsubstituted nitrogen containing heterocycle such as morpholine, piperidine, piperazine, or pyrrolidine.

In certain embodiments, in Formula I,

B represents $M_nR_8$;
Ar represents an aryl or heteroaryl ring;
V represents O, S, or N-CN;
W represents C(=O), C(=S), $SO_2$ or $CH_2$;
R' represents, independently for each occurrence, H, lower alkyl, a metal counterion, or alkaline earth metal counterion;
R" represents, independently for each occurrence, H or lower alkyl;
$R_5$ represents H, P(=O)(OR')$_2$, $M_nJK$, or $M_nQ$;
$R_6$ represents H, OH, or $M_nQ$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$;
K represents OR', N(R")$_2$, or N(R')$SO_2$R";
M, independently for each occurrence, represents a substituted or unsubstituted methylene group (including C(=S) and C(=O)), NR", O, S, S(O), or S(O2);
n represents an integer from 1-4 when present in B, from 0-6 when present in $R_5$ and from 1-3 when present in $R_6$; and
Q represents a substituted or unsubstituted: nitrogen-containing heteroaryl ring, secondary amino substituent, tertiary amino substituent, or nitrogen-containing heterocycle;
with the proviso that said compound is not compound **A37.**

In a preferred embodiment, only one of $R_5$ and $R_6$ represents H.

In certain embodiments, K represents OR' or N(R')$SO_2$R";

In certain embodiments, Q represents a tertiary amino substituent, *e.g.*, dialkyl amine, or a substituted or unsubstituted nitrogen containing heterocycle such as morpholine, piperidine, piperazine, or pyrrolidine.

In certain embodiments, in Formula I,

B represents $M_nR_8$;
Ar represents an aryl or heteroaryl ring;
V represents O, S, or N-CN;
W represents O, S, S($O_2$), C(=O), C(=S), $CH_2$, or NR";
R' represents, independently for each occurrence, H, lower alkyl, a metal counterion, or alkaline earth metal counterion;
R" represents, independently for each occurrence, H or lower alkyl;
$R_5$ represents H, P(=O)(OR')$_2$, $M_nJK$, or $M_nQ$;
$R_6$ represents H, OH, or $M_nQ$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$;
K represents OR', N(R")2, or N(R')$SO_2$R";
M, independently for each occurrence, represents a substituted or unsubstituted methylene group (including C(=S) and C(=O)), NR", O, S, S(O), or S($O_2$) ;
n represents an integer from 1-4 when present in B, from 0-6 when present in $R_5$ and from 1-3 when present in $R_6$; and
Q represents a substituted or unsubstituted secondary amino substituents;
with the proviso that said compound is not compound A37.

In a preferred embodiment, only one of $R_5$ and $R_6$ represents H.

In certain embodiments, K represents OR' or N(R')$SO_2$R";

In other embodiments, in Formula I,

B represents $M_nR_8$;

Ar represents an aryl or heteroaryl ring;
V represents O, S, or N-CN;
W represents O, S, $S(O_2)$, C(=O), C(=S), $CH_2$, or NR";
R' represents, independently for each occurrence, H, lower alkyl, a metal counterion, or alkaline earth metal counterion;
R" represents, independently for each occurrence, H or lower alkyl;
$R_5$ represents $M_nJK$;
$R_6$ represents H, OH, or $M_{nQ}$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$;
K represents OR', $N(R")_2$, or $N(R')SO_2R"$;
M, independently for each occurrence, represents a substituted or unsubstituted methylene group (including C(=S) and C(=O)), NR", O, S, S(O), or S(O2);
n represents an integer from 1-4 when present in B, from 0-6 when present in $R_5$ and from 1-3 when present in $R_6$; and
Q represents a substituted or unsubstituted: nitrogen-containing heteroaryl ring, secondary amino substituent, tertiary amino substituent, or nitrogen-containing heterocycle;
with the proviso that said compound is not compound **A37.**

In a preferred embodiment, $R_5$ is not $CH_2COOH$.
In certain embodiments, K represents OR' or $N(R')SO_2R"$;
In certain embodiments, Q is a substituted or unsubstituted nitrogen-containing heteroaryl ring, while $R_8$ may represent substituted or unsubstituted morpholino, piperazinyl, or cyclohexyl. In certain embodiments, R" may represent H.
M may also represent $CH_2$. In certain embodiments, W represents $CH_2$ and at least one occurrence of M represents substituted NR".
In certain embodiments, Q represents a substituted or unsubstituted secondary amino group. In certain embodiments, Q represents a substituted or unsubstituted tertiary amino group. In certain embodiments, Q represents a substituted or unsubstituted nitrogen-containing heterocycle.
In certain embodiments, Q represents a substituted or unsubstituted: nitrogen-containing heteroaryl ring, tertiary amino substituent, or nitrogen-containing heterocycle.
In certain embodiments, $R_5$ represents $M_nQ$ and Q represents a substituted or unsubstituted: nitrogen-containing heteroaryl ring, tertiary amino substituent, or nitrogen-containing heterocycle.
In certain embodiments, Q represents a substituted or unsubstituted tertiary amino group.
In certain embodiments, Q represents a substituted or unsubstituted nitrogen-containing heterocycle.
In certain embodiments, $R_5$ represents $M_nQ$ and Q represents a substituted or unsubstituted secondary amino group.
In certain embodiments, $R_5$ represents $M_nQ$ and Q is a substituted or unsubstituted nitrogen-containing heteroaryl ring.
In certain embodiments, $R_8$ represents substituted or unsubstituted morpholino, piperazinyl, or cyclohexyl.
In certain embodiments, R" represents H.
In certain embodiments, W represents $CH_2$.
In certain embodiments, M when attached to Q is $CH_2$, $S(O_2)$, C(=S), or C(=O).
In certain embodiments, M when attached to Q is $CH_2$.
In certain embodiments, the occurrence of M attached to Q is $CH_2$, $S(O_2)$ C(=S), or C(=O).
In certain embodiments, V is O, $M_n$ in B represents NH, and R8 has the structure:

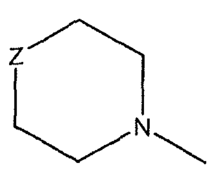

where Z represents O or NR".
In certain embodiments, Ar represents a phenyl ring and $R_6$ and $R_7$ represent H for all occurrences.

Certain embodiments include a compound, or a prodrug, isomeric, tautomeric, pharmaceutically acceptable salt, N-oxide, or stereoisomeric form thereof, having a structure of Formula II:

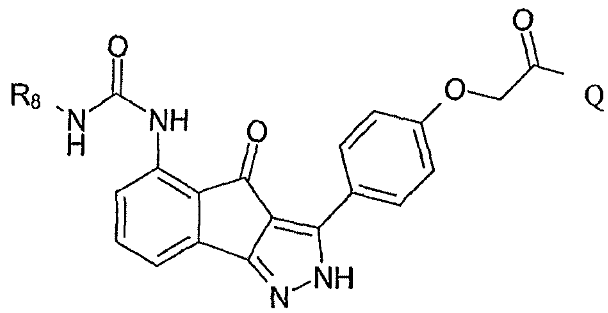

wherein

$R_8$ represents a substituted or unsubstituted heterocycle;
Q represents a substituted or unsubstituted: secondary amino substituent, tertiary amino substituent, or substituted or unsubstituted nitrogen-containing heterocycle.

As noted above, $R_8$ may represent a morpholino or piperazinyl ring in certain embodiments.
In certain embodiments, as noted above, Q may represent piperazine, morpholine, piperidine, pyridine, pyrrole, oxazole, isoxazole, imidazole, or pyrazole.
In certain embodiments, the invention provides novel treatment methods of the various disease conditions described above, using a compound, or a prodrug, isomeric, tautomeric, pharmaceutically acceptable salt, N-oxide, or stereoisomeric form thereof, having a structure of Formula III:

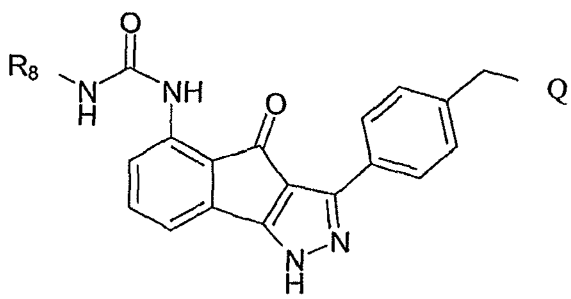

**Formula III**

wherein

$R_8$ represents a substituted or unsubstituted heterocycle;
Q represents a substituted or unsubstituted secondary amino substituents, tertiary amino substituent, or substituted or unsubstituted nitrogen-containing heterocycle.

In one embodiment, Formula III does not include a compound selected from A47, A49, A51 and A82.
In one embodiment, $R_8$ represents a morpholino or piperazine ring.
In one embodiment, Q represents pyrrolidine.
In one embodiment, the compound of formula III is selected from the group of B 1 to B20 and C2.
In one embodiment, the compound is **B16:**

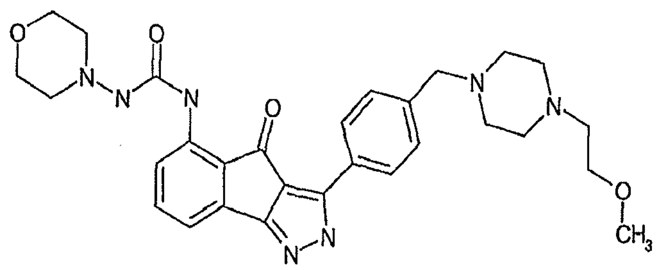

Certain embodiments include the use of compounds selected from the group of A34, A36, A37, A44, A46, and A76 to A81, or prodrugs, isomers, tautomers, pharmaceutically acceptable salts, N-oxides, or stereoisomeric forms thereof.

Certain embodiments include the use of compounds selected from the group of A47, A49, A51 and A82, or prodrugs, isomers, tautomers, pharmaceutically acceptable salts, N-oxides, or stereoisomeric forms thereof.

Certain embodiments may include pharmaceutical compositions comprising a pharmaceutically acceptable excipient and a compound of any of the type disclosed herein, while certain embodiments include a method of treating cancer, proliferative, degenerative or other diseases including a hyperproliferative disorder, comprising administering to an animal a compound of any of the type disclosed herein.

In certain embodiments, the compounds disclosed herein may be applied to methods of inhibiting proliferation of a cell, comprising contacting the cell with a compound of the type disclosed herein, or to methods of treating a viral infection (such as infection caused by a human immunodeficiency virus (HIV)), comprising administering to a mammal a compound of the type disclosed herein. In preferred embodiments, the proliferation of cells occurs in benign tumors or hyperplasia. In other preferred embodiments, the proliferation of cells occurs in malignant tumors cancers (solid or leukemia / lymphoma). Such tumors / cancers may have been subjected to other treatments, such as chemotherapy / radiotherapy, but have relapsed, with or without new mutations in the tumor / cancer. Alternatively, no such tumors / cancers have been treated before by any other means.

In yet other embodiments, the compounds disclosed herein may be applied to methods of treating one or more disease conditions, comprising contacting the cell with a compound of the type disclosed herein. In certain embodiments, the disease conditions are selected from those listed in Table I. Certain embodiments contemplate methods for the treatment or prevention of alopecia induced by chemotherapy or radiation therapy, comprising administering to a mammal a compound of the type disclosed herein conjointly with one or more chemotherapeutics or radiation therapy. The compounds disclosed herein may also be used for the manufacture of a medicament or packaged pharmaceuticals.

In certain embodiments, the present invention provides a novel packaged pharmaceutical composition comprising a pharmaceutically acceptable carrier and a therapeutically effective amount of a compound of formula (I) or (II), or any other compound disclosed herein, or an isomeric, prodrug, tautomeric, pharmaceutically acceptable salt, N-oxide or stereoisomeric form thereof. The packaged pharmaceutical composition may additionally include specific instructions for treating cancer, proliferative, degenerative or other diseases.

In another embodiment, the present invention provides a novel method of treating cancer, proliferative, degenerative or other diseases comprising administering to a host in need of such treatment a therapeutically effective amount of a compound of formula (I) or (II), or any other compound disclosed herein, or an isomeric, prodrug, tautomeric, pharmaceutically acceptable salt, N-oxide or stereoisomeric form thereof.

In another embodiment, the present invention provides a novel method of treating cancer, proliferative, degenerative or other diseases comprising administering to a host in need of such treatment a therapeutically effective amount of: (a) a compound of formula (I) or (II), or any other compound disclosed herein, or an isomeric, prodrug, tautomeric, pharmaceutically acceptable salt, N-oxide or stereoisomeric form thereof; and (b) at least one compound selected from anti-cancer agents and anti-proliferative agents.

As described herein, the inhibitors of this invention are capable of inhibiting a large number of non-CDK kinases which are involved in a variety of disease conditions including cancer. Thus such compounds would be useful for treating subjects having disorders associated with excessive cell proliferation, such as hyperplasia or cancer (including drug-resistant cancer or relapsed cancer, or cancer that has progressed to an advanced stage including invasion and/or metastasis), psoriasis, immunological disorders involving unwanted leukocyte proliferation, in the treatment of restenosis and other smooth muscle cell disorders, and the like.

In other embodiments, the disease is not taken from those diseases or disorders where involvement of cyclin-dependent kinases (CDKs) has been shown, such as cancer, psoriasis, immunological disorders involving unwanted leukocyte proliferation, in the treatment of restenosis and other smooth muscle cell disorders, or in the inhibition of human immunodeficiency virus type I (HIV-I) transcription. In a preferred embodiment, the disease is not taken from

diseases or disorders wherein treatment is essentially mediated and/or effected by inhibiting a cyclin-dependent kinase.

In one embodiment, the subject compounds are used to inhibit a kinase selected from the list shown in Table II. In certain embodiments, such kinase is inhibited by administering to a host, individual or patient in need of such treatment a therapeutically effective amount of said compound.

In another embodiment, the subject compounds are used to treat a disease susceptible to treatment by inhibition of a kinase selected from the list shown in Table II. In certain embodiments, such treatment comprises administering to a host, individual or patient in need of such treatment a therapeutically effective amount of said compound.

In other embodiments, the subject compounds can be used to inhibit a protein related to a kinase listed in Table II, or another kinase listed herein.

In certain embodiments, the kinase does not include one or more certain specified kinase selected from the group consisting of: protein kinase C (PKC), PKA, her2, raf 1, MEK1, MAP kinase, EGF receptor, PDGF receptor, IGF receptor, PI3 kinase, wee1 kinase, Src, and Ab1.

All embodiments disclosed above are intended to be combined with one or more other embodiments, regardless of whether they are initially disclosed under the same aspect of the invention, so long as the combination(s) does not render the invention inoperable.

## Brief Description of the Drawings

Figure 1 illustrates the irreversible effect of compound **A37** on clongeneic survival ofHCT-116 tumor cells, as represented by (a) dose response; and (b) time-course.

Figure 2 depicts the irreversible effect of compound **B16** on clongeneic survival of HCT-116 tumor cells, as represented by time-course.

Figure 3 presents results obtained from the HCT-116 xenograft tumor assay with various compounds of the invention.

Figure 4 shows the results obtained from the A2780 xenograft tumor assay with compound **A37,** represented by (a) time-course of tumor size at various doses; and (b) table of salient metrics from the assay.

Figure 5 shows the results obtained from the PC3 xenograft tumor assay with compound **A37,** represented by (a) time-course of tumor size at various doses; and (b) table of salient metrics from the assay.

Figure 6 shows the results obtained from the A2780 xenograft tumor assay with compound **B16,** represented by (a) time-course of tumor size at various doses; and (b) table of salient metrics from the assay.

## Detailed Description of Exemplary Embodiments

### I. Overview

The invention pertains to the novel use of classes of compounds previously known as cyclin dependent kinase inhibitors (CKi's), as inhibitors of other non-CDK kinases and signaling transduction pathways associated therewith. As described herein, the inhibitors of this invention are capable of inhibiting a number of non-CDK kinases, including receptor tyrosine kinases, Ser/Thr kinases, and consequently may be useful in modulating cell growth, differentiation, apoptosis, and other associated cellular functions. Such compounds would thus be useful for treating subjects having disorders associated with excessive kinase activities, such as those caused by overexpression of wild-type kinases and/or mutation-activated kinase activities. The compounds of the invention can be used for the treatment of cancer and other disorders (see greater details below).

The invention is partly based on the surprising discovery that certain compounds previously identified as CDK inhibitors also inhibit a broad range of kinases: not only Cyclin Dependent Kinases (CDK) regulating cell cycle, such as Cdk2, Cdk4, Cdk6, but also other protein kinases including those described herein, and protein kinase C, her2, raf 1, MEK1, MAP kinase, EGF receptor, PDGF receptor, IGF receptor, PI3 kinase, wee1 kinase, Src, Ab1 and thus be effective in the treatment of diseases associated with these other protein kinases. Since many different signal transduction pathways utilize several shared downstream kinase cascades, inhibition of certain key downstream kinases may indirectly inhibit abnormal activities originating from some upstream kinases, even though the inhibitors may not directly inhibit those abnormal upstream kinases per se. For example, if a subject compound does not inhibit PDGF receptor per se, but inhibits its downstream MAPK, diseases associated with abnormal PDGF receptor activity may nevertheless be treated by the inhibitor, since the inhibitor blocks the "bottleneck" downstream kinase necessary to transmit the excessive signal from the PDGF receptor.

Another aspect of the invention relates to the ability of the subject compounds to treat drug-resistant cancers, including those that have been previously treated by other chemotherapeutic agents.

A further aspect of the invention relates to the surprising finding that the subject compounds can inhibit wild-type as well as mutant forms of certain kinases, such as the CML-associated c-Abl kinase, and GIST-associated c-kit kinase. This characteristic is especially valuable since other kinase inhibitors, such as the commercially available inhibitor GLEEVEC®, can only effectively inhibit the wild-type (such as the catalytic domain with wild-type sequences of the p210 Bcr-Ab1 kinase), but not the mutant form of the kinase (such as the Bcr-Abl T315I mutant).

Inhibition of a broad spectrum of kinases by two of the representative subject compounds, **A37** and **B16,** are listed below in Table II.

Not surprisingly, **A37** inhibits DNA replication (24 hour incubation, $IC_{50}$ =16 nM); induces cell cycle arrest - predominantly G1 arrest, leading to endo-reduplication and apoptosis; and inhibits Rb phosphorylation at Serine-780.

Similarly, **B16** also inhibits DNA replication (24 hour incubation, $IC_{50}$ =10 nM); induces cell cycle arrest - predominantly G1 arrest, leading to endo-reduplication and apoptosis.

Interestingly, **B16** is more active on quiescent tumor cells ($IC_{50}$ = 0.011 μM) as compared to that on normal cells ($IC_{50}$ =10.9 μM).

Another aspect of the invention provides uses of the information that a subject compound inhibits or binds to a kinase disclosed herein. Such use can lead to the identification of novel kinase inhibitors. In one embodiment of this aspect, the information that a subject compound binds to a given kinase disclosed herein is used. However, as will be readily understood, in other embodiments of this aspect, the information that a given subject compound inhibits or binds to more than one kinase, such as 2, about 5, about 8, about 10, about 15, about 20 or about 25 kinases, may be readily utilized. In certain embodiments of this aspect, the kinases do not include CDKs. In another embodiment, the information that a given kinase is inhibited or bound by more than one subject kinase, such as 2, more than 5, more than 10 or more than about 20 subject compounds, may be readily utilized.

In another certain embodiment or this aspect, the invention provides a method to design (*e.g.,* de novo or to optimize an existing structure) novel kinase inhibitors, or improve the binding affinity and/or specificity of certain subject kinase inhibitors, based on the co-crystal structure of a subject kinase inhibitor and the kinase it inhibits. Mutational analysis based on the crystal structure of an imatinib-related compound bound to the Abl kinase established the relevance of amino acids 315 and 253 as critical for efficient imatinib (GLEEVEC®) binding (Corbin et al., Blood 96: 470a, 2000; Corbin et al., J. Biol. Chem. 277: 32214-32219, 2002; Schindler et al., Science 289: 1938-1942, 2000). Similar analysis can be used to design compounds either structurally similar to imatinib or one or more of the subject compounds (*e.g.*, **A37** and/or **B16),** which compounds may exhibit more superior binding affinity specificity to a given kinase target. Obviously, the similar analysis can also be used to improve the binding between any of the subject compound with its kinase target(s), thus generating better fit inhibitor / target complexes for better efficacy and/or less side effect. In one embodiment, such a method is used to design a compound with improved specificity to one or more kinases, or to reduce specificity to one or more kinases. For example, following the disclosure herein of the binding and inhibition of JNK kinases by compound **A37** herein, it can be possible to uses this information and structure-based design to design or identify a compound that is different to **A37** with improved characteristics, such as improved specificity to JNK kinases, potentially providing such second compound with improved therapeutic utility for treating inflammatory diseases.

NCBI (National Center for Biotechnology Information) maintains a constantly being updated structure database, which contains many kinases - inhibitor co-crystal structures. Software for computer-assisted drug design (CCAD) are widely available. For example, Computer-Assisted Drug Design (J. Phillip Bowen and Michael Cory, published in Encyclopedia of Pharmaceutical Technology, ISBN: 0-8247-2826-2) provides an introduction to computational chemistry, molecular modeling, and CADD theory. Coupled with computational chemistry, such tools can be readily used to facilitate the de novo or improvement design of new candidate inhibitors, based on the known co-crystal structure.

Another related aspect of the invention relates to any compounds designed or improved based on the method described above.

Another aspect of the invention relates to a method of identifying additional kinase inhibitors to specific kinase targets, comprising using any of the kinase inhibition assays to screen among a library of candidate compounds that might inhibit the target kinase. In one embodiment, the assay is a binding competition assay comprising a subject compound and a kinase selected from Table II. In such an embodiment, candidate compounds are tested for their ability to competitively displace the binding of the subject compound from the kinase. Such binding or displacement can be detected by a number of methods known in the art. In another embodiment, since it is now know that certain mutant kinases associated with refractory or relapsed diseases, such as cancer, can still be inhibited by compounds such as those disclosed herein, these mutant kinases, which might previously have been thought of as uninhibitable, can now serve as targets in such kinase inhibitor screens.

Another related aspect of the invention relates to any compounds obtained through such a screen.

## II. Definitions

As used herein, the following terms and expressions have the indicated meanings. The compounds of the present invention may contain an asymmetrically substituted carbon atom, and may be isolated in optically active or

racemic forms. It is well known in the art how to prepare optically active forms, such as by resolution of racemic forms or by synthesis from optically active starting materials. All chiral, diastereomeric, racemic forms and all geometric isomeric forms of a structure are intended, unless the specific stereochemistry or isomer form is specifically indicated. All processes used to prepare compounds of the present invention and intermediates made therein are considered to be part of the present invention.

The present invention is intended to include all isotopes of atoms occurring on the present compounds. Isotopes include those atoms having the same atomic number but different mass numbers. By way of general example and without limitation, isotopes of hydrogen include tritium and deuterium. Isotopes of carbon include $^{12}C$ and $^{14}C$.

The term "alkyl" is intended to include both branched and straight-chain saturated aliphatic hydrocarbon groups having the specified number of carbon atoms. Examples of alkyl include but are not limited to, methyl, ethyl, *n*-propyl, *i*-propyl, *n*-butyl, *s*-butyl, *t*-butyl, *n*-pentyl, and *s*-pentyl. In addition, the term is intended to include both unsubstituted and substituted alkyl groups, the latter referring to alkyl moieties having one or more hydrogen substituents replaced by, but not limited to, halogen, hydroxyl, carbonyl, alkoxy, ester, ether, cyano, phosphoryl, amino, imino, amido, sulfhydryl, alkythio, thioester, sulfonyl, nitro, heterocyclo, aryl or heteroaryl. It will also be understood by those skilled in the art that the substituted moieties themselves can be substituted as well when appropriate. The term "lower alkyl" refers to those alkyl groups having from 1 to 6 carbon atoms, preferably from 1 to 4 carbon atoms, and the term "lower alkoxy" refers to such lower alkyl groups attached to an oxygen atom. In certain embodiments, alkyl substituents are preferably lower alkyl substituents.

The terms "halo" or "halogen" as used herein refer to fluoro, chloro, bromo and iodo.

The term "aryl" is intended to mean an aromatic moiety such as, but not limited to phenyl, indanyl or naphthyl.

The terms "cycloalkyl", and "bicycloalkyl" are intended to mean any stable ring system, which may be saturated or partially unsaturated. Examples of such include, but are not limited to, cyclopropyl, cyclopentyl, cyclohexyl, norbornyl, bicyclo[2, 2]nonane, adamantyl, or tetrahydronaphthyl (tetralin).

As used herein, "carbocycle" or "carbocyclic residue" is intended to mean any stable 3- to 7-membered monocyclic or bicyclic or 7- to 13-membered bicyclic or tricyclic, any of which may be saturated, partially unsaturated, or aromatic. Examples of such carbocycles include, but are not limited to, cyclopropyl, cyclobutyl, cyclopentyl, cyclohexyl, cycloheptyl, adamantyl, cyclooctyl, [3.0]bicyclooctane, [4.0]bicyclononane,[4.0]bicyclodecane (decalin), [2.2]bicyclooctane, fluorenyl, phenyl, naphthyl, indanyl, adamantyl, or tetrahydronaphthyl (tetralin).

As used herein, the term "heterocycle" or "heterocyclic system" is intended to mean a stable 5- to 7-membered monocyclic or bicyclic or 7- to 10-membered bicyclic heterocyclic ring which is saturated, partially unsaturated, or unsaturated (aromatic/heteroaryl), and which consists of carbon atoms and from 1 to 4 heteroatoms independently selected from the group consisting of N, O and S and including any bicyclic group in which any of the above-defined heterocyclic rings is fused to a benzene ring. The nitrogen and sulfur heteroatoms may optionally be oxidized. The heterocyclic ring may be attached to its pendant group at any heteroatom or carbon atom that results in a stable structure. The heterocyclic rings described herein may be substituted on carbon or on a nitrogen atom if the resulting compound is stable. If specifically noted, a nitrogen in the heterocycle may optionally be quaternized. In certain embodiments, when the total number of S and O atoms in the heterocycle exceeds 1, then these heteroatoms need not be adjacent to one another. It is preferred that the total number of S atoms in the heterocycle is not more than 1. As used herein, the term "aromatic heterocyclic system" is intended to mean a stable 5- to 7-membered monocyclic or bicyclic or 7- to 10-membered bicyclic heterocyclic aromatic ring which consists of carbon atoms and from 1 to 4 heterotams independently selected from N, O and S. It is preferred that the total number of S and 0 atoms in the aromatic heterocycle is not more than 1. Examples of heterocycles include, but are not limited to, 1H-indazole, 2-pyrrolidonyl, 2H16H dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazalonyl, carbazolyl, 4aH-carbazolyl, P-carbolinyl, chromanyl, chromenyl, cinnolinyl, decahydroquinolinyl, 2H,6H dithiazinyl, dihydrofuro[2,3-b]tetrahydrofuran, furanyl, furazanyl, imidazolidinyl, imidazolinyl, imidazolyl, 1H-indazolyl, indolenyl, indolinyl, indolizinyl, indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazoiy1,1,2,4-oxadiazolyl, 1,2,5-oxadiazolyl, 1,3,4-oxadiazolyl, oxazolidinyl, oxazolyl, oxazolidinylperimidinyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, piperidonyl, 4-piperidonyl, pteridinyl, purinyl, pyranyl, pyrazinyl, pyrazolidinyl, pyrazolinyl, pyrazolyl, pyridazinyl, pyridooxazole, pyridoimidazole, pyndothiazole, pyridinyl, pyridyl, pyrimidinyl, pyrrolidinyl, pyrrolinyl, pyrrolyl, quinazolinyl, quinolinyl, 4H-quinolizinyl, quinoxalinyl, quinuclidinyl, carbolinyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazoly1,1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl,1,2,5-triazolyl, 1,3,4-triazolyl, xanthenyl. Preferred heterocycles include, but are not limited to, pyridinyl, furanyl, thienyl, pyrrolyl, pyrazolyl, imidazolyl, indolyl, benzimidazolyl, 1H-indazolyl, oxazolidinyl, benzotriazolyl, benzisoxazolyl, oxindolyl, benzoxazolinyl, or isatinoyl. Also included are fused ring and spiro compounds containing, for example, the

above heterocycles.

The term "metabolite", as used herein, refers to any substance produced by the metabolism or by a metabolic process. Metabolism, as used herein, refers to the various physical/chemical/biochemical/phamacological reactions involved in the transformation of molecules or chemical compounds occurring in the cell, tissue, system, body, animal, individual, patient or human therein. The term "metabolite", as used herein, also includes a substance derived from a drug by physical, chemical, biological or biochemical processes in the body or cell after the drug is administered. Raynaud et al. (1996 Cancer Chemother. Pharmacol. 38: 155-162) shows exemplary metabolites of satraplatin.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids.

For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, EtOAc, ethanol, isopropanol, or acetonitrile are preferred. Lists of suitable salts are found in Remington's Pharmaceutical Sciences, 18th ed., Mack Publishing Company, Easton, PA, 1990, p. 1445, the disclosure of which is hereby incorporated by reference.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

"Prodrugs", as the term is used herein, are intended to include any covalently bonded carriers which release an active parent drug of the present invention *in vivo* when such prodrug is administered to a mammalian subject. Since prodrugs are known to enhance numerous desirable qualities of pharmaceuticals (*i.e.*, solubility, bioavailability, manufacturing, etc.) the compounds of the present invention may be delivered in prodrug form. Thus, the present invention is intended to cover prodrugs of the presently claimed compounds, methods of delivering the same, and compositions containing the same. Prodrugs of the present invention are prepared by modifying functional groups present in the compound in such a way that the modifications are cleaved, either in routine manipulation or *in vivo,* to the parent compound. Prodrugs include compounds of the present invention wherein a hydroxy, amino, or sulfhydryl group is bonded to any group that, when the prodrug of the present invention is administered to a mammalian subject, it cleaves to form a free hydroxyl, free amino, or free sulfydryl group, respectively. Examples of prodrugs include, but are not limited to, acetate, formate, and benzoate derivatives of alcohol and amine functional groups in the compounds of the present invention.

"Substituted" is intended to indicate that one or more hydrogens on the atom indicated in the expression using "substituted" is replaced with a selection from the indicated group(s), provided that the indicated atom's normal valency is not exceeded, and that the substitution results in a stable compound. When a substituent is keto or oxo (*i.e.*, =O) group, then 2 hydrogens on the atom are replaced. Keto/oxo substituents are not present on aromatic moieties. Exemplary substituents include, for example, an alkyl, a perfluoroalkyl (such as trifluoromethyl), a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a carbocyclyl, a heterocyclyl, an aralkyl, a heteroaralkyl, or an aromatic or heteroaromatic moiety. It will be understood by those skilled in the art that substituents, such as heterocyclyl, aryl, alkyl, etc., can themselves be substituted, if appropriate.

The term "therapeutically effective amount" of a compound of this invention means an amount effective to inhibit the class of enzymes known as cyclin-dependent kinases or treat the symptoms of cancer or other proliferative or other diseases in a host.

As used herein, the term "anti-cancer" or "anti-proliferative" agent includes, but is not limited to, altretamine, busulfan, chlorambucil, cyclophosphamide, ifosfamide, mechlorethamine, melphalan, thiotepa, cladribine, fluorouracil, floxuridine, gemcitabine, thioguanine, pentostatin, methotrexate, 6-mercaptopurine, cytarabine, carmustine, lomustine, streptozotocin, carboplatin, cisplatin, oxaliplatin, iproplatin, tetraplatin, lobaplatin, JM216, JM335, satraplatin, fludarabine,

aminoglutethimide, flutamide, goserelin, leuprolide, megestrol acetate, cyproterone acetate, tamoxifen, anastrozole, bicalutamide, dexamethasone, diethylstilbestrol, prednisone, bleomycin, dactinomycin, daunorubicin, doxirubicin, idarubicin, mitoxanthrone, losoxantrone, mitomycin-c, plicamycin, paclitaxel, docetaxel, topotecan, irinotecan, 9-amino camptothecan, 9-nitro camptothecan, GS-211, JM 118, etoposide, teniposide, vinblastine, vincristine, vinorelbine, procarbazine, asparaginase, pegaspargase, octreotide, estramustine, and hydroxyurea.

III. Kinase Inhibitor Compounds

In another embodiment, the present invention also provides novel treatment methods of the various disease conditions described above, using compounds, including isomeric, prodrug, tautomeric, pharmaceutically acceptable salt, N-oxide, or stereoisomeric forms thereof, having a structure of Formula I:

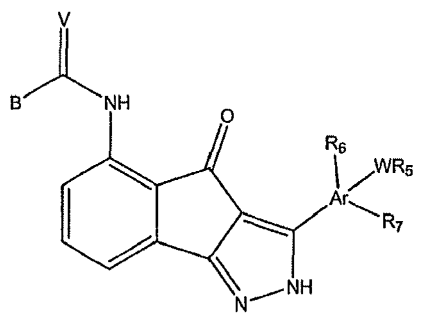

wherein

B represents $M_nR_8$.
Ar represents an aryl or heteroaryl ring, such as a phenyl ring;
V represents O, S, or N-CN, preferably O or S;
W represents O, S, $S(O_2)$, C(=O), C(=S), $CH_2$, or NR";
R' represents, independently for each occurrence, H, lower alkyl, or a metal counterion, such as an alkali or alkaline earth metal counterion;
R" represents, independently for each occurrence, H or lower alkyl, preferably H;
$R_5$ represents H, $P(=O)(OR')_2$, $M_nJK$, or $M_nQ$;
$R_6$ represents H, OH, or $M_nQ$, preferably provided that one and only one of $R_5$ and $R_6$ represents H;
$R_7$, independently for each occurrence, represents H, halogen, hydroxyl, lower alkyl, such as methyl, or lower alkoxyl, such as methoxy;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$;
K represents OR', $N(R")_2$ or $N(R')SO_2R"$;
M, independently for each occurrence, represents a substituted or unsubstituted methylene group (*e.g.*, substituted with lower alkyl, oxo, hydroxyl, etc.), NR", O, S, S(O), or $S(O_2)$, preferably NR" or $CH_2$, or, when attached to W or Q, $CH_2$, $S(O_2)$, C(=S), or C(=O);
n represents an integer from 0-10, preferably 1-7 or even 1-4 when present in B, from 0-6 when present in $R_5$ and from 1-3 when present in $R_6$; and
Q represents a substituted or unsubstituted: nitrogen-containing heteroaryl ring, *e.g.,* pyrrole, tetrazole, oxazole, oxadiazole, isoxazole, imidazole, or pyrazole; secondary amino substituent, *e.g.*, monoalkyl amine, arylalkyl amine, heteroarylalkyl amine; tertiary amino substituent, *e.g.,* a dialkylamine; or nitrogen-containing heterocycle such as morpholine, piperidine, piperazine, pyridine, or pyrrolidine.

In a preferred embodiment, said treatment is the treatment of a disease or disorder wherein such treatment is essentially mediated and/or effected by inhibiting a kinase not being a cyclin-dependent kinase. Particularly preferred is the treatment of a disease or disorder listed in Table I.

In certain embodiments, K represents OR' or $N(R')SO_2R"$;

In certain embodiments, when K represents $N(R')SO_2R"$, R" represents lower alkyl.

In certain embodiments where $R_5$ is $M_nJK$, $R_5$ is not $CH_2COOH$.

In certain embodiments, appropriate substituents include, independently for each occurrence, alkyl, oxo, hydroxyl, alkoxy, hydroxy-alkoxy, carbonyl, sulfonyl, ester, amide, $N(R'')_2$, alkyl halide, acyl amino, or substituted or unsubstituted aryl, heteroaryl, heterocyclyl, cycloalkyl, oligo(ethylene glycol) etc. It will be apparent to those skilled in the art that aryl and heteroaryl may employ any suitable substituent, including any of those listed above.

In certain embodiments, $R_8$ represents any of the following substituents: alkyl, alkenyl, alkynyl, alkoxy, hydroxylalkoxy, aryl, amine, or heteroaryl. In certain embodiments, any of the aforementioned substituents may, in turn, optionally be substituted by any of the mentioned substituents, or even by halo, -CN, $N_3$, $NO_2$, or haloalkyl. Other suitable substituents may also include, for example, cyclohexyl, =O, carbonyl, sulfonyl, carboxyl, sulfoxyl, amide, heterocycle, ester, or ether.

In certain embodiments, at least one occurrence of M is substituted NR" when attached to $R_8$ and when present in $R_5$.

In certain embodiments, including any of the embodiments above, $R_8$ has the following form:

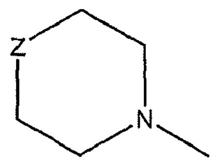

where Z represents O or NR". In certain embodiments $R_8$ represents morpholino or cyclohexyl. In certain such embodiments, $M_n$ is NR", preferably NH. In certain embodiments V is O.

In certain embodiments, W represents $CH_2$. In certain such embodiments, at least one occurrence of M is substituted NR".

In preferred embodiments, $R_5$ W and $R_6$ are adjacent (ortho) to each other on Ar, and are preferably not adjacent (ortho) to the bond to the tricyclic core.

In certain embodiments, V represents S or N-CN. In some embodiments, Ar represents a heteroaryl ring.

In certain embodiments of Formula I, W represents O, S or NR". In certain embodiments $R_5$ represents H, P(=O)(OR')$_2$, or $M_nQ$. In certain embodiments $R_7$ represents, independently for each occurrence, halogen, hydroxyl, lower alkyl, such as methyl, or lower alkoxyl, such as methoxy. In certain embodiments n represents an integer from 0-5, preferably from 1-5, and more preferably from 2-4 when present in $R_5$.

In certain embodiments of Formula I, W represents O, $CH_2$, C(=O), C(=S), or $SO_2$. In certain embodiments, $R_5$ represents $M_nJK$ or $M_nQ$. In certain embodiments, $R_6$ and $R_7$ represent H. In certain embodiments, M represents C(=O) or $CH_2$. In certain embodiments, n is preferably 1, while in other embodiments n may be 0. In certain embodiments, J is preferably C(=O), and K is OR' or N(R')$SO_2$R". In certain embodiments, N(R')$SO_2$R" is $NHSO_2$R".

In certain embodiments, Q represents a substituted or unsubstituted nitrogen-containing heteroaryl ring. In certain embodiments, Q represents a substituted or unsubstituted heteroaryl ring, *e.g.,* a five-membered or six-membered ring, containing at least two nitrogen atoms. In certain embodiments, Q may be substituted or unsubstituted occurrences of tetrazole or oxadiazole. In certain embodiments Q may be substituted or unsubstituted occurrences of pyridine, piperidine, or piperazine.

In certain embodiments, Q represents a secondary amino substituent. In certain such embodiments, the substituent on the secondary amino substituent is selected from alkyl, alkoxyalkyl, hydroxyalkyl, and hydroxyalkoxyalkyl.

In certain embodiments of Formula I, W represents C(=O), $SO_2$, or C(=S), $R_6$ and $R_7$ represent H, and $R_5$ represents $M_nQ$, where n represents 0 and Q represents a substituted or unsubstituted nitrogen-containing heteroaryl ring. In certain embodiments, W represents $CH_2$, $R_6$ and $R_7$ represent H, and $R_5$ represents $M_nQ$, where n represents 0 and Q represents a substituted or unsubstituted nitrogen-containing heteroaryl ring.

In certain embodiments, W represents S, O, or NR", R6 and $R_7$ represent H, and $R_5$ represents $M_nJK$, where n is an integer from 1-3, J is C(=O), and K is OR' or N(R')$SO_2$R".

In certain embodiments, W represents S, O, or NR", $R_6$ and $R_7$ represent H, and $R_5$ represents $M_nQ$, where n is an integer from 1-3, and Q is a substituted or unsubstituted five-membered nitrogen-containing heterocycle. In such embodiments, n is preferably 1. In certain embodiments Q contains at least two nitrogen atoms.

In certain embodiments, W represents S, O, or NR", $R_6$ and $R_7$ represent H, and $R_5$ represents $M_nQ$, where n represents an integer from 1-3, and Q is a substituted or unsubstituted six-membered nitrogen-containing heterocycle. In certain of such embodiments, n is 2, and $M_n$ represents $CH_2C(=O)$.

In certain embodiments, W represents O, S, or NR", $R_6$ and $R_7$ represent H, and $R_5$ represents $M_nQ$, where M is $CH_2$, n is an integer from 1-3, and Q is a substituted or unsubstituted nitrogen-containing heterocycle.

In certain embodiments, where Q represents a substituted nitrogen-containing heterocycle, *e.g.*, piperazine, morpholine, piperidine, pyridine, thiazole, oxadiazole, tetrazole, pyrrole, etc., suitable substituents include substituted

or unsubstituted occurrences of alkyl, amino-alkyl, alkoxyl, aralkyl (*e.g.,* benzyl), aryl (*e.g.*, phenyl), and heteroaryl, *e.g.*, oxazyl, piperazyl, pyridyl, pyrrolyl. In certain such embodiments where Q contains a nitrogen not attached to M, that nitrogen is substituted, *e.g.*, by such a substituent.

In certain embodiments, the present invention provides novel treatment methods of the various disease conditions described above, using compounds, including isomeric, prodrug, tautomeric, pharmaceutically acceptable salt, N-oxide, or stereoisomeric forms thereof, having a structure of Formula Ia:

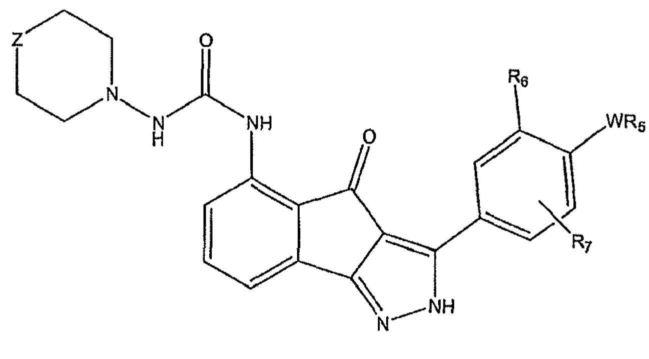

wherein

- W and Z, independently, represent O or NR'';
- R' represents, independently for each occurrence, H, lower alkyl, or a metal counterion, such as an alkali or alkaline earth metal counterion;
- R'' represents, independently for each occurrence, H or lower alkyl, preferably H;
- $R_5$ represents H, P(=O)(OR')$_2$, or $M_nQ$;
- $R_6$ represents H, OH, or $M_nQ$, preferably provided that one and only one of $R_5$ and $R_6$ represents H;
- $R_7$, independently for each occurrence, represents hydrogen, halogen, lower alkyl, such as methyl, or lower alkoxyl, such as methoxy;
- M, independently for each occurrence, represents a substituted or unsubstituted methylene group (*e.g.,* substituted with lower alkyl, oxo, hydroxyl, etc.), NR'', O, S, S(O), or S($O_2$), preferably $CH_2$, or, when attached to W or Q, $CH_2$, S($O_2$), C(=S), or C(=O);
- n represents an integer from 1-5, preferably from 2-4 when present in $R_5$ and from 1-3 when present in $R_6$; and
- Q represents a nitrogen-containing heteroaryl ring, *e.g.,* pyrrole, oxazole, isoxazole, imidazole, or pyrazole, a tertiary amino substituent, *e.g.,* a dialkylamine, or a substituted or unsubstituted nitrogen-containing heterocycle such as morpholine, piperidine, piperazine, or pyrrolidine.

In certain embodiments of Formula Ia, $R_6$ is H and $R_7$ represents a methyl or methoxy substituent ortho to W. Preferably, $R_7$ is methyl.

In certain embodiments, the subject compounds with the structure of Formula I do not include compounds with the structure of Formula Ia.

In certain embodiments Q represents a tertiary amino substituent, *e.g.*, dialkyl amine. In certain embodiments Q represents a substituted or unsubstituted nitrogen containing heterocycle such as morpholine, piperidine, piperazine, or pyrrolidine. In certain embodiments, Q represents a nitrogen-containing heteroaryl ring, a tertiary amino substituent, or a substituted or unsubstituted nitrogen-containing heterocycle.

Exemplary compounds of Formula I and Ia include those shown in Table A.

The invention also provides novel treatment methods of the various disease conditions described above, using compounds having a structure selected from A3, A7 to A29, A31, A33 to A37, A40, A41, A44 to A47, A49, A51, A56, A57, A65, A69 to A82, C1, C2, and C5, including isomeric, prodrug, tautomeric, pharmaceutically acceptable salt, N-oxide, or stereoisomeric forms thereof. In a certain embodiments, the invention uses a compound having a structure **A37**, including isomeric, prodrug, tautomeric, pharmaceutically acceptable salt, N-oxide, or stereoisomeric forms thereof.

In an alterative embodiment, the present invention provides for an isolated prodrug or pharmaceutically acceptable salt of a metabolite of compound A37. A preferred such embodiment is a prodrug or pharmaceutically acceptable salt of compound A68 or C5.

In another embodiment, the present invention uses compounds having a structure selected from B1 to B20, and C1, C2 and C5, including isomeric, prodrug, tautomeric, pharmaceutically acceptable salt, N-oxide, or stereoisomeric forms thereof. In a preferred embodiment, the invention uses a compound having a structure **B16** or C5, including

isomeric, prodrug, tautomeric, pharmaceutically acceptable salt, N-oxide, or stereoisomeric forms thereof. In another embodiment, the invention uses a compound having a structure B3, including isomeric, prodrug, tautomeric, pharmaceuticaly acceptable salt, N-oxide, or stereoisomeric forms thereof.

In an alterative embodiment, the present invention provides for an isolated prodrug or pharmaceutically acceptable salt of a metabolite of compound **B16.** A preferred such embodiment is a prodrug or pharmaceutically acceptable salt of compound B3.

In certain embodiments, the invention provides novel treatment methods of the various disease conditions described above, using a compound, or a prodrug, isomeric, tautomeric, pharmaceutically acceptable salt, N-oxide, or stereoisomeric form thereof, having a structure of Formula II:

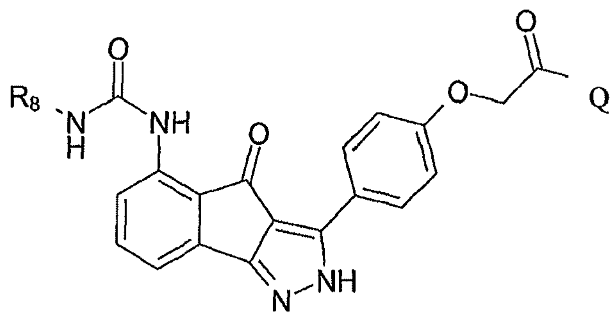

wherein

$R_8$ represents a substituted or unsubstituted heterocycle; and
Q represents a substituted or unsubstituted: tertiary amino substituent, or nitrogen-containing heterocycle.

In certain embodiments, the invention provides novel treatment methods of the various disease conditions described above, using a compound, or a prodrug, isomeric, tautomeric, pharmaceutically acceptable salt, N-oxide, or stereoisomeric form thereof, having a structure of Formula III:

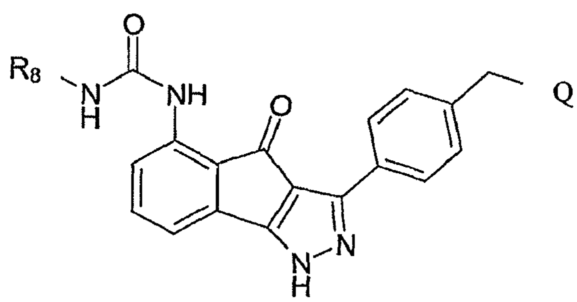

**Formula III**

wherein

$R_8$ represents a substituted or unsubstituted heterocycle;
Q represents a substituted or unsubstituted secondary amino substituent tertiary amino substituent, or substituted or unsubstituted nitrogen-containing heterocycle.

In one embodiment, Formula III does not include a compound selected from A47, A49, A51 and A82.

In one embodiment, $R_8$ represents a morpholino or piperazine ring.

In one embodiment, Q represents pyrrolidine.

In one embodiment, the compound of formula III is selected from the group of B 1 to B20 and C2.

In one embodiment, the compound is:

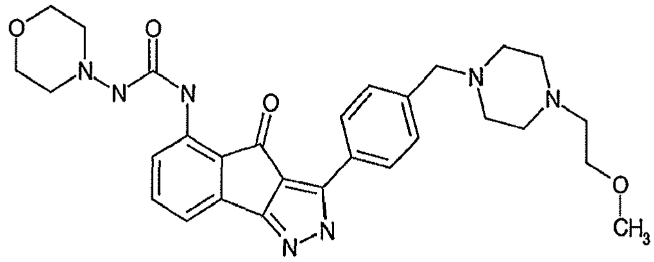

**B16**

In another embodiment, the present invention provides a pharmaceutical composition for the novel treatment of cancer, proliferative, degenerative and other diseases, comprising a pharmaceutically acceptable carrier and a therapeutically effective amount of a compound of Formula I, Ia, II, III, or any compound disclosed herein, or an isomeric, prodrug, tautomeric, pharmaceutically acceptable salt, N-oxide, or stereoisomeric form thereof. In a preferred embodiment, such pharmaceutical composition comprises a therapeutically effective amount of a compound selected from A1 to A82, B1 to B20 and C1 to C5, or an isomeric, prodrug, tautomeric, pharmaceutically acceptable salt, N-oxide, or stereoisomeric form thereof. In alterative embodiment, such pharmaceutical composition comprises a therapeutically effective amount of a prodrug or pharmaceutically acceptable salt of a metabolite of compound **A37** or **B16,** preferably a metabolite having the structure A68 or C5.

In another embodiment, the present invention provides a novel method of treating cancer, proliferative, degenerative and other diseases as described above, comprising administering to a host in need of such treatment a therapeutically effective amount of a compound of Formula I, Ia, II, III, or any compound disclosed herein, or an isomeric, prodrug, tautomeric, pharmaceutically acceptable salt, N-oxide, or stereoisomeric form thereof. In certain embodiments, at least one compound selected from anti-cancer agents and anti-proliferative agents may be administered conjointly with a compound of Formula I, Ia, II, III, or any compound disclosed herein, or an isomeric, prodrug, tautomeric, pharmaceutically acceptable salt, N-oxide, or stereoisomeric form thereof. In a preferred embodiment, such methods of treatment comprise suitable administration of a therapeutically effective amount of a compound selected from A1 to A82, B1 to B20 and C1 to C5, or a isomeric, prodrug, tautomeric, pharmaceutically acceptable salt, N-oxide, or stereoisomeric form thereof. Conjoint administration, as the term is used herein, encompasses therapies wherein two therapeutics are combined in a single preparation, are administered, *e.g.*, simultaneously or at different times, in separate preparations, or are otherwise administered to a patient as part of a therapeutic regimen.

In another embodiment, the invention provides a method for formulating a pharmaceutical composition for the novel treatment of cancer, proliferative, degenerative and other diseases, comprising a therapeutically effective amount of a compound of Formula I, Ia, II, III, or any compound disclosed herein, or an isomeric, prodrug, tautomeric, pharmaceutically acceptable salt, N-oxide, or stereoisomeric form thereof, and optionally a pharmaceutically acceptable carrier. In a preferred embodiment, such pharmaceutical composition comprises a therapeutically effective amount of a compound selected from A1 to A82, B1 to B20, and C1 to C5, or an isomeric, prodrug, tautomeric, pharmaceutically acceptable salt, N-oxide, or stereoisomeric form thereof. In alterative embodiment, such pharmaceutical composition comprises a therapeutically effective amount of a prodrug or pharmaceutically acceptable salt of a metabolite of compound **A37** or **B16,** preferably a metabolite having the structure A68 or C5.

In a further embodiment, the invention provides packaged pharmaceuticals encompassing the above pharmaceutical compositions, and instructions for using such pharmaceutical compositions in treating various disease conditions described above.

In further embodiments, the pharmaceutical compositions of the invention are for use in treating a disease, such as cancer, proliferative, degenerative or other diseases, including any disease or condition discussed above and below.

In certain embodiments of the present invention, where substituted groups are used, suitable substituents can include, for example, a halogen, a hydroxyl, a carbonyl (*e.g.*, ketones, aldehydes, carboxyls, esters, acyls), a thiocarbonyl (*e.g.*, thioester, a thioacetate, a thioformate), an alkoxyl, a phosphoryl (*e.g.*, phosphonate, phosphinate), a phosphate, a phosphonate, a phosphinate, an amino, an amino-alkyl, an amido, an amidine, an imine, a cyano, a nitro, an azido, a sulfhydryl, an alkylthio, ethers, $-CF_3$, alkyls, alkenyls, alkynyl, cycloalkyl, alkoxyl, silyl, sulfonyl (*e.g.*, sulfate, sulfonamido, sulfamoyl, sulfonate), a heterocyclyl, an aralkyl (*e.g.*, benzyl), or an aromatic or heteroaromatic moiety (*e.g.*, phenyl, oxazyl, piperazyl, pyridyl, pyrryl). Such substituents may also, themselves, be substituted or unsubstituted.

IV. Novel Kinase Targets and Diseases Associated Therewith

The subject kinase inhibitor compounds effectively inhibit not only CDKs functioning in cell-cycle regulation, but also a broad spectrum of other kinases not directly related to cell-cycle regulation.

For example, the activity of a panel of a number of kinases were assayed in the presence of 10 μM of exemplary compound **A37** (and 10 μM ATP). Fifteen (15) sensitive kinases were identified as being inhibited, with $IC_{50}$'s of less than 100 nM. Among them, a few Ser/Thr kinases were inhibited with an $IC_{50}$ of less than 3 nM, including GSK3β and CK1; and several Ser/Thr kinases were inhibited with an $IC_{50}$ between 100 nM and 10 nM, including MAPK 1, MAPK 6, c-JNK 1α 1, JNK 2α2, JNK 3, AMPK, and Ribosomal protein S6 kinase 3 (Rsk3). Overall, average $IC_{50}$ among the 8 Ser/Thr kinases is about 29 nM. Among the Tyr kinases, a few were inhibited with an $IC_{50}$ between 100 nM and 10 nM (average of about 37 nM), including c-Src, Lyn kinase, Fyn, Lymphocyte kinase, Yes, and Abelson kinase (Abl). As comparison, 6 CDKs were inhibited with an average $IC_{50}$ of about 42 nM. Meanwhile, average $IC_{50}$ by Flavopiridol (the first CDK inhibitor to be evaluated in clinic, Aventis) for CDK1, CDK2, and CDK4 was about 650 nM.

The compound used in this example, A37, is a dual specificity (serine/threonine & tyrosine) protein kinase inhibitor, which inhibits multiple signal transduction pathways essential for cancer cell survival, at low nanomolar range. It demonstrates favorable activity in many drug resistant cell lines, and has high efficacy in the A2780 and PC3 xenograft tumor models. Thus **A37** has suitable pharmaceutical properties for further development.

The structure of **A37** is listed below:

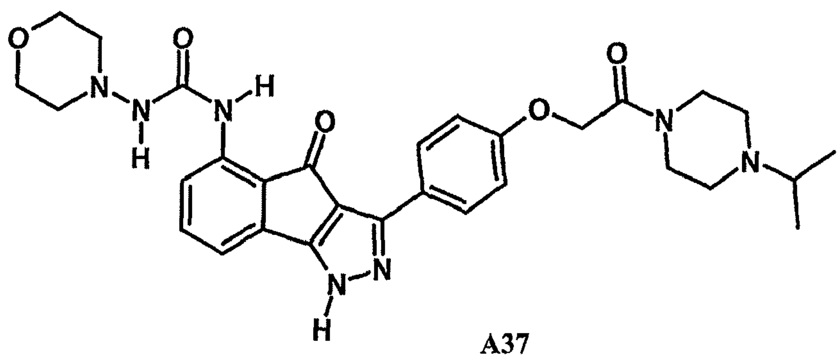

**A37**

**A37** has a M.W. of 610.10, three pKa's of 2.03, 7.18, and 10.53. Aqueous solubility is aboyt 15-20 mg/ml. Solution stability at pH6 is >1 mo. at room temperature and kept in dark.

Similarly, exemplary compound **B16** was also evaluated in the same panel of kinases, and it was found that 19 kinases were inhibited with $IC_{50}$ values <100 nM. Among them, 8 non-CDK Ser/Thr kinases were inhibited with an average $IC_{50}$ of about 50 nM. These include GSK3β, CK1, MEK1, MKK6, JNK1α1, JNK2α2, JNK3, AMPK. In addition, 8 Tyr kinases were inhibited with an average $IC_{50}$ of about 55 nM: Abl, c-Src, Fes, Yes, Blk, Lyn, Fyn, Lck. For comparison, the $IC_{50}$ of Abl T315I by **B16** is about 100 nM (or 0.1 μM), while the $IC_{50}$ for the marketed kinase inhibitor drug GLEEVEC® is no less than 30 μM. Also, the 6 CDKs were inhibited with an average $IC_{50}$ of about 17 nM. The $IC_{50}$ for another drug Flavopiridol Ave. is 650 nM (CDK1, CDK2, CDK4).

Table II below lists examples of the protein kinases inhibited by the subject inhibitor compounds as represented in the formulae described below, and the diseases associated with deregulation, mutation or otherwise associated with the kinase as suggested by the corresponding scientific publication. For example, Table S2 (supplemental material) of Manning et al., The Protein Kinase Complement of the Human Genome. Science 298: 1912-34 (2002) listed several characteristics of about 625 kinases, including kinase name, accession number, group, kinase family and subfamily, "Ensembl map position," and some of their known disease associations, etc. The entire content of this book is incorporated herein by reference. Furthermore, such literature can be used to identify other proteins related to the subject kinases.

These compounds can be used in specific medical applications for the treatment of specific diseases, based on the inhibition of the associated / underlying kinases.

Thus, in one aspect of the invention is the subject compounds are used to inhibit a kinase selected from the list shown in Table II. In certain embodiments, such kinase is inhibited by administering to a host, individual or patient in need of such treatment a therapeutically effective amount of said compound.

In another aspect, the subject compounds are used to treat a disease susceptible to treatment by inhibition of a kinase selected from the list shown in Table II. In certain embodiments, such treatment comprises administering to a host, individual or patient in need of such treatment a therapeutically effective amount of said compound

Yet another aspect of the invention provides a method of treating the various disease conditions as listed in

Table I below, where the disease is mediated by kinases associated with the disease. The method comprises using the subject compounds represented in the formulae in the instant application, and functional equivalents or derivatives thereof. In certain embodiments of this aspect, such mediation is caused or enhanced by excessive activities of a kinase associated with the disease, such as a kinase selected from Table II. In particular embodiments, the mediation is brought about by mutation of the kinase, including germ-line or somatic-mutations, such as point mutations, insertions, deletions of chromosomal rearrangements.

In another embodiment of the invention, the subject kinase includes one or more proteins related to any of the kinases disclosed herein. A related protein can include a mutant, variant, isoform, polymorphism of a given kinase, and can further include proteins that are homologous or orthologous to the given kinase for example the corresponding kinase from a different organism or species. In a certain embodiment, the kinase includes a protein that is a member of the same kinase class, group, family or subfamily of a given kinase listed herein, where such classifications can be made according to Manning *et al* (Supra).

In certain embodiments, the kinase does not include one or more kinases selected from: protein kinase C, her2, raf 1, MEK1, MAP kinase, EGF receptor, PDGF receptor, IGF receptor, PI3 kinase, weel kinase, Src, Abl.

**CK 1**: For example, aberrantly phosphorylated tau accumulates in such disorders as Alzheimer disease (AD), Down syndrome (DS), progressive supranuclear palsy (PSP), corticobasal degeneration, and parkinsonism dementia complex of Guam (PDC). The hyperphosphorylated tau forms both straight and paired helical filaments that accumulate within neurofibrillary tangles (NFT), neuropil threads, and dystrophic neurites (DN) in amyloid-containing plaques. In Pick's disease (PiD), abnormal tau accumulates in Pick bodies and ballooned neurons. In several tauopathies linked to chromosome 17 (Hong et al., Science 282: 1914-1917, 1998; Hutton et al., Nature 393: 702-705, 1998; Poorkaj et al., Ann Neurol 43: 815-825,1998), including pallido-ponto-nigral degeneration (PPND), the disruption of the cytoskeleton can be directly linked to mutations in the tau gene (Clark, Proc. Natl. Acad. Sci. USA 95: 13103-13107, 1998; Hong, supra; Hutton, supra; and Poorkaj, supra). In the more common "tauopathies" such as AD, other pathological processes lead to aberrant tau phosphorylation, accumulation of tau filaments, disruption of the cytoskeleton, and finally death of neurons.

Efforts to identify phosphotransferases potentially involved in tau hyperphosphorylation have led to the identification of casein kinase I (CK1) as one expected of a pathological tau protein kinase. Schwab et al. (Neurobiol Aging 21 (4): 503-10,2000) studied CK 1 by immunohistochemistry and correlated with other pathological hallmarks in Alzheimer's disease (AD), Down syndrome (DS), progressive supranuclear palsy (PSP), parkinsonism dementia complex of Guam (PDC), Pick's disease (PiD), pallido-ponto-nigral degeneration (PPND), Parkinson's disease (PD), dementia with Lewy bodies (DLB), amyotrophic lateral sclerosis (ALS), and elderly controls. CK 1 was found to be associated generally with granulovacuolar bodies and tau-containing neurofibrillary tangles in AD, DS, PSP, PDC, PPND, and controls, and Pick bodies and ballooned neurons in PiD. The colocalization of the kinase CK 1 and its apparent substrate tau suggests a function for CK 1 in the abnormal processing / phosphorylation of tau, and thus the pathological process of diseases including Alzheimer's disease (AD), Down syndrome (DS), progressive supranuclear palsy (PSP), parkinsonism dementia complex of Guam (PDC), Pick's disease (PiD), pallido-ponto-nigral degeneration (PPND).

Therefore, inhibiting the activity of CK 1 by using the subject kinase inhibitors provides a method to treat or alleviate the symptoms of the above diseases in patients suffering such diseases. The method may additionally help to prevent or delay the onset and/or progression of such diseases, especially in high-risk populations predisposed to such diseases due to, for example, genetic and/or environmental reasons.

**Src (and its related kinases, *e.g.,* Yes, Hck, Fyn, Lyn, Lck, Blk, Fgr or Yrk):** Activating mutations of the proto-oncogene Src is frequently found in colon cancers, particularly those advanced ones that metastasize to the liver. Thus the subject kinase inhibitors can be used in a method to treat cancer patients, where activating mutations of the Src and Src-related kinases (Yes, Fyn, etc.) contributes to the proliferation and progression of cancer cells, especially primary colon cancers, and its metastasis in the liver.

Alzheimer's disease is a neurodegenerative disorder of the brain, which is accompanied at the cellular level by a massive loss of neurons in the limbic system and in the cerebral cortex. In the brain areas affected, protein deposits, so-called plaques, can be detected at the molecular level, which are an essential characteristic of Alzheimer's disease. The protein occurring most frequently in these plaques is a peptide of 40 to 42 amino acids in size, which is designated as Aβ-peptide. This peptide is a cleavage product of a larger protein of 695 to 770 amino acids, the so-called Amyloid Precursor Protein (APP).

Williamson et al. J. Neurochem. 22: 10-20, 2002 described the rapid phosphorylation of neuronal proteins including Tau and Focal adhesion kinase (FAK) in response to amyloid-β peptide exposure and an involvement of Src family protein kinases. Slack and Berse described in Society for Neuroscience Abstracts, Vol. 24, No. 1-2, pp. 208, 1998 (Conference/Meeting Information: 28th Annual Meeting of the Society for Neuroscience, Part 1, Los Angeles, California, USA, Nov. 7-12, 1998 Society for Neuroscience, ISSN: 0190-5295) a role for tyrosine kinases in the stimulation of APP release by action of muscarinic m3 acetylcholine receptors. They described a role for Src tyrosine kinase in the regulation of APPsec release by muscarinic receptors. They demonstrated that an increase in the active form of Src leads to a

decrease in secAPP. Thus the subject compounds demonstrating inhibitory function against Src may also be used to treat AD.

WO03013540A1 describes that compounds inhibiting the c-Src protein tyrosine kinase activity are effective against leukemia. Furthermore, it was surprisingly found that the effect in treating leukemia of a combination which comprises (a) at least one compound decreasing the c-Src activity, and, (b) ST1571 (GLEEVEC®) or the monomethane sulfonate salt thereof is greater than the effects that can be achieved with either type of combination partner alone, *i.e.* greater than the effects of a monotherapy using only one of the combination partners (a) and (b) as defined herein.

Thus in a broader sense, the present invention relates to a method of treating a warm-blooded animal having leukemia, comprising administering to the animal at least one subject kinase inhibitor compound to inhibit the activity of a member of the Src kinase family, in particular c-Src, v-Src, c-Yes, v-Yes, Fyn, Lyn, Lck, Blk, Hck, c-Fgr, v-Fgr, p56Lck, Tkl, Csk, Ctk or Yrk, or the activity of a member of the Btk or Tec kinase family, in a quantity which is therapeutically effective against leukemia alone or in combination with a Bcr-Ab1 inhibitor, in particular STI571 or a Raf kinase inhibitor, *e.g.*, BAY 43-9006 (the first compound to target both the RAF/MEK/ERK signaling pathway to inhibit cell proliferation and the VEGFR-2/PDGFR-β signaling cascade to inhibit tumor angiogenesis).

The term leukemia as used herein (especially for this particular embodiment here) includes, but is not limited to, chronic myelogenous leukemia (CML) and acute lymphocyte leukemia (ALL), especially Philadelphia-chromosome positive acute lymphocyte leukemia ($Ph^+$ ALL). Preferably, the variant of leukemia to be treated by the methods disclosed herein is CML.

Other diseases related to abnormal Src activity, and thus can be treated by the subject compounds include: hyperproliferative diseases, haematologic diseases, osteoporosis, neurological diseases (*e.g.* Alzheimer's Disease, epilepsy, etc.), autoimmune diseases (*e.g.* lupus erythematosus), allergic/immunological diseases (*e.g.* anaphylaxis), or viral (*e.g.* HIV) or bacterial infections. See Src-related diseases disclosed in US20040077663A1.

In one embodiment, the hyperproliferative disease is cancer, such as cancers of brain, lung, liver, spleen, kidney, lymph node, small intestine, blood cells, pancreas, colon, stomach, breast, endometrium, prostate, testicle, ovary, skin, head and neck, esophagus, bone marrow and blood tissue.

**FES and its related kinases (c-fes/fps, v- fes/fps, p94-c-fes-related protein, and Fer):** Fes is a tyrosine kinase that contains SH2 domain, but no SH3 domain or carboxy terminal regulatory phosphotyrosine, such as that found in the Src family of kinases. Fes has a unique N-terminal domain of over 400 amino acids of unknown function. It has been implicated in signaling by a variety of hematopoietic growth factors, and is predominantly a nuclear protein. Although originally identified as a cellular homolog of several transforming retroviral oncoproteins, Fes was later found to exhibit strong expression in myeloid hematopoietic cells and to play a direct role in their differentiation. (See Yates, Role of c-Fes in normal and neoplastic, hematopoiesis. Stem Cells 1996 Jan; 14(1):117). The members of Fes family include, but are not limited to: c-fes/fps, v-fps/fes, p94-c-fes-related protein, Fer, Fes-homologs, and Fer-homologs. It is noted that these examples are present here for illustrative, rather then limiting purposes, so that other peptides that share structural and / or functional similarities with the members of Fes family are fall within the scope of this invention.

The examples of diseases associated with abnormal activity of members of Fes family of tyrosine kinases include, but are not limited to carcinoma, neuroblastoma, and tumors of hematopoietic origin, especially acute promyelocytic leukemia (APL). See Hagemeijer et al., Hum. Genet. 61: 223-227, 1982. Co-administration of a DNA methylation inhibitor in conjunction with one or more inhibitor(s) of member(s) of the subject kinase inhibitors may be specifically advantageous in treatment of tumors of mesenchymal and hematopoietic origin.

**Abl and related kinases:** The Philadelphia chromosome, with the Bcr-Abl oncogene detectable at the molecular level, is present at diagnosis in 95% of patients. Thus the subject kinase inhibitors can be used to treat CML resulting from the presence of the Ph chromosome. For example, see section XI below.

**AMPK**: The adenosine monophosphate (AMP)-activated protein kinase (AMPK) is the downstream component of a protein kinase cascade that is activated by rising AMP coupled with falling ATP, these changes in cellular nucleotides signaling a fall in cellular energy status. Following activation, the kinase switches on catabolic pathways while switching off many ATP-requiring processes, both through direct phosphorylation of metabolic enzymes and through effects on gene expression. In 1999, it was proposed that activation of AMPK was a promising target for the development of new drugs aimed at treatment of Type 2 diabetes. See WO 04/024942 A1.

This view has been greatly strengthened by recent findings that the existing anti-diabetic drugs, metformin and rosiglitazone, activate AMPK *in vivo* and/or in cultured cells, albeit by distinct mechanisms. Activators of AMPK are therefore potential new drugs aimed at treatment of Type 2 diabetes, with metformin and rosiglitazone being the prototypes. Since AMPK also inhibits fatty acid and triglyceride synthesis and stimulates fatty acid oxidation, and mediates at least some of the effects of the "satiety" hormone leptin, they may also be effective in treatment of obesity. Indeed, metformin treatment has been shown to reduce body weight and fat content, in obese, non-diabetic subjects. See WO 041024942A1. Dominant negative AMPK expression in the hypothalamus is also sufficient to reduce food intake and body weight. See Minokoshi, Nature 428(6982):569-74, 2004.

AMPK has also been associated with cardiac diseases. During myocardial ischemia, changes in glucose and

fatty acid metabolism leads ultimately to the undesirable proton accumulation in the heart. AMPK is a key player contributing to this result. Hopkins et al. (Biochem Soc Trans. 31(Pt 1): 207-12, 2003) suggests that decreasing the ischaemic-induced activation of AMPK or preventing the downstream decrease in malonyl-CoA levels may be a therapeutic approach to treating ischaemic heart disease. There is also evidence that gain of function mutations in AMPK may cause hypertrophic cardiomyopathies. The the subject inhibitors may also be used to treat AMPK-mediated cardiac disease. See also Sambandam and Lopaschuk, Prog. Lipid Res. 42 (3): 238-56, 2003, which reports that AMPK is a key player in the ischemia-induced alteration in fatty acid and glucose metabolism. Activation of AMPK during myocardial ischemia both increases glucose uptake and glycolysis, while also increasing fatty acid oxidation during reperfusion. Gain-of function mutations of AMPK in cardiac muscle may also be causally related to the development of hypertrophic cardiomyopathies. Therefore, AMPK could be a therapeutic target in treating ischemia reperfusion injuries.

AMPK exists as heterotrimeric complexes comprising a catalytic $\alpha$ subunit and accessory $\beta$ and $\gamma$ subunits. Each subunit occurs as multiple isoforms encoded by distinct genes so that there are twelve possible heterotrimeric combinations. AMP activates the complex via a complex multi-step mechanism that results in an ultrasensitive response, such that over the critical range of AMP concentrations there is a large activation in response to a small rise in AMP. The effects of AMP are to: (i) bind to AMPK and cause allosteric activation; (ii) bind to AMPK and make it a better substrate for the upstream kinase, AMPKK; (iii) bind to AMPK and make it a worse substrate for protein phosphatases; (iv) allosterically activate the upstream kinase, AMPKK. The simplest model to explain effects (i) through (iii) is that they are due to binding of AMP to a single allosteric site on the complex, although until now this has not been directly addressed experimentally. Effects (i) through (iii) are also antagonized by high concentrations of ATP, which may therefore compete for binding at the same site as AMP, although once again until now this has not been directly tested. See W004024942A1.

In certain embodiments, the disease does not include a disease selected from: (1) cancer, benign prostate hyperplasia, familial adenomatosis polyposis, neurofibromatosis, psoriasis, fungal infections, endotoxic shock, hypertrophic scar formation, inflammatory bowel disease, transplant rejection, vascular smooth muscle cell proliferation associated with atherosclerosis, psoriasis, pulmonary fibrosis, arthritis, glomerulonephritis, restenosis following angioplasty or vascular surgery, and other post-surgical stenosis and restenosis; (2) proliferative diseases, specifically cancer, autoimmune diseases, viral diseases, fungal diseases, neurodegenerative disorders and cardiovascular disease; (3) carcinoma, specifically that of the bladder, breast, colon, kidney, liver, lung, small cell lung cancer, esophagus, gall bladder, ovary, pancreas, stomach, cervix, thyroid, prostate, and skin, squamous cell carcinoma; hematopoietic tumors of lymphoid lineage, leukemia, acute lymphocytic leukemia, acute lymphoblastic leukemia, B-cell lymphoma, T-cell lymphoma, Hodgkins lymphoma, non-Hodgkins lymphoma, hairy cell lymphoma, and Burkett's lymphoma; hematopoietic tumors of myeloid lineage, acute and chronic myelogenous leukemias, myelodysplastic syndrome, and promyelocytic leukemia; tumors of mesenchymal origin, specifically fibrosarcoma and rhabdomyosarcoma; tumors of the central and peripheral nervous system, specifically astrocytoma, neuroblastoma, glioma, and schwannomas; and other tumors, specifically melanoma, seminoma, teratocarcinoma, osteosarcoma, xenoderoma pigmentosum, keratoctanthoma, thyroid follicular cancer, and Kaposi's sarcoma; (4) Alzheimer's disease; (5) cancer, viral infections (specifically herpesvirus, poxvirus, Epstein-Barr virus, Sindbis virus and adenovirus), prevention of AIDS development in HIV-infected individuals, autoimmune diseases (specifically systemic lupus, erythematosus, autoimmune mediated glomerulonephritis, rheumatoid arthritis, psoriasis, inflammatory bowel disease, and autoimmune diabetes mellitus), neurodegenerative disorders (specifically Alzheimer's disease, AIDS-related dementia, Parkinson's disease, amyotrophic lateral sclerosis, retinitis pigmentosa, spinal muscular atrophy and cerebellar degeneration), myelodysplastic syndromes, aplastic anemia, ischemic injury associated with myocardial infarctions, stroke and reperfusion injury, arrhythmia, atherosclerosis, toxin-induced or alcohol related liver diseases, hematological diseases (specifically chronic anemia and aplastic anemia), degenerative diseases of the musculoskeletal system (specifically osteoporosis and arthritis) aspirin-sensitive rhinosinusitis, cystic fibrosis, multiple sclerosis, kidney diseases and cancer pain; (6) viral infections (specifically HIV, human papilloma virus, herpesvirus, poxvirus, Epstein-Barr virus, Sindbis virus, and adenovirus; (7) chemoprevention of cancer. Chemoprevention is defined as inhibiting the development of invasive cancer by either blocking the initiating mutagenic event or by blocking the progression ofpre-malignant cells that have already suffered an insult or inhibiting tumor relapse; (8) tumor angiogenesis and metastasis; (9) hair loss that ordinarily accompanies many traditional chemotherapeutic regimens.

V. Kinase Inhibitors used as Broad Spectrum Inhibitors

The subject compounds show significantly broader spectrum of activity than other kinase inhibitors (including two currently undergoing clinical trials). This allows the use of the subject compounds to simultaneously inhibit several kinases, especially those kinases that cooperate in certain disease conditions.

To illustrate, in the case of cell-cycle kinases, a broad spectrum inhibitor might act faster due to independence from the exact stage of cell cycle the target cells are in. In addition, by simultaneously inhibiting several control points in the cell cycle, administering a single drug is more likely to completely halt the progression of cell cycle, since cells

escaping one check point and advance to the next stage of cell cycle are still subject to the inhibitory effect of the drug at a different stage of cell cycle.

Similarly, progression of many cancers depends on the cooperation of multiple pathways involving several different key kinases. A broad spectrum kinase inhibitor maybe more likely to simultaneously block several related signaling pathways leading to a disease condition, thus resulting in a better overall effect, likely with less amount of drug.

For instance, Table 5 and Table II shows that compound A37 inhibits CDK1-7 with an $IC_{50}$ of about 3-155 nM (average 42 nM). It also inhibits a panel of Ser/Thr kinases with a comparable average $IC_{50}$ of 44 nM; a panel of Tyr kinases with a comparable average $IC_{50}$ of 35.5 nM. The same is true for representative compound **B16.**

As a result, **A37** impacts numerous signal transduction pathways, including Wnt signaling - GSK3-β and CK1; AKT and STAT signaling - c-SRC and c-SRC related kinases; MAP kinase signaling - Mek1 and MKK6; Stress activated signaling - JNK1-3. This may lead to enhanced efficacy through the simultaneous inhibition of many targets relevant to cancer.

In certain aspects of the invention, the subject inhibitors bind to or inhibit more than one kinase, such as 2, about 5, about 8, about 10, about 15, about 20 or about 25 kinases. In one embodiment of this aspect, the disease is susceptible to treatment through the inhibition of such number of kinases. In other embodiments of this aspect, the disease is susceptible to treatment through the inhibition or blocking of one or more signaling pathways described herein, such as 1, 2, about 3, about 5, about 8, about 10 or more than 10 signaling pathways.

VI. Kinase Inhibitors for Treating Drug-Resistant Cancers / Tumors

Clinical drug resistance, either intrinsic or acquired, is a major barrier to overcome before chemotherapy can become curative for most patients presenting with cancer. In many common cancers (for example, non-small cell lung, testicular and ovarian cancers), substantial tumor shrinkage can be expected in more than 50% of cases with conventional chemotherapy. In other cases, response rates are lower; 10-20% of patients with renal cell carcinoma, pancreatic and esophageal cancers respond to treatment. In almost all cases, drug resistance eventually develops shortly and is often fatal. If this could be treated, prevented or overcome, the impact would be substantial.

The subject compounds may be used to treat cancers that (1) have been shown to be, (2) are suspected of becoming, or (3) are not then known to be (but are, will be or are suspected of being or becoming) resistant or refractory to other drugs, therapeutics, or cytotoxic agents, especially cancers resistant or refractory to treatment by taxanes (*e.g.*, taxol) and platinum-based therapeutic agents (*e.g.*, cisplatin, etc.). Such resistance or refractory phenotype may be brought about by a variety of mechanisms. For example, compound **B16** shows potency in inhibiting cell viability in cells lines representing the following classes of resistance mechanisms: (i) p-gylocoprotein mediated multi-drug resistance (MDR); (ii) mutant topoisomerase mediated atypical MDR; (iii) tubulin mutation mediated resistance to taxanes; and (iv) resistance to cisplatin.

Specifically, cancers or tumors that are resistant or refractory to treatment of a variety of therapeutic agents may benefit from treatment with the methods of the present invention. Preferred tumors are those resistant to chemotherapeutic agents other than the subject compounds disclosed herein. In certain embodiments of the instant invention, the subject compounds may be useful in treating tumors that are refectory to platinum-based chemotherapeutic agents, including carboplatin, cisplatin, oxaliplatin, iproplatin, tetraplatin, lobaplatin, DCP, PLD-147, JM118, JM216, JM335, and satraplatin. Such platinum-based chemotherapeutic agents also include the platinum complexes disclosed in EP 0147926, US 5072011, US 5244919, 5519155, 6503943 (LA-12/PLD-147), 6350737, and WO 01/064696 (DCP). Resistance to these platinum-based compounds can be tested and verified using the methods described in U.S.S.N. 60/546097. For example, one may use the colorimetric cytotoxicity assay described for anticancer drug screening in Shekan et al., J. Natl. Cancer. Inst. 82: 1107-12 (1990). For another example, one may determine the viability of a tumor cell by contacting the cell with a dye and viewing it under a microscope. Viable cells can be observed to have an intact membrane and do not stain, whereas dying or dead cells having "leaky" membranes do stain. Incorporation of the dye by the cell indicates the death of the cell. A dye useful for this purpose is trypan blue.

Suitable agents for which the subject compounds are not cross-resistant are described in the following section, which may be taken as non-limiting examples of "anti-cancer therapeutic agents."

*1. Taxanes*

Resistance to taxanes like pacitaxel and docetaxol is a major problem for all chemotherapeutic regimens utilizing these drugs. Taxanes exert their cytotoxic effect by binding to tubulin, thereby causing the formation of unusually stable microtubules. The ensuing mitotic arrest triggers the mitotic spindle checkpoint and results in apoptosis. Other mechanisms that mediate apoptosis through pathways independent of microtubule dysfunction have been described as well, including molecular events triggered by the activation of Cell Division Control-2 (cdc-2) Kinase, phosphorylation of BCL-2 and the induction of interleukin 1β (IL-1β) and tumor necrosis factor-α (TNF-α). Furthermore, taxanes have

been shown to also exert anti-tumor activity via other mechanisms than the direct activation of the apoptotic cascade. These mechanisms include decreased production of metalloproteinases and the inhibition of endothelial cell proliferation and motility, with consequent inhibition of angiogenesis.

As shown in the Examples, Applicants have demonstrated that exemplary subject compounds, including **A37** and **B16**, maintain their therapeutic efficacy in tumor cell lines resistant to taxanes. In other words, tumor cell lines that are resistant to taxane treatment do not show resistance to treatment with the exemplary subject compounds. Thus, one embodiment of the present invention relates to methods of treating patients with tumors resistant to taxanes by administering a subject compound as disclosed in various formulae, preferably **A37** and **B16.**

By the term "taxane", it is meant to include any member of the family of terpenes, including, but not limited to paclitaxel (Taxol) and docetaxel (Taxotere), which were derived primarily from the Pacific yew tree, Taxus brevifolia, and which have activity against certain tumors, particularly breast, lung and ovarian tumors (See, for example, Pazdur et al. Cancer Treat Res. 1993.19:3 5 1; Bissery et al. Cancer Res. 1991 51:4845). In the methods and packaged pharmaceuticals of the present invention, preferred taxanes are paclitaxel, docetaxel, deoxygenated paclitaxel, TL-139 and their derivatives. See Annu. Rev. Med. 48:353-374 (1997).

The term "paclitaxel" includes both naturally derived and related forms and chemically synthesized compounds or derivatives thereof with antineoplastic properties including deoxygenated paclitaxel compounds such as those described in U.S. Pat. No. 5,440,056, U.S. Patent No. 4942184, which are herein incorporated by reference, and that sold as TAXOL® by Bristol-Myers Oncology. Paclitaxel has been approved for clinical use in the treatment of refractory ovarian cancer in the United States (Markman et al., Yale Journal of Biology and Medicine, 64:583, 1991; McGuire et al., Ann. Intern. Med., 111:273, 1989). It is effective for chemotherapy for several types of neoplasms including breast (Holmes et al., J. Nat. Cancer Inst., 83:1797, 1991) and has been approved for treatment of breast cancer as well. It is a potential candidate for treatment of neoplasms in the skin (Einzig et al., Proc. Am. Soc. Clin. Oncol., 20:46) and head and neck carcinomas (Forastire et al. Sem. Oncol., 20:56, 1990). The compound also shows potential for the treatment of polycystic kidney disease (Woo et al, Nature, 368:750, 1994), lung cancer and malaria. Docetaxel (N-debenzoyl-N-tert-butoxycarbonyl-10-deacetyl paclitaxel) is produced under the trademark TAXOTERE® by Aventis. In addition, other taxanes are described in "Synthesis and Anticancer Activity of Taxol other Derivatives," D. G. 1. Kingston et al., Studies in Organic Chemistry, vol. 26, entitled "New Trends in Natural Products Chemistry" (1986), Atta-urRabman, P. W. le Quesne, Eds. (Elvesier, Amsterdam 1986), pp 219-235 are incorporated herein. Various taxanes are also described in U.S. 6380405, the entirety of which is incorporated herein.

Methods and packaged pharmaceuticals of the present invention are applicable for treating tumors resistant to treatment by any taxane, regardless of the resistance mechanism. Known mechanisms that confer taxane resistance include, for example, molecular changes in the target molecules, *i.e.*, α-tublin and/or β-tubulin, up-regulation of P-glycoprotein (multidrug resistance gene MDR-1), changes in apoptotic regulatory and mitosis checkpoint proteins, changes in cell membranes, overexpression of interleukin 6 (IL-6; Clin Cancer Res (1999) 5, 3445-3453; Cytokine (2002) 17, 234-242), the overexpression of interleukin 8 (IL-8; Clin Cancer Res (1999) 5, 3445-3453; Cancer Res (1996) 56, 1303-1308) or the overexpression of monocyte chemotactic protein-1 (MCP-1; (MCP-1; Clin Cancer Res (1999) 5, 3445-3453), changes in the levels of acidic and basic fibroblast growth factors, transmembrane factors, such as p185 (HER2; Oncogene (1996) 13, 1359-1365) or EGFR (Oncogene (2000) 19, 6550-6565; Bioessays (2000) 22, 673-680), changes in adhesion molecules, such as β1 integrin (Oncogene (2001) 20, 4995-5004), changes in house keeping molecules, such as glutathione-S-transferase and/or glutathione peroxidase (Jpn J Clin Oncol (1996) 26, 1-5), changes in molecules involved in cell signaling, such as interferon response factor 9, molecules involved in NF-κB signaling, molecules involved in the PI-3 kinase/AKT survival pathway, RAF-1 kinase activity, PKC α/β or PKC β/β2 and via nuclear proteins, such as nuclear annexin IV, the methylation controlled J protein of the DNA J family of proteins, thymidylate synthetase or c-jun.

Another known mechanism that confers taxane resistance is, for example, changes in apoptotic regulatory and mitosis checkpoint proteins. Such changes in apoptotic regulatory and mitosis checkpoint proteins include the over-expression of Bcl-2(Cancer Chemother Pharmacol (2000) 46, 329-337; Leukemia (1997) 11, 253-257) and the over-expression of Bcl-xL (Cancer Res (1997) 57, 1109-1115; Leukemia (1997) 11, 253-257). Over-expression of Bcl-2 may be effected by estradiol (Breast Cancer Res Treat (1997) 42, 73-81).

Taxane resistance may also be conferred via changes in the cell membrane. Such changes include the change of the ratio of fatty acid methylene:methyl (Cancer Res (1996) 56, 3461-3467), the change of the ratio of oholine:methyl (Cancer Res (1996)56, 3461-3467) and a change of the permeability of the cell membrane (J Cell Biol (1986) 102, 1522-1531).

A further known mechanism that confers taxane resistance is via changes in acidic and basic fibroblast growth factors (Proc Natl Acad Sci USA (2000) 97, 8658-8663), via molecules involved in cell signaling, such as interferon response factor 9 (Cancer Res (2001) 61, 6540-6547), molecules involved in NF-κB signaling (Surgery (2991) 130, 143-150), molecules involved in the PI-3 kinase/AKT survival pathway (Oncogene (2001) 20, 4995-5004), RAF-1 kinase activity (Anticancer Drugs (2000) 11, 439-443; Chemotherapy (2000) 46, 327-334), PKC α/β (Int J Cancer (1993)

54" 302-308) or PKC β/β2 (Int J Cancer (2001) 93, 179-184, Anticancer Drugs (1997) 8, 189-198).

Taxane resistance may also be conferred via changes in nuclear proteins, such as nuclear annexin IV (Br J Cancer (2000) 83, 83-88), the methylation controlled J protein of the DNA J family of proteins (Cancer Res (2001) 61, 4258-4265), thymidylate synthetase (Anticancer Drugs (1997) 8, 189-198) or c-jun (Anticancer Drugs (1997) 8, 189-198), via paracrine factors, such as LPS (J Leukoc Biol (1996) 59,280-286), HIF-1 (Mech Dev (1998) 73, 117-123), VEGF (Mech Dev (1998) 73, 117-123) and the lack of decline in bcl-XL in spheroid cultures (Cancer Res (1997) 57, 2388-2393).

*2. Indole Alkaloid*

As shown in the Examples, Applicants have demonstrated that exemplary subject compounds maintain its therapeutic efficacy in tumors resistant to camptothecin, an indole alkaloid. In other words, tumors that are resistant to camptothecin treatment do not show resistance to treatment with the exemplary subject compounds. Thus, one embodiment of the present invention relates to methods of treating patients with tumors resistant to an indole alkaloid by administering a subject compound as disclosed in the various formulae, preferably **A37** and **B16**.

Exemplary indole alkaloids include bis-indole alkaloids, such as vincristine, vinblastine and 5'-nor-anhydrovinblastine (hereinafter: 5'-nor- vinblastine). It is known that bis-indole compounds (alkaloids), and particularly vincristine and vinblastine of natural origin as well as the recently synthetically prepared 5'-nor-vinblastine play an important role in the antitumor therapy. These compounds were commercialized or described, respectively in the various pharmacopoeias as salts (mainly as sulfates or difumarates, respectively).

Preferred indole alkaloids are camptothecin and its derivatives and analogues. Camptothecin is a plant alkaloid found in wood, bark, and fruit of the Asian tree Camptotheca acuminata. Camptothecin derivatives are now standard components in the treatment of several malignancies. See Pizzolato and Saltz, 2003. Studies have established that CPT inhibited both DNA and RNA synthesis. Recent research has demonstrated that CPT and CPT analogues interfere with the mechanism of action of the cellular enzyme topoisomerase I, which is important in a number of cellular processes (*e.g.*, DNA replication and recombination, RNA transcription, chromosome decondensation, etc.). Without being bound to theory, camptothecin is thought to reversibly induce single-strand breaks, thereby affecting the cell's capacity to replicate. Camptothecin stabilizes the so-called cleavable complex between topoisomerase I and DNA. These stabilized breaks are fully reversible and non-lethal. However, when a DNA replication fork collides with the cleavable complex, single-strand breaks are converted to irreversible double-strand breaks. Apoptotic cell death is then mediated by caspase activation. Inhibition of caspase activation shifts the cells from apoptosis to transient G1 arrest followed by cell necrosis. Thus, the mechanisms of cell death need active DNA replication to be happening, resulting in cytotoxic effects from camptothecin that is S-phase-specific. Indeed, cells in S-phase *in vitro* have been shown to be 100-1000 times more sensitive to camptothecin than cells in G1 or G2.

Camptothecin analogues and derivatives include, for example, irinotecan (Camptosar, CPT-11), topotecan (Hycamptin), BAY 38-3441, 9-nitrocamptothecin (Orethecin, rubitecan), exatecan (DX-8951), lurtotecan (GI-147211C), gimatecan, homocamptothecins diflomotecan (BN-80915) and 9-aminocamptotheein (IDEC-13'). See Pizzolato and Saltz, The Lancet, 361:2235-42 (2003); and Ulukan and Swaan, Drug 62: 2039-57 (2002). Additional Camptothecin analogues and derivatives include, SN-38 (this is the active compound of the prodrug irinotecan; conversion is catalyzed by cellular carboxylesterases), ST1481, karanitecin (BNP1350), indolocarbazoles, such as NB-506, protoberberines, intoplicines, idenoisoquinolones, benzo-phenazines and NB-506. More camptothecin derivatives are described in W003101998: NITROGEN-BASED HOMO-CAMPTOTHECIN DERIVATIVES; US6100273: Water Soluble Camptothecin Derivatives, US 5587673, Camptothecin Derivatives.

The methods and pharmaceutical compositions of the present invention are useful for treating tumors resistant to any one or more of above-listed drugs.

*3. Platinum-based therapeutic agents*

In an alternative embodiment, the methods, packaged pharmaceuticals and pharmaceutical compositions of the present invention are useful for treating tumors resistant to platinum-based chemotherapeutic agents.

Such platinum-based chemotherapeutic agents may include: carboplatin, cisplatin, oxaliplatin, iproplatin, tetraplatin, lobaplatin, DCP, PLD-147, JM118, JM216, JM335, and satraplatin. Such platinum-based chemotherapeutic agents also include the platinum complexes disclosed in EP 0147926, US 5072011, US 5244919, 5519155, 6503943 (LA-12/PLD-147), 6350737, and WO 01/064696 (DCP).

As is understood in the art, the platinum-based chemotherapeutic agents, or platinum coordination complexes, typified by cisplatin [cis-diamminedichloroplatinum (II)] (Reed, 1993, in Cancer, Principles and Practice of Oncology, pp. 390-4001), have been described as "the most important group of agents now in use for cancer treatment". These agents, used as a part of combination chemotherapy regimens, have been shown to be curative for testicular and ovarian cancers and beneficial for the treatment of lung, bladder and head and neck cancers. DNA damage is believed to be the major

determinant of cisplatin cytotoxicity, though this drug also induces other types of cellular damage.

In addition to cisplatin, this group of drugs includes carboplatin, which like cisplatin is used clinically, and other platinum-containing drugs that are under development. These compounds are believed to act by the same or very similar mechanisms, so that conclusions drawn from the study of the bases of cisplatin sensitivity and resistance are expected to be valid for other platinum-containing drugs.

Cisplatin is known to form adducts with DNA and to induce interstrand crosslinks. Adduct formation, through an as yet unknown signaling mechanism, is believed to activate some presently unknown cellular enzymes involved in programmed cell death (apoptosis), the process which is believed to be ultimately responsible for cisplatin cytotoxicity (see Eastman, 1990, Cancer Cells 2: 275-2802).

Applicants have demonstrated that the subject compounds are effective in treating resistant tumors in which resistance is mediated through at least one of the following three mechanisms: multidrug resistance, tubulins and topoisomerase I. This section describes these three resistance mechanisms and therapeutic agents for which resistance arises through at least one of these mechanisms. One of skill in the art will understand that tumor cells may be resistant to a chemotherapeutic agent through more than one mechanism. For example, the resistance of tumor cells to paclitaxel may be mediated through via multidrug resistance, or alternatively, via tubulin mutation(s).

In a preferred embodiment, the methods and pharmaceutical compositions of the present invention are useful for treating tumors resistant to certain chemotherapeutic agents.

<u>a. Resistance mediated through tubulins</u>

Microtubules are intracellular filamentous structures present in all eukaryotic cells. As components of different organelles such as mitotic spindles, centrioles, basal bodies, cilia, flagella, axopodia and the cytoskeleton, microtubules are involved in many cellular functions including chromosome movement during mitosis, cell motility, organelle transport, cytokinesis, cell plate formation, maintenance of cell shape and orientation of cell microfibril deposition in developing plant cell walls. The major component of microtubules is tubulin, a protein composed of two subunits called alpha and beta. An important property of tubulin in cells is the ability to undergo polymerization to form microtubules or to depolymerize under appropriate conditions. This process can also occur *in vitro* using isolated tubulin.

Microtubules play a critical role in cell division as components of the mitotic spindle, an organelle which is involved in distributing chromosomes within the dividing cell precisely between the two daughter nuclei. Various drugs prevent cell division by binding to tubulin or to microtubules. Anticancer drugs acting by this mechanism include the alkaloids vincristine and vinblastine, and the taxane-based compounds paclitaxel and docetaxel {see, for example, E. K. Rowinsky and R. C. Donehower, Pharmacology and Therapeutics, 52, 35-84 (1991)}. Other antitubulin compounds active against mammalian cells include benzimidazoles such as nocodazole and natural products such as colchicine, podophyllotoxin, epithilones, and the combretastatins.

Certain therapeutic agents may exert their activities by, for example, binding to α-tubulin, β-tubulin or both, and/or stabilizing microtubules by preventing their depolymerization. Other modes of activity may include, down regulation of the expression of such tubulin proteins, or binding to and modification of the activity of other proteins involved in the control of expression, activity or function of tubulin.

In one embodiment, the resistance of tumor cells to a therapeutic agent is mediated through tubulin. By "mediated through tubulin", it is meant to include direct and indirect involvement of tubulin. For example, resistance may arise due to tubulin mutation, a direct involvement of tubulin in the resistance. Alternatively, resistance may arise due to alterations elsewhere in the cell that affect tubulin and/or microtubules. These alterations may be mutations in genes affecting the expression level or pattern of tubulin, or mutations in genes affecting microtubule assembly in general. Mammals express 6 α- and 6 β-tubulin genes, each of which may mediate drug resistance.

Specifically, tubulin-mediated tumor resistance to a therapeutic agent may be conferred via molecular changes in the tubulin molecules. For example, molecular changes include mutations, such as point mutations, deletions or insertions, splice variants or other changes at the gene, message or protein level. In particular embodiments, such molecular changes may reside in amino acids 250-300 of β-tubulin, or may affect nucleotides 810 and/or 1092 of the β-tubulin gene. For example, and without wishing to be limited, the paclitaxel-resistant human ovarian carcinoma cell line 1A9-PTX10 is mutated at amino acid residues β 270 and β 364 of β-tubulin (see Giannakakou *et al.,* 1997). For another example, two epothilone-resistant human cancer cell lines has acquired β-tubulin mutations at amino acid residues β274 and β282, respectively (See Giannakakou *et al.,* 2000). These mutations are thought to affect the binding of the drugs to tubulins. Alternatively, mutations in tubulins that confer drug resistance may also be alterations that affect microtubule assembly. This change in microtubule assembly has been demonstrated to compensate for the effect of drugs by having diminished microtubule assembly compared to wild-type controls (Minotti, A. M., Barlow, S. B., and Cabral, F. (1991) J Biol Chem 266, 3987-3994). It will also be understood by a person skilled in the art that molecular changes in α-tubulin may also confer resistance to certain compounds. WO 00/71752 describes a wide range of molecular changes to tubulin molecules and the resistance to certain chemotherapeutic compounds that such molecular changes

may confer on a cell. WO 00/71752, and all references therein, are incorporated in their entirety herein.

Tubulin-mediated tumor resistance to therapeutic agents may also be conferred via alterations of the expression pattern of either α-tubulin or the β-tubulin,, or both. For example, several laboratories have provided evidence that changes in the expression of specific β-tubulin genes are associated with paclitaxel resistance in cultured tumor cell lines (Haber, M., Burkhart, C. A., Regl, D. L., Madafiglio, J., Norris, M. D., and Horwitz, S. B. (I 995) J Biol Chem. 270, 31269-75; Jaffrezou, J. P., Durnontet, C., Deny, W. B., Duran, G., Chen, G., Tsuchiya, E., Wilson, L., Jordan, M. A., and Sikic, B. 1. (I 995) Oncology Res. 7, 517-27; Kavallaris, M., Kuo, D. Y. S., Burkhart, C. A., RegI, D. L., Norris, M. D., Haber, M., and Horwitz, S. B. (I 997) J Clin. Invest. 100, 1282-93; and Ranganathan, S., Dexter, D. W., Benetatos, C. A., and Hudes, G. R. (1998) Biochin7. Biophys. Acta 1395, 237-245).

Tubulin-mediated tumor resistance to therapeutic agents may also be conferred via an increase of the total tubulin content of the cell, an increase in the α-tubulin content or the expression of different electrophoretic variants of α-tubulin. Furthermore, resistance may be conferred via alterations in the electrophoretic mobility of β-tubulin subunits, overexpression of the Hβ2 tubulin gene, overexpression of the Hβ3 tubulin gene, overexpression of the Hβ4 tubulin gene, overexpression of the Hβ4a tubulin gene or overexpression of the Hβ5 tubulin gene.

Tubulin-mediated tumor resistance to therapeutic agents may also be conferred via post-translational modification of tubulin, such as increased acetylation of α-tubulin (Jpn J Cancer Res (85) 290-297), via proteins that regulate microtubule dynamics by interacting with tubulin dimmers or polymerized microtubules. Such proteins include but are not limited to stathmin (Mol Cell Biol (1999) 19, 2242-2250) and MAP4 (Biochem Pharmacol (2001) 62, 1469-1480).

Exemplary chemotherapeutic agents for which resistance is at least partly mediated through tubulin include, taxanes (paclitaxel, docetaxel and Taxol derivatives), vinca alkaloids (vinblastine, vincristine, vindesine and vinorelbine), epothilones (epothilone A, epothilone B and discodermolide), nocodazole, colchicin, colchicines derivatives, allocolchicine, Halichondrin B, dolstatin 10, maytansine, rhizoxin, thiocolchicine, trityl cysterin, estramustine and nocodazole. See WO 03/099210 and Giannakakou et al., 2000. Additional exemplary chemotherapeutic agents for which resistance is at least partly mediated through tubulin include, colchicine, curacin, combretastatins, cryptophycins, dolastatin, auristatin PHE, symplostatin 1, eleutherobin, halichondrin B, halimide, hemiasterlins, laulimalide, maytansinoids, PC-SPES, peloruside A, resveratrol, S-allylmercaptocysteine (SAMC), spongistatins, taxanes, vitilevuamide, 2-methoxyestradiol (2-ME2), A-289099, A-293620/A-318315, ABT-751/E7010, ANG 600 series, anhydrovinblastine (AVLB), AVE806, bivatuzumab mertansine, BMS-247550, BMS-310705, cantuzumab mertansine, combretastatin, combretastatin A-4 prodrug (CA4P), CP248/CP461, D-24851/D-64131, dolastatin 10, E7389, EPO906, FR182877, HMN-214, huN901-DM1/BB-10901TAP, ILX-651, KOS-862, LY355703, mebendazole, MLN591DM1, My9-6-DM1, NPI-2352 and NPI-2358, Oxi-4503, R440, SB-715992, SDX-103, T67/T607, trastuzumab-DMI, TZT-1027, vinflunine, ZD6126, ZK-EPO.

Resistance to these and other compounds can be tested and verified using the methods described in the Examples. The methods and pharmaceutical compositions of the present invention are useful for treating tumors resistant to any one or more of above-listed agents.

Preferred chemotherapeutic agents for which resistance is at least partly mediated through tubulin are taxanes, including, but not limited to paclitaxel and docetaxel (Taxotere), which were derived primarily from the Pacific yew tree, *Taxus brevifolia,* and which have activity against certain tumors, particularly breast and ovarian tumors (See, for example, Pazdur et al. Cancer Treat Res. 1993.19:3 5 1; Bissery et al. Cancer Res. 1991 51:4845).

b. Resistance mediated through multidrug resistance

In another embodiment, the resistance of tumor cells to a therapeutic agent is mediated through multidrug resistance. The term "multidrug resistance (MDR)", as used herein, refers to a specific mechanism that limits the ability of a broad class of hydrophobic, weakly cationic compounds to accumulate in the cell. These compounds have diverse structures and mechanisms of action *yet al*l are affected by this mechanism.

Experimental models demonstrate that multidrug resistance can be caused by increased expression of ATP-binding cassette (ABC) transporters, which function as ATP-dependent efflux pumps. These pumps actively transport a wide array of anti-cancer and cytotoxic drugs out of the cell, in particular natural hydrophobic drugs. In mammals, the superfamily of ABC transporters includes P-glycoprotein (P-gp) transporters (MDR1 and MDR3 genes in human), the MRP subfamily (already composed of six members), and bile salt export protein (ABCB11; Cancer Res (1998) 58, 4160-4167), MDR-3 (Nature Rev Cancer (2002) 2, 48-58), lung resistance protein (LRP) and breast cancer resistant protein (BCRP). See Kondratov *et al.,* 2001 and references therein; Cancer Res (1993) 53,747-754; J Biol Chem (1995) 270, 31269-31275; Leukemia (1994) 8, 465-475; Biochem Pharmacol (1997) 53, 461-470; Leonard et al (2003), The Oncologist 8:411-424). These proteins can recognize and efflux numerous substrates with diverged chemical structure, including many anticancer drugs. Overexpression of P-gp is the most common cause for MDR. Other causes of MDR have been attributed to changes in topoisomerase II, protein kinase C and specific glutathione transferase enzymes. See Endicott and Ling, 1989.

The methods of the present invention are useful for treating tumors resistant to a therapeutic agent, in which

resistance is at least partially due to MDR. In a preferred embodiment, the drug resistance of the tumor is mediated through overexpression of an ABC transporter. In a further preferred embodiment, the drug resistance of the tumor is mediated through the overexpression of P-gp. Numerous mechanisms can lead to overexpression of P-gp, including amplification of the MDR-1 gene (Anticancer Res (2002) 22, 2199-2203), increased transcription of the MDR-1 gene (J Clin Invest (1995) 95, 2205-2214; Cancer Lett (1999) 146, 195-199; Clin Cancer Res (1999) 5, 3445-3453; Anticancer Res (2002) 22, 2199-2203), which may be mediated by transcription factors such as RGP8.5 (Nat Genet 2001 (27), 23-29), mechanisms involving changes in MDR-1 translational efficiency (Anticancer Res (2002) 22, 2199-2203), mutations in the MDR-1 gene (Cell (1988) 53, 519-529; Proc Natl Acad Sci USA (1991) 88, 7289-7293; Proc Nat1 Acad Sci USA (1992) 89, 4564-4568) and chromosomal rearrangements involving the MDR-1 gene and resulting in the formation of hybrid genes (J Clin Invest (1997) 99, 1947-1957).

In other embodiments, the methods of the present invention are useful for treating tumors resistant to a therapeutic agent, in which resistance is due to other causes that lead to MDR, including, for example, changes in topoisomerase II, protein kinase C and specific glutathione transferase enzyme.

Therapeutic agents to which resistance is conferred via the action of P-gp include, but is not limited to: vinca alkaloids (*e.g.*, vinblastine), the anthracyclines (*e.g.*, adriamycin, doxorubicin), the epipodophyllotoxins (*e.g.*, etoposide), taxanes (*e.g.*, paclitaxel, docetaxel), antibiotics (*e.g.*, actinomycin D and gramicidin D), antimicrotubule drugs (*e.g.*, colclicine), protein synthesis inhibitors (*e.g.*, puromycin), toxic peptides (*e.g.*, valinomycin), topoisomerase Inhibitors (*e.g.*, topotecan), DNA intercalators (*e.g.*, ethidium bromide) and anti-mitotics. See WO 99/20791. The methods and pharmaceutical compositions of the present invention are useful for treating tumors resistant to any one or more of above-listed drugs.

c. Resistance mediated through topoisomerase I

In a further embodiment, the resistance of tumor cells to a therapeutic agent is mediated through topoisomerase. Exemplary therapeutic agents that belong to this category include those that target topoisomerase, either directly or indirectly.

DNA normally exists as a supercoiled double helix. During replication, it unwinds, with single strands serving as a template for synthesis of new strands. To relieve the torsional stress that develops ahead of the replication fork, transient cleavage of one or both strands of DNA is needed. Without wishing to be bound to any mechanism, it is believed that Topoisomerases facilitate this process as follows: Topoisomerase II causes transient double-stranded breaks, whereas topoisomerase I causes single-strand breaks. This action allows for rotation of the broken strand around the intact strand. Topoisomerase I then re-ligates the broken strand to restore integrity of double-stranded DNA.

In one embodiment, resistance of tumor cells to a therapeutic agent is mediated through topoisomerase. By "mediated through topoisomerase", it is meant to include direct and indirect involvement of topoisomerase. For example, resistance may arise due to topoisomerase mutation, a direct involvement of topoisomerase in the resistance. Alternatively, resistance may arise due to alterations elsewhere in the cell that affect topoisomerase. These alterations may be mutations in genes affecting the expression level or pattern of topoisomerase, or mutations in genes affecting topoisomerase function or activity in general. In preferred embodiments said topoisomerase is topoisomerase I. In other embodiments said topoisomerase is Topoisomerase II.

Without being bound by theory, compounds that act on topoisomerase I bind to the topoisomerase I-DNA complex in a manner that prevents the relegation of DNA. Topoisomerase I initially covalently interacts with DNA. Topoisomerase I then cleaves a single strand of DNA and forms a covalent intermediate via a phosphodiester linkage between tyrosine-273 of topoisomerase I and the 3'-phosphate group of the scissile strand of DNA. The intact strand of DNA is then passed through the break and then topoisomerase I religates the DNA and releases the complex. Drugs such as camptothecins bind to the covalent complex in a manner that prevents DNA relegation. The persistent DNA breaks induce apoptosis, likely via collisions between these lesions and or replication or transcription complexes.

Preferred therapeutic agents to which resistance is mediated through topoisomerase I include camptothecin and its derivatives and analogues, such as 9-nitrocamptothecin (IDEC-132), exatecan (DX-8951f), rubitecan (9-nitrocamptothecin), lurtotecan (GI-147211C), the homocamptothecins such as diflomotecan (BN-80915) and BN-80927, topotecan, NB-506, J107088, pyrazolo [1,5-a] indole derivatives, such as GS-5, lamellarin D, SN-38, 9-aminocamptothecin, ST1481 and karanitecin (BNP1350) and irinotecan (CPT-11). Other related camptothecins can be found in The Camptothecins: Unfolding Their Anticancer Potential, Annals of the New York Academy of Science, Volume 922 (ISBN 1-57331-291-6).

Without wishing to be bound by any particular theory, it is believed that camptothecins inhibit topoisomerase I by blocking the rejoining step of the cleavage/religation reaction of topoisomerase I, resulting in accumulation of a covalent reaction intermediate, the cleavable complex. Specifically, topoisomerase I-mediated tumor resistance to therapeutic agents may be conferred via molecular changes in the topoisomerase I molecules. For example, molecular changes include mutations, such as point mutations, deletions or insertions, splice variants or other changes at the

gene, message or protein level.

In particular embodiments, such molecular changes reside near the catalytic tyrosine residue at amino, acid position 723. Residues at which such molecular changes may occur include but are not limited to amino acid positions 717, 722, 723, 725, 726, 727, 729, 736 and 737 (see Oncogene (2003) 22, 7296-7304 for a review).

In equally preferred embodiments, such molecular changes reside between amino acids 361 and 364. Residues at which such molecular changes may occur include but are not limited to amino acid positions 361, 363 and 364.

In other equally preferred embodiments, such molecular changes reside near amino acid 533. Residues at which such molecular changes may occur include but are not limited to amino acid positions 503 and 533.

In other equally preferred embodiments, such molecular changes may also reside in other amino acids of the topoisomerase I protein. Residues at which such molecular changes may occur include but are not limited to amino acid positions 418 and 503.

In other embodiments, such molecular changes may be a duplication. In one embodiment such a duplication may reside in the nucleotides corresponding to amino acids 20-609 of the topoisomerase I protein.

In other embodiments, topoisomerase I-mediated tumor resistance may also be conferred via cellular proteins that interact with topoisomerase-1. Proteins that are able to do so include, but are not limited to, nucleolin.

In particular embodiments, such molecular changes may reside in amino acids 370 and/or 723. For example, and without wishing to be limited, the camptothecin-resistant human leukemia cell line CEM/C2 (ATCC No. CRL-2264) carries two amino acid substitution at positions 370 (Met→Thr) and 722 (Asn→Ser) (Cancer Res (1995) 55, 1339-1346). The camptothecin resistant CEM/C2 cells were derived from the T lymphoblastoid leukemia cell line CCRF/CEM by selection in the presence of camptothecin *in vitro* (Kapoor et al., 1995. Oncology Research 7; 83-95, ATCC). The CEM/C2 resistant cells display atypical multi-drug resistance and express a form of topoisomerase I that is less sensitive to the inhibitory action of camptothecin than that from CCRF/CEM cells at a reduced level relative to the parental cells. In addition to resistance to camptothecin, the CEM/C2 cells exhibit cross resistance to etoposide, dactinomycin, bleomycin, mitoxantrone, daunorubicin, doxorubicin and 4'-(9-acridinylamino)methanesulfon-m-anisidide.

In other embodiments, topoisomerase I-mediated tumor resistance to therapeutic agents may also be conferred via alterations of the expression pattern the topoisomerase I gene (Oncol Res (1995) 7, 83-95). In further embodiments, topoisomerase I-mediated tumor resistance may also be conferred via altered metabolism of the drug. In yet further embodiments, topoisomerase I-mediated tumor resistance may also be conferred via inadequate and/or reduced accumulation of drug in the tumor, alterations in the structure or location of topoisomerase 1, alterations in the cellular response to the topoisomerase I-drug interaction or alterations in the cellular response to drug-DNA-ternary complex formation (Oncogene (2003) 22, 7296-7304; Ann N Y Acad Sci (2000) 922, 46-55).

Topoisomerase I is believed to move rapidly from the nucleolus to the nucleus or even cytoplasm after cellular exposure to camptothecins.

In one embodiment topoisomerase I-mediate tumor resistance is mediated through factors involved in the relocation of topoisomerase I from the nucleolus to the nucleus and/or the cytoplasm, such as factors involved in the ubiquitin/26S proteasome pathway or SUMO.

In other embodiments topoisomerase I-mediated tumor resistance is mediated through factors involved in DNA replication, DNA checkpoint control and DNA repair.

Factors of the DNA checkpoint control include proteins of the S-checkpoint control, such as Chk1, ATR, ATM, and the DNA-PK multimer.

In other embodiments topoisomerase I-mediated tumor resistance is mediated via factors of apoptosis pathways or other cell death pathways. This includes, but is not limited to, the overexpression of bcl-2 and the overexpression of $p21^{Wafl/Cíp1}$.

In other embodiments topoisomerase I-mediated tumor resistance is mediated via post-translational modifications of topoisomerase I. Such post-translational modifications are ubiquitination and sumoylation. Furthermore, such post-translational modifications may involve other cellular proteins, such as Ubp11, DOA4 and topor.

Therapeutic agents to which resistance is mediated through topoisomerase II include epipodophyllotoxins, such as VP16 and VM26, [1,5-a], pyrazolo [1,5-a] indole derivatives, such as GS-2, GS-3, GS-4 and GS-5.

d. Resistance to Mitoxanthrone

In another embodiment, tumor cells resistant to Mitoxantrone can be treated using the subject compounds.

Resistance to the anticancer drug mitoxantrone has been associated with several mechanisms, including drug accumulation defects and reduction in its target proteins topoisomerase II α and β 4. Recently, overexpression of the breast cancer resistance half transporter protein (BCRP1) was found to be responsible for the occurrence of mitoxantrone resistance in a number of cell lines (Ross et al., J. Natl. Cancer Inst. 91: 429-433, 1999; Miyake et al., Cancer Res. 59: 8-13, 1999; Litman et al., J. Cell Sci. 113(Pt 11): 2011-2021, 2000; Doyle et al., Proc. Natl. Acad. Sci. U.S.A. 95: 15665-15670, 1998). However, not all mitoxantrone resistant cell lines express BCRP1 (Hazlehurst et al., Cancer Res.

59: 1021-1028, 1999; Nielsen et al., Biochem. Pharmacol. 60: 363-370, 2000). The efflux pump responsible for the mitoxantrone resistance in these cell lines is less clear. Boonstra et al. (Br J Cancer. 2004 May 18 [Epub ahead of print]) report that overexpression of the ABC transporter ABCA2 may lead to the efflux of mitoxantrone by exploring estramustine, which is able to block mitoxantrone efflux in the mitoxantrone resistant GLC4 sub line GLC4-MITO (does not overexpress BCRP1).

The methods of the present invention are useful for treating tumors resistant to a mitoxanthrone.

VII. Assays for effectiveness of treatment

In one embodiment, the compounds of the present invention kill tumor cells resistant to a other various therapeutic agents. Viability of a tumor cell can be determined by any methods known in the art. For example, one may use the colorimetric cytotoxicity assay described for anticancer drug screening in Skekan et al., J. Natl. Cancer. Inst. 82: 1107-12 (1990). For another example, one may determine the viability of a tumor cell by contacting the cell with a dye and viewing it under a microscope. Viable cells can be observed to have an intact membrane and do not stain, whereas dying or dead cells having "leaky" membranes do stain. Incorporation of the dye by the cell indicates the death of the cell. A dye useful for this purpose is trypan blue.

The exemplary composition of the present invention may induce apoptosis, a mode of cell death, in resistant tumor cells. Apoptosis is recognized by a characteristic pattern of morphological, biochemical and molecular changes. Cells going through apoptosis appear shrunken and rounded. They also can be observed to become detached from a culture dish in which they are maintained. The morphological changes involve a characteristic pattern of condensation of chromatin and cytoplasm which can be readily identified by microscopy. When stained with a DNA-binding dye, *e.g.*, H33258, apoptotic cells display classic condensed and punctuate nuclei instead of homogenous and round nuclei.

A typical characteristic of apoptosis is endonucleolysis, a molecular change in which nuclear DNA is initially degraded at the linker sections of nucleosomes to give rise to fragments equivalent to single and multiple nucleosomes. When these DNA fragments are subjected to gel electrophoresis, they reveal a series of DNA bands which are positioned approximately equally distant from each other on the gel. The size difference between the two bands next to each other is about the length of one nucleosome, *i.e.*, 120 base pairs. This characteristic display of the DNA bands is called a DNA ladder and it indicates apoptosis of the cell. Apoptotic cells can also be identified by flow cytometric methods based on measurement of cellular DNA content, increased sensitivity of DNA to denaturation, or altered light scattering properties. These methods are well known in the art. It should be recognized however, that modes of programmed cell death, including apoptosis, may following a number of mechanisms or show other phenotypes/properties to those described above. In such cases, these mechanisms may also be characterized, classified or considered as "apoptosis".

For example, incubation of cells with A37 for 72 hours at 5 X of the $IC_{50}$ concentration induced apoptosis, as evidenced by positive TUNEL staining in about 60% of the cells. In addition, PARP cleavage was also observed. Similarly, cells incubated with B16 are 90% TUNEL positive. Such assays can be used for apoptosis detection in any cells in contact with the subject compounds.

Cytotoxicity may also be measured using the SRB assay according to Shekan et al (J Natl Cancer Inst (1990) 82, 1107-112), as described in the Examples.

Additional assays for cell viability are described in Chapter 15 of Handbook of Fluorescent Probes and Research Products (Molecular Probes Handbook), which is incorporated in its entirety herein.

In another embodiment, the compounds of the present invention inhibit the growth of tumor cells resistant to various therapeutic agents. The growth inhibition of resistant tumor cells caused by a subject compound can be either partial (slowing down cell growth) or complete inhibition (*i.e.*, arresting cells at a certain point in cell cycle). Cell growth can be measured by any techniques known in the art. Such techniques include, for example, MTT assay (based on reduction of the tetrazolium salt 3, [4,5-dimethylthiazol-2-yl]-2,5-diphenytetrazolium bromide), and PicoGreen assay using the DNA-binding dye Picogreen, both of which are described in Torrance, et al., Nat. Biotech. 19:940-945 (2001), incorporated herein in its entirety. Other assays for cell proliferation/growth are described in Chapter 15 of Handbook of Fluorescent Probes and Research Products (Molecular Probes Handbook).

Progression of disease, cancer or tumor in response to treatment using the subject compounds can be monitored using any standard technique known in the art. For example, tumor size can be monitored and assessed to see if tumor size reduction has occurred as a result of the treatment. Monitoring and assessment may be aided by a variety of means including biopsies, manual inspection, microscopy, whole or partial body imaging and scans, and various molecular-based diagnostic and prognostic methods including those that investigate tumor-specific markers or mutations.

VIII. Tumors and Other Proliferative Disorders

The subject compounds are useful to treat various disorders, including proliferative disorders resistant to other therapeutic agents. The term "proliferative disorder" is also art recognized and includes a disorder affecting an animal

in a manner which is marked by aberrant, or otherwise unwanted, proliferation of a subset of cells of an animal. Cancer and tumors are proliferative disorders. Cells comprising or derived from a tumor will generally be understood to be a proliferating cell, typically a hyper-proliferating cell, and in other circumstances, a tumor cell may be dysplastic, or may have proliferated.

It will be apparent to a person skilled in the art, on reading the disclosure of the instant invention, that the methods, pharmaceutical compositions and packaged pharmaceuticals comprising the subject compounds will be useful for the treatment of other proliferative disorders, or for killing or inhibiting proliferating cells including tumor cells.

Tumors that are resistant or refractory to treatment of a variety of chemotherapeutic agents may benefit from treatment with the methods and pharmaceutical compositions of the present invention. Suitable tumors may be solid tumors, which are cancer of body tissues other than blood, bone marrow, or the lymphatic system. Preferred tumors are those resistant to chemotherapeutic agents other than the same class of agents disclosed in the formulae. Suitable tumors may also be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, skin cancer, cervical cancer, ovarian cancer, lung cancer, bronchial, small and non-small-cell lung cancer, gastric, prostate, pancreas, and head and neck cancer.

Hematological tumors may be leukemia, such as Acute Myelogenous Leukemia (AML), Acute Lymphoblastic Leukemia (ALL), Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin Lymphoma, Non-Hodgkin Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Extramedullaiy myelonia, Granulocytic Sarcomae.

In certain embodiments, the methods and compositions of the present invention can be used to treat tumors resistant or refractory to taxanes. Such tumors include, for example, breast cancer, cervical cancer, colorectal cancer, peritoneal cancer, ovarian cancer, bronchial cancer, small cell lung cancer, non-small cell lung cancer, gastric, prostate, and head and neck cancer, including recurrent squamous cell carcinomas.

In other embodiments, the methods and compositions of the present invention can be used to treat cancers, proliferative, degenerative and other diseases, including those listed in Table I.

In other embodiments, the methods and compositions of the present invention are applicable for treating tumors resistant or refractory to camptothecin and its analogues. Such tumors include any tumor for which camptothecin and its analogues have shown activities. Examples of such tumors include ovarian cancer and small-cell lung cancer, for which both topotecan and irinotecan have shown effectiveness, colorectal cancer, upper gastrointestinal malignancies, non-small-cell lung cancer, mesothelioma, and head and neck cancer, for which irinotecan has shown effectiveness, small-cell lung cancer, cervical cancer, breast cancer, prostate cancer, rectal cancer, leukemia, lymphoma cancers and malignant melanoma. See Pizzolato and Saltz, The Lancet 361:2235-42 (2003).

Refractory cancers or tumors include those that fail or are resistant to treatment with chemotherapeutic agents alone, radiation alone or combinations thereof. For the purposes of this specification, refractory cancers or tumors also encompass those that appear to be inhibited by treatment with chemotherapeutic agents and/or radiation but recur up to five years, sometimes up to ten years or longer, after treatment is discontinued.

The term "resistant", as used herein, include both partially resistant and completely resistant. Thus, a tumor that is only partially resistant to another therapeutic agent may nonetheless benefit from treatment with the subject compounds. Indeed, in certain embodiments it may be beneficial to treat a tumor if such resistance is merely suspected, may not yet be known, or even before such resistance has developed. In such cases, a co-administration, combination therapy or treatment regime may be envisioned, by appropriate use of the subject compounds together with appropriate other therapeutic agents. In alternative embodiments of all aspects of the instant invention, the subject compounds will be useful in treating individuals suffering from a cancer or a tumor that has been previously treated with any of the other therapeutic agents. In such embodiments, it may be subsequently determined that the cancer or tumor was resistant or refractory to the other therapeutic agents.

Cell lines according to the invention that may be used to evaluate whether the compounds of this invention possess cytotoxic activity against drug resistant cell lines include but are not limited to the taxane-resistant tubulin-mutated cell lines 1A9-PTX10 and 1A9-PTX22 and their parental cell line 1A9 (J Biol Chem (1997) 272, 17118-17124), the adriamycin-resistant Pgp overexpressing cell line NCI-Adr resistant (Vickers et al., 1989. Mol Endocrinology 3 (1): 157-164), the camptothecin-resistant cell lines CEM/C1 and CEM/C2 and their parental cell line CEM (Kapoor et al.,

1995. Oncology Research 7; 83-95), the TWIST overexpressing cell line HNE1-T3 and its parental cell line (Oncogene (2004) 23, 474-482) and the paclitaxel-resistant subclones of the human ovarian cancer cell line SKOV-3 and SKOV-3 (Clin Cancer Res (2003) 9, 2778-2785).

The subject compounds are also believed useful in treating other types of proliferative disorders, especially those associated with abnormal kinase activity, including proliferative disorders which are characterized by benign indications. Such disorders may also be known as "cytoproliferative" or "hyperproliferative" in that cells are made by the body at an atypically elevated rate. Such disorders include, but are not limited to, the following: hemangiomatosis in newborns, secondary progressive multiple sclerosis, chronic progressive myelodegenerative disease, neurofibromatosis, ganglioneuromatosis, keloid formation, Paget's Disease of the bone, fibrocystic disease of the breast, Peronies and Duputren's fibrosis, restenosis, and cirrhosis.

IX. Combination Therapy

The subject pharmaceutical compositions can be co-administered, *e.g.*, in the same or different formulation, with a variety of other drugs. For example, the subject pharmaceutical compositions can be used as part of a regiment of treatment in which they are combined with other drugs for the various described disease conditions, such as chemotherapeutic agents including anti-cancer therapeutic agents that inhibit cancer growth, anti-angiogenesis agents and anti-metastatic agents. The subject pharmaceutical compositions may also be combined with immunomodulators.

In a preferred embodiment, the subject pharmaceutical compositions are co-administered with an agent that tackles a specific drug resistance mechanism. For example, when a tumor drug resistance is caused by an increase in drug efflux brought about by an overexpression of ATP-dependent pumps such as P-glycoprotein, pharmacological agents that reverse the excessive drug efflux through P-glycoprotein can be used in combination with the subject pharmaceutical compositions for treating resistance tumors. Such suitable pharmacological agents include, for example, phenothiazines, verapamil, tamoxifen, quinidine, phenothiazines, cyclosporine A, methylenedioxymethamphetamine, methylenedioxyethylamphetamine, paramethoxyamphetamine, pervilleine F, PSC 833 and LY335979, among others. For a general discussion of the pharmacologic implications for the clinical use of P-glycoprotein inhibitors, see Lum et al., Drug Resist. Clin. Onc. Hemat., 9: 319-336 (1995); Schinkel et al., Eur. J. Cancer, 31A: 1295-1298 (1995).

Most suitable drugs to combine with the subject pharmaceutical compositions is PSC-833 (Valspodar), an analogue of cyclosporine. PSC-833 was found to be an P-gp inhibitor 10 times more potent than cyclosporine, and without its side-effects of nephrotoxicity and immunosuppression. Although combination phase I and II studies in different tumor types showed that PSC-833 had a profound effect on the pharmacokinetics of the co-administered chemotherapy, this effect could be adjusted for by reducing the dose of chemotherapy given. See Baird and Kaye, 2003.

In another embodiment, when a tumor drug resistance is caused by a mutation in β-tubulin, cancer therapeutic agents that have cellular targets other than microtubules may be used in combination with the subject pharmaceutical compositions.

In a further embodiment, the subject compounds are administered to a patient to whom is also administered an anti-emetic agent. Anti-emetic agents according to this invention are any anti-emetic agents known to the skill artisan, including, but are not limited to, serotonin-3 receptor antagonists like granisetron, ondansetron and tropisetron, NK1 receptor antagonists, antihistamines such as cinnarizine, cyclizine and promethazine, histamine H2 receptor antagonists such as ranitidine (Zantac), phenothiazines such as chlorpromazine, droperidol, haloperidol, methotrimeprazine, perphenazine, trifluoperazine and prochlorperazine, domperidone, and metoclopramide.

In other embodiments, the subject compound is administered to a patient, to whom is also administered an anti-diarrheal such as loperamid, corticosteroide such as cortisone, growth hormone or growth factor such as GCSF or erythropoietin, a diuretica such as furosemid, steroidal or non-steroidal analgesics such as an opiate, *e.g.* morphine, or paracetamol or anti-hyperuricemics such as allopurinol.

In other embodiments, the subject compound is administered to a patient, who is also treated with thrombocytes, erythrocytes or whole blood.

In other embodiments, the subject compound is administered to a patient, who is also treated with stem cells from bone marrow.

In other embodiments, the instant invention also relate to a method of therapeutic patient care. In the method, a patient who is administered the subject compound receives food parenterally.

The term "co-administer" or "co-administered", as used herein, include administering two or more different therapeutic agents concurrently, sequentially or intermittently in all of the various aspects of the method of the invention. Thus, the subject compounds may be administered before, after, or together with one or more other therapeutic agents to an individual in need of. In one embodiment, two or more therapeutic agents are formulated together with one or more of the subject compound in a single pill.

The methods of the present invention can also be combined with other methods of cancer treatment, such as radiation therapy, surgery, or immunotherapy.

X. Packaged Pharmaceuticals and Other Methods

The present invention also provides a packaged pharmaceutical comprising a pharmaceutical composition of the subject compounds and instructions to administer an effective amount of the pharmaceutical composition to a patient, such as one who is previously treated with other therapeutic agents. In a particular embodiment, the packaged pharmaceutical also includes an anti-emetic or anti-diarrheal therapeutic composition and/or instructions to administer an effective amount of the anti-emetic therapeutic composition to said patient.

The present invention further provide methods of conducting a pharmaceutical business, which comprises: a) compiling data including: i) bioequivalence data for a subject compound and their metabolites compared to a marketed originator compound; ii) clinical data demonstrating the effectiveness of said subject compound in treating patients; b) submitting said compiled data to a drug regulatory authority for the purpose or obtaining regulatory or marketing approval for said compound for the treatment of patients previously treated with another therapeutic agent; and c) preparing to manufacture, import, package/re-package, label/re-label or market said subject compound, or license rights to said approval, for the treatment of patients.

In a particular embodiment, the methods for conducting a pharmaceutical business further includes marketing, distributing or selling said subject compound for the treatment of patients.

Such methods may be followed, for example, by a generic pharmaceutical company wishing to introduce a generic version of a previously approved therapeutic compound on to the market.

XI. Kinase Inhibitors for Mutant Kinases

Yet another surprising feature of the subject compounds is their ability to inhibit the activity of certain mutations of kinases, preferably those mutation forms that exhibit resistant to other kinase inhibitors.

For example, Chronic Myelogenous Leukemia (CML) is a hematopoietic stem cell disorder characterized by the Philadelphia chromosome, the result of a (9;22) translocation that fuses BCR sequence with the ABL gene and produces the constitutively active, Bcr-Abl tyrosine kinase (Deininger et al., Blood 96: 3343-3356, 2000). The disease progresses through three phases: chronic, accelerated and blast crisis, with disease progression likely due to an accumulation of additional genetic abnormalities (Sawyers, N. Engl. J. Med. 340:.1330-1340, 1999).

The clinical success of imatinib mesylate (STI571, GLEEVEC®), a selective inhibitor of Bcr-Abl kinase activity, validates the therapeutic strategy of rationally targeting the causative molecular abnormality of CML (Druker et al., N. Engl. J. Med. 344: 1038-1042, 2001; Druker et al., N. Engl. J. Med. 344: 1031-1037, 200 1). Ninety-five percent of patients treated in chronic phase achieve a complete hematologic remission and 60% a major cytogenetic response, however, most blast crisis patients either fail to respond or quickly relapse following an initial response to imatinib (Kantarjian et al., N. Engl. J. Med. 346: 645-652, 2002; Sawyers et al., Blood 99: 3530-3539, 2002).

Mutations within the Bcr-Abl kinase domain are the most commonly identified mechanism associated with relapse (Gorre et al., Science 293: 876-880, 2001; Hochhaus et al., Leukemia 16: 2190-2196, 2002). Mutational analysis based on the crystal structure of an imatinib-related compound bound to the Abl kinase established the relevance of amino acids 315 and 253 as critical for efficient imatinib binding (Corbin et al., Blood 96: 470a, 2000; Corbin et al., J. Biol. Chem. 277: 32214-32219, 2002; Schindler et al., Science 289: 1938-1942, 2000). Without being bound by theory, it is believed that Threonine 315 is involved in the formation of a critical hydrogen bond with GLEEVEC®. This critical bond may be prevented if isoleucine is substituted for threonine at position 315, and the bulky side chain of isoleucine may sterically hinder the binding of GLEEVEC®. Mutations of these two amino acids along with amino acids 255 and 351, were subsequently identified in 60% of patients with kinase domain mutations at the time of disease relapse, with an overall mutation frequency between 30% and 90% (Gorre, supra; Hochhaus, supra; Hochhaus et al., Science 293: 2163, 2001; Branford et al., Blood 99: 3472-3475, 2002; Hofmann et al., Blood 99: 1860-1862, 2002; Roche-Lestienne et al., Blood 100: 1014-1018, 2002; Shah et al., Cancer Cell. 2: 117, 2002; von Bubnoff et al., Lancet 359: 487-491, 2002). The marked decrease in sensitivity of these mutants to imatinib implicates them as the likely cause of resistance.

Additional mutations including M244V, G250E, Q252H, F311L, F317L, E355G, F359V, V379I, L387M, H396P/R were identified in patients, albeit at a lower frequency (Hochhaus, supra; Branford, supra; Roche-Lestienne, supra; Shah, supra; von Bubnoff, supra).

Although GLEEVEC® (STI-571) is a ~ 400 nM inhibitor ($IC_{50}$) of the wild-type form of c-Abl, it is non-potent inhibitor ($IC_{50}$ 30 μM) of the T315I mutant of c-Abl (a reduction of about 75-fold). In contrast, exemplary kinase inhibitors of formula I A37 and B16, respectively, show $IC_{50}$'s of < 50nM and < 100 nM for inhibition of wild-type c-Abl, *yet also* show significant potency as inhibitors of the T315I mutant form with $IC_{50}$'s of< 100 nM and ~100nM.

Clinical studies have shown that chronic myeloid leukemia (CML) patients with advanced disease often respond initially to GLEEVEC® but then relapse. In one study, of such relapsed patients, GLEEVEC® resistance was associated with a single base substitution that leads to the T315I mutation in six out of nine patients (Gorre et al., Science 293:876, 2001). The subject compounds that show activity against mutant kinases such as the T315I mutant of c-Abl, may have

further utility in treating cancers, including CML, that have become refractory to other therapeutics including GLEEVEC®.

Other cancers associated with mutant forms of kinase include gastrointestinal stromal tumors (GISTs). GISTs belong to a group of cancers called soft tissue sarcomas. Sarcomas are a rare type of cancer that can occur in connective tissues, bones, muscles, fat, nerves, blood vessels, and cartilage. Sarcomas are derived from the general class of cells known as "mesenchymal cells."

Gastrointestinal stromal tumor (GIST) occurs in 10-20 per one million people; one out of 3-4 is malignant. It is the most common mesenchymal tumor of the gastrointestinal tract. Currently, it is believed to originate from an intestinal pacemaker cell called the interstitial cell of Cajal. GIST may develop anywhere along the gastrointestinal tract, but most often it arises in the stomach and, less commonly, in the intestine. Occasionally, GIST develops outside the gastrointestinal tract in the mesentery, omentum, or retroperitoneum.

Most (50-80%) GISTs arise because of a mutation in the transmembrane receptor c-kit, which is a receptor for the growth factor SCF (stem cell factor). The c-kit/CD117 receptor is expressed on ICCs and a large number of other cells. The c-kit molecule comprises a long extracellular domain, a transcellular segment, and an intracellular part. Mutations generally occur in the DNA encoding the intracellular part (exon 11), which acts as a tyrosine kinase to activate other enzymes. Mutations make c-kit function independent of activation by SCF, leading to a high cell division rate and possibly genomic instability. It is likely that additional mutations are needed for a cell with a c-kit mutation to develop into a GIST, but the c-kit mutation is probably the first step of this process. The tyrosine kinase function of c-kit is vital in the therapy for GISTs.

Until recently, GISTs were notorious for being resistant to chemotherapy, with a success rate of<5%. Recently, imatinib (GLIVEC®/ GLEEVEC®), a drug initially marketed for chronic myelogenous leukemia, turned out to inhibit the c-kit tyrosine kinase, leading to a 40-70% response rate in metastatic or inoperable GISTs. However, certain point mutations in the c-kit oncogene may lead to increased kinase activity but prevent the binding of GLEEVEC® to the c-kit receptor, thus hindering the treatment of GIST by GLEEVEC®. Certain subject compounds, including **A37** and, **B16**, are shown to be inhibitors of c-kit and may also have utility in the treatment of GIST patients that do not respond to GLEEVEC®.

Thus another aspect of the invention provides a method for treating an individual with a disease condition associated with a mutant kinase such as a mutant c-Abl or c-kit, comprising administering to said individual an effective amount of a subject compound, or isomeric, prodrug, tautomeric, pharmaceutically acceptable salt, N-oxide, or stereoisomeric form thereof.

In a related embodiment, the invention provides a use of a subject compound for the manufacture of a medicament for treating an individual with a disease condition associated with a mutant kinase such as a mutant c-Abl or c-kit, comprising administering to said individual an effective amount of a subject compound, or isomeric, prodrug, tautomeric, pharmaceutically acceptable salt, N-oxide, or stereoisomeric form thereof.

Thus, in one aspect of the invention is the subject compounds are used to inhibit a mutant kinase. In certain embodiments, such mutant kinase is inhibited by administering to a host, individual or patient in need of such treatment a therapeutically effective amount of said compound.

In another aspect, the subject compounds are used to treat a disease susceptible to treatment by inhibition of a mutant kinase. In certain embodiments, such treatment comprises administering to a host, individual or patient in need of such treatment a therapeutically effective amount of said compound

In certain embodiments, the mutant kinase is a Bcr-Ab1 or a AblT315I mutation. In other embodiments, the disease condition is selected from GIST or CML.

In another embodiment, the disease, host or patient has become resistant or refractory to GLEEVEC®. In yet another embodiment, the disease, host or patient is suspected of being or of becoming resistant to a therapeutic agent. In other embodiments, the patient has been previously treated with a therapeutic agent or is co-administered with the therapeutic agent and a subject compound. In certain embodiments, the therapeutic agent is an inhibitor of a kinase, such as GLEEVEC®.

"Mutant kinase" generally refers to a kinase that is not wild-type (such as the Bcr-Ab1 p210 kinase). The term preferably includes kinases with one or more mutations (*e.g.*, a point mutation, small in-frame deletion / insertion, etc.) occurring at the kinase active site, such as at residues catalyzing the phosphate transfer, residues important for substrate binding, or residues otherwise important for the kinase reaction (*e.g.* a T315I mutation of the c-Abl or the Bcr-Abl kinases). Such mutant kinases typically have altered (*e.g.*, enhanced) kinase activity as compared to their wild-type counterparts. Kinases which have mutations at residues not appreciably affecting kinase activity are generally not considered a mutant kinase for this particular embodiment.

XII. <u>Dosage and Formulation</u>

The kinase inhibitors of this invention can be administered as treatment for cancer or proliferative or other diseases by any means that produces contact of the active agent with the agent's site of action in the body of a mammal.

They can be administered by any conventional means available for use in conjunction with pharmaceuticals, either as individual therapeutic agents or in a combination of therapeutic agents. The chemical features of the inhibitors described herein bestow favorable solubility properties on the compounds, rendering them suitable for administration as intravenous formulations, topical formulations, oral formulations, and others as discussed in greater detail below. They can be administered alone, but preferably are administered with a pharmaceutical carrier selected on the basis of the chosen route of administration and standard pharmaceutical practice. Suitable vehicles and their formulation are described, for example, in the book Remington's Pharmaceutical Sciences (Remington's Pharmaceutical Sciences. Mack Publishing Company, Easton, Pa., USA 1985).

In another aspect, the present invention provides pharmaceutically acceptable compositions which comprise a therapeutically effective amount of one or more compounds of the subject invention, such as described above, formulated together with one or more pharmaceutically acceptable carriers (additives) and/or diluents. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intrarectally, for example, as a pessary, cream or foam. In certain embodiments, the pharmaceutical preparations may be non-pyrogenic, *i.e.,* do not elevate the body temperature of a patient.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples ofpharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

The dosage administered will, of course, vary depending upon known factors, such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient can be expected to be about 0.001 to about 1000 milligrams per kilogram of body weight, with the preferred dose being about 0.1 to about 30 mg/kg.

Dosage forms of compositions suitable for administration contain from about 1 mg to about 100 mg of active ingredient per unit. In these pharmaceutical compositions the active ingredient will ordinarily be present in an amount of about 0.95% by weight based on the total weight of the composition. The active ingredient can be administered orally in solid dosage forms, such as capsules, tablets and powders, or in liquid dosage forms, such as elixirs, syrups and suspensions. It can also be administered parenterally, in sterile liquid dosage forms.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of inhibitor which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association an inhibitor of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. An inhibitor of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin,

polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered inhibitor moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulations so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active inhibitor(s) of the present invention, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active inhibitor.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active prenyltransferase inhibitor, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present

invention to the body. Such dosage forms can be made by dissolving or dispersing an inhibitor of the present invention in the proper medium. Absorption enhancers can also be used to increase the flux of the drug across the skin. The rate of such flux can be controlled by either providing a rate controlling membrane or dispersing the compound of the present invention in a polymer matrix or gel.

Ophthalmic formulations, eye ointments, powders, solutions and the like, are also contemplated as being within the scope of this invention.

Pharmaceutical compositions of this invention suitable for parenteral administration comprise one or more inhibitors of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the therapeutic effect of an inhibitor, it is desirable to slow the absorption of the inhibitor from subcutaneous or intramuscular injection. This may be accomplished by the use of a liquid suspension of crystalline or amorphous material having poor water solubility. The rate of absorption of the inhibitor then depends upon its rate of dissolution which, in turn, may depend upon crystal size and crystalline form. Alternatively, delayed absorption of a parenterally administered inhibitor form is accomplished by dissolving or suspending the inhibitor in an oil vehicle.

Injectable depot forms are made by forming microencapsuled matrices of the subject inhibitors in biodegradable polymers such as polylactide-polyglycolide. Depending on the ratio of drug to polymer, and the nature of the particular polymer employed, the rate of drug release can be controlled. Examples of other biodegradable polymers include poly (orthoesters) and poly(anhydnides). Depot injectable formulations are also prepared by entrapping the drug in liposomes or microemulsions which are compatible with body tissue.

When the compounds of the present invention are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

The preparations of the present invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

Regardless of the route of administration selected, the CDK inhibitors useful in the subject method may be used in a suitable hydrated form, and/or the pharmaceutical compositions of the present invention, are formulated into pharmaceutically acceptable dosage forms by conventional methods known to those of skill in the art.

Gelatin capsules contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract. Solid compositions of a similar type are also employed as fillers in soft and hard-filled gelatin capsules; preferred materials in this connection also include lactose or milk sugar as well as high molecular weight polyethylene glycols. A preferred formulation is a solution or suspension in an oil, for example olive oil, Miglyol, or Capmul, in a soft gelatin capsule. Antioxidants may be added to prevent long-term degradation as appropriate.

Liquid dosage forms for oral administration can contain coloring and flavoring to increase patient acceptance. In general, water, a suitable oil, saline, ethanol, aqueous dextrose (glucose), and related sugar solutions, glycols such as propylene glycol or polyethylene glycols, or mixtures of these are suitable carriers for parenteral solutions.

For intravenous administration, compounds disclosed above may be formulated as a sterile solution of the active ingredient, either in its free or salt form, in physiological buffer or sterile water. Sugar-containing carrier liquids (such as Ringer's lactate, or other glucose or dextrose solutions) can be used if desired, provided that the total sugar content does not cause undesired levels of lactic acidosis. Intravenous administration can be either through bolus injection (preferably several times per day), or through continuous infusion over a sustained period of time. Total preferred dosages for bolus injection or infusion may vary substantially, depending on a patient's physical condition; in general, they will usually range from about 25 mg/kg to about 250 mg/kg.

Solutions for parenteral administration preferably contain a water-soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts, and sodium EDTA. In addition, parenteral solutions can contain preservatives, such as benzalkonium chloride, methyl- or propyl-paraben, and chlorobutanol. Suitable pharmaceutical carriers are described in Remington's Pharmaceutical Sciences, 18th ed., Mack Publishing Company, Easton, PA, 1990, a standard reference text in this field, the disclosure of which is hereby incorporated by reference.

## XIII. Therapeutic Applications

Due to the key role of CDKs in the regulation of cellular proliferation in general, the compounds disclosed herein may act as reversible cytostatic agents which may be useful in the treatment of any disease process which features abnormal cellular proliferation, such as hyperproliferative diseases, including cancer, benign prostate hyperplasia, familial adenomatosis polyposis, neurofibromatosis, psoriasis, fungal infections, endotoxic shock, hypertrophic scar formation, inflammatory bowel disease, transplant rejection, vascular smooth muscle cell proliferation associated with atherosclerosis, psoriasis, pulmonary fibrosis, arthritis, glomerulonephritis, restenosis following angioplasty or vascular surgery, and other post-surgical stenosis and restenosis. See, for example, U.S. Patent Nos. 6,114,365 and 6,107,305.

The compounds disclosed herein are expected to be useful in the therapy of proliferative or hyperproliferative diseases such as cancer, autoimmune diseases, viral diseases, fungal diseases, neurodegenerative disorders and cardiovascular disease.

More specifically, the compounds disclosed herein are useful in the treatment of a variety of cancers, including (but not limited to) the following: carcinoma, including that of the bladder, breast, colon, kidney, liver, lung, including small cell lung cancer, esophagus, gall bladder, ovary, pancreas, stomach, cervix, thyroid, prostate, and skin, including squamous cell carcinoma; hematopoietic tumors of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma, and Burkett's lymphoma; hematopoietic tumors of myeloid lineage, including acute and chronic myelogenous leukemias, myelodysplastic syndrome, and promyelocytic leukemia; tumors of mesenchymal origin, including fibrosarcoma and rhabdomyosarcoma; tumors of the central and peripheral nervous system, including astrocytoma, neuroblastoma, glioma, and schwannomas; and other tumors, including melanoma, seminoma, teratocarcinoma, osteosarcoma, xenoderoma pigmentosum, keratoctanthoma, thyroid follicular cancer, and Kaposi's sarcoma.

Compounds disclosed herein may also be useful in the treatment of Alzheimer's disease, as suggested by the recent finding that cdk5 is involved in the phosphorylation of tau protein (J. Biochem, 117, 741-749 (1995)).

Compounds disclosed herein may induce or inhibit apoptosis. The apoptotic response is aberrant in a variety of human diseases. Compounds described herein, as modulators of apoptosis, will be useful in the treatment of cancer (including but not limited to those types mentioned hereinabove), viral infections (including but not limited to herpesvirus, poxvirus, Epstein-Barr virus, Sindbis virus and adenovirus), prevention of AIDS development in HIV-infected individuals, autoimmune diseases (including but not limited to systemic lupus, erythematosus, autoimmune mediated glomerulonephritis, rheumatoid arthritis, psoriasis, inflammatory bowel disease, and autoimmune diabetes mellitus), neurodegenerative disorders (including but not limited to Alzheimer's disease, AIDS-related dementia, Parkinson's disease, amyotrophic lateral sclerosis, retinitis pigmentosa, spinal muscular atrophy and cerebellar degeneration), myelodysplastic syndromes, aplastic anemia, ischemic injury associated with myocardial infarctions, stroke and reperfusion injury, arrhythmia, atherosclerosis, toxin-induced or alcohol related liver diseases, hematological diseases (including but not limited to chronic anemia and aplastic anemia), degenerative diseases of the musculoskeletal system (including but not limited to osteoporosis and arthritis) aspirin-sensitive rhinosinusitis, cystic fibrosis, multiple sclerosis, kidney diseases and cancer pain.

Compounds disclosed herein, as inhibitors of the CDKs, can modulate the level of cellular RNA and DNA

synthesis. These agents would therefore be useful in the treatment of viral infections (including but not limited to HIV, human papilloma virus, herpesvirus, poxvirus, Epstein-Barr virus, Sindbis virus, and adenovirus).

Compounds disclosed herein may also be useful in the chemoprevention of cancer. Chemoprevention is defined as inhibiting the development of invasive cancer by either blocking the initiating mutagenic event or by blocking the progression of pre-malignant cells that have already suffered an insult or inhibiting tumor relapse.

Compounds disclosed herein may also be useful in inhibiting tumor angiogenesis and metastasis.

Compounds disclosed herein may also be employed in the prevention of hair loss that ordinarily accompanies many traditional chemotherapeutic regimens. For example, a CDK inhibitor of the invention may be used to inhibit proliferation of cells in hair follicles, thereby sparing them from attack by a cytotoxic agent that targets proliferating cells.

Compounds disclosed herein may also act as inhibitors of other protein kinases, *e.g.*, protein kinase C, her2, raf 1, MEK1, MAP kinase, EGF receptor, PDGF receptor, IGF receptor, PI3 kinase, wee1 kinase, Src, Abl and thus be effective in the treatment of diseases associated with other protein kinases.

The compounds of this invention may also be useful in combination (administered together or sequentially) with known anti-cancer treatments such as radiation therapy or with cytostatic or cytotoxic agents, such as for example, but not limited to, DNA interactive agents, such as cisplatin or doxorubicin; topoisomerase II inhibitors, such as etoposide; topoisomerase I inhibitors such as CPT-11 or topotecan; tubulin interacting agents, such as paclitaxel, docetaxel or the epothilones; hormonal agents, such as tamoxifen; thymidilate synthase inhibitors, such as 5-fluorouracil; and anti-metabolites, such as methotrexate. In such combinations, the compounds and formulations of the present invention may be useful for the prevention or reduction of incidence of alopecia, which is often induced by radiation therapy or chemotherapy.

If formulated as a fixed dose, such combination products employ the compounds of this invention within the dosage range described below and the other pharmaceutically active agent or treatment within its approved dosage range. For example, the cdc2 inhibitor olomucine has been found to act synergistically with known cytotoxic agents in inducing apoptosis (J. Cell Sci., 108, 2897 (1995)). Compounds described herein may also be administered sequentially with known anticancer or cytotoxic agents when a combination formulation is inappropriate. The invention is not limited in the sequence of administration; compounds described herein may be administered either prior to or after administration of the known anticancer or cytotoxic agent. For example, the cytotoxic activity of the cyclin-dependent kinase inhibitor flavopiridol is affected by the sequence of administration with anticancer agents. Cancer Research, 57, 3375 (1997).

A number of PCT and co-pending U.S. utility applications describe in detail about many CDK inhibitor compounds identical or similar to those disclosed herein, in terms of the structure, function, manufacture, various uses, including dosage forms, effective doses for various uses, etc. Most, if not all of these compounds can be used for the instant invention. The entire contents of these PCT and U.S. utility applications are incorporated herein by reference. Such applications include: U.S.S.N. 10/321284, published as US20030162797; U.S.S.N. 10/492116, filed on April 9, 2004; U.S.S.N. 10/820453, filed on April 7, 2004; W03/033499; U.S.S.N. 10/819899, filed on April 6, 2004; and PCT/US04/10381, filed on April 6, 2004.

XIV. Exemplification

While the invention has been described and exemplified in sufficient detail for those skilled in this art to make and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims. Those skilled in the art will also recognize that all combinations of embodiments or features of the claims described herein are within the scope of the invention.

It will be readily apparent to a person skilled in the art that varying substitutions and modifications may be made to the invention disclosed herein without departing from the scope and spirit of the invention.

It should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Synthesis of subject compounds

Subject compounds can be synthesized by a variety routes. For example, WO 03/033499 describes a number

of schemes for the synthesis of subject compounds.

A further general process to synthesize compounds of the invention is shown in Scheme 1 below, using the synthesis of compound A37 as a specific example.

Scheme 1

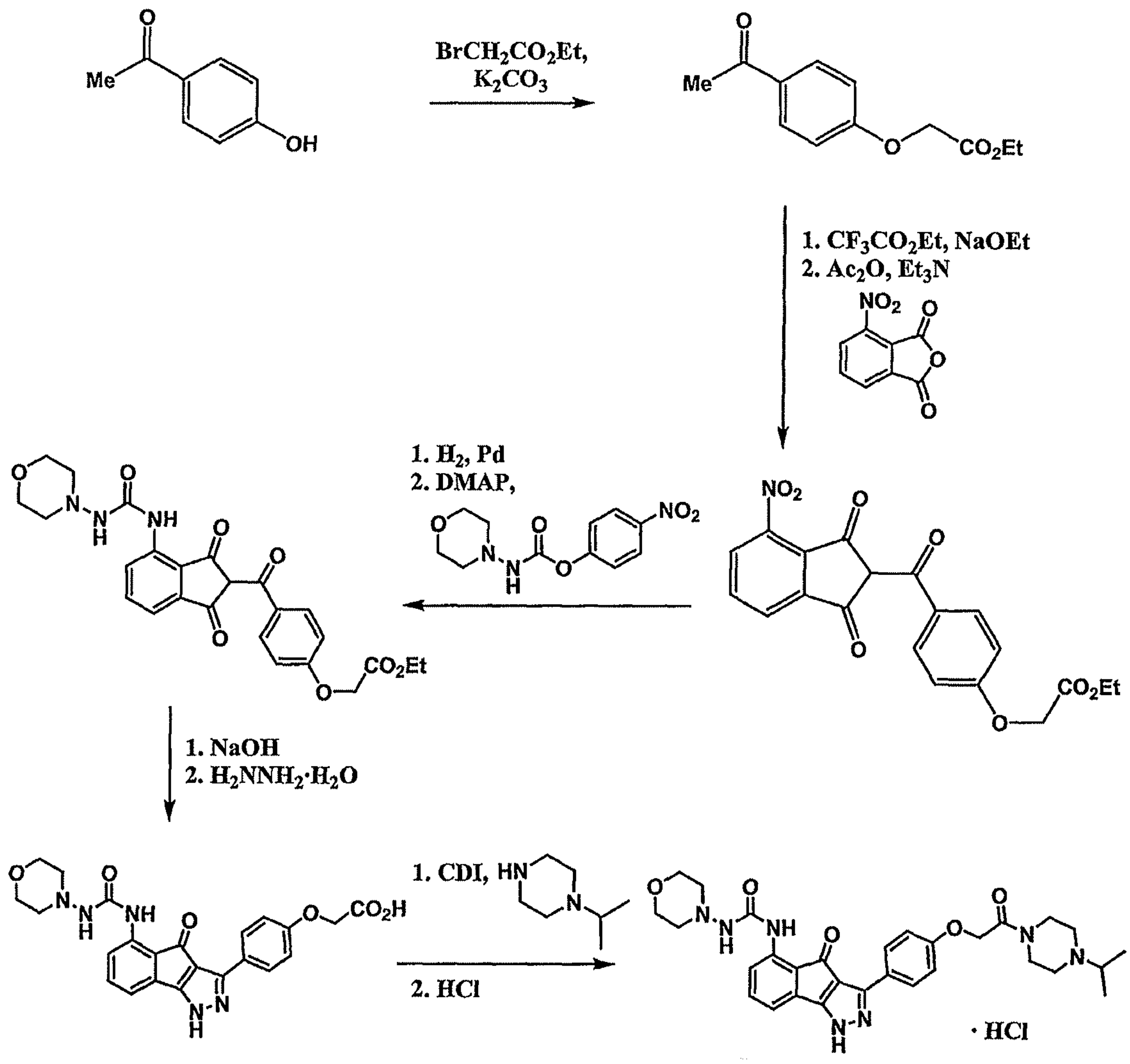


A 37

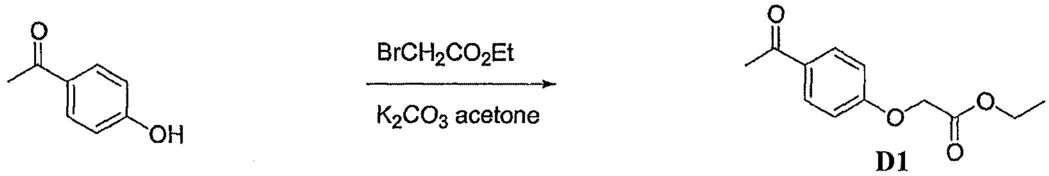


D1

**Step 1.** To a solution of 4- hydroxyacetophenone (13.6 g, 100 mmol) in acetone (200 mL) was added $K_2CO_3$ (16.6 g, 120 mmol) followed by ethyl bromoacetate (11.1 mL, 100 mmol). The reaction mixture was stirred at room temp. for 18 hours. The mixture was concentrated to half the volume under reduced pressure and the suspension was partitioned

between BtOAc and 1N NaOH. The organic layer was washed with brine, dried, and concentrated under reduced pressure to give **D1** (22.2 g, 100% yield).

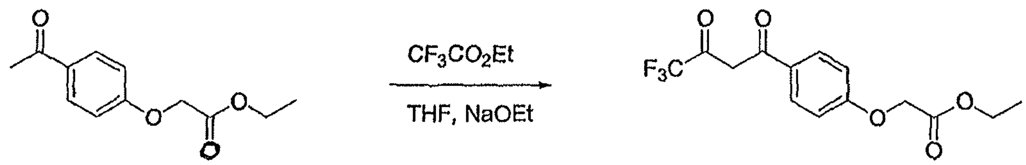


D1 D2

**Step 2.** A 21 wt.% solution of NaOEt in EtOH (204 mL, 542 mmol) was added drop-wise via addition funnel over 40 min to a solution of **D1** (110 g, 493 mmol) and ethyl trifluoroacetate (84.1 g, 592 mmol) in THF (1000 mL) at 0˚C. The ice bath was removed and the reaction was allowed to warm to room temp. overnight. 2N HCl (300 mL) and brine (300 mL) were added and the layers separated. The aqueous layer was extracted with EtOAc (2 X 250 mL). The combined organic layers were washed with brine (2 X 200 mL), dried ($MgSO_4$), and concentrated under reduced pressure to give **D2** as a tan solid (155 g, 99% yield).

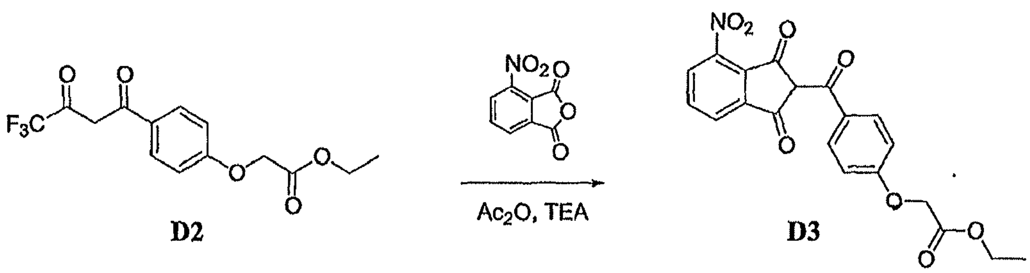


D2 D3

**Step 3.** Triethylamine (280 mL, 2.01 mol) was added to a suspension of **D2** (160 g, 503 mmol) and 3-nitrophthalic anhydride (97.1 g, 503 memo1) in acetic anhydride (332 mL) at 0˚C. The reaction mixture was allowed to warm to room temp. overnight and slowly turned deep red, becoming homogeneous after 30 minutes. The reaction mixture was cooled to 0˚C and 1.5N HCl (4000 mL) was added. The mixture was mechanically stirred for 1 hour at room temp. until a brown granular precipitation formed. The brown solid was collected by filtration, suspended in $H_2O$ (2000 mL) and stirred for 20 min. The brown solid was collected by filtration, rinsed with $H_2O$ (500 mL), and dried under vacuum to give 224 g of crude product. The crude reaction product was suspended in EtOH (800 mL) and heated to boiling. The solution slowly turned deep red and the solid became bright yellow. The suspension was allowed to cool to room temp. then placed in a freezer overnight. The product was collected by filtration, rinsed with cold EtOH (300 mL), and dried under vacuum to give **D3** as a bright yellow solid (126 g, 63% yield).

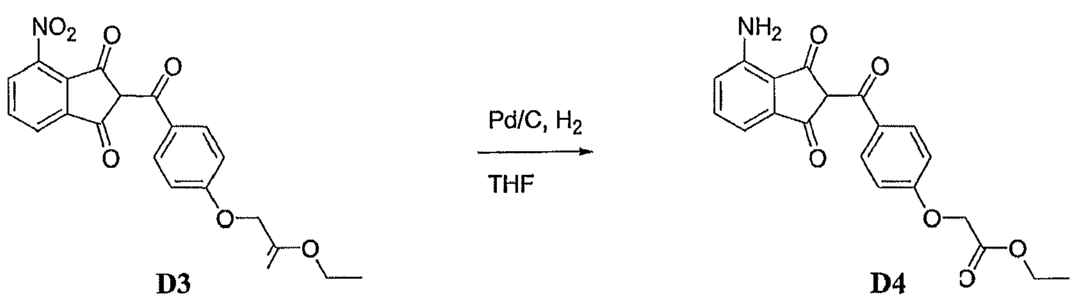


D3 D4

**Step 4.** To a solution of **D3** (108 g, 273 mmol) in THF (3000 mL) under argon was added 10 % Pd/C (17.0 g, 16.0 mmol). The argon was exchanged for $H_2$ under balloon pressure and the reaction mixture was stirred overnight. The catalyst was removed by filtration through a plug of celite and the solvent was evaporated under reduced pressure

to give **D4** as a yellow solid (90.3 g, 90% yield).

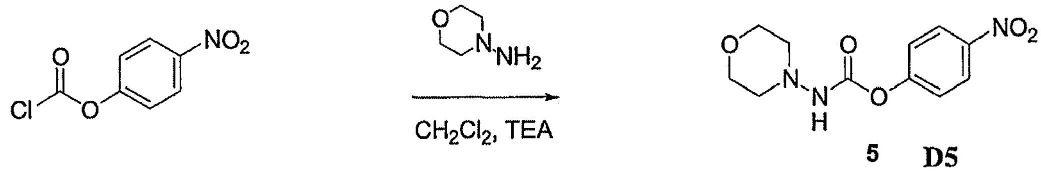


5 D5

**Step 5.** A solution of aminomorpholine (116 g, 1.14 mol) and triethylamine (174 mL, 1.25 mol) in $CH_2Cl_2$ (210 mL) was added via addition funnel over 2 hours to a solution of 4-nitrophenyl chloroformate (275 g, 1.36 mol) in $CH_2Cl_2$ (3000 mL) at 0˚C under mechanical stirring. A white precipitation formed during the addition. The reaction was stirred for 1 hour after the addition was complete. The product was collected by filtration, re-suspended in $CH_2Cl_2$ (1000 mL), stirred for 20 min, and collected by filtration (198 g, 65% yield). The combined $CH_2Cl_2$ filtrates were washed with 1N HCl (2 X 500 mL) and brine (2 X 350 mL), then dried ($MgSO_4$) and concentrated. The off-white solid was suspended in $Et_2O$ (1000 mL), stirred for 20 min and collected by filtration. The $Et_2O$ rinse was repeated to give a second crop of product (81.2 g, 27% yield). The combined batches contain ~ 2 wt % TEA HCl and a small amount of p-nitrophenol.

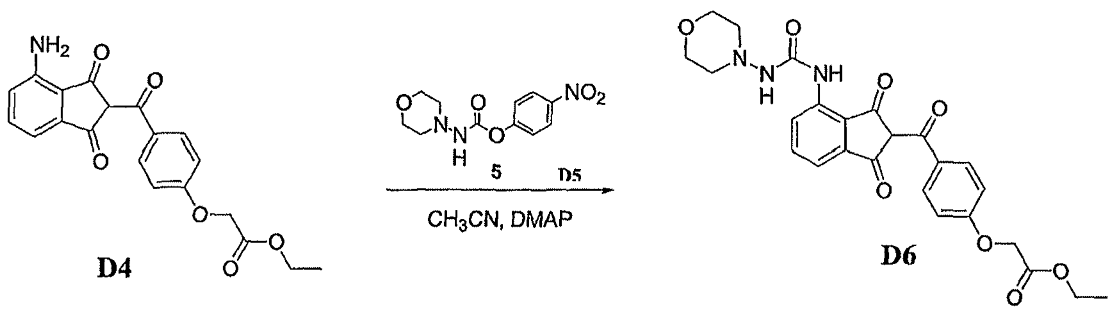


D4 D6

**Step 6.** Dimethylaminopyridine (1.50 g, 12.3 mmol) was added to a suspension of **D4** (90.3 g, 246 mmol) and **D5** (79.0 g, 295 mmol) in $CH_3CN$ (850 mL) at room temp. The reaction mixture was heated at reflux for 4 hours. The reaction mixture became homogeneous upon heating forming a yellow precipitation after 1.5 hours. After cooling to 0˚C, the bright yellow solid was collected by filtration, rinsed with cold $CH_3CN$ (150 mL), followed by $Et_2O$ (2 x 200 mL), and dried under vacuum to give **D6** (84.5 g, 69 % yield) as a yellow solid.

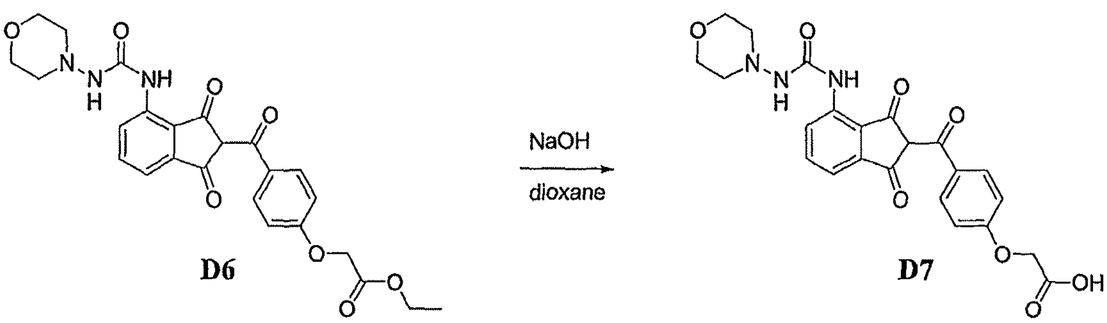


D6 D7

**Step 7.** 1N NaOH (375 mL, 375 mmol) was added to a suspension of **D6** (84.5 g, 171 mmol) in dioxane (1750 mL) at room temp. The reaction mixture became homogeneous forming a yellow precipitation after 15 min. The reaction mixture was stirred for 3 hours. The precipitation was collected by filtration, rinsed with EtOAc (2 x 500 mL), suspended in 1N HCl (500 mL) and stirred for 20 min. After collecting the product by filtration, the HCl wash was repeated. The solid was collected by filtration, rinsed with $H_2O$ (2 X 400 mL) and dried in a vacuum oven overnight at 75˚C to give **D7** (77.4 g, 97% yield) as a yellow solid.

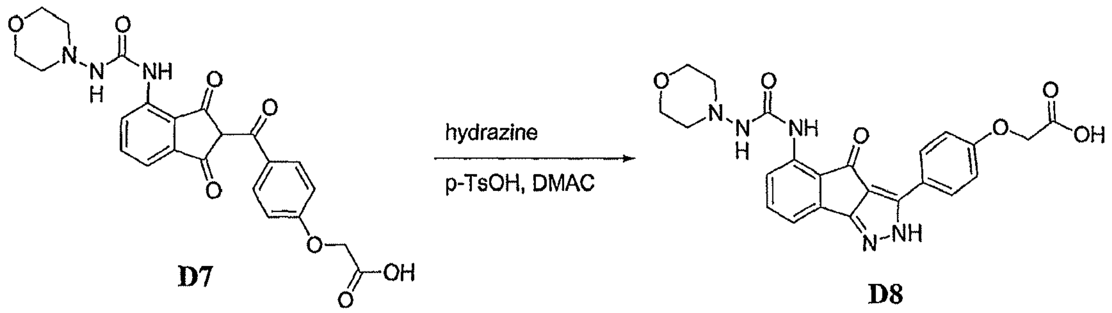


D7

D8

**Step 8.** Hydrazine monohydrate (12.2 mL, 252 mmol) was added to a solution of **D7** (23.5 g, 50.4 mmol) and p-TsOH (479 mg, 2.52 mmol) in DMAC (150 mL). The reaction mixture darkened and was heated at 50 ˚C overnight forming a yellow precipitation after 1.5 hours. The reaction mixture was cooled to room temp. The yellow solid was collected by filtration, rinsed with EtOH (150 mL), then $Et_2O$ (150 mL), and dried under vacuum to give the hydrazine salt of **D8** (21 g, 84 % yield). The hydrazine salt was suspended in 1N HCl (200 mL), stirred for 20 min, and collected by filtration. The yellow solid was rinsed with $H_2O$ (150 mL), EtOH (150 mL), and $Et_2O$ (150 mL) to give **D8** (17.2 g, 74 % yield) as the free acid.

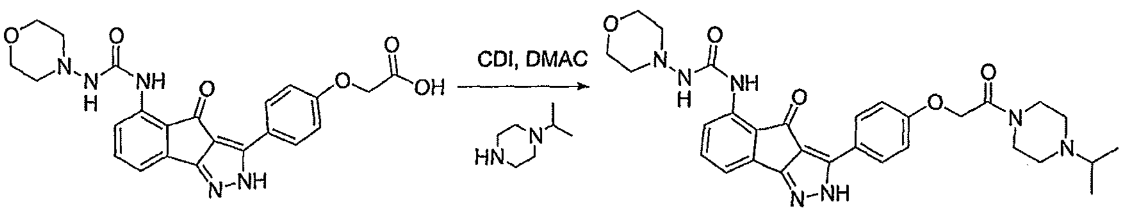


D8

D9

Step 9. N, N'-carbonyldiimidazole (11.0g, 67.5 mmol) was added to a solution of **D8** (14.9 g, 32.2 mmol) in DMAC (100 mL) at room temp. Vigorous gas evolution was evident. The reaction mixture was stirred for 1 hour forming a yellow precipitation after 15 min. Additional DMAC (50 mL) was added to aid stirring. *i*-Propylpiperazine (9.5 g, 74.0 mmol) was added and the reaction mixture became homogeneous. The reaction mixture was stirred overnight, forming a yellow precipitation, then poured into $H_2O$ (1000 mL). The solid was collected by filtration, suspended in EtOH (300 mL) and heated to boiling. After cooling slightly, the solid was collected by filtration, rinsed with EtOH (50 mL), then $Et_2O$ (100 mL), and dried under vacuum to give **D9** (17.4 g, 94 % yield) as a yellow solid.

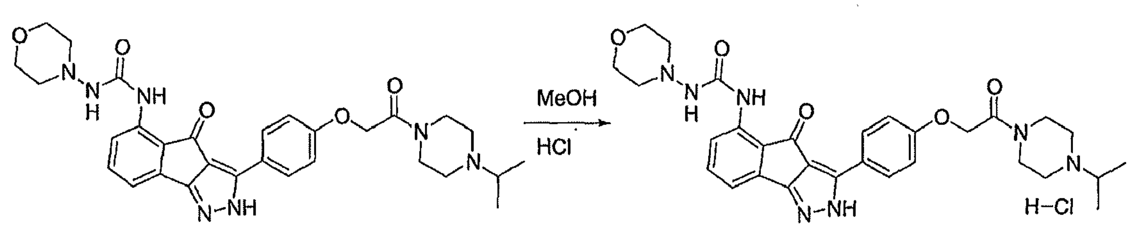


D9

D10

**Step 10.** A suspension of **D9** (11.5 g, 20.0 mmol) in MeOH (400 mL) was heated to near boiling and a solution of 4N HCl in dioxane (5.50 mL, 22.0 mmol) was added. The mixture became homogeneous forming a yellow precipitation within 5 min. After cooling to room temp. overnight, the solid was collected by filtration, rinsed with EtOH (100 mL) then $Et_2O$ (200 ml), and dried in a vacuum oven (75 ˚C, 48 hours) to give compound **A37** (**D10**) (11.4 g, 91 % yield) as a yellow solid.

An alterative general process to synthesize certain compounds of the invention is shown in Scheme 2 below, using the synthesis of compound B16 as a specific example:

Scheme 2

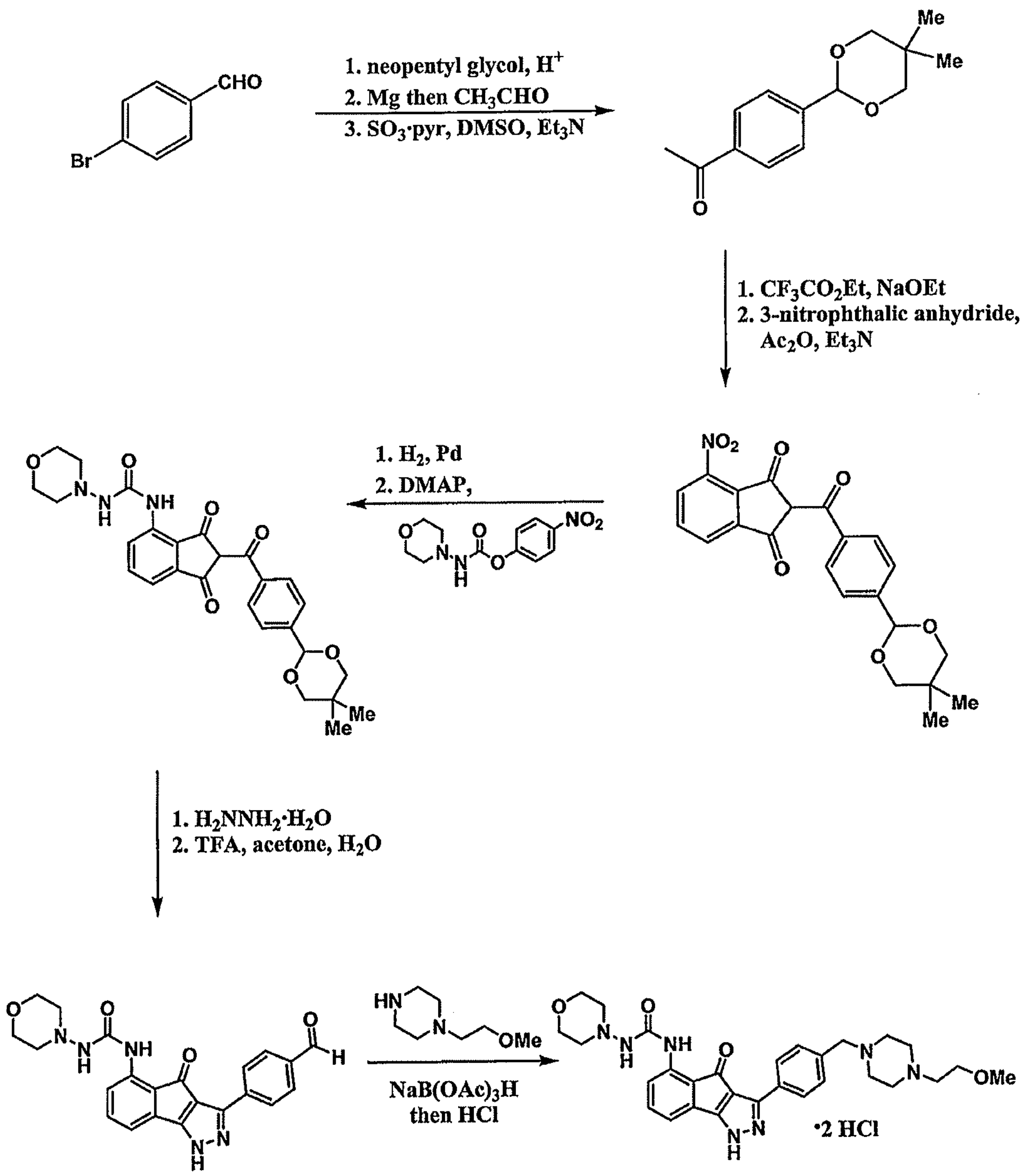


B16

Procedures:

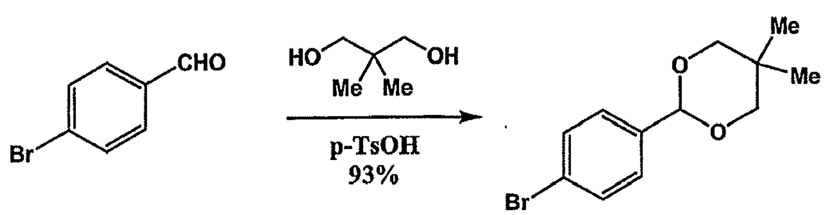

Synthesis of 2-(4-bromophenyl)-5,5-dimethyl-1,3-dioxane:

A mixture of 4-bromobenzaldehyde (100 g, 0.54 mole), neopentyl glycol (115 g, 1.10 mole), and *p*-toluenesulfonic acid (800 mg, 4 mmole) in benzene (800 mL) was heated to reflux using a Dean Stark apparatus for 16h. The reaction mixture was cooled to room temperature and most of the benzene was removed. The residue was partitioned between ethyl acetate (500 mL) and cold water (150 mL). The organic phase was washed with water (2 x 150 mL) and brine (1 x 150 mL), then dried ($Na_2SO_4$) and concentrated to give the desired product (136 g, 93%).

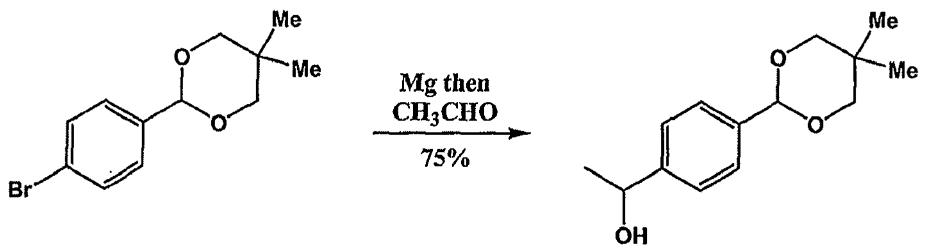


**Synthesis of 1-[4-(5,5-dimethyl-1,3-dioxan-2-yl)phenyl]ethan-1-ol:**

To a suspension of Mg (14.4 g, 0.59 mole) in THF (1400 mL) was added 1,2-dibromoethane (0.4 mL). The suspension was then heated to 30 ˚C. After 10 minutes a solution of the aryl bromide (146.4 g, 0.54 mole) in THF (500 mL) was added drop-wise and the reaction was stirred at 35 ˚C overnight. The resulting dark gray solution was cooled to -5 ˚C in an ice/salt bath, and was treated with acetaldehyde (45.4 mL, 0.81 mole). The reaction was stirred at 0 ˚C for 1h, then poured onto ice. The reaction mixture was then extracted with MTBE (750 mL), and the aqueous layer was extracted with MTBE (2 x 500 mL). The organic layers were combined, washed with sat. $NaHCO_3$ (750 mL), brine (750 mL), dried ($Na_2SO_4$), and concentrated to give the desired product as a red oil (128 g, contains ~25% reduced product by wt.; corrected yield is 96 g, 75%).

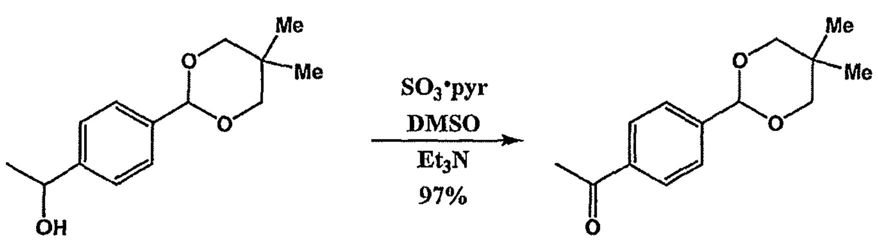


**Synthesis of 1-[4-(5,5-dimethyl-1,3-dioxan-2-yl)phenyl] ethan-1-one:**

To a solution of the alcohol (54 g, 0.228 mole) in dichloromethane (1100 mL) at 0 ˚C was added triethylamine (95 mL, 0.684 mole), followed by a suspension of sulfur trioxide pyridine complex (72.6 g, 0.456 mole) in DMSO (160 mL), keeping the temperature below 5 ˚C. The reaction was allowed to warm up to room temperature, and was stirred at room temperature for 16h. The reaction mixture was diluted with dichloromethane (400 mL) and washed with 1N HCl (500 mL), sat. $NaHCO_3$ (500mL), and brine (500 mL). The organic phase was then dried ($Na_2SO_4$) and concentrated to give 52g (97%) of the desired product.

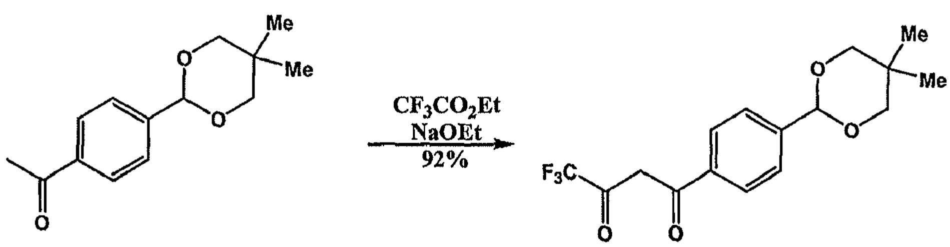

**Synthesis of 1-[4-(5,5-dimethyl(1,3-dioxan-2-yl))phenyl]-4,4,4-trifluorobutane-1,3-dione:**

To a solution of the ketone (147.6 g, 0.63 mole) and ethyl trifluoroacetate (90.2 mL, 0.76 mole) in THF (1250 mL) at -4 ˚C was added a 2 1 % solution of NaOEt in EtOH (308 mL, 0.82 mole) over 45 min. The resulting solution was kept at 0 ˚C for 1h then warmed to room temp and stirred for 2.5h. The reaction was then diluted with MTBE (1500 mL) and treated with 1N HCl (700 mL) and brine (500 mL). The layers were then separated and the organic phase was washed with brine (2 x 500 mL) then dried over $Na_2SO_4$, and concentrated in vacuo, to afford the β-diketone (191.3 g, 92%) as a brown solid.

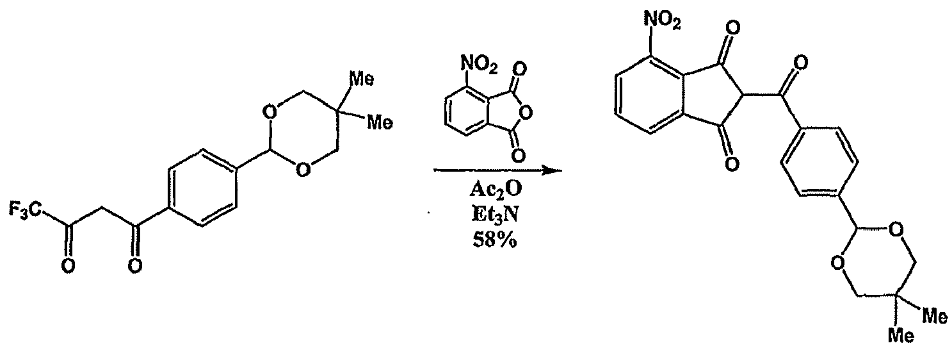


**Synthesis of 2-{[4-(5,5-dimethyl(1,3-dioxan-2-yl))phenyl]carbonyl}-4-nitro-2-hydrocyclopenta[1,2-a]benzene-1,3-dione:**

To a suspension of β-diketone (191.3 g, 0.58 mole) and 3-nitrophthalic anhydride (111.8 g, 0.58 mole) in acetic anhydride (383 mL, 4.1 mole) at 0 ˚C was added triethylamine (323 mL, 2.3 mole) over 15 min. The resulting dark red solution was stirred at 0 ˚C for 1.5h then warmed to room temp and stirred for 16h. The reaction was then cooled to 0 ˚C and treated with 1N HCl (2500 mL). The brown tarry solid was then stirred vigorously for 30 min. The liquid was then decanted and the resulting sticky brown solid was suspended in $H_2O$ (~4 L) and stirred vigorously at room temp for 45 min. The decanting/resuspension sequence was repeated twice more and the resulting brown granular solid was dried under vacuum to yield 249 g of crude product. The crude product was then suspended in MTBE (750 mL) and heated to boiling. The resulting suspension was then placed in a 4 ˚C refrigerator for 16h then filtered. The solid was then filtered, washed with cold MTBE (500 mL), and dried under vacuum to yield the desired nitrotriketone (136 g, 58%).

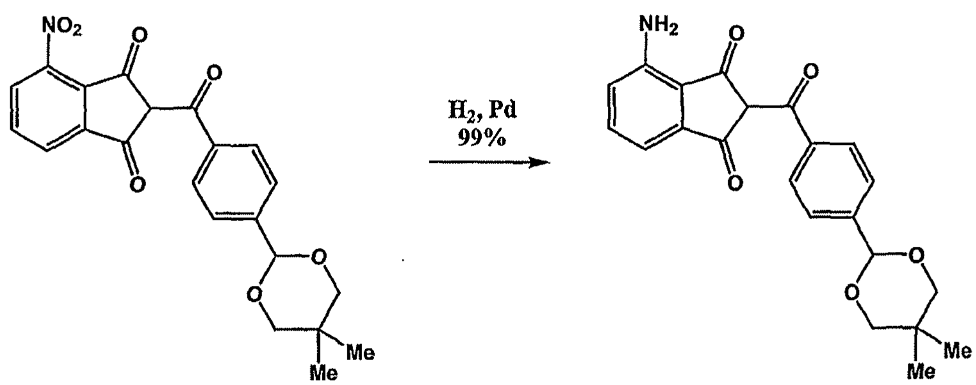


**Synthesis of 4-amino-2-{[4-(5,5-dimethyl(1,3-dioxan-2-yl))phenyl]carbonyl}-2-hydrocyclopenta[1,2-a]benzene-1,3-dione:**

A solution of the nitrotriketone (136 g, 0.33 mole) in THF (2500 mL) was hydrogenated using a hydrogen balloon in the presence of 10% Pd on C (2.5g) for 18h. The catalyst was removed by filtration through a celite pad. The filtrate was then evaporated to yield the desired amine (124 g, 99%) as a yellow foam.

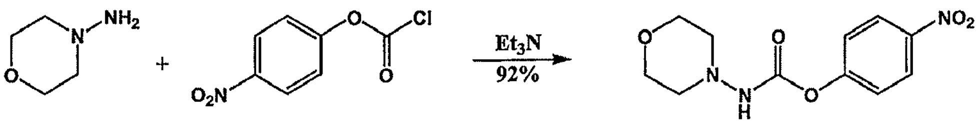


**Synthesis of N-morpholin-4-yl(4-nitrophenoxy)carboxamide:**

A solution of aminomorpholine (116 g, 1.14 mole) and triethylamine (174 mL, 1.25 mole) in $CH_2Cl_2$ (210 mL) was added via addition funnel over 2 hours to a solution of 4-nitrophenyl chloroformate (275 g, 1.36 mole) in $CH_2Cl_2$ (3000 mL) at 0˚C under mechanical stirring. A white precipitation formed during the addition. The reaction was stirred for 1 hour after the addition was complete. The product was collected by filtration, re-suspended in $CH_2Cl_2$ (1000 mL), stirred for 20 min, and collected by filtration (198 g, 65% yield). The combined $CH_2Cl_2$ filtrates were washed with 1N HCl (2 x 500 mL) and brine (2 x 350 mL), then dried ($MgSO_4$) and concentrated. The off-white solid was suspended in $Et_2O$ (1000 mL), stirred for 20 min and collected by filtration. The $Et_2O$ rinse was repeated to give a second crop of product (81.2 g, 27% yield). The combined batches contain ~ 2 wt % triethylamine hydrochloride and a small amount of p-nitrophenol.

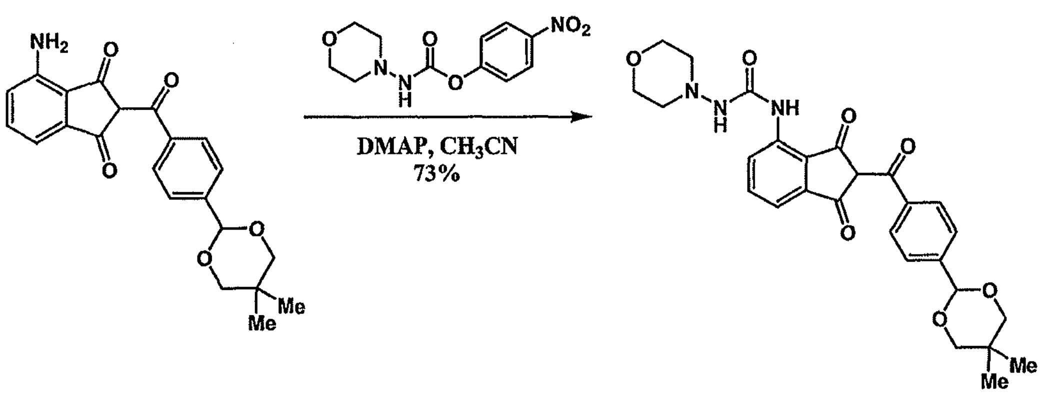


**Synthesis of N-(2-{[4-(5,5-dimethyl(1,3-dioxan-2-yl))phenyl]carbonyl}-1,3-dioxo(2-hydrocyclopenta[2,1-b]benzen-4-yl))(morpholin-4-ylamino)carboxamide:**

The aminotriketone (17g, 0.045 mole), N-morpholin-4-yl(4-nitrophenoxy)carboxamide (15.6g, 0.058 mole), and DMAP (0.27g) were suspended in $CH_3CN$ (200mL) and heated to reflux for 18 h. The reaction mixture was then placed in a 4 ˚C refrigerator for 12h. The grayish yellow solid was isolated by filtration and dried under vacuum to give the desired product (16.7g, 73%).

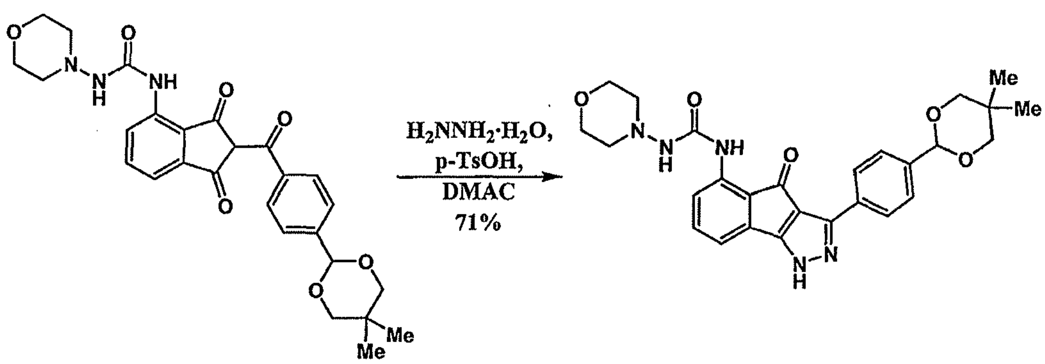

**Synthesis of N-{3-[4-(5,5-dimethyl(1,3-dioxan-2-yl))phenyl]-4-oxoindeno[2,3-d]pyrazol-5-yl}(morpholin-4-ylamino)carboxamide:**

Hydrazine hydrate (56.5 mL, 1.2 mole) was added to a suspension of the triketone (118 g, 0.23 mole) and p-toluenesulfonic acid (2.2 g, 12 mmole) in DMAC (750 mL). The reaction darkens and becomes homogeneous. The reaction was then heated to 50 ˚C for 18h. After about 2h of heating a thick yellow precipitate formed and additional DMAC (100 mL) was added to facilitate stirring. Upon completion of heating, the reaction was placed in a 4 ˚C refrigerator for 16h. The resulting yellow precipitate was then filtered and washed with cold ethanol (500 mL) and $H_2O$ (500 mL). The solid was then dried under vacuum to yield the desired pyrazole (83.4 g, 71 %; contains ~8% by wt. DMAC).

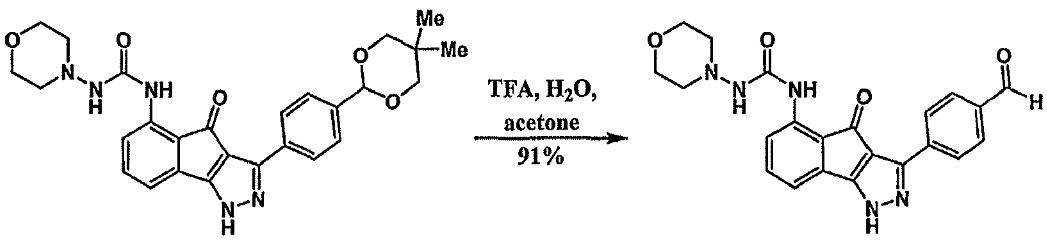


**Synthesis of N-[3-(4-carbonylphenyl)-4-oxoindeno[2,3-d]pyrazol-5-yl](morpholin-4-ylamino)carboxamide:**

To a solution of the indenopyrazole (13.1g, 0.026 mole) in TFA (160mL) was added acetone (75 mL) followed by water (12 mL). Solid product precipitated out of the red clear solution. The reaction mixture was stirred vigorously for 20h, then diluted with acetone/water (100 mL, 1:1) mixture and placed in a 4 ˚C refrigerator for 16h. The solid was collected by filtration and washed with water (100 mL), and acetone (50 mL). The solid was then dried under vacuum to give the desired product (9.8 g, 91%).

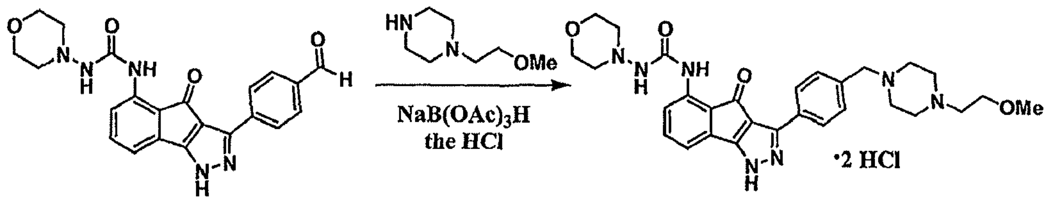


**Synthesis of N-[3-(4-{[4-(2-methoxyethyl)piperazinyl]methyl}phenyl)-4-oxoindeno[2,3-d]pyrazol-5-yl](morpholin-4-ylamino)carboxamide dihydrochloride (compound B16):**

Acetic acid (5.76 g, 96 mmole) was added to a suspension of aldehyde (10 g, 24 mmole) and piperazine (6.91 g, 48 mole) in NMP (150 mL). The reaction was stirred at room temp for 16h then treated with $NaB(OAc)_3H$ (12.7 g, 60 mmole). The reaction was stirred at room temp for 20h during which time the reaction becomes very viscous. 1N NaOH (200 mL) was then added and the reaction was stirred for 1h. The reaction was then poured onto $H_2O$ (750 mL) and filtered. The solid was washed with $H_2O$ (2 x 350 mL), EtOH (100 mL), and $Et_2O$ (200 mL). The solid was then dried under vacuum to yield the desired amine as the free base (9.98 g, 76%). The free base was then suspended in EtOH (200 mL) and heated to boiling. The suspension was then treated with 4N HCl in dioxane (15 mL). The suspension clears then after ~ 15 min, a thick precipitate forms. Additional EtOH (200 mL) was added to facilitate stirring. Once the suspension cooled to room temp, it was filtered and the solid was washed with EtOH (200 mL) and $Et_2O$ (200 mL). The solid was then dried under vacuum to yield the desired bis-hydrochloride salt (10.3 g) designated compound B16.

**Assay Protocols and Results**

The biological activity and utility of the compounds of the invention are demonstrated by one or more assays including those described in more detail below:

Assay 1. Reduced viability of a broad range of 60 cell-lines derived from various human tumors as represented by the NCI panel, on exposure to compounds of the invention (results shown in Table 2).

Assay 2. Irreversible effect of compounds of the invention on cells in a clonogenic survival assay (results shown in Table 2, Figure 1 and Figure 2).

Assay 3. Reduced viability of HCT-116 and IMR90 cells exposed to compounds of the invention as estimated using a Calcein AM assay (results shown in Tables 3 and 6).

Assay 4. Inhibitory activity of compounds of the invention in certain kinase biochemical assays (results shown in Tables 5 and 6).

Assay 5. Activity of compounds in xenograft tumor models (results shown in Figures 3, 4, 5 and 6).

***Assay 1: Evaluation of CDK inhibitors in the NCI panel of human tumor cell lines***

The evaluation of compounds at the National Cancer Institute in their panel of 60 cell lines provides a wealth of information regarding efficacy in a wide range of tumor types and genetic backgrounds. Included within this panel are cell lines derived from leukemia, melanoma and cancers of the lung, colon, brain, ovary, breast, prostate and kidney. Use of this panel provides a measure of the efficacy of compounds in cells with alterations in many genes that are associated with neoplastic transformation including p53 and Her2/Neu as well as those involved in metabolism and those which confer multi-drug resistance. The data generated in these cell lines with the protocol described below can be used to evaluate the activity of compounds.

Results of the NCI panel assays are presented in Table 2 (NCI panel) represented by two informative metrics: (a) the Mean-Graph Mid-point - the average $IC_{50}$ over the whole cell panel except that an $IC_{50}$ ($\mu$M) of less than 10 nM is calculated as being equal to 10 nM for this estimate; and (b) the $IC_{50}$ ($\mu$M) of the inhibitory activity of the compound against an adriamycin resistant cell line (ADR-res).

Additional compounds of the invention showed the following activity in the NCI assay: (i) compound **A37:** Mean-Graph Mid-point < 50 nM (about 21-26 nM) and $IC_{50}$ of inhibition of growth of ADR-res cells < 100 $\mu$M; Ave TGI = 2.06 $\mu$M; Ave $LC_{50}$ = 67 $\mu$M; (ii) compound **B16:** Mean-Graph Mid-point < 50 nM (about 20-33 nM) and $IC_{50}$ of inhibition of growth of ADR-res cells < 10 $\mu$M.

*Methodology of the in vitro cancer screen*

Cells were grown in RPMI-1640 10% FCS and plated in 96 well micro-titer plates at densities ranging from 5,000 to 40,000 cells/well. The plates were incubated for 24 hours at 37˚C, 5% $CO_2$ for 24 hours. Media containing twice the desired final concentration of the compound (5 doses spanning 4 logs) was prepared and 100 $\mu$l was added to each well containing 100 $\mu$l media plus cells to yield the desired final concentration. The plates were then incubated for an additional 48 hours.

The effect of the compound on cellular viability was determined with the Sulforhodamine B (SRB) assay, which measures total protein. Cells were fixed with cold TCA to a final concentration of 10% and incubated at 4 ˚C for 60 minutes. The supernatant was discarded and the plates were washed five times with water and air-dried. SRB solution at 4% (w/v) in 1% acetic acid was added to each well and the plates were incubated for 10 minutes at room temperature. The plates were washed five times with 1% acetic acid and air-dried. The bound stain was solubilized with 10 mM trizma base and the absorbance was read on a plate reader at 515 nM.

***Assay 2: Protocol for Clonogenic survival assay with HCT-116 cells***

This assay was used to determine the concentration of a compound that results in irreversible loss of viability after a specified period of exposure. Essentially, cells are exposed to compound for a period of 1, 2 or 5 days, and are then transferred to compound-free growth medium. After continued incubation in the compound-free medium for a number of days, the number of colonies recovered is counted as an estimate of the number of surviving cells.

Results of such survival assays for various compounds of the invention are presented in Table 2 (clonogenic) as the concentration ($\mu$M) of compound that inhibits colony recovery by 50% ($IC_{50}$). Figure 1 displays irreversible inhibition of cellular activity in HCT-116 cells, and the time-course of such inhibition by compound A37, with an $IC_{50}$ of < 50 nM with 24 hour compound exposure. Compound **B16** shows an $IC_{50}$ of < 100 nM in the same assay, and $IC_{50}$ reached within 30 to 60 min at 100 nM (Figure 2).

*Method to measure cell survival after exposure to compound*

Media (RPMI-1640, 10% FCS, pen/strep) was pre-warmed to 37˚C in a water bath. Cells were incubated and grown at 37 ˚C, 5% $CO_2$. Cells were recovered by trypsinization from sub-confluent plates and counted using a hemocytometer. 1 X $10^4$ cells were plated in 25 mls of media in a 15 cm tissue culture dish. 14 plates were set up for each test compound, and were incubated overnight at 37 ˚C. The compound was diluted into media at 7 concentrations and

the media on the cells was replaced with that containing the test compound. Two plates were set up for each concentration of the compound to be tested, as well as two control plates without compound. Plates were incubated as above for 24, 48 or 74 hours, media was removed and replaced with fresh media, and the plates were incubated an additional 7 days and washed with PBS. Colonies were stained with crystal violet solution (0.4% crystal violet, 20 % ethanol) for 5 minutes, washed twice with distilled water, and counted.

***Assay 3: Use of the Calcein AM viability assay for the evaluation of CDK inhibitors in the presence and absence of serum proteins***

The potency of the subject kinase inhibitors, as measured by loss of cellular viability, was determined with the Calcein AM assay (Molecular Probes). Calcein AM is a substrate of intracellular esterases, which is cleaved only in viable cells to generate a fluorescent product that can be quantified with a fluorescent plate reader. The fluorescent signal is proportional to the number of viable cells, and thus loss of signal in response to the exposure of cells to the subject kinase inhibitors correlates with a loss of viability. This assay not only distinguishes cell cycle arrest, in which cells may still by viable, from loss of viability, and is thus well suited for the evaluation of the subject kinase inhibitors. A compound that is a potent cytotoxic agent may cause significant loss of cell viability in such an assay.

Cellular $IC_{50}$'s were determined in the human colorectal carcinoma cell line, HCT-116, and the normal human fibroblast, IMR90. Protein adjusted $IC_{50}$'s were also determined in HCT-116. Results of such viability assays are presented in Table 2 (HCT-116 (viability/protein adjusted) and IMR-90). $IC_{50}$'s (μM, non-protein adjusted) for the viability assay against HCT-116 cells are shown for further compounds of the invention in Table 4.

Analogous cell viability assays against other cell lines were conducted as above. The $IC_{50}$ (μM) for other compounds of the invention were found to be: (i) compound **A37:** HCT-116 (<50 nM), HCT-116 protein-adjusted (< 500 nM), A2780 (<10 nM), IMR90 (<50 nM); (ii) compound **B16:** HCT-116 (<10 nM), HCT-116 protein-adjusted (< 500 nM), A2780 (<10 nM), IMR90 (<100 nM).Protocol for the Calcein AM viability assay.

HCT-116 or IMR90 cells were recovered from sub-confluent plates by trypsinization and 1,000 or 4,000 cells, respectively, were plated in 24-well dishes and incubated overnight at 37˚C, 5% $CO_2$. HCT-116 cells were cultured in RPMI-1640, 10% FCS, and IMR90 cells were cultured in Minimum Essential Medium-alpha, 10% FCS. After overnight incubation to allow adherence, the media was aspirated from each well and replaced with media containing a test compound at a concentration from 0 to 250 nM, spanning a total of 7 doses. The plates were returned to the incubator and cultured for an additional 72 hours (3 days). The media used for the determination of protein-adjusted $IC_{50}$'s was RPMI-1640, 10% FCS, plus 1 mg/ml alpha acidic glycoprotein (Sigma G-9885), and 45 mg/ml human serum albumin (Sigma A3782). After 72-hours incubation with the test compound, the cells were washed twice with 1X PBS, taking special care to remove all residual buffer.

A 5 μM Calcein AM solution was prepared by dissolving a 50 μg aliquot of Calcein (Molecular Probes catalog # C3100) in 50 μl DMSO. After the Calcein had completely dissolved (10 minutes at room temperature), it was diluted into 10 ml PBS. Calcein/PBS (0.5 ml) was added to each well. The plates were incubated for 75 minutes at 37 ˚C (protected from light) and the fluorescent signal was read on a fluorescent plate reader (excitation 485/20 and emission 530/25).

***Assay 4: Inhibition of Biochemical Kinase Assay***

Certain embodiments of the invention call for the assay of kinase activity (*e.g.* before / after treatment by the subject kinase inhibitors). Listed below are a few exemplary biochemical kinase activity assays, some of which might be generally adapted to other kinases. In general, kinase activity can be conducted according to those in the art.

*Enzymes:* Cdc2 / cyclin B was obtained from commercial sources. Cdk2 / his-cyclin $E_{short}$ was expressed in Sf9 cells. Cdk2 / cyclin A, cdk4 / cyclin D1, and cdk6 / cyclin D2 were expressed in Sf9 cells. Protein kinase A (catalytic subunit, from bovine heart) and protein kinase C (mixed isozymes from rat brain) were obtained from commercial sources.

*Substrates:* Histone H1 was from commercial sources. GST-Rb is glutathione-S-transferase fused to the N-terminal of residues 379-928 of the Rb protein.

*Assays:* Cdc2/cyclinB activity was determined by measuring incorporation of radioactivity from adenosine [γ-$^{32}$P]triphosphate into Histone H1 using a TCA precipitation assay. Cdc2/cyclin B kinase and Histone H1 were obtained from commercial sources. The final assay solution contained 50 mM Tris.HCl, 10 mM $MgCl_2$, 1 mM dithiothreitol, 50 μM adenosine triphosphate, 2 μCi $^{32}$P, 10% dimethylsulfoxide (from compounds), pH 7.5, 20 μg Histone H1, 6 U enzyme in a 50 μL volume. Compounds were added at various concentrations between 1 nM and 10 μM. The reaction was started with the addition of enzyme, allowed to proceed for 20 min at 30 ˚C, and stopped by the addition of 20 μL of stop solution (237 mM disodium ethylenediamine tetraacetate, 105 mM adenosine triphosphate, pH 8.0). The protein was precipitated by the addition of 35 μL 70% (w/v) trichloroacetic acid, and the precipitate was captured on a 96-well glass fiber filter plate (Millipore, Inc.), which had been wet with 25% (w/v) trichloroacetic acid. The filter was washed ten

times with 25% (w/v) trichloroacetic acid, and the amount of incorporated $^{32}P$ was determined by scintillation counting after adding 100 μL scintillant (Microscint 20, Packard Instruments). Relative activity was determined by dividing the amount of radioactivity incorporated in the presence of compound by the amount of radioactivity incorporated in a control experiment containing DMSO alone but no compound. The background radioactivity, determined in an experiment containing 50 mM EDTA in place of compound, was subtracted from all results before calculations. The concentration of compound for 50% inhibition ($IC_{50}$) was determined by fitting the data to the standard equation:

$$P = min + (max - min)\,(1/(1 + (IC_{50} / [I])^{s}))\quad (1)$$

where P (= 1 - relative activity) is relative inhibition, *[I]* is concentration of compound, *max* and *min* are the maximum and minimum relative inhibition (1 and 0, respectively) and *s* is the so-called Hill slope.

Cdk2/cyclin E, Cdk2/cyclin A, Cdk4/cyclin D1, and Cdk6/cyclin D2 activity was determined using a glutathione-sepharose capture assay. The enzymes were expressed in Sf9 insect cells as heterodimers, and the substrate (GST-Rb) was glutathione-S-transferase fused to residues 379 to 928 of Rb retinoblastoma protein, expressed in E. *coli.* The assay solution contained 50 mM Tris.HCl, 10 mM $MgCl_2$, 1 mM dithiothreitol, 50 μM adenosine triphosphate, 2 μCi [γ-$^{33}P$]adenosine triphosphate, 10% dimethylsulfoxide (from compounds), pH 7.5, 40 μg GST-Rb, and enzyme in a 100 μL volume. Compounds were added at various concentrations between 1 nM and 10 μM. The reaction was allowed to proceed for 15 min at 30 °C and was stopped by the addition of 70 μL of stop solution (237 mM disodium ethylenediamine tetraacetate, 105 mM adenosine triphosphate, pH 8.0). The GST-Rb was captured by binding to glutathione-sepharose bead (Amersham) for 110 min, and the suspension was filtered through a glass fiber filter. After washing the retained beads five time with phosphate-buffered saline containing 0.3 % (w/v) Tween-20, the amount of $^{33}P$ incorporated was determined by scintillation counting after adding 100 μL scintillant. Relative activity was determined by dividing the amount of radioactivity incorporated in the presence of compound by the amount of radioactivity incorporated in a control experiment containing DMSO alone but no compound. The background radioactivity, determined in an experiment containing 50 mM disodium ethylenediamine tetraacetate in place of compound, was subtracted from all results before calculations. The concentration of compound for 50% inhibition ($IC_{50}$) was determined by fitting the data to equation (1).

Protein kinase C and protein kinase A were assayed using a TCA precipitation assay with Histone H1 as a substrate. For protein kinase A, the final assay contained 50 mM Tris, 10 mM $MgCl_2$, 1 mM dithiothreitol, pH 7.5, 12 μM adenosine triphosphate, 10% (v/v) dimethylsulfoxide (from compounds), 20 μg Histone H1, 2 μCi [γ-$^{32}P$] adenosine triphosphate, 0.2 U protein kinase A in a 100 μL assay. A protein kinase C assay contained 50 mM Tris, 10 mM $MgCl_2$, 1 mM dithiothreitol, 0.8 mM $CaCl_2$, pH 7.5, 5 μM adenosine triphosphate, 10% (v/v) dimethylsulfoxide (from compounds), 20 μg Histone H1, 2 μCi [γ-$^{32}P$] adenosine triphosphate, 0.01 U protein kinase C in a 50 μL assay. The assays were started by the addition of enzyme, allowed to react for 10 min at 30 °C, and stopped by adding 0.4 volumes of 237 mM disodium ethylenediamine tetraacetate, 105 mM adenosine triphosphate, pH 8.0. The protein was precipitated from the stopped reaction by adding 0.5 volume 75 % (w/v) trichloroacetic acid and captured by filtering through a 96-well glass fiber filtration apparatus (Millipore). The filters were washed ten times with 25% (w/v) trichloroacetic acid, and the amount of incorporated [$^{32}P$]phosphate was determined by adding 100 μl Microscint and scintillation counting. The concentration of compound for 50% inhibition ($IC_{50}$) was determined by fitting the data to equation (1).

Results from the above assays are presented in Table 3 and Table 4. Further inhibition results for subject compounds of the invention against other CDKs and further kinases are shown in Table 5, Table II and described in Example 6.

***Assay 5: Xenograft Tumor Models***

Xenograph tumor models may be used to assess the effectiveness of the subject compounds in treating certain tumors, such as tumors resistant to certain chemotherapeutic agents, but are not resistant to the subject compounds in *in vitro* tests. The following assay may be adapted for this and other similar purposes.

*Drugs.* Compounds of the invention were synthesized and prepared for i.v. administration in a biocompatible vehicle. CPT-11 (Camptosar®, Pharmacia) was obtained as the pharmaceutical drug and was prepared in 5% dextrose-water (D5W). All preparations were made fresh weekly and injection volumes were adjusted to body weight (0.2 ml/20 g mouse).

*Mice/Husbandry.* Female nu/nu mice were obtained from Charles River, housed in static microisolators, and provided *ad libitum* with water and an irradiated standard rodent diet (Purina Pico-Lab Rodent Diet 20).

*Determination of maximum tolerated dose (MTD).* Mice at 8 weeks of age were pair-matched into groups of 5-8 animals and preliminary toxicity studies were performed with unknown test compounds. Animals were treated i.v. daily for 10 consecutive days with test compound and were weighed twice weekly. Mice were examined frequently for

clinical signs of any adverse drug-related effects. Acceptable toxicity for anti-cancer drugs in mice is defined by the NCI as no mean group weight loss of over 20% and not more than 10% toxic death in treated animals.

A similar scheme can also be employed in experimental rats and other animals. For example, to determine MTD in rats, 50-80 mg/kg of **A37** was dosed i/v in a QDx5/2/5 schedule. It was found that **A37** did not induce cardiotoxicity as seen with other analogs.

For **B16,** in nu/nu mice, single dose MTD is about 120 mg/kg. **B16** MTD as measured by QDX5 is about 100 mg/kg. In CD-1 mice, **B16** MTD as measured by 5/2/5 dose is 40 mg/kg.

*Standard Protocol.* Athymic nude mice (male or female, 6-7 weeks) were implanted s.c. with single 1 $mm^3$ tumor fragments (tumor brie) or alternatively, 5-10 x $10^6$ tissue culture-derived cells into the flank. Animals were initially monitored twice weekly for tumor growth and then daily as the implants approached the desired size of approximately 100 $mm^3$. When the tumors grew to between 62-221 mg in calculated tumor weight, the animals were pair-matched into appropriate experimental treatment groups (8-10 animals/group) and treatment with test compounds was initiated. A positive control was dosed according to historical controls. Tumor weights were calculated and body weights were taken twice weekly and animals were observed frequently for adverse drug effects. The protocol called for any animal whose tumor mass reached 1000 mg to be immediately euthanized.

Tumors were measured by determining the length and width of the tumor with a digital caliper. Tumor weight was estimated using the following formula:

$$\textbf{Tumor Weight (mg)} = (w^2 \, x \, l) / 2$$

where w = width and l = length in mm of the tumor. These values can also be expressed in volumetric units ($mm^3$).

Experimental treatment may cause partial regression (PR) or complete regression (CR) of tumors. PR is where the tumor size is 50% or less of the starting (day 1) size but greater than 0.0 mg for three consecutive days during the course of the study, whereas a CR occurs when there is no measurable tumor mass for three consecutive days. Cures are defined as animals whose tumor shrinks to 0 mg and remains that way until the completion of the experiment.

Log cell kill (LCK) is a calculation that determines the percentage of tumor cells that are killed after the initiation of treatment and can be used as a quantitative measure of efficacy:

$$\textbf{Log Cell Kill (LCK)} = \textbf{(T-C) / (3.32)(Td)}$$

where T = is the mean time required for the treatment group of mice to reach 1000 mg in size, C = the mean time for the control group tumors to reach 1000 mg in size, Td = is the tumor doubling time estimated from the linear regression analysis from a semi-log growth plot of the control group tumors during exponential growth and 3.32 = the number of doublings required for a population to increase 1-log10 unit. Each LCK unit represents 1-log10 unit of cell killing (*e.g.* 1 LCK = 90% kill, 2 LCK = 99% kill, etc.). We consider compounds to be significantly active when they have LCK values > 1, which corresponds to 90% tumor cell kill.

Tumor growth inhibition (TGI) is a calculation that describes the amount of tumor growth that is inhibited by treatment with a compound over a defined period of time. It is expressed as:

$$\textbf{\%TGI} = 100(1\text{-}T/C)$$

where T is the mean tumor size of a compound treated group on a given day, and C is the mean tumor size of the vehicle control group on the same day.

Toxic deaths are defined as deaths caused by compound treatment and not by advanced disease state. A death is considered toxic if the animal dies within 1 week after the final compound treatment and the tumor size has not reached 1000 mg. Non-tumor related deaths after this point are recorded, but not considered toxic deaths.

Tumor regression is defined according the following conventions: a regression is defined as partial (PR) if the tumor weight decreases to < 50% of the starting weight (< 50 mg). A regression is defined as complete (CR) if the tumor weight decreases below measurable weight during the experimental period. A cure is defined as a tumor-free animal at end of the observation period.

*Results.* Figure 4 shows results achieved for several compounds of the invention in a HCT116 xenograft tumor model. Figure 4 shows the results of an A2780 xenograft tumor model achieved from compound **A37.** Figure 5 shows

the results of a PC3 xenograft tumor model achieved from compound **A37.** Figure 6 shows the results of a A2780 xenograft tumor model achieved from compound **B16.**

All references, patents, and publications cited herein are hereby incorporated by reference in their entirety.

Table 1.Range of compound concentrations used in Assay 1.

| **Concentration of Compound** | 0 | 5 nM | 10 nM | 25 nM | 50 nM | 100 nM | 250 nM |
|---|---|---|---|---|---|---|---|

Table 2.

Results for certain compounds of the invention for the following *in-vitro* cellular activity assays described above: viability and clonogenic survival assays with HCT-116 cells, viability assays with IMR90 cells, and two measures of activity against the NCI cell panel ("Mean-Graph MID-point and $IC_{50}$ against an adriamycin resistant cell line)

| **Compound** | **HCT-116 $UC_{50}$ ($\mu$M)** | | | | | **IMR90 ($\mu$M)** | **NCI panel** | |
|---|---|---|---|---|---|---|---|---|
| | **Viability** | **Protein adjusted** | **Clonogenic** | | | | **MG-MID ($\mu$M)** | **ADR-res ($\mu$M)** |
| | | | **24 h** | **48 h** | **72 h** | | | |
| A | <0.1 | <1 | <1 | <0.1 | <0.1 | <0.1 | <0.1 | <1 |
| B | <1 | | | | | <1 | | |
| C | <0.1 | <1 | <1 | | | <0.1 | <0.1 | <0.1 |
| D | <0.01 | | <0.1 | | | <0.01 | | |
| E | <0.1 | <1 | <0.1 | | <0.1 | <0.1 | <0.1 | <1 |
| F | <0.1 | <0.1 | <1 | | <0.1 | <0.1 | <0.1 | <1 |
| G | <0.1 | <1 | <0.1 | <0.1 | | <0.1 | <0.1 | <1 |
| H | <0.1 | <1 | <1 | <0.1 | | <0.1 | <0.1 | 10 |
| I | <0.1 | <1 | <0.1 | <0.1 | | <0.1 | <0.1 | <1 |
| J | <1 | | | | | <0.1 | | |
| K | <0.1 | <1 | <1 | | | 0.1 | <0.1 | <10 |
| L | <0.1 | <1 | | <0.1 | | 0.1 | 10 | 10 |
| M | <0.1 | <1 | <0.1 | | | <0.1 | | |
| N | <1 | <1 | | | | 0.1 | | |
| O | <0.1 | <1 | <0.01 | | | <0.1 | | |
| P | <0.01 | | <0.1 | | | <0.1 | | |
| Q | <0.1 | <1 | <0.1 | | <0.1 | <0.1 | <0.1 | <10 |

Table 3. Results for certain compounds of the invention ($IC_{50}$ as $\mu$M) for biochemical inhibition assays described above.

| **Compound** | **Cdk2/ Cyclin E** | **Cdk2/ Cyclin A** | **Cdk4/ Cyclin D** | **Cdc2/ Cyclin B** | **Cdk6/ Cyclin D2** | **PKA** | **PKC** | **c-Abl** |
|---|---|---|---|---|---|---|---|---|
| A | <0.1 | <0.1 | <1 | <1 | <1 | | | |
| B | <0.01 | <0.1 | <10 | <0.1 | | | | |
| C | <0.1 | <0.1 | <1 | <1 | | >10 | >10 | >10 |
| D | <0.1 | <0.1 | <1 | <1 | | | | |
| E | <0.01 | <0.01 | <0.01 | <0.1 | <0.01 | <10 | <10 | <10 |
| F | <0.1 | <0.1 | <0.01 | <0.1 | <0.01 | | | |

(continued)

| Compound | Cdk2/ Cyclin E | Cdk2/ Cyclin A | Cdk4/ Cyclin D | Cdc2/ Cyclin B | Cdk6/ Cyclin D2 | PKA | PKC | c-Abl |
|---|---|---|---|---|---|---|---|---|
| G | <0.1 | <0.1 | <0.01 | <0.1 | | >10 | | >10 |
| H | <0.1 | <0.1 | <0.1 | <0.1 | | | | |
| I | <0.1 | <0.1 | <0.01 | <0.1 | | | | |
| J | <0.1 | | | | | | | |
| K | <0.1 | | | | | | | |
| L | <0.1 | | <0.1 | <0.1 | | | | |
| M | <0.1 | | | | | | | |
| N | <0.1 | | | <0.01 | | | | |
| O | <0.1 | | | | | | | |
| P | <0.1 | | | | | | | |
| Q | <0.01 | <0.1 | <0.01 | <0.1 | <0.1 | | | |

Table 4. Results for additional compounds in the biochemical inhibition and HCT-116 viability assays (non-protein adjusted) described above.

| | $IC_{50}$ (μM) | | | |
|---|---|---|---|---|
| Compound | Cdk2/ cyclin E | Cdk4/ cyclin D1 | Cdc2/ cyclin B | MCT-116 viability |
| A1 | <0.01 | <1 | <0.1 | <0.1 |
| A2 | <0.01 | <10 | <1 | <0.1 |
| A3 | <0.1 | <0.1 | <0.1 | <0.1 |
| A4 | <0.01 | <0.1 | <0.1 | <0.1 |
| A5 | <0.1 | <10 | <1 | <0.1 |
| A6 | <0.01 | <0.1 | <0.01 | <0.1 |
| A7 | <0.1 | <0.1 | <0.1 | <0.1 |
| A8 | <0.1 | <0.1 | <0.1 | <0.1 |
| A9 | <0.1 | <0.1 | <0.01 | <0.1 |
| A10 | <0.1 | <1 | <0.01 | <0.1 |
| A11 | <0.1 | <0.1 | <0.1 | <0.1 |
| A12 | <0.1 | <0.1 | <0.1 | <0.1 |
| A13 | <0.1 | <0.1 | <0.1 | <0.01 |
| A14 | <0.1 | <0.1 | <0.1 | <0.1 |
| A15 | <0.1 | <0.1 | <0.01 | <0.1 |
| A16 | <0.1 | <1 | <0.1 | <0.1 |
| A17 | <0.1 | <0.1 | <0.01 | <0.1 |
| A18 | <0.1 | <0.1 | <0.1 | <0.1 |
| A19 | <0.1 | <1 | <0.1 | <0.1 |
| A20 | <0.01 | <0.1 | <0.01 | <0.1 |
| A21 | <0.01 | <1 | <0.1 | <0.1 |

(continued)

| Compound | IC50 (μM) Cdk2/ cyclin E | Cdk4/ cyclin D1 | Cdc2/ cyclin B | MCT-116 viability |
|---|---|---|---|---|
| A22 | <0.01 | <0.1 | <0.1 | <0.1 |
| A23 | <0.01 | <0.1 | <0.1 | <0.1 |
| A24 | <0.01 | <0.1 | <0.01 | <0.01 |
| A25 | <0.1 | <0.1 | <0.1 | <0.01 |
| A26 | <0.1 | <0.1 | <0.1 | <0.01 |
| A27 | <0.1 | <0.1 | <0.01 | <0.1 |
| A28 | <0.1 | <1 | <0.1 | <0.1 |
| A29 | <0.01 | <0.1 | <0.1 | <0.1 |
| A30 | <0.1 | <1 | <1 | <0.1 |
| A31 | <0.1 | <0.1 | <0.1 | <0.1 |
| A32 | <0.1 | <0.1 | <1 | <0.1 |
| A33 | <0.1 | <0.1 | <0.1 | <0.1 |
| A34 | <0.01 | <0.1 | <0.1 | <0.1 |
| A35 | <0.1 | <0.1 | <0.01 | <0.1 |
| A36 | <0.1 | <0.1 | <0.1 | <0.01 |
| A37 | <0.1 | <0.1 | <0.1 | <0.1 |
| A38 | <0.1 | <1 | <1 | <0.1 |
| A39 | <0.1 | <1 | <1 | <0.1 |
| A40 | <0.1 | <0.1 | <0.1 | <0.1 |
| A41 | <0.1 | <0.1 | <1 | <0.1 |
| A42 | <0.1 | <1 | <1 | <0.1 |
| A43 | <0.1 | <1 | <1 | <0.1 |
| A44 | <0.1 | <0.1 | <0.01 | <0.1 |
| A45 | <0.1 | <0.1 | <0.01 | <0.01 |
| A46 | <0.1 | <1 | <0.01 | <0.1 |
| A47 | <0.1 | | <0.1 | <0.01 |
| A48 | <0.1 | | <1 | <0.1 |
| A49 | <0.1 | | <0.1 | <0.1 |
| A50 | <0.1 | | <1 | <0.1 |
| A51 | <0.1 | | | <0.1 |
| A52 | <0.1 | <1 | <1 | <0.1 |
| A53 | <1 | <10 | <1 | <0.01 |
| A54 | <0.01 | <1 | <0.01 | <0.1 |
| A55 | <0.1 | <10 | <0.1 | <0.1 |
| A56 | <0.1 | <1 | <0.1 | <0.1 |
| A57 | <0.01 | <0.1 | <0.01 | <0.1 |
| A58 | <0.01 | <10 | <10 | <0.1 |

(continued)

| Compound | IC$_{50}$ (μM) Cdk2/ cyclin E | Cdk4/ cyclin D1 | Cdc2/ cyclin B | MCT-116 viability |
|---|---|---|---|---|
| A59 | <0.1 | <1 | <0.1 | <0.1 |
| A60 | <0.1 | <10 | <1 | <0.1 |
| A61 | <0.1 | <1 | <0.1 | <0.1 |
| A62 | <0.1 | <10 | <0.1 | <0.1 |
| A63 | <0.1 | <1 | <0.1 | <0.1 |
| A64 | <0.1 | <1 | <0.1 | <0.1 |
| A65 | <0.1 | <0.1 | <0.01 | <0.1 |
| A66 | <0.1 | <10 | <0.1 | <0.1 |
| A67 | <0.01 | | <0.1 | <0.1 |
| A68 | <0.01 | <0.1 | <0.1 | <1 |
| A74 | <0.1 | | <0.1 | 0.25 |
| A76 | <0.1 | <0.1 | <0.1 | <0.1 |
| A77 | | | | <0.1 |
| A78 | | | | <0.01 |
| A79 | | | | <0.1 |
| A80 | | | | <0.1 |
| A81 | | | | <0.1 |
| A82 | <0.1 | | | <0.1 |
| B1 | <0.01 | | | <1 |
| B2 | <0.1 | | <0.01 | <0.1 |
| B3 | <0.1 | | <0.01 | <0.1 |
| B4 | <0.1 | | | <0.1 |
| B5 | <0.1 | | | <0.1 |
| B6 | <0.1 | | | <0.1 |
| B7 | <0.1 | | | <1 |
| B8 | | | <0.1 | <0.1 |
| B9 | | | <0.1 | <0.1 |
| B10 | | | <0.1 | <0.1 |
| B11 | | | <0.1 | <1 |
| B12 | | | <0.1 | <0.1 |
| B13 | | | <0.1 | <0.01 |
| B14 | | | | <0.01 |
| B15 | | | | <0.01 |
| B16 | <0.01 | | <0.01 | <0.01 |
| B17 | | | | <0.01 |
| C1 | | | | <0.1 |
| C3 | <0.1 | | | <0.1 |

(continued)

| Compound | IC50 (μM) Cdk2/ cyclin E | Cdk4/ cyclin D1 | Cdc2/ cyclin B | MCT-116 viability |
|---|---|---|---|---|
| C4 | <0.01 | | | <0.1 |
| C5 | | | | <0.25 |

Table 5. Inhibition data ($IC_{50}$/μM) of subject compounds of the invention assayed against cyclin-dependent kinases.

| | Inhibition ($IC_{50}$) | |
|---|---|---|
| **Kinase** | **A37 (μM)** | **B16 (μM)** |
| Cdk1 | <0.05 | <0.005 |
| Cdk2 | <0.05 | <0.005 |
| Cdk3 | <0.005 | <0.005 |
| Cdk4 | <0.05 | >0.1 |
| Cdk5 | <0.005 | <0.005 |
| Cdk6 | <0.1 | <0.05 |
| Cdk7 | <0.5 | <0.05 |

Table A

| Compound | Structure |
|---|---|
| E | 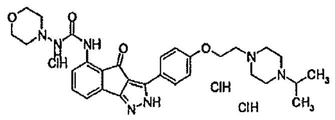 |
| F | 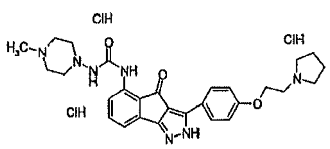 |
| G | 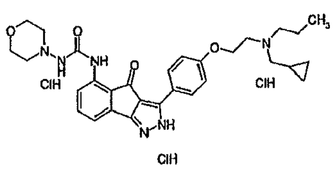 |

(continued)

| Compound | Structure |
|---|---|
| H | 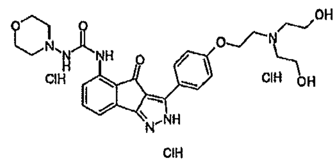 |
| I | 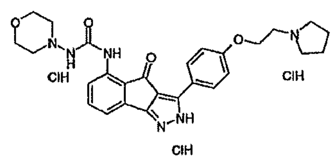 |
| J | 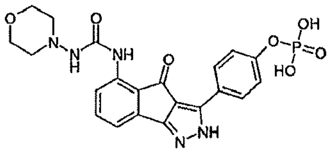 |
| K | 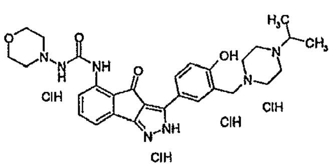 |
| L | 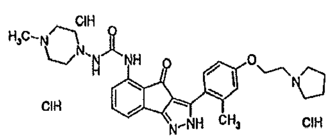 |
| N | 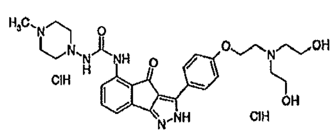 |

(continued)

| Compound | Structure |
|---|---|
| M | 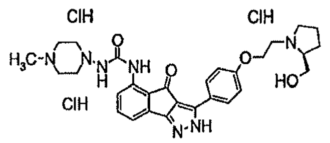 |
| O | 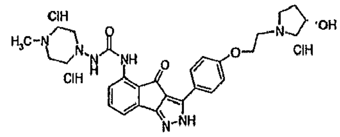 |
| P | 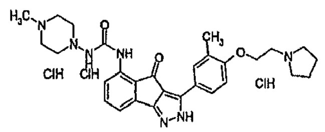 |
| Q | 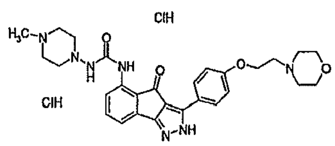 |
| A3 | 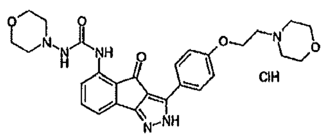 |
| A7 | 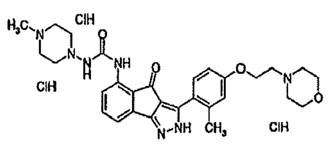 |

(continued)

| Compound | Structure |
|---|---|
| A8 | 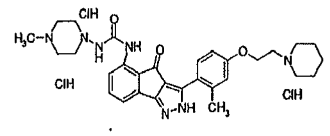 |
| A9 | 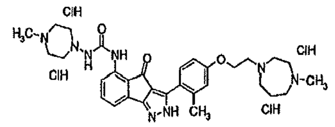 |
| A10 | 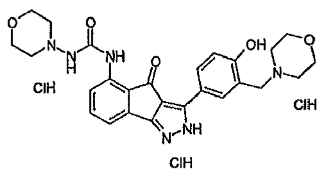 |
| A11 | 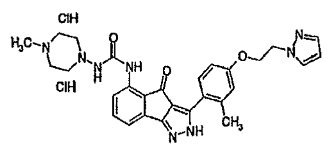 |
| A12 | 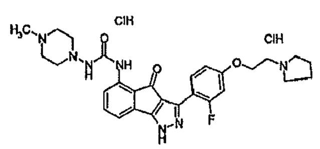 |
| A13 | 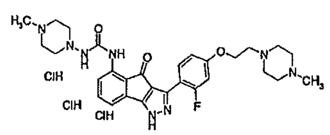 |

(continued)

| Compound | Structure |
|---|---|
| A14 | 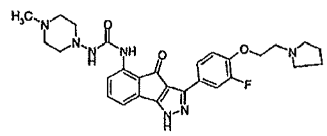 |
| A15 | 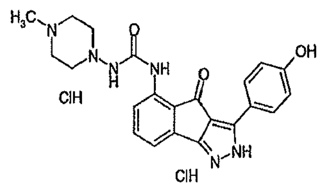 |
| A16 | 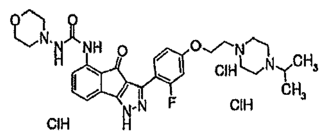 |
| A17 | 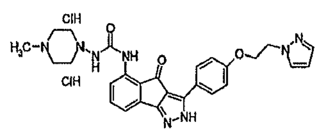 |
| A18 | 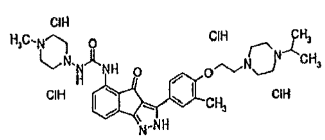 |
| A19 | 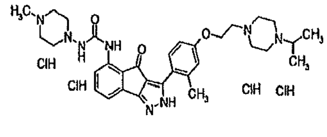 |

(continued)

| Compound | Structure |
|---|---|
| A20 | 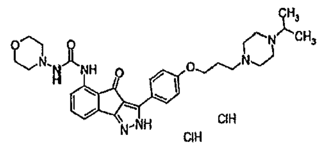 |
| A21 | 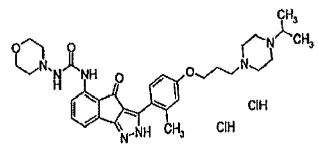 |
| A22 | 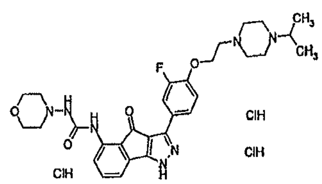 |
| A23 | 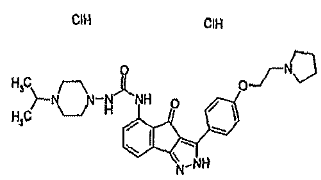 |
| A24 | 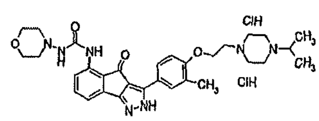 |
| A25 | 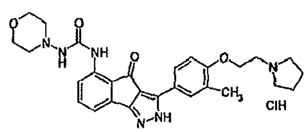 |

(continued)

| Compound | Structure |
|---|---|
| A26 | 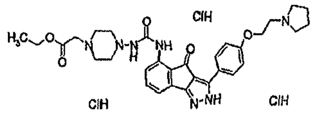 |
| A27 | 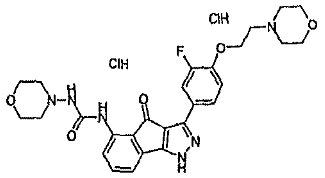 |
| A28 | 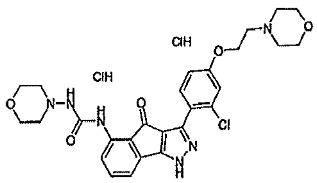 |
| A29 | 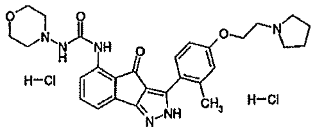 |
| A31 | 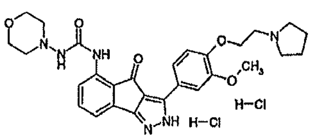 |
| A33 | 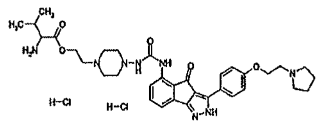 |

(continued)

| Compound | Structure |
|---|---|
| A34 | 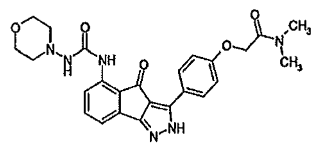 |
| A35 | 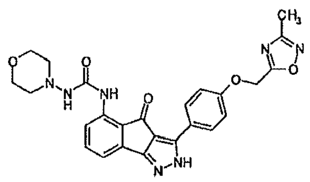 |
| A36 | 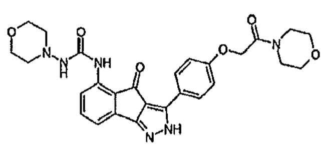 |
| A37 | 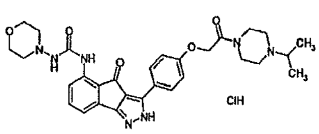 |
| A40 | 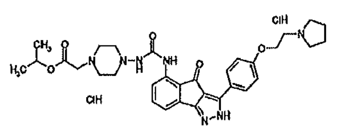 |
| A41 | 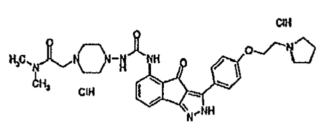 |

(continued)

| Compound | Structure |
|---|---|
| A44 | 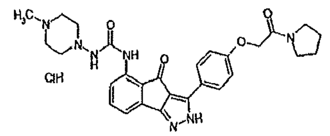 |
| A45 | 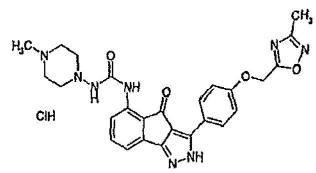 |
| A46 | 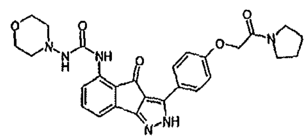 |
| A47 | 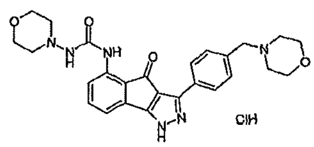 |
| A49 | 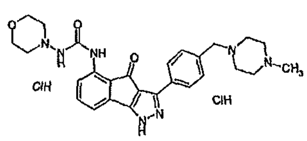 |
| A51 | 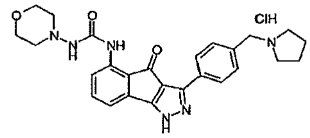 |

(continued)

| Compound | Structure |
|---|---|
| A56 | 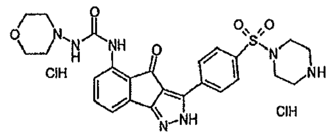 |
| A57 | 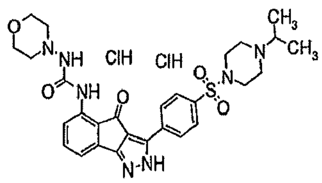 |
| A65 | 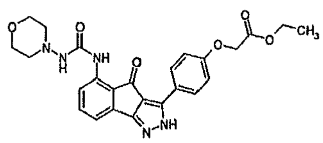 |
| A68 | 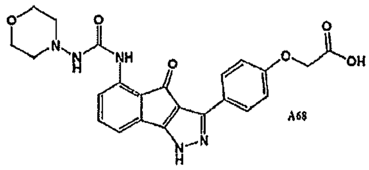 A68 |
| A69 | 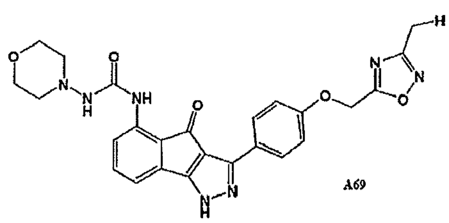 A69 |
| A70 | 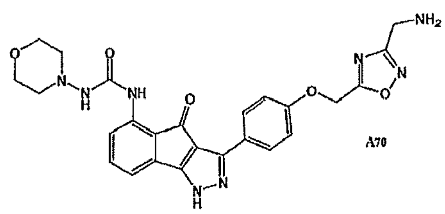 A70 |

(continued)

| Compound | Structure |
| --- | --- |
| A71 | 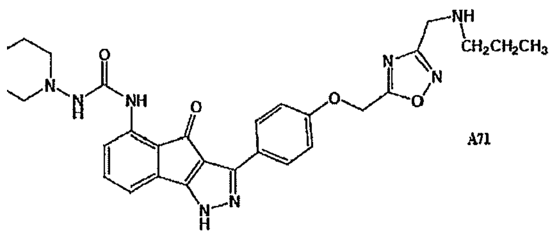 A71 |
| A72 | 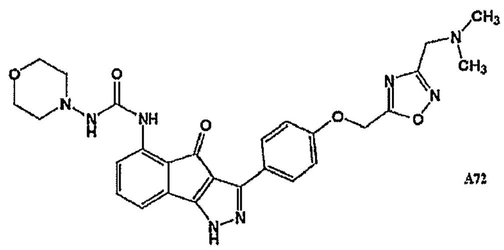 A72 |
| A73 | 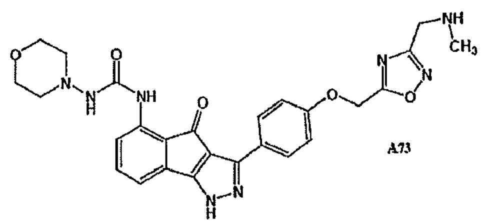 A73 |
| A74 | 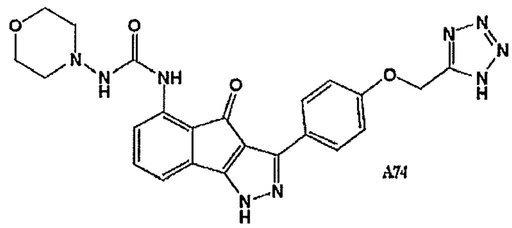 A74 |
| A75 | 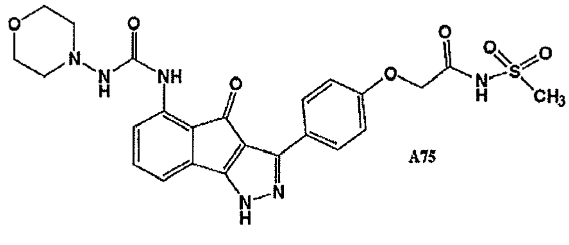 A75 |
| A76 | 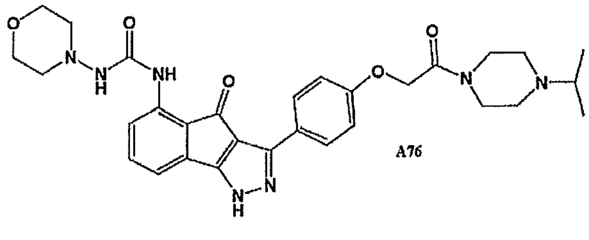 A76 |

(continued)

| Compound | Structure |
|---|---|
| A77 | 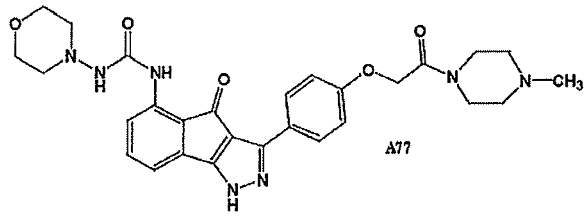 A77 |
| A78 | 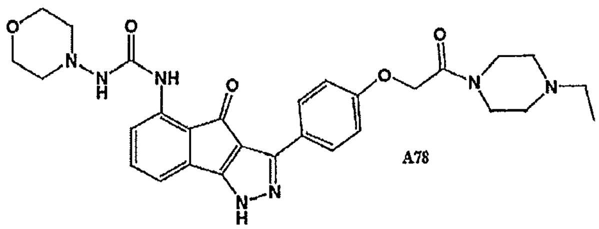 A78 |
| A79 | 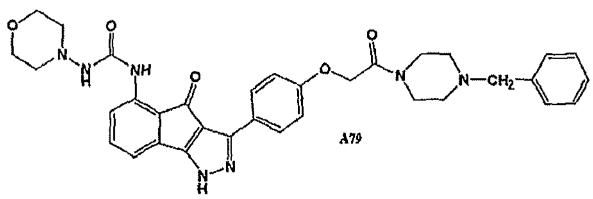 A79 |
| A80 | 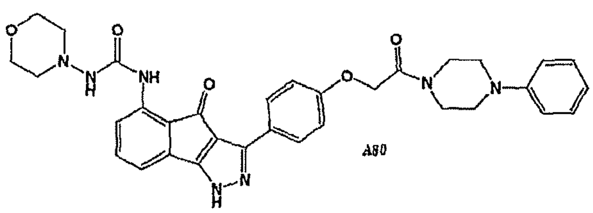 A80 |
| A81 | 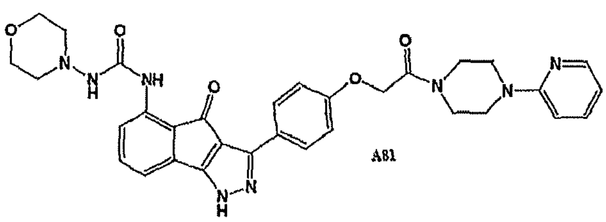 A81 |

(continued)

| Compound | Structure |
|---|---|
| A82 | 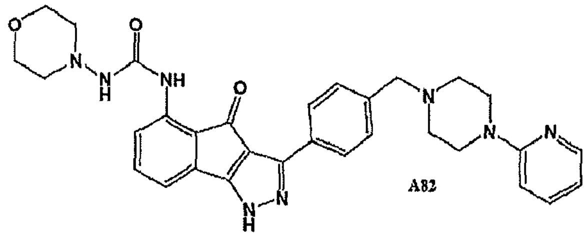 A82 |
| B1 | 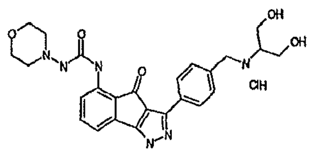 |
| B2 | 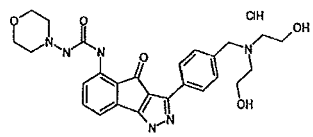 |
| B3 | 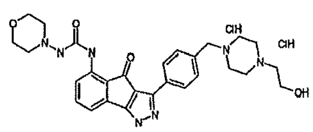 |
| B4 | 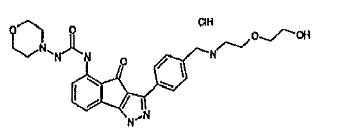 |

(continued)

| Compound | Structure |
|---|---|
| B5 | 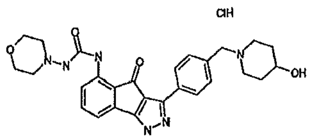 |
| B6 | 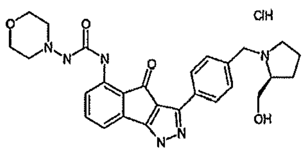 |
| B7 | 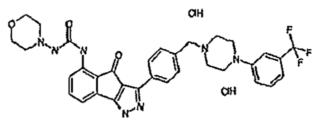 |
| B8 | 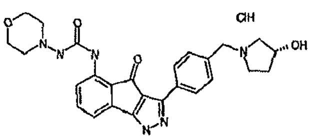 |
| B9 | 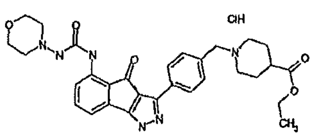 |

(continued)

| Compound | Structure |
|---|---|
| B10 | 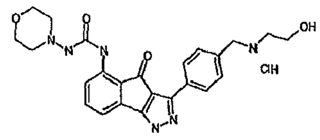 |
| B11 | 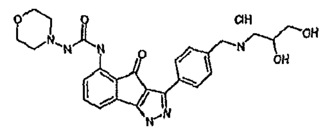 |
| B12 | 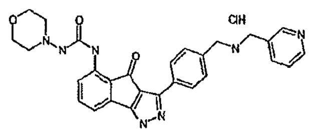 |
| B13 | 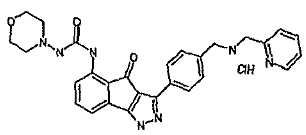 |
| B14 | 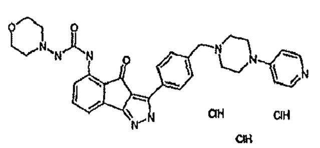 |
| B15 | 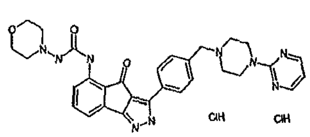 |

(continued)

| Compound | Structure |
| --- | --- |
| B16 | 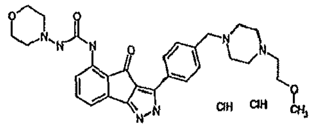 |
| B17 | 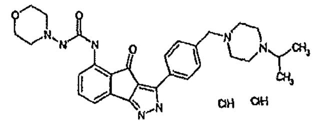 |
| B18 | 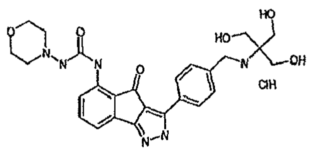 |
| B19 | 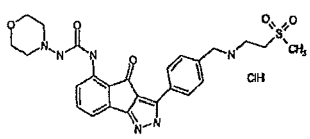 |
| B20 | 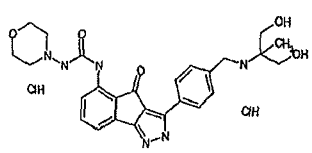 |
| C1 | 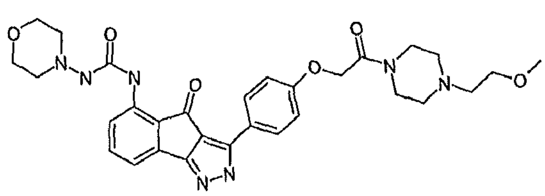 |

(continued)

| Compound | Structure |
|---|---|
| C2 | 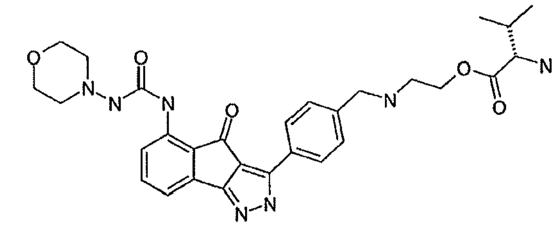 |
| C5 | 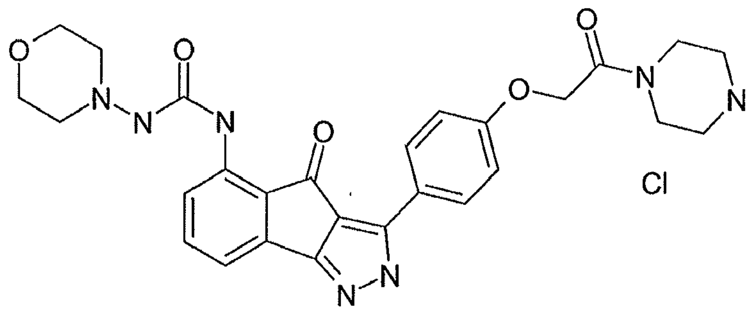 |

Table B

Other compounds suitable for the compositions and methods of the invention result from selecting appropriate features from the table of possible features below. For example, compound A77 results from the following selections: none-morpholino-aryl-$OCH_2(CO)$-piperazine-$CH_3$.

| **Left-hand substituent** | **Left-hand ring** | **Aryl or heteroaryl** | **Ring substituent** | **Nitogen feature** | **Right-hand substituent** |
|---|---|---|---|---|---|
| CH3 | morpholino | aryl | OCH2 | NHM | alkyl |
| isopropyl | piperazine | thiopene | OCH2(CO) | NMM | alkoxy |
| CH3CH2O (CO)CH2 | | | SO2 | morpholino | alcohol |
| none | | | OCH2(CO) OCH2 | piperazine | substituted amine |
| | | | | piperidine | acid |
| | | | | pyrazole | ester |
| | | | | pyrrolodine | $CH_2CH_2OCH_3$ |
| | | | | | $CH_2CH_2OH$ |
| | | | | | $CH_2NH_2$ |
| | | | | | $CH_2NHCH_2CH_2CH_3$ |
| | | | | | $CH_2NHCH_3$ |
| | | | | | $CH_2NHCHCH_3CH_3$ |
| | | | | | $CH_3$ |
| | | | | | $CHCH_3CH_3$ |
| | | | | | $COOCH_2CH_3$ |
| | | | | | none |

Table I. Exemplary Protein Kinases Inhibited by the Subject Kinase Inhibitors, and Diseases Associated Therewith

| **Kinase/class** | ***Associated diseases*** | ***References*** |
|---|---|---|
| Serine-Threonine Kinases | | |
| GSK3b | inflammation and hyperproliferative disorders | WO0147533A2 |
| GSK3b | organ transplant rejection, tumor growth, chemotherapy-induced alopecia, chemotherapy-induced thrombocytopenia, chemotherapy-induced leukopenia, mucocitis, plantar-palmar syndrome, restenosis, atherosclerosis, rheumatoid arthritis, angiogenesis, hepatic cirrhosis, glomerutonephritis, diabetic nephropathy, malignant nephrosclerosis, thrombotic microangiopathy, glomerulopathy, psoriasis, diabetes mellitus, inflammation, neurodegenerative disease, macular degeneration, actinic keratosis and hyperproliferative disorders. | EP1180105 B1 |
| GSK3b | organ transplant rejection, tumor growth, chemotherapy-induced atopecia, chemotherapy-induced thrombocytopenia, chemotherapy-induced leukopenia, mucocitis, plantar-palmar syndrome, restenosis, atherosclerosis, rheumatoid arthritis, angiogenesis, hepatic cirrhosis, glomerulonephritis, diabetic nephropathy, malignant nephrosclerbsis, thrombotic microangiopathy, glomerulopathy, psoriasis, diabetes mellitus, inflammation, neurodegenerative disease, macular degeneration, actinic keratosis and hyperproliferative disorders. | US20040072836 A1 |
| GSK3b | neurodegenerative disease is selected from the group consisting of Alzheimer's disease, Parkinson's disease, tauopathies, vascular dementia; acute stroke, traumatic injuries; cerebrovascular accidents, brain cord trauma, spinal cord trauma; peripheral neuropathies; retinopathies or glaucoma. non-insulin dependent diabetes; obesity; manic depressive illness; schizophrenia; alopecia; or cancers, such as breast cancer, non-small cell lung carcinoma, thyroid cancer, T or B-cell leukemia or virus-induced tumors. | US20030187004A1; WO0170729A1 |

(continued)

| Kinase/class | *Associated diseases* | *References* |
|---|---|---|
| Serine-Threonine Kinases | | |
| GSK3b | bipolar disorder (in particular manic depression), diabetes, Alzheimer's disease, leukopenia, FTDP-1 7 (Fronto-temporal dementia associated with Parkinson's disease), cortico-basal degeneration, progressive supranuclear palsy, multiple system atrophy, Pick's disease, Niemann Pick's disease type C, Dementia Pugilistica, dementia with tangles only, dementia with tangles and calcification, Down syndrome, myotonic dystrophy, Parkinsonism-dementia complex of Guam, aids related dementia, Postencephalic Parkinsonism, prion diseases with tangles, subacute sclerosing panencephalitis, frontal lobe degeneration (FLD), argyrophilic grains disease, subacute sclerotizing pariencephalitis (SSPE) (late complication of viral infections in the central nervous system), inflammatory diseases, cancer, dermatological disorders, neuronal damage, schizophrenia, pain. | WO03037891A1 |
| | dementia, Alzheimer's Disease, Parkinson's Disease, Frontotemporal dementia Parkinson's Type, Parkinson dementia complex of Gaum, HIV dementia, diseases with associated neurofibrillar tangle pathologies, amyotrophic lateral sclerosis, corticobasal degeneration, dementia pugilistica, Down syndrome, Huntington's Disease, postencephelatic parkinsonism, progressive supranuclear palsy, Pick's Disease Niemann-Picks Disease, stroke, head trauma and other chronic neurodegenerative diseases, Bipolar Disease, affective disorders, depression, schizophrenia, cognitive disorders, Type I and Type II diabetes, diabetic neuropathy, hair loss and contraceptive medication. | WO03004478A1; WO02066480A2 |
| GSK3 | Inhibition of GSK3 may be most beneficial to treat neurodegenerative disorders such as Alzheimer and other dementias, and diabetes | Progress in Cell Cycle Research 5, 489-496 |
| CK1 | Parkinson's disease | WO03020702A2; US20030211040A1 |
| CK1 | treating or preventing neurodegenerative disorders (*e.g.* Alzheimer's disease), diabetes, inflammatory pathologies, cancers | WO0141768A2 |

(continued)

| Kinase/class | *Associated diseases* | *References* |
|---|---|---|
| Serine-Threonine Kinases | | |
| CK1 | treatment, alleviation and/or prevention of disorders, including metabolic diseases such as obesity and other body-weight regulation disorders as well as related disorders such as eating disorder, cachexia, diabetes mellitus, hypertension, coronary heart disease, hyper-cholesterolemia, dyslipidemia, osteoarthritis, gallstones, and others, in cells, cell masses, organs and/or subjects. | WO03066086A2 |
| CK1 | The distribution of casein kinase 1 delta (Cki delta) was correlated with other pathological hallmarks in Alzheimer's disease (AD), Down syndrome (DS), progressive supranuclear palsy (PSP), parkinsonism dementia complex of Guam (PDC), Pick's disease (PiD), pallido-ponto-nigral degeneration (PPND), Parkinson's disease (PD), dementia with Lewy bodies (DLB), amyotrophic lateral sclerosis (ALS), and elderly controls. CK1 is highly associated with Alzheimer disease (AD) brain-derived tau filaments and granulovacuolar bodies. | Schwab, Neurobio! Aging. 21(4):503-10, 2000. Yasojima, Brain Res. 865 (1):116-20, 2000. |
| MEK1 | reduce tissue damage resulting from ischemia and/or reperfusion, particularly brain damage associated with ischemia resulting from stroke. | US6319955 |
| MEK1 | reduce tissue damage resulting from ischemia and/or reperfusion, particularly brain damage associated with ischemia resulting from stroke. | US6150401 |
| MEK1 | condition characterized by ischemia: ischemic stroke; or at risk of having an ischemic stroke | WO9934792A1 |
| MEK1 | a condition characterized by glutamate toxicity; a condition characterized bv hypoxia | WO0228388A2 |
| MEK1 | prophylaxis and/or treatment of virally induced hemorrhagic fever and/or hemorrhagic shock syndromes and/or inflammatory conditions;<br>regulating and/or inhibiting virally induced TNF-$\alpha$ production;<br>treatment of virally induced (especially filovirus-induced) TNF-a mediated diseases, such as hemorrhagic fever diseases and hemorrhagic shock syndromes. | WO02069960A2 |

(continued)

| Kinase/class | *Associated diseases* | *References* |
|---|---|---|
| Serine-Threonine Kinases | | |
| MEK1 | Joint therapy with a chondrogenesis promoter comprising a low molecular weight compound having a chondrogenesis promoting effect (*e.g*. the ones disclosed in EP1399211A1) for cartilage disease selected from the group consisting of osteoarthritis, chronic rheumatoid arthritis, osteochondritis dissecans, articular cartilage damage, herniated intervertebral disk, anotia, and microtia cartilage defect. | EP1391211A1 |
| MEK1 | hyperproliferative condition, such as cancer. | WO0031106A1 |
| MKK6 | metabolic diseases and disorders, eg. obesity | WO04035082 A2 |
| MKK6 | neurodegenerative disorders | W00013015A1 |
| MKK6 | cardiac hypertrophy-induced disease | US20020056144 A1 |
| MKK6 | Treating cancer that is: a) a squamous epithelial carcinoma, preferably a squamous epithelial carcinoma of the head, neck, skin or stomach, or b) a colon-, breast- or hepatocellular carcinoma, or c) a fibrosarcoma of the stomach. | US20040067883A1, WO0205792A2 |
| JNK(JNK1, JNK2 & JNK3) | neurological conditions such as Huntington's disease and Alzheimer's disease | US20030148395 A1 |
| JNK(JNK1, JNK2 & JNK3) | neurological conditions such as Huntington's disease and Alzheimer's disease | US20020058245 A1 |
| JNK (JNK1, JNK2 & JNK3) | neurological conditions such as Huntington's disease and Alzheimer's disease | WO9958982 A1 |
| JNK (JNK1, JNK2 & 3) | apoptotic disorders, eg. cancer | US6221850 |
| JNK (JNK1, JNK2 & JNK3) | hyperproliferative diseases and inhibiting tumor growth | W09909214 A1 |

(continued)

| Kinase/class | *Associated diseases* | *References* |
|---|---|---|
| Serine-Threonine Kinases | | |
| JNK | inflammatory disease (*e.g.* acute pancreatitis, chronic pancreatitis, asthma, allergies, or adult respiratory distress syndrome), autoimmune disease (*e.g.* glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, or graft vs. host disease), destructive bone disorder (*e.g.* osteoarthritis, osteoporosis or multiple myeloma-related bone disorder), proliferative disorder (*e.g.* acute myelogenous leukemia, chronic myelogenous leukemia, metastatic melanoma, Kaposi's sarcoma, multiple myeloma), infectious disease, neurodegenerative disease (*e.g.* Alzheimer's disease, Parkinson's disease, amyotrophic lateral sclerosis, Huntington's disease, cerebral ischemia or neurodegenerative disease caused by traumatic injury, glutamate neurotoxicity or hypoxia), allergy, reperfusion / ischemia in stroke (*e.g.* treat or prevent ischemia/reperfusion in stroke or myocardial ischemia, renal ischemia, heart attacks, organ hypoxia or thrombin-induced platelet aggregation), heart attack, angiogenic disorder (*e.g.* solid tumors, ocular neovasculization, or infantile haemangiomas), organ hypoxia, vascular hyperplasia, cardiac hypertrophy, thrombin-induced platelet aggregation, or a condition associated with proinflammatory cytokines;<br>a condition associated with T-cell activation or pathologic immune responses.<br>hypercalcemia, restenosis, hypercalcemia, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, or allergic rhinitis. | US20040097531A1 |

(continued)

| Kinase/class | *Associated diseases* | *References* |
|---|---|---|
| Serine-Threonine Kinases | | |
| | hypercalcemia, restenosis, hypercalcemia, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, allergic rhinitis; autoimmune diseases, allergies, rheumatoid arthritis, or leukemia; melanoma, leukemia, or a cancer selected from colon, breast, gastric, ovarian, cervical, melanoma, renal, prostate, lymphoma, neuroblastoma, pancreatic, leukemia and bladder. | |
| JNK | JNKs as therapeutic targets for indications such as inflammation, vascular disease, neurodegenerative disease, metabolic and oncological diseases; Inflammation: activated immune cells express many genes encoding inflammatory molecules, and many of these ar regulated by the JNK pathway. Monocytes, tissue macrophages nd tissue mast cells are key sources of TNFa. The JNK pathwayregulated TNF production. Inhiiition of JNK actvation effectively modulates TNFa secretion. Baed on their important role in regulating the immune system, JNK inhibition may be beneficial in disease such as rheumatoid arthritis, multiple sclerosis, asthma, infmallatory bowel disease and psoriasis, and chronic transplant rejection; Neurodegeneration: Alzheimer, Parkinson, Huntigton and strole share synaptic loss, neuronal atrophy and death as common pathological hallmarks. JNKs play an integral role in neuronal deat. Hence inhibition of JNKs may have application in these diseases; Metabolic disease: as a mediator of obesity and insulin resitance, as well as many other cellular processes including apoptosis and neuronal differentiation, JNK is a potential target for obesity and type 2 diabetes; | Natur Rev. Drug Discov. 2, 554-565 |

(continued)

| Kinase/class | *Associated diseases* | *References* |
|---|---|---|
| Serine-Threonine Kinases | | |
| JNK | Neurodegenerative pathological conditions including Alzheimer's, Parkinson's, Huntington's Diseases and stroke | Bozyczko-Coyne et al; Curr Drug Targets CNS Neurol Disord. 2002 Feb;1 (1):31-49. |
| JNK | JNK inhibitors in inflammatory, vascular, neurodegenerative, metabolic and oncological diseases | Manning et al; Nat Rev Drug Discov. 2003 Jul;2 (7):554-65. |
| JNK | JNK overexpression is clearly a basis for resistance to DNA-damaging drugs; drug resistance | Vasilevskaya et al; Drug Resist Updat. 2003 Jun;6 (3):147-56 |
| JNK | JNK inhibitors for chronic inflammatory diseases; diabetes, insulin resistance and obesity | Bennett et al; Curr Opin Pharmacol. 2003 Aug;3 (4):420-5. |
| JNK | Treatment of inflammation; regulation of TNF-dependent apoptosis | Varfolomeev et al, Cell. 2004 Feb 20:116(4): 491-7. |
| AMPK | disorders associated with energy metabolism such as diabetes, obesity, and myopathy | WO0120003 A2 |
| AMPK | cardiac disease, including ischemic conditions and hypertrophic cardiomyopathies | Sambandam and Lopaschuk, 2003, Prog. Lipid Res. 42 (3): 238-56; Hopkins, *et al*. 2003, 31 (Pt1):207-212 |
| AMPK | metabolism regulation | WO03064466 A1 |
| Rsk (Rsk1, Rsk2, Rsk3 & Rsk4) | neoplastic disease | WO03105766 A2 |
| Rsk (RSK-1, RSK, RSK-B) | hyperproliferative diseases, such as (1) cancer, specifically lung, head and neck, pancreatic, prostate, renal, bone, testicular, breast, cervical, gastrointestinal, lymphoma, brain, breast, ovarian, leukemia, myeloma, colorectal, esophageal, skin, thyroid, liver, or bladder cancer; (2) rheumatoid arthritis, inflammatory bowel disease, osteo-arthritis, adenoma, leiomyoma, lipoma, hemangioma, fibroma, restenosis, pre-neoplastic lesions, vascular occlusion, or psoriasis; (3) vascular occlusion; and (4) restenosis. | W00071096A2 |

(continued)

| Kinase/class | *Associated diseases* | *References* |
|---|---|---|
| Serine-Threonine Kinases | | |
| Abl | chronic myelogenous leukemia (CML) or acute lymphocytic leukemia (ALL) | US 2003190688 A1 |
| Abl | bcr-abl related diseases | WO 2003037322 A1 |
| Abl | retard, prevent, suppress, and/or reverse the depigmentation of hair; a disease characterized by hair depigmentation, such as vitiligo or piebaldism; | WO03043591A1 |
| c-Src | leukemia | WO03013540 A1<br>WO03013540 A |
| c-Src | hyperactivation of the immune response | US20040101850 A1 |
| c-src | Alzheimer's disease. | WO04038422A2 |
| c-src | hyperproliferative diseases (such as cancer), hematologic diseases, osteoporosis, neurological diseases (*e.g.* Alzheimer's Disease, epilepsy, etc.), autoimmune diseases (*e.g.* lupus erythematosus), allergic/immunological diseases (*e.g.* anaphylaxis), or viral infections (*e.g.* HIV infection);<br>alter cell morphology, migration, adhesion, cell cycle progression, secretion, differentiation, proliferation, anchorage-independent growth, vascular endothelial growth factor expression, microtubule binding by tau, viral infectivity, or bone reabsorption. Disease cell can be from cells of brain, lung, liver, spleen, kidney, lymph node, small intestine, blood cells, pancreas, colon, stomach, breast, endometrium, prostate, testicle, ovary, skin, head and neck, esophagus, bone marrow and blood tissue. | US20040077663A1 |
| c-src | activating mutations in colon cancers, particularly those metastatic to the liver. | Irby, Nat Genet. 21(2): 187-90, 1999. Soriano, Cell. 64(4): 693-702, 1991. |
| c-src related | transplant rejection, rheumatoid arthritis, psoriasis or inflammatory bowel disease | EP1206265B1 |

(continued)

| Kinase/class | *Associated diseases* | *References* |
|---|---|---|
| Serine-Threonine Kinases | | |
| c-src | inflammatory disease (*e.g.* acute pancreatitis, chronic pancreatitis, asthma, allergies, or adult respiratory distress syndrome), autoimmune disease (*e.g.* glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, or graft vs. host disease), destructive bone disorder (*e.g.* osteoarthritis, osteoporosis or multiple myeloma-related bone disorder), proliferative disorder (acute myelogenous leukemia, chronic myelogenous leukemia, metastatic melanoma, Kaposi's sarcoma, or multiple myeloma), infectious disease, neurodegenerative disease (*e.g.* Alzheimer's disease, Parkinson's disease, amyotrophic lateral sclerosis, Huntington's disease, cerebral ischemia or neurodegenerative disease caused by traumatic injury, glutamate neurotoxicity or hypoxia), allergy, reperfusion/ischemia in stroke, heart attack, angiogenic disorder (e.g solid tumors, ocular neovasculization, or infantile haemangiomas), organ hypoxia, vascular hyperplasia, cardiac hypertrophy, thrombin-induced platelet aggregation or a condition associated with proinflammatory cytokines; T-cell activation or pathologic immune responses; ischemia/reperfusion in stroke or myocardial ischemia, renal ischemia, heart attacks, organ hypoxia or thrombin-induced platelet aggregation. hypercalcemia, restenosis, hypercalcemia, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, or allergic rhinitis. | US20030207873A1 |

(continued)

| Kinase/class | *Associated diseases* | *References* |
|---|---|---|
| Serine-Threonine Kinases | | |
| c-src | hypercalcemia, restenosis, osteoporosis, osteoarthritis, symptomatic treatment of bone metastasis, rheumatoid arthritis, inflammatory bowel disease, multiple sclerosis, psoriasis, lupus, graft vs. host disease, T-cell mediated hypersensitivity disease, Hashimoto's thyroiditis, Guillain-Barre syndrome, chronic obtructive pulmonary disorder, contact dermatitis, cancer, Paget's disease, asthma, ischemic or reperfusion injury, allergic disease, atopic dermatitis, or allergic rhinitis. | US20030171389A1 |
| c-src | breast cancer, carcinoma, myeloma, leukemia, and neuroblastoma | W003065995A2 |
| c-src | diseases associated with elevated bone loss | Bone. 1999. 24:437. Curr. Pharm. Des. 2002. 8:2049 |
| Fes | inflammation, benign tumors, malignant tumors, leukemia (chronic myelogenous leukemia), asthma, allergy-associated chronic rhinitis, autoimmune diseases and mastolocytosis. | WO03065995 A2 |
| Fes | angiogenesis, fibros s, cancer | WO9807835 A2 |
| Fes | agammaglobulinemia, AIDS, ALL, angiogenesis, breast cancer, carcinoma, chromic myelogenous leukemia, colon carcinoma, colorectal cancer, diabetes, erythroleukemia, gastric cancer, hematopoiesis, Kaposi's Sarcoma, leukemia, liver regeneration, Lyme disease, megakaryocytopoiesis, melanoma, neuroblastoma, organogenesis, osteopetrosis, ovarian hyperstimulation syndrome, placental development, severe combined immunodeficiency, ulcerative colitis or Wilms tumor. | WO9816638 A1 |
| Fes | tumor of mesenchymal origin and tumor of hematopoietic origin | W003065995A2 |
| Fes | Activated FES may be involved in the breakpoints in translocation (15;17) observed in patients with acute promyelocytic leukemia (APL). | Hagemeijer, Hum. Genet. 61: 223-227,1982. |
| Yes | breast cancer, carcinoma, myeloma, leukemia, and neuroblastoma. | WO03065995 A2 |
| Yes | angiogenesis | US6685938 |
| Yes | Of the primary colon tumors, 64% of the tumors contained elevated activities of both pp60(c-src) and pp62(c-yes). Patients with either synchronous or metachronous liver metastases and elevated pp62 (c-yes) kinase activity have biologically more aggressive disease and a worse prognosis than patients without elevated pp62(c-yes) activity in their liver metastases. | Han, Clin Cancer Res. 2 (8): 1397-404, 1996. |
| Blk | apoptosis | US6190912 |
| Blk | apoptosis | US6600024 |

(continued)

| Kinase/class | *Associated diseases* | *References* |
|---|---|---|
| Serine-Threonine Kinases | | |
| Blk | apoptosis | WO 9950414 A1 |
| Lyn | prostate cancer | US20020019346 A1 |
| Lyn | prostate cancer | W00247710 A2 |
| Lyn | prostate cancer | US20020151497 A1 |
| Fyn | cancer ("tumorous diseases") | WO04010986 A1 |
| Fyn | autoimmune diseases | CA 2084120 AA |
| Fyn & Lck | vascular remodeling, restinosis | Curr. Pharm. Des. 2000.6: 59 |
| Fyn & Lck | asthma, inflammatory bowel diseease, atopic dermatitis | Expert Opin. Biol. Ther. 2004. 4:837. Biochim Biophys Acta 2004.1697: 53-69. Science 2002.296: 1639 JBC 1996. 271:695 |
| Lck | immunological diseases | DE 10237423 A1 |
| Lck | breast cancer | WO 2004037996 A2 |
| Lck | reperfusion injury | WO 2004032709 A2 |
| Lck | autoimmune disease, allergies, rheumatoid arthritis, or leukemia | US20030171389A1 |
| Lck | viral (CVB3)-induced heart disease also meningitis, hepatitis and pancreatitis | Nature Medicine. 2000. 6: 429 |
| c-Kit | leukemia, anemia, AIDS cancer | EP0639979 B1 |
| c-Kit | leukemia, anaemia, AIDS cancer | US20030103937 A1 |
| c-Kit | bacterial infections | WO03035049A2 |
| c-Kit | myeloma | W003028711 A2 |
| c-kit | retard, prevent, suppress, and/or reverse the depigmentation of hair; a disease characterized by hair depigmentation, such as vitiligo or piebaldism. | WO03043591A1 |
| c-kit | mastocytosis, the presence of one or more mast cell tumors, asthma, and allergy associated chronic rhinitis, small cell lung cancer, non-small cell lung cancer, acute myelocytic leukemia, acute lymphocytic leukemia, myelodysplastic syndrome, chronic myelogenous leukemia, a colorectal carcinoma, a gastric carcinoma, a gastrointestinal stromal tumor, a testicular cancer, a glioblastoma, and an astrocytoma. | US20020010203A1 |
| c-kit | neoplastic diseases such as mastocytosis, canine mastocytoma, human gastrointestinal stromal tumor ("GIST"), small cell lung cancer, non-small cell lung cancer, acute myelocytic leukemia, acute lymphocytic leukemia, myelodysplastic syndrome, chronic myelogenous leukemia, colorectal carcinomas, gastric carcinomas, gastrointestinal stromal tumors, testicular cancers, glioblastomas, and astrocytomas. | W004014903A1 |

(continued)

| Kinase/class | *Associated diseases* | *References* |
|---|---|---|
| Serine-Threonine Kinases | | |
| | allergic diseases such as asthma, allergic rhinitis, allergic sinusitis, anaphylactic syndrome, urticaria, angioedema, atopic dermatitis, allergic contact dermatitis, erythema nodosum, erythema multifortne, cutaneous necrotizing venulitis and insect bite skin inflammation and blood sucking parasitic infestation. inflammatory diseases such as rheumatoid arthritis, conjunctivitis, rheumatoid spondylitis, osteoarthritis, gouty arthritis and other arthritic conditions. autoimmune diseases such as multiple sclerosis, psoriasis, intestine inflammatory disease, ulcerative colitis, Crohn's disease, rheumatoid arthritis and polyarthritis, local and systemic scleroderma, systemic 1upus erythematosus, discoid 1upus erythematosus, cutaneous 1upus, dermatomyositis, polymyositis, Sjogren's syndrome, nodular panarteritis, autoimmune enteropathy, as well as proliferative glomerulonephritis. Graft-versus-host disease or graft rejection in any organ transplantation including kidney, pancreas, liver, heart, lung, and bone marrow. | |
| **Mutant forms** | | |
| Abl T315I | | W002102976 A2 |
| Bcr-abl | leukemia | W09718232 A3 |
| Bcr-abl | leukemias, including CML, ALL and AML. | WO9509365 A1 |
| Bcr-abl | leukemias, including CML, ALL and AML. | EP0721586 B1 |
| Bcr-abl | leukemias, including CML, ALL and AML. | US6066463 |
| Bcr-abl | leukemias, including CML, ALL and AML. | WO9625520 A1 |
| Bcr-abl | leukemias, including CML, ALL and AML. | US6107457 |

Table II. $IC_{50}$ of Ser/Thr kinases and Tyr kinases by A37 and B16

| Class | | | Example accession #, identifier or reference | | | | Inhibition ($IC_{50}$) | |
|---|---|---|---|---|---|---|---|---|
| Group | Family | Sub-family | GenBank Accession No. | GenBank or other enzyme source quoted by Upstate | Refseq Acc. No. | Hugo Symbol | A37 (μM) | B16 (μM) |
| CMGC | GSK | | BC012760 | EMBL L33801 | NM_ 002093 | GSK3B | <0.005 | <0.005 |
| CK1 | CK1 | | AF119911 | AB063114 or S. pombe CK1 | NM_ 018548 | CSNK1A1 | <0.005* | <0.05 |

(continued)

| Class | | | Example accession #, identifier or reference | | | | Inhibition ($IC_{50}$) | |
|---|---|---|---|---|---|---|---|---|
| Group | Family | Sub-family | GenBank Accession No. | GenBank or other enzyme source quoted by Upstate | Refseq Acc. No. | Hugo Symbol | A37 (μM) | B16 (μM) |
| STE | STE7 | | L11284 | SWISS PROT Q02750 | NM_ 002755 | MAP2K1 | <0.05 | <0.1 |
| STE | STE7 | | U39064 | EMBL U39657 | NM_ 031988 | MAP2K6 | <0.5 | <0.1 |
| CMGC | MAPK | JNK | L26318 | EMBL L26318 | NM_ 002750 | MAPK8 | <0.05 | <0.1 |
| CMGC | MAPK | JNK | U09759 | SWISS PROT P45984 | NM_ 139069 | MAPK9 | <0.05 | <0.1 |
| CMGC | MAPK | JNK | U07620 | NM_ 138980 | NM_ 002753 | MAPK10 | <0.05 | <0.05 |
| CAMK | CAMKL | AMPK | U06454 | Purified from rat-human #=U06454 | NM_ 006252 | PRKAA2 | <0.1 | <0.05 |
| AGC | RSK | RSK | L07598 | XM-004469 | NM_ 021135 | RPS6KA2 | <0.05 | <0.5 |
| TK | Abl | | M14752 | U-07563 | NM_ 005157 | ABL1 | <0.05 | <0.1 |
| TK | Src | | AF077754 | EMBL K03218 or SWISS PROT P12931 | NM_ 005417 | SRC | <0.05 | <0.05 |
| TK | Fer | | X52192 | X06292 | NM_ 002005 | FES | <0.1 | <0.1 |
| TK | Src | | M15990 | M15990 | NM_ 005433 | YES1 | <0.05 | <0.05 |
| TK | Src | | BC004473 | M30903 | NM_ 032657 | BLK | <0.1 | <0.1 |
| TK | Src | | M16038 | EMBL M64608 | NM_ 002350 | LYN | <0.05 | <0.1 |
| TK | Src | | AK056699 | EMBL M14333 | NM_ 002037 | FYN | <0.1 | <0.05 |
| TK | Src | | M26693 | EMBL X13529 | NM_ 005356 | LCK | <0.05 | <0.05 |
| TK | PDGFR | | S67773 | | NM_ 000222 | KIT | <0.5 | <0.5 |
| | | | U07563 | U07563 | | | <0.1 | <0.1 |

## Examples

While the invention has been described and exemplified in sufficient detail for those skilled in the art to make and use it, various alternatives, modifications, and improvements should be apparent without departing from the spirit and scope of the invention.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defmed by the scope of the claims.

It will be readily apparent to a person skilled in the art that varying substitutions and modifications may be made to the invention disclosed herein without departing from the scope and spirit of the invention.

It should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

### EXAMPLE 1. Efficacy of A37 and B16 is maintained in taxane-resistant tumors

A. Taxane-resistant tumor cells in which resistance is mediated through P-glycoprotein

We observed the surprising finding that subject compounds of the invention were useful in inhibiting or killing tumor cells that were resistant to other chemotherapeutic agents, where such resistance is mediated through p-glycoprotein.

Cells expressing P-glycoprotein (NCI-Adr resistant subline) were highly resistant to Paclitaxel and Taxotere in the absence of verapamil, but remained susceptible in the presence of verapamil, an inhibitor of P-glycoprotein (relative resistance ("RR") in individual experiments were between 78- and 120-fold. Upon treatment with **A37** or **B16**, there were significant reduction in the relative resistance - down to about 25-fold in both cases - about 3 to 4 fold improvement.

In these experiments, the P-glycoprotein expressing NCI-Adr resistant subline (Vickers et al., 1989. Mol Endocrinology 3 (1):157-164) was used. 3,000-15,000 cells/well were exposed to the test compounds for 72 hours at various concentrations in the presence and absence of verapamil at 12.5 μg/ml in order to calculate the $IC_{50}$ values shown in Table W, and cytotoxicity was measured using the SRB assay according to Shekan et al (J Natl Cancer Inst (1990) 82, 1107-112). Briefly, cells were plated in 96 well dishes 24 hours prior to compound addition. Where noted, they were exposed to verapamil for 4 hours prior to compound addition and then throughout the treatment period (72 hours). The assay was terminated with the addition of cold TCA to a final concentration of 10% and the plates were incubated for one hour at 4˚C. The plates were then washed 5 times with water and 100 μl of a Sulforhodamine B solution (4%) was added to each well. The plate was then incubated for 10 minutes at room temperature before removal of unbound dye by washing with 1% acetic acid. The bound dye was solubilized with 10 mM Trizma base and the absorbance read at $OD_{570}$.

B. Taxane-resistant tumor cells in which resistance is mediated through tubulin

We observed the surprising finding that subject compounds of the invention were useful in inhibiting or killing tumor cells that were resistant to other chemotherapeutic agents, where such resistance is mediated through tubulin.

Tubulin-mutated cells (1A9-PTX10) were highly resistant to paclitaxel and taxotere - highly resistance in individual experiments ranged between 37.4 to 142-fold - yet remained relatively susceptible to treatment with **A37** or **B16 -** relative resistance in individual experiments between 2.3 to 5-fold. The parental non-mutated cell line, 1A9, was used as a control.

The tubulin mutated, paclitaxel-resistant human ovarian carcinoma cell line 1A9-PTX10 and its parental paclitaxel-sensitive cell line 1A9 were derived from the A2780 human ovarian carcinoma cell line (J Biol Chem (1997) 272, 17118-17124). About 1,000-4,000 cells/well were exposed to the test compounds for 72 hours at various concentrations in order to calculate the $IC_{50}$ values shown in Table W, as above, and cytotoxicity was measured using the SRB assay according to Shekan et al. (J Natl Cancer Inst (1990) 82, 1107-112) (see Example 1A).

Table W. Cellular $IC_{50}$'s of **A37** and **B16** in Paclitaxel resistant cells

| Compound | 1A9 | 1A9-PTX10 | | NCI-Adr resistant | | |
|---|---|---|---|---|---|---|
| | | | | -verapamil | +verapamil | |
| | $IC_{50}$ [nM] | $IC_{50}$ [nM] | RR | $IC_{50}$ [nM] | $IC_{50}$ [nM] | RR |
| Paclitaxel | 4 +/- 2.6 (n=3) | 235 +/- 62.1 (n=3) | 59 | 3960 +/- 2449 (n=3) | 39 +/- 16.5 (n=3) | 101.5 |
| Taxotere | 1.75+/-0.5 (n=4) | 160 +/- 84.8 (n=4) | 91.4 | 3310 +/- 2172 (n=3) | 36.3 +/- 20.3 (n=3) | 91.2 |
| **A37** | 13.3+/-4.1 (n=3) | 44.3 +/- 15 (n=3) | 3.3 | 3433 +/- 1199 (n=3) | 135 +/- 64.5 (n=3) | 25.4 |
| **B16** | 10.7 +/- 1.1 (n=$_3$) | 25 +/- 6.2 (n=3) | 2.3 | 1873 +/- 530 (n=3) | 80 +/- 10 (n=$_3$) | 23.4 |
| Legend: | | | | | | |

Table W shows the $IC_{50}$ values as determined in the experiments described in Example 1. Numbers in parentheses indicate how often experiments were performed. In each single experiment a minimum of three replica wells was used for each drug concentration and cell line. Shown are mean values and standard deviations of the $IC_{50}$'s, as determined in the individual experiments. RR denominates the relative resistance, *i.e.* the level of resistance conferred to the indicated drugs by the respective resistance mechanisms, and is calculated as the ratio of the means of the resistant cells compared to the sensitive cells.

**Example 2. Efficacy of A37 and B16 is maintained in Camptothecin-resistant tumor cells**

We observed the surprising finding that subject compounds were useful in inhibiting or killing tumour cells that were resistant to other chemotherapeutic agents, where such resistance is mediated through topoisomerase I.

Topoisomerase-mutated cells (CEM/C2) were highly resistant to camptothecin - relative resistance in individual experiments ranged between 857 to 1959-fold - yet remained highly susceptible to treatment with **A37** and **B16 -** relative resistance in individual experiments between 0.385 to 10.31-fold. The parental non-mutated cell line, CEM, was used as a control.

The Camptothecin resistant CEM/C2 cells were derived from the T lymphoblastoid leukemia cell line CCRF/CEM by selection in the presence of Camptothecin *in vitro* (Kapoor et al., 1995. Oncology Research 7; 83-95, ATCC).

The effects of **A37** and **B16** were evaluated in the CCRF/CEM and CEM/C2 cells using the same SRB assay as described above, with 72-hour drug exposure.

Table X. Cellular $IC_{50}$'s of **A37** and **B16** in Camptothecin resistant cells

| Compound | CEM $IC_{50}$ (nM) | CEM/C2 $IC_{50}$ (nM) | RR |
|---|---|---|---|
| Camptothecin | 3.5 +/- 0.5 (n=5) | 4823+/-1415 (n=5) | 1378 |
| **A37** | 21.3+/-12.7 (n=$_3$) | 15.3 +/-11.1 (n=$_3$) | 0.72 |
| **B16** | 25 +/- 7.6 (n=3) | 25.3 +/- 8.0 (n=3) | 1.01 |
| Legend: | | | |

Table X shows the $IC_{50}$ values as determined in the experiments described in Example 2. Numbers in parentheses indicate how often experiments were performed. In each single experiment, a minimum of three replica wells was used for each drug concentration and cell line. Shown are mean values and standard deviations of the $IC_{50}$'s, as determined in the individual experiments. RR denominates the relative resistance, *i.e.* the level of resistance conferred to the indicated drugs through atypical multi-drug resistance and mutant topoisomerase I.

**Example 3. Efficacy of A37 and B16 is maintained in Cisplatin-resistant tumor cells**

We observed the surprising finding that subject compounds were useful in inhibiting or killing tumour cells that were resistant to other therapeutic compounds, such as cisplatin.

The A129 cp80 cell line (received from Tito Fojo, NIH), derived from the ovarian carcinoma A2780, was highly resistant to cisplatin - relative resistance in individual experiments ranged between 35.6 to 106-fold - yet remained highly susceptible to treatment with **A37** and **B16 -** relative resistance in individual experiments between 0.5 to 2.5-fold (Table Y). The parental non-mutated cell line A129 was used as a control.

About 1,000-5,000 cells/well were exposed to the test compounds for 72 hours at various concentrations in order to calculate the $IC_{50}$ values shown in Tables Y. Cytotoxicity was measured using the SRB assay according to Shekan *et al.* as described above in Example 1A.

**Table Y. Cellular $IC_{50}$'s of A37 and B16 in Cisplatin resistant cells**

| Compound | Cell line | | RR |
|---|---|---|---|
| | A129 $IC_{50}$ (nM) | A129 cp80 $IC_{50}$ (nM) | |
| Cisplatin | 324 +/- 181 (n=5) | 16720 +/- 536 (n=5) | 51.6 |
| **A37** | 5.7 +/-4.0 (n=3) | 4 +/- 5.0 (n=3) | 0.7 |
| **B16** | 8.7 +/-10.0 (n=3) | 4.7 +/- 0.7 (n=3) | 0.5 |
| Legend: | | | |

Table Y shows the $IC_{50}$ values as determined in the experiments described in Example 3. Numbers in parentheses indicate how often experiments were performed. In each single experiment a minimum of three replica wells was used for each drug concentration and cell line. Shown are mean values and standard deviations of the $IC_{50}$'s, as determined in the individual experiments. RR denominates the relative resistance, *i.e.* the relative level of resistance conferred to the indicated drugs by the mechanism that confers cisplatin resistance.

**Example 4. Efficacy of B16 is maintained in Mitoxanthrone-resistant tumor cells**

We observed the surprising finding that subject compounds were useful in inhibiting or killing tumor cells that were resistant to other therapeutic compounds, such as mitoxanthrone.

The HT29/mit cell line, derived from the human colon carcinoma cell line HT29 cell line by exposure to increasing drug concentrations up to 0.3 μg/ml (Perego et al., Cancer Research 61: 6034-6037, 2001), was highly resistant to mitoxanthrone - relative resistance in individual experiments ranged between 104 to 239-fold - yet remained relatively susceptible to treatment with **B16** - relative resistance in individual experiments between 6.9 to 36.1-fold (Table Z). The parental non-mutated cell line HT29 was used as a control.

About 1,000-5,000 cells/well were exposed to the test compounds for 72 hours at various concentrations in order to calculate the $IC_{50}$ values shown in Tables **Z**. Cytotoxicity was measured using the SRB assay according to Shekan *et al.* as described above in Example 1A.

**Table Z. Cellular $IC_{50}$'s of B16 in Mitoxanthrone resistant cells**

| Compound | Cell line | | RR |
|---|---|---|---|
| | HT29 $IC_{50}$ (μM) | HT29/mit $IC_{50}$ (μM) | |
| Mitoxanthrone | 1.23 +/- 0.52 (n=4) | 188.3 +/- 22.4 (n=4) | 153.1 |
| **B16** | 0.077 +/- 0.01 (n=3) | 1.70 +/- 0.7 (n=3) | 22.1 |
| Legend: | | | |

Table Z shows the $IC_{50}$ values as determined in the experiments described in Example 4. Numbers in brackets indicate how often experiments were performed. In each single experiment a minimum of three replica wells was used for each drug concentration and cell line. Shown are mean values and standard deviations of the $IC_{50}$'s, as determined in the individual experiments. RR denominates the relative resistance, *i.e.* the relative level of resistance conferred to the indicated drugs by the mechanism that confers mitoxanthrone resistance.

In summary, cisplatin-resistant cells and atypical MDR cells has no resistance against compounds **A37** and **B16.** Cells resistant against taxane due to tubulin mutations have no significant resistance against compounds **A37** and **B16.** There is only moderate resistance against **A37** and **B16** in cells exibiting P-glycoprotein-mediated MDR, and only moderate resistance against mitoxanthrone resistance for **B16.**

## References

R.D. Baird and S.B. Kaye, Drug resistance reversal - are we getting closer?, European Journal of Cancer, 39: 2450-61 (2003).

P. Giannokakou et al., J. Biol. Chem. 272: 17118-125 (1997).

P. Giannokakou et al., Proc. Natl. Acad. Sci. U.S.A. 97: 2904-2909 (2000)

Kelland L R. An update on satraplatin: the first orally available platinum anticancer drug. Expert Opin Investig Drugs 9(6):1373-1382, 2000.

**Example 5. Inhibition of Mutant Kinases and Proliferation Inhibition of Bcr-Abl cells by A37 and B16**

We observed the surprising finding that subject compounds were useful in inhibiting mutant kinases, including those that are not subject to GLEEVEC® inhibition. The table below shows inhibitory activity of **A37** and **B16** against c-Abl and the T315I mutation, which is frequently found in CML patients. The table also shows inhibition of proliferation of KU812 leukemia cells with the Philadelphia chromosome that express the Bcr-Abl kinase (ATCC: CRL-2099; Kishi, Leuk. Res. 9: 381-390, 1985).

Table **AA**

| | $IC_{50}$ (μM) | | |
|---|---|---|---|
| Compounds | Abl | Abl T315I | KU812 cells |
| Gleevec® | 0.05 | >30 | 0.137 |
| **A37** | 0.035 | 0.087 | 0.085 |
| **B16** | 0.052 | 0.1 | 0.054 |

It is evident that both **A37** and **B16** can inhibit the mutant form of Abl (T315I) almost equally well as compared to wild-type Ab1- at most about 2-fold less effective. In contrast, GLEEVEC® is at least a 600-time worse inhibitor of Abl (T315I) mutant as compared to the wild-type Abl. This probably contributes to the inability of GLEEVEC® to treat CML patients that relapse or become resistant/refractory after initial response to GLEEVEC, in which patients the Abl (T315I) mutation is frequently found. See Gorre et al., Science 293:876-80, 2001; von Bubnoff et al., Lancet 359:487-91, 2002. Compounds **A37** and **B16** are potent inhibitors, not only of the c-Abl and T315I mutant, but also of proliferation of leukemia that expresses the Bcr-Ab1 kinase, and inhibit the proliferation of such cells up to 2.5-fold more effective than GLEEVEC. Leukemia cells expressing Bcr-Abl, are often subsequently found to contain mutations such as the T3151 mutation as described in section XI.

Examples of the methods used to assay the inhibition of mutant kinases such as Abl T315I and other kinases such as c-Abl and c-Kit are described in Example 6.

The proliferation of KU812 cells was assayed using the calcein viability assay described in Assay 3 but adapted for quantification by flow cytometry, briefly as follows: cells were recovered from sub-confluent plates by trypsinization and replated at a density of 1,000 cells/well in 24 well plates and incubated 24 hours to allow adherence. The media (RPMI-1640 supplemented with 10% FCS and Pen/Strep) was aspirated from each well and replaced with media containing the compound at a range from 0 to 0.250 μM. The plates were incubated for 3 days corresponding to approximately three cell cycles. Cellular viability was determined with the Calcein AM assay as follows. The cells were washed twice in PBS and incubated with 5μM Calcein AM/PBS for 75 minutes before determining fluorescent units with a fluorescent plate reader. The Calcein AM assay was adapted for the flow cytometer by including an additional 1:16,000 dilution of the Calcein AM in PBS of which 200 ul was added to each sample of cells. The cells were incubated for 90 minutes at 37oC and washed once in PBS before the fluorescence was quantified on the flow cytometer, from which the $IC_{50}$ values shown in Tables **AA** where estimated.

**Example 6. Kinase Assays and Protocols**

Kinase assays conducted using generally available protocols such as Upstate Biotech's (Charlotteville, VA)

Chemilluminescent MBP Assay Kit or the KinaseProfiler™ Selectivity Testing Services, the PanQuinase Activity Assay service from ProQuinase(Frieburg, Germany), or those assays described below show that compounds disclosed herein may inhibit a broad range of kinases, including non-CDK kinases. Table3, Table 4, Table 5 and Table II show the results obtained for inhibition of a broad range of such kinases for subject compounds of the invention.

**Technical Note on scintillation counting:**

Allow the radiolabeled substrate to bind to the filter paper for 30 seconds before immersing the paper into a 50 ml conical tube containing 40 ml 0.75% phosphoric acid. Gently shake the assay squares for 5 minutes on a rotator. Discard the wash in a liquid radioisotope waste container, (dispose of per institutional regulations) and repeat the wash step twice. Wash the squares in 20 ml of acetone for 5 minutes. Drain and add scintillation cocktail.

**Example 6a Kinase Assay Protocol - GSK3β**

**Upstate catalog # 14-306**

**Stock Solutions:**

1. Reaction Buffer: 8mM MOPS, pH 7.0, 0.2mM EDTA, 10mM magnesium acetate.
2. GSK3β, active: 5-40ng per assay point. Dilute to 0.5-4ng/μl in Reaction Buffer containing 0.03% Brij-35.
3. Phospho-Glycogen Synthase Peptide-2 (Catalog # 12-241): Prepare a 250μM stock. Use 10μl per assay point for a final assay concentration of 62.5 μM per assay point.
4. [$\gamma$-$^{32}$P]ATP: Stock 1mCi/100μl (3000Ci/mmol, obtained from Perkin Elmer, Cat. # BLU002A). Make 10μl aliquots (100μCi/vial). Before starting the assay, dilute an aliquot to 1μCi/μl with 90μl of 75mM magnesium chloride and 500μM unlabeled ATP in 20mM MOPS, pH 7.2, 25mM β-glycerol phosphate, 5mM EGTA, 1mM sodium orthovanadate, 1mM dithiothreitol.

**Assay Protocol:**

1. Add 10μl of Reaction Buffer per assay.
2. Add 10μl of substrate peptide [final assay concentration of 62.5μM].
3. Add 10μl of **GSK3β, active** [5-40ng per assay].
4. Add 10μl of the diluted [$^{32}$P]ATP mixture.
5. Incubate for 30 minutes at 30˚C.
6. Spot 25μl onto the center of a 2cm x 2cm P81 paper square.
7. Wash the assay squares five times with 0.75% phosphoric acid.
8. Wash the assay squares once with acetone.
9. Transfer the assay squares to vial and add 5ml scintillation cocktail.
10. Read in scintillation counter. Compare cpm of enzyme samples to cpm of control samples that contain no enzyme (background control).

**Example 6b Kinase Assay Protocol - CKI-yeast (A37 assay)**

**Upstate catalog # 14-112**

**Stock Solutions:**

1. Assay Dilution Buffer I (ADBI, Catalog # 20-108): 20mM MOPS, pH 7.2, 25mM β-glycerol phosphate, 5mM EGTA, 1mM sodium orthovanadate, 1mM dithiothreitol.
2. Casein Kinase 1, active: Dilute to 10-50ng/ul Use 10μl (100-500ng) per assay point.
3. Casein Kinase 1 Substrate Peptide (Catalog # 12-423): Prepare a 1mM sock in ADBI. Use 10μl per assay for a final concentration of 250μM per assay point.

4. [γ-$^{32}$P]ATP: Stock 1mCi/100μl (3000Ci/mmol, obtained from Perkin Elmer, Cat. # BLU002A). Make 10μl aliquots (100μCi/vial). Before starting the assay, dilute an aliquot with 90μl of 500μM cold ATP and 75mM magnesium chloride in ADBI.

**Assay Protocol:**

1. Add 10μl of ADBI to a microcentrifuge tube.
2. Add 10μl of **Casein Kinase 1, active** (100-500ng).
3. Add 10μl (250μM) Casein Kinase 1 Substrate Peptide.
4. Add 10μl of diluted [γ-$^{32}$P]ATP mixture.
5. Incubate for 10 minutes at in a shaking incubator at 30˚C.
6. Transfer 25μl onto the center of a 2cm x 2cm P81 paper.
7. Wash the assay squares three times with 0.75% phosphoric acid.
8. Wash the assay squares once with acetone.
9. Transfer the assay squares to a scintillation vial and add 5ml scintillation cocktail.
10. Read in scintillation counter. Compare cpm of enzyme samples to cpm of control samples that contain no enzyme (background control).

**Example 6c Kinase Assay Protocol - CK1-delta (B16 assay)**

**Upstate catalog # 14-520**

**Stock Solutions:**

1. **5X Reaction Buffer:** 40mM MOPS, pH 7.0, 1mM EDTA.
2. **KRRRALS(p)VASLPGL:** Use at a final assay concentration of 200μM. Prepare a 2mM stock. Use 2.5μl of stock.
3. **CK1 delta:** Dilute to 0.2-2ng/μl with 20mM MOPS, pH 7.0, 1mM EDTA, 0.01% Brij 35, 5% glycerol, 0.1% 2-mercaptoethanol, 1mg/ml BSA. Use 2.5μl per assay point.
4. **[γ-$^{32}$P]ATP:** Stock 1mCi/100μl (3000Ci/mmol, obtained from Perkin Elmer, Catalog # BLU002A). Before starting the assay, dilute a 10μl aliquot to 1μCi/μl by adding 90μl of 75mM $MgCl_2$ and 500μM cold ATP in 20mM MOPS, pH 7.2, 25mM β-glycerol phosphate, 5mM EGTA, 1mM sodium orthovanadate, 1mM dithiothreitol (Magnesium/ATP cocktail, Catalog # 20-113).

**Assay Procedure:**

1. Add 5μl of5X Reaction Buffer per assay.
2. Add 2.5μl of KRRRALS(p)VASLPGL.
3. Add 2.5μl (0.5-5ng) of **Casein Kinase 1δ, active.**
4. Add 5μl sterile, distilled water.
5. Add 10μl of the diluted [γ-$^{32}$P]ATP.
6. Incubate for 10 minutes at 30˚C.
7. Spot 20μl onto the center of a 2cm x 2cm P81 paper square (Catalog # 20-134).
8. Wash the assay squares 3 times for 5 minutes each with 0.75% phosphoric acid.
9. Wash the assay squares once for 5 minutes with acetone.
10. Transfer the assay squares into scintillation vials and add 5ml scintillation cocktail.
11. Read in scintillation counter. Compare cpm of enzyme samples to cpm of control samples.

**Examples 6d Kinase Assay Protocol - MEK1**

**Upstate catalog # 14-429**

**Stock Solutions:**

1. Assay Dilution Buffer I (ADBI, Catalog # 20-108): 20mM MOPS, pH 7.2, 25mM β-glycerol phosphate, 5mM EGTA, 1mM sodium orthovanadate, 1mM dithiothreitol.
2. Magnesium/ATP Cocktail (Catalog # 20-113): 500μM cold ATP and 75mM magnesium chloride in ADBI.
3. MEK1, active: Dilute to 0.5-5ng/μl with ADBI containing 0.03% Brij-35 and 1mg/ml BSA. Use 5μl per assay point.
4. MAP Kinase 2/Erk2, unactive (Catalog # 14-198): Use 1μg per assay point.
5. MBP Substrate (Catalog # 13-104): Prepare a 3.33mg/ml stock solution with ADBI. Use 2.5μl per assay for a final concentration of 0.33mg/ml per assay point.
6. Magnesium/ATP Cocktail (Catalog # 20-113): 75mM $MgCl_2$ and 500μM ATP in 20mM MOPS, pH 7.2, 25mM β-glycerol phosphate, 5mM EGTA, 1mM sodium orthovanadate, 1mM dithiothreitol.
7. [$\gamma$-$^{32}$P]ATP: Stock 1mCi/100μl (3000Ci/mmol, obtained from Perkin Elmer, Catalog # BLU002A). Make 10μl aliquots (100μCi/vial). Before starting the assay, dilute the Magnesium/ATP Cocktail 1:1 with sterile, distilled water. Then dilute an aliquot of [$\gamma$-$^{32}$P]ATP to 1μCi/μl with 90μl of the diluted Magnesium/ATP Cocktail. Use 10μl per assay point for a final ATP concentration of 100μM per assay point.

**Assay Procedure:**

Stage One: *Activation of MAP Kinase*

1. Add 11μl of ADBI to a microcentrifuge tube.
2. Add 5μl of Magnesium/ATP cocktail.
3. Add 5μl **of MEK1, active** (2.5-25ng)
4. Add 4μl (1μg) of unactive MAP Kinase 2/Erk 2.
5. Incubate at 30˚C for 30 minutes.
6. Remove 1μl of this mixture and add to Stage Two component mixture.

Stage Two: *Assay of MAP Kinase Activity*

1. Add 12.5μl of ADBI.
2. Add 2.5μl of MBP stock solution.
3. Add 10μl of diluted [$\gamma$-$^{32}$P] ATP.
4. Incubate for at 30˚C 10 minutes.
5. Transfer a 20μl aliquot onto the center of a 2cm x 2cm P81 paper square.
6. Wash the paper squares three times with 0.75% phosphoric acid for 5 minutes per wash.
7. Wash the paper squares once with acetone for five minutes.
8. Transfer the paper squares to a scintillation vial and add 5m1 scintillation cocktail.
9. Read in scintillation counter. Compare cpm of enzyme samples to cpm of control samples that contain no enzyme (background control).

**Example 6e Kinase Assay Protocol - MKK6**

**Upstate catalog # 14-303**

**Stock Solutions:**

1. Reaction Buffer (RB): 50mM Tris-HCl, pH 7.5, 0.1mM EGTA, 0.1% 2-mercaptoethanol, 0.1mM sodium orthovanadate, 10mM MgAc.
2. Magnesium/ATP Cocktail (Catalog # 20-113): 500μM cold ATP and 75mM magnesium chloride in 20mM MOPS, pH 7.2, 25mM β-glycerol phosphate, 5mM EGTA, 1mM sodium orthovanadate, 1mM dithiothreitol.
3. MKK6/SKK3, active: Dilute to 10ng/μl with Reaction Buffer containing 0.03% Brij-35. Use 5μl (50ng) per assay point.
4. p38α/SAPK2a, unactive (Catalog # 14-252): Use 4μl per assay point.
5. Myelin Basic Protein (MBP, Catalog # 13-104): Prepare a 2mg/ml stock solution in 20mM MOPS, pH 7.2, 25mM β-glycerol phosphate, 5mM EGTA, 1mM sodium orthovanadate, 1mM dithiothreitol. Use 10μl per assay point.
6. [γ-$^{32}$P]ATP: Stock 1mCi/100μl (3000Ci/mmol, obtained from Perkin Elmer, Catalog # BLU002A). Make 10μl aliquots (100μCi/vial). Before starting the assay, dilute the Magnesium/ATP Cocktail 1:1 with sterile, distilled water. Then dilute an aliquot of [γ-$^{32}$P]ATP to 1μCi/μl with 90μl of the diluted Magnesium/ATP Cocktail. Use 10μl per assay point for a final ATP concentration of 100μM per assay point.

**Assay Procedure:**

Stage One: *Phosphorylation and A ctivation of p38 α by MKK6/SKK3*

1. On ice, add 1μl RB to a microcentrifuge tube.
2. Add 4μl (2μg) p38α/SAPK2a, unactive.
3. Add 10μl of Magnesium/ATP Cocktail.
4. Add 5μl **(50ng) MKK6/SKK3, active.**
5. Incubate for 30 minutes shaking at 30°C.
6. Place on ice and proceed to Stage Two.

Stage Two: *Phosphorylation of Myelin Basic Protein by Activated p38 α*

1. Remove 10μl of the mixture prepared in Stage One and add to a new microcentrifuge tube.
2. Add 20μl of RB.
3. Add 10μl of MBP.
4. Add 10μl of the diluted [γ-$^{32}$P] ATP mixture (1μCi/μl).
5. Incubate for 15 minutes shaking at 30°C.
6. Spot 40μl on a 2cm x 2cm P81 paper.
7. Wash assay squares three times with 0.75% phosphoric acid for 5 minutes per wash.
8. Wash assay squares once with acetone for five minutes.
9. Transfer the assay squares to a scintillation vial and add 5ml scintillation cocktail.
10. Read in scintillation counter. Compare cpm of enzyme samples to cpm of control samples that contain no active enzyme (background control).

**Example 6f Kinase Assay Protocol - JNK1α1**

**Upstate catalog # 14-327**

**Stock Solutions:**

1. 10X Reaction Buffer: 500mM Tris-HCl pH 7.5, 1mM EGTA, 1% 2-mercaptoethanol.
2. Enzyme Dilution Buffer (EDB): 50mM Tris-HCl pH 7.5, 0.1mM EGTA, 0.1% 2-mercaptoethanol, 1mg/ml BSA.
3. JNK1α1/SAPK1c, active: Dilute to 0.4-8ng/μl with EDB. Use 2.5μl per assay point.
4. ATF2 (amino acids 19-96) (Catalog # 12-367): Prepare a 1.08mg/ml stock. Use 2.5μl per assay point for a final concentration of 3μM per assay point.
5. Magnesium/ATP Cocktail (Catalog # 20-113): 75mM MgCl2 and 500μM ATP in 20mM MOPS, pH 7.2, 25mM β-glycerol phosphate, 5mM EGTA, 1mM sodium orthovanadate, 1mM dithiothreitol.

6. [$\gamma$-$^{32}$P]ATP: Stock 1mCi/100μl (3000Ci/mmol, obtained from PerkinElmer, Cat. # BLU002A). Make 10μl aliquots (100μCi/vial). Before starting the assay, dilute an aliquot to 1μCi/μl with 90μl of Magnesium/ATP Cocktail diluted 1:2 with sterile, distilled water. Use 10μl per assay point for a final ATP concentration of 100μM per assay point.

**Assay Procedure:**

1. Add 2.5μl of 10X Reaction Buffer per assay.
2. Add 2.5μl of ATF2 (amino acids 19-96).
3. Add 2.5μl **(1-20ng) JNK1α1/SAPK1c, active.**
4. Add 7.5μl of sterile, distilled water.
5. Add 10μl of the diluted [$\gamma$-$^{32}$P]ATP mixture.
6. Incubate for 10 minutes at 30˚C.
7. Transfer a 20μl aliquot onto the center of a 2cm x 2cm P81 paper.
8. Wash the assay squares three times for 5 minutes with 0.75% phosphoric acid.
9. Wash the assay squares once for 5 minutes with acetone.
10. Transfer the assay squares to vial and add 5ml scintillation cocktail.
11. Read in scintillation counter. Compare cpm of enzyme samples to cpm of control samples that contain background control.

**Example 6g Kinase Assay Protocol - JNK2α2**

**Upstate catalog # 14-329**

**Stock Solutions:**

1. 10X Reaction Buffer: 500mM Tris-HCl, pH 7.5, 1mM EGTA, 1% 2-mercaptoethanol.
2. ATF2 (amino acids 19-69) (Catalog # 12-367): Prepare a 30μM stock. Use 2.5μl per assay point for a final concentration of 3μM per assay.
3. JNK2α2/SAPK1a, active: Dilute to 4-40ng/μl with 1X Reaction Buffer containing 0.03% Brij-35. Use 2.5μl per assay point.
4. Magnesium/ATP Cocktail (Catalog # 20-113): 75mM $MgCl_2$ and 500μM ATP in 20mM MOPS, pH 7.2, 25mM β-glycerol phosphate, 5mM EGTA, 1mM sodium orthovanadate, 1mM dithiothreitol.
5. [$\gamma$-$^{32}$P]ATP: Stock 1mCi/100μl (3000Ci/mmol, obtained from PerkinElmer, Catalog # BLU002A). Make 10μl aliquots (100μCi/vial). Before starting the assay, dilute the Magnesium/ATP Cocktail 1:1 with sterile, distilled water. Then dilute an aliquot of [$\gamma$-$^{32}$P]ATP to 1 μCi/μl with 90μl of the diluted Magnesium/ATP Cocktail. Use 10μl per assay point for a final ATP concentration of 100μM per assay point.

**Assay Procedure:**

1. Add 2.5μl of 10X Reaction Buffer to a microcentrifuge tube.
2. Add 2.5μl (3μM) of ATF2 (amino acids 19-69).
3. Add 2.5μl **(10-100ng) of JNK2α2/SAPK1a, active.**
4. Add 7.5μl of sterile, distilled water.
5. Add 10μl of the diluted [$\gamma$-$^{32}$P]ATP solution.
6. Incubate with agitation for 10 minutes at 30˚C.
7. Transfer a 20μl aliquot onto the center of a 2cm x 2cm P81 paper square (Catalog # 20-134).
8. Wash the paper squares three times with 0.75% phosphoric acid for 5 minutes.
9. Wash the paper squares once for 5 minutes with acetone.
10. Transfer the paper squares to a scintillation vial and add 5ml scintillation cocktail.
11. Read in scintillation counter. Compare the cpm of enzyme samples to the cpm of control samples that contain no enzyme (background control).

**Example 6h Kinase Assay Protocol - JNK3**

**Upstate catalog # 14-501**

**Stock Solutions:**

1. 10X Reaction Buffer: 500mM Tris-HCl pH 7.5, 1mM EGTA, 1% 2-mercaptoethanol.
2. Enzyme Dilution Buffer (EDB): 50mM Tris-HCl pH 7.5, 0.1mM EGTA, 0.1% 2-mercaptoethanol, 1mg/ml BSA, 0.03% Brij-35.
3. JNK3/SAPK1b, active: Dilute to 4-24ng/μl with EDB. Use 2.5μl per assay point.
4. ATF2 (amino acids 19-96) (Catalog # 12-367): Prepare a 1.08mg/ml stock. Use 2.5μl per assay point for a final concentration of 3μM per assay point.
5. [γ-$^{32}$P]ATP: Stock 1mCi/l00μl (3000Ci/mmol, obtained from PerkinElmer, Cat. # BLU002A). Make 10μl aliquots (100μCi/vial). Before starting the assay, dilute an aliquot to 1μCi/μl with 90μl of 75mM MgCl2 and 500μM cold ATP in 20mM MOPS, pH 7.2, 25mM β-glycerol phosphate, 5mM EGTA, 1mM sodium orthovanadate, 1mM dithiothreitol.

**Assay Procedure:**

1. Add 2.5μl of 10X Reaction Buffer per assay.
2. Add 2.5μl of ATF2 (amino acids 19-96).
3. Add 2.5μl **(10-60ng) JNK3/SAPK1b, active.**
4. Add 7.5μl of sterile, distilled water.
5. Add 10μl of the diluted [γ-$^{32}$P]ATP mixture.
6. Incubate for 10 minutes at 30˚C.
7. Transfer a 20μl aliquot onto the center of a 2cm x 2cm P81 paper.
8. Wash the assay squares three times for 5 minutes with 0.75% phosphoric acid.
9. Wash the assay squares once for 5 minutes with acetone.
10. Transfer the assay squares to vial and add 5ml scintillation cocktail.
11. Read in scintillation counter. Compare cpm of enzyme samples to cpm of control samples that contain background control.

**Example 6i Kinase Assay Protocol - AMPK**

**Upstate catalog # 14-305**

**Stock Solutions:**

1. AMPK Reaction Buffer: 20mM HEPES-NaOH, pH 7.0,0.4mM dithiothreitol, 0.01% Brij-35, with or without 300μM AMP.
2. SAMS Substrate Peptide (Catalog # 12-355): Use 7μl per reaction for final assay concentration of 100μM.
3. AMP-Activated Protein Kinase: Dilute enzyme to 2-10MU/μl in AMPK Reaction Buffer with or without 300μM AMP. AMP can be purchased from Sigma-Aldrich, Catalog # A1752.
4. [γ-$^{32}$P]ATP: Stock 1mCi/100μl (3000Ci/mmol, obtained from PerkinElmer, Cat. # BLU002A). Make 10μl aliquots (100μCi/vial). Before starting the assay, dilute an aliquot to 1μCiul with 90μl of 75mM magnesium chloride and 500μM unlabeled ATP in 20mM MOPS, pH 7.2, 25mM β-glycerophosphate, 5mM EGTA, 1mM $Na_3VO_4$, 1mM dithiothreitol.

**Assay Protocol:**

**NOTE: Negative controls should be run with all reagents containing no AMP.**

1. Add 13μl of Reaction Buffer per assay.
2. Add 7μl of SAMS substrate peptide [100μM final assay concentration].
3. Add 10μl **of AMP-Activated Protein Kinase** [20-100mU].
4. Add 10μl of the diluted [$^{32}$P]ATP mixture.
5. Incubate for 15 minutes at 30˚C in a shaking incubator.
6. Spot 35μl onto the center of a 2cm x 2cm P81 paper square.
7. Wash the assay squares three times with 0.75% phosphoric acid for 5 minutes.
8. Wash the assay squares once with acetone for 5 minutes.
9. Transfer the assay squares to vial and add 5ml scintillation cocktail.
10. Read in scintillation counter. Compare cpm of enzyme samples to cpm of control samples that contain no enzyme (background control).

**Example 6j Kinase Assay Protocol - Rsk3**

**Upstate catalog # 14-462**

**Stock Solutions:**

1. 2.5X Reaction Buffer: 20mM MOPS, pH 7.0, 1mM EGTA.
2. Enzyme Dilution Buffer (EDB): 20mM MOPS, pH 7.0, 1mM EDTA, 0.01% Brij-35, 5% glycerol, 0.1% β-mercaptoethanol, 1mg/ml BSA.
3. RSK3, active: Dilute to 4-40ng/μl with EDB. Use 2.5μl per assay point.
4. MAPKAP Kinase 2 substrate peptide (Catalog #12-240): Prepare a 2.5mM stock. Use 2.5μl per assay point for a final concentration of 250μM per assay.
5. Magnesium/ATP Cocktail (Catalog # 20-113): 75mM $MgCl_2$ and 500μM ATP in 20mM MOPS, pH 7.2, 25mM β-glycerol phosphate, 5mM EGTA, 1mM sodium orthovanadate, 1mM dithiothreitol.
6. [γ-$^{32}$P]ATP: Stock 1mCi/100μl (3000Ci/mmol, obtained from PerkinElmer, Catalog # BLU002A). Make 10μl aliquots (100μCi/vial). Before starting the assay, dilute the Magnesium/ATP Cocktail 1:1 with sterile, distilled water. Then dilute an aliquot of [γ-$^{32}$P]ATP to 1μ.Ci/μl with 90μl of the diluted Magnesium/ATP Cocktail. Use 10μl per assay point for a final ATP concentration of 100μM per assay point.

**Assay Protocol:**

1. Add 10μl of 2.5X Reaction Buffer per assay.
2. Add 2.5μl of MAPKAP Kinase 2 substrate peptide.
3. Add 2.5μl of **RSK3, active** (10-100ng).
4. Add 10μl of the diluted [γ$^{32}$P]ATP mixture.
5. Incubate for 10 minutes at 30˚C.
6. Transfer a 20μl aliquot onto the center of a 2cm x 2cm P81 paper.
7. Wash the assay squares three times for 5 minutes with 0.75% phosphoric acid.
8. Wash the assay squares once for 5 minutes with acetone.
9. Transfer the assay squares to vial and add 5ml scintillation cocktail.
10. Read in scintillation counter. Compare cpm of enzyme samples to cpm of control samples that contain background control.

**Example 6k Kinase Assay Protocol - Abl**

**Upstate catalog # 14-529**

**Stock Solutions:**

1. 5X Reaction Buffer: 40mM MOPS, pH 7.0, 1mM EDTA.

2. Enzyme Dilution Buffer (EDB): 20mM MOPS, pH 7.0, 1mM EDTA, 0.01% Brij-35, 5% glycerol, 0.1% β-mercaptoethanol, 1mg/ml BSA.
3. Abl, active: Dilute to 0.2-8ng/μl with EDB. Use 2.5μl per assay point.
4. Abltide (EAIYAAPFAKKK), (Catalog # 12-493): Prepare a 500μm stock. Use 2.5μl per assay point for a final assay concentration of 50μM per assay point.
5. [γ-$^{32}$P]ATP: Stock 1mCi/100μl (3000Ci/mmol, obtained from PerkinElmer, Cat. # BLU002A). Make 10μl aliquots (100μCi/vial). Before starting the assay, dilute an aliquot to 1μCi/μl with 90μl of 75mM $MgCl_2$ and 500μM cold ATP in 20mM MOPS, pH 7.2, 25mM β-glycerol phosphate, 5mM EGTA, 1mM sodium orthovanadate, 1mM dithiothreitol.

**Assay Protocol:**

1. Add 5μl of 5X Reaction Buffer per assay.
2. Add 2.5μl of Abltide (50μM).
3. Add 2.5μl of **Abl (human), active** (0.5-20ng).
4. Add 5μl sterile, distilled water.
5. Add 10μl of the diluted [γ-$^{32}$P]ATP solution.
6. Incubate for 10 minutes at 30˚C.
7. Transfer a 20μl aliquot onto the center of a 2cm x 2cm P81 paper.
8. Wash the assay squares three times for 5 minutes with 0.75% phosphoric acid.
9. Wash the assay squares once for 5 minutes with acetone.
10. Transfer the assay squares to vial and add 5ml scintillation cocktail.
11. Read in scintillation counter. Compare cpm of enzyme samples to cpm of control samples that contain background control.

**Example 61 Kinase Assay Protocol - c-Src**

**Upstate catalog # 14-326**

**Stock Solutions:**

1. Src Kinase Reaction Buffer (SrcRB), Catalog # 20-121: 100mM Tris-HCl, pH 7.2, 125mM $MgCl_2$, 25mM $MnCl_2$, 2mM EGTA, 0.25mM sodium orthovanadate, 2mM dithiothreitol.
2. [γ-$^{32}$P]ATP: Stock 1mCi/100μl (3000Ci/mmol, obtained from DuPont-NEN). Make 10μl aliquots (100μCi/vial). Before starting the assay dilute an aliquot to 1μCi/μl with 90μl of 500μM unlabeled ATP containing 75mM $MnCl_2$ 20mM MOPS, pH 7.2, 25mM β-glycerophosphate, 5mM EGTA, 1mM $Na_3VO_4$, 1mM dithiothreitol.
3. Src Kinase Substrate Peptide (Catalog # 12-140): Use a 0.6-1.5mM stock. Rehydrate 1mg peptide with 400μl distilled water or SrcRB for a 1.5mM stock or with 998μl for a 0.6mM stock.
4. Src, active: Use 20-80 Units per assay point. Dilute as required with SrcRB.

**Assay Protocol:**

1. Add 10μl of SrcRB into a microfuge tube.
2. Add 10μl Src Kinase substrate peptide (93-375μM/assay).
3. Add 4-16μl **of Src, active** (20-80Units/assay).
4. Add 10μl of the diluted [γ-$^{32}$P]ATP.
5. Incubate for 10 minutes at 30˚C.
6. Add 20μl of 40% TCA and incubate for 5 minutes at room temperature.
7. Transfer 25μl to the center of a 2cm x 2cm P81 paper square.
8. Wash the assay squares five times for 5 minutes each with 0.75% phosphoric acid.
9. Wash the assay squares once with acetone for 5 minutes.
10. Transfer the assay squares to a scintillation vial and add 5ml scintillation cocktail.
11. Read in scintillation counter. Compare cpm of enzyme samples to cpm of control samples that contain no enzyme

(background control).

**Example 6m Kinase Assay Protocol - Fes**

**Upstate catalog # 14-473**

**Stock Solutions:**

1. 2.5X Reaction Buffer: 50mM MOPS, pH 7.0, 2.5mM EDTA.
2. Poly($Glu_4$-Tyr) (4:1): Prepare a 1mg/ml stock in sterile distilled water. Add 2.5μl per assay point for a final concentration of 0.1mg/ml.
3. Enzyme Dilution Buffer (EDB): 20mM MOPS, pH 7.0, 1mM EDTA, 5% glycerol, 0.01% Brij-35, 0.1% β-mercaptoethanol, 1mg/ml BSA.
4. Fes/Fps, active: Dilute to 4-40ng/μl with EDB. Use 2.5μl per assay.
5. Magnesium/ATP Cocktail (Catalog # 20-113): 75mM $MgCl_2$ and 500μM ATP in 20mM MOPS, pH 7.2, 25mM β-glycerol phosphate, 5mM EGTA, 1mM sodium orthovanadate, 1mM dithiothreitol.
6. [γ-$^{32}$P]ATP: Stock 1mCi/100μl (3000Ci/mmol, obtained from PerkinElmer, Catalog # LU002A). Make 10μl aliquots (100μCi/vial). Before starting the assay, dilute the Magnesium/ATP Cocktail 1:1 with sterile, distilled water. Then dilute an aliquot of [γ-$^{32}$P]ATP to 1μCi/μl with 90μl of the diluted Magnesium/ATP Cocktail. Use 10μl per assay point for a final ATP concentration of 100μM per assay point.

**Assay Protocol:**

1. Add 10μl of 2.5X Reaction Buffer per assay.
3. Add 2.5μl **of Fes/Fps, active** (10-100ng).
4. Add 10μl of the diluted [γ-$^{32}$P]ATP mixture.
5. Incubate for 10 minutes at 30˚C.
6. Spot 20μl onto the center of a 2cm x 2cm **Whatman No.1** paper circle.
7. Wash the assay squares three times for 5 minutes with 0.75% phosphoric acid.
8. Wash the assay squares once for 5 minutes with acetone.
9. Transfer the assay squares to vials and add 5ml scintillation cocktail.
10. Read in scintillation counter. Compare cpm of enzyme samples to cpm of control samples.

**Example 6n Kinase Assay Protocol - Yes**

**Upstate catalog # 14-478**

**Stock Solutions:**

1. 2.5X Reaction Buffer: 20mM MOPS, 0.5mM EDTA.
2. Enzyme Dilution Buffer (EDB): 20mM MOPS pH 7.0, 1mM EDTA, 0.01% Brij-35, 5% glycerol, 0.1% β-mercaptoethanol, 1mg/ml BSA.
3. Yes, active: Dilute to 0.4ng-4ng/μl with EDB. Use 2.5μl per assay point.
4. Poly($Glu_4$-Tyr) substrate: Prepare a 1mg/ml stock solution. Use 2.5μl per assay point for a final concentration of 0.1mg/ml per assay point.
5. [γ-$^{32}$P]ATP: Stock 1mCi/100μl (3000Ci/mmol, obtained from PerkinElmer, Cat. # BLU002A).Make 10/μl aliquots (100μCi/vial). Before starting the assay, dilute an aliquot to 1μCi/μl with 90μl of 75mM $MgCl_2$ and 500μM cold ATP in 20mM MOPS, pH 7.2, 25mM β-glycerol phosphate, 5mM EGTA, 1mM sodium orthovanadate, 1mM dithiothreitol.

**Assay Protocol:**

1. Add 10μl of 2.5X Reaction Buffer per assay.
2. Add 2.5/μl of Poly($Glu_4$-Tyr) substrate (0.1mg/ml).
3. Add 2.5μl of **Yes, active** (1ng-10ng).
4. Add 10μl of the diluted [γ-$^{32}$P]ATP solution.
5. Incubate for 10 minutes at 30°C.
6. Transfer a 20μl aliquot onto the center of a 2cm x 2cm P81 paper.
7. Wash the assay squares three times for 5 minutes with 0.75% phosphoric acid.
8. Wash the assay squares once for 5 minutes with acetone.
9. Transfer the assay squares to vial and add 5ml scintillation cocktail.
10. Read in scintillation counter. Compare cpm of enzyme samples to cpm of control samples that contain background control.

**Example 60 Kinase Assay Protocol -Blk**

**Upstate catalog # 14-517**

**Stock Solutions:**

1.10X Reaction buffer: 500mM Tris-HCl pH 7.5, 1mM EGTA, 1mM sodium orthovanadate, 1% 2-mercaptoethanol.
2. Enzyme Dilution Buffer (EDB): 50mM Tris-HCl pH 7.5, 0.1mM EGTA, 0.1mM sodium orthovanadate, 0.1% 2-mercaptoethano10.03% Brij-35.
3. Blk (human), active: Dilute to 4-40ng/μl with EDB. Use 2.5μl per assay point.
4. Poly($Glu_4$-Tyr) (4:1): Prepare a 1mg/ml stock. Use 2.5μl per assay point for a final assay concentration of 0.1mg/ml per assay point.
5. [γ-$^{32}$P]ATP: Stock 1mCi/100μl (3000Ci/mmol, obtained from PerkinElmer, Cat. # BLU002A). Make 10μl aliquots (100μCi/vial). Before starting the assay, dilute an aliquot to 1μCi/μl with 90μl of 75mM $MgCl_2$ and 500μM cold ATP in 20mM MOPS, pH 7.2, 25mM β-glycerol phosphate, 5mM EGTA, 1mM sodium orthovanadate, 1mM dithiothreitol.

**Assay Procedure:**

1. Add 2.5μl of 10X Reaction Buffer per assay.
2. Add 2.5μl (0.1mg/ml) of Poly (Glu4-Tyr) (4:1).
3. Add 2.5μl (10-100ng) of **Blk (human), active.**
4. Add 7.5μl sterile, distilled water.
5. Add 10μl of the diluted [γ-$^{32}$P]ATP solution.
6. Incubate for 10 minutes at 30°C.
7. Transfer a 20μl aliquot onto the center of a 2.5cm **Whatman No. 1** paper circle.
8. Wash the assay squares three times for 5 minutes with 0.75% phosphoric acid.
9. Wash the assay squares once for 5 minutes with acetone.
10. Transfer the assay squares to vial and add 1 ml scintillation cocktail.
11. Read in scintillation counter. Compare cpm of enzyme samples to cpm of control samples that contain background control.

**Example 6p Kinase Assay Protocol - Lyn**

**Upstate catalog # 14-510**

**Stock Solutions:**

1.10X Reaction Buffer: 500mM Tris-HCl, pH 7.5, 1mM EGTA, 1mM sodium orthovanadate, 1% 2-mercaptoethanol.
2. Enzyme Dilution Buffer (EDB): 50mM Tris-HCl, pH 7.5, 1mM EGTA, 0.1mM sodium orthovanadate, 0.03% Brij-35, 0.1% 2-mercaptoethanol, 1mg/ml BSA.

3. Lyn, active: Dilute to 2-16ng/μl with EDB. Use 2.5μl per assay point.
4. Poly($Glu_4$-Tyr) (4:1): Prepare a 1mg/ml stock. Use 2.5μl per assay point for a final concentration of 0.1mg/ml per assay point.
5. [γ-$^{32}$P]ATP: Stock 1mCi/100μl (3000Ci/mmol, obtained from PerkinElmer, Cat. # BLU002A). Make 10μl aliquots (100μCi/vial). Before starting the assay, dilute an aliquot to 1μCi/μl with 90μl of 75mM MgCl2 and 500μM cold ATP in 20mM MOPS, pH 7.2, 25mM β-glycerol phosphate, 5mM EGTA, 1mM sodium orthovanadate, 1mM dithiothreitol.

**Assay Procedure:**

1. Add 2.5μl of 10X Reaction Buffer per assay.
2. Add 2.5μl of Poly (Glu4-Tyr) (4:1).
3. Add 2.5μl **(5-40ng) Lyn, active.**
4. Add 7.5μl of sterile, distilled water.
5. Add 10μl of the diluted [γ-$^{32}$P]ATP mixture.
6. Incubate for 10 minutes at 30°C.
7. Transfer a 20μl aliquot onto the center of a 2cm x 2cm P81 paper.
8. Wash the assay squares three times for 5 minutes with 0.75% phosphoric acid.
9. Wash the assay squares once for 5 minutes with acetone.
10. Transfer the assay squares to vial and add 5ml scintillation cocktail.
11. Read in scintillation counter. Compare cpm of enzyme samples to cpm of control samples that contain background control.

**Example 6q Kinase Assay Protocol - Fyn**

**Upstate catalog # 14-441**

**Stock Solutions:**

1. Assay Buffer: 200mM Tris-HCl pH 7.5, 0.4mM EGTA, 0.4mM sodium orthovanadate.
2. Magnesium/ATP Cocktail (Catalog # 20-113): 500μM cold ATP and 75mM magnesium chloride in 20mM MOPS, pH 7.2, 25mM β-glycerol phosphate, 5mM EGTA, 1mM sodium orthovanadate, 1mM dithiothreitol.
3. [γ-$^{32}$P]ATP: Stock 1mCi/100μl (3000Ci/mmol, obtained from DuPont-NEN). Make 10μl aliquots (100μCi/vial). Before starting the assay, dilute an aliquot with 90μl of Magnesium/ATP Cocktail.
4. Enzyme Dilution Buffer: 20mM MOPS pH 7.5, 1mM EDTA, 0.01% Brij-35, 5% glycerol, 0.1% β-mercaptoethanol, 1mg/ml BSA.
5. Fyn: Dilute with Enzyme Dilution Buffer to prepare a 4ng-40ng/μl stock. Use 2.5μl per assay point.
6. Src Substrate Peptide (Catalog# 12-140): Dilute to 2.5mM with distilled water. Use 2.5μl per ssay point.

**Assay Protocol:**

1. Add 6.25μl of Assay Buffer per assay.
2. Add 2.5μl of **diluted Fyn.**
3. Add 2.5μl Src Substrate peptide (250μM).
4. Add 3.75μl distilled water.
5. Add 10μl of the [γ̃-$^{32}$P]ATP solution.
6. Incubate for 10 minutes at 30°C.
7. Transfer a 20μl aliquot onto the center of a 2cm x 2cm P81 paper.
8. Wash assay squares three times with 0.75% phosphoric acid for 5 minutes per wash.
9. Wash assay squares once with acetone for 1-2 minutes.
10. Transfer assay squares to a scintillation vial and add 1ml scintillation cocktail.
11. Read in scintillation counter. Compare cpm of enzyme samples to cpm of control samples that contain no enzyme (background control).

**Example 6r Kinase Assay Protocol - Lck**

**Upstate catalog # 14-442**

**Stock Solutions:**

1. Assay Buffer: 200mM Tris-HCl pH 7.5, 0.4mM EGTA, 0.4mM sodium orthovanadate.
2. Enzyme Dilution Buffer (EDB): 20mM MOPS, pH 7.5, 1mM EDTA, 0.01% Brij-35, 5% glycerol, 0.1% β-mercaptoethanol, 1mg/ml BSA.
3. Lck, active: Dilute to 10-100ng/μl with EDB. Use 2.5μl per assay point.
4. Src Substrate Peptide (Catalog # 12-140): Prepare a 2.5mM stock with sterile, distilled water. Use 2.5μl per assay point for a final concentration of 250μM per assay.
5. Magnesium/ATP Cocktail (Catalog # 20-113): 500μM cold ATP and 75mM magnesium chloride in 20mM MOPS, pH 7.2, 25mM β-glycerophosphate, 5mM EGTA, 1mM $Na_3VO_4$, 1mM dithiothreitol.
6. [γ-$^{32}$P]ATP: Stock 1mCi/100μl (3000Ci/mmol, obtained from PerkinElmer, Catalog # BLU002A). Make 10μl aliquots (100μCi/vial). Before starting the assay, dilute the Magnesium/ATP Cocktail 1:1 with sterile, distilled water. Then dilute an aliquot of [γ-$^{32}$P]ATP to 1μCi/μl with 90μl of the diluted Magnesium/ATP Cocktail. Use 10μl per assay point for a final ATP concentration of 100μM per assay point.

**Assay Protocol:**

1. Add 6.25μl of Assay Buffer per assay.
2. Add 2.5μl **(25-250ng) of Lck, active.**
3. Add 2.5μl Src Substrate peptide (250)μM).
4. Add 3.75μl distilled water.
5. Add 10μl of the diluted [γ-$^{32}$P]ATP solution.
6. Incubate for 10 minutes at 30˚C.
7. Transfer a 20μl aliquot onto the center of a 2cm x 2cm P81 paper.
8. Wash assay squares five times with 0.75% phosphoric acid for 5 minutes per wash.
9. Wash assay squares once with acetone for 5 minutes.
10. Transfer assay squares to a scintillation vial and add 5ml scintillation cocktail.
11. Read in scintillation counter. Compare cpm of enzyme samples to cpm of control samples that contain no substrate (background control).

**Example 6s Kinase Assay Protocol - c-Kit**

**Proquinase**

c-kit can be assayed, for example, using the PanQinase Activity Assay or ProQuinase (Freiburg, Germany). Briefly:

Assay buffer- 60 mM HEPES-NaOH, pH 7.5, 3 mM $MgCl_2$, 3 mM $MnCl_2$, 3 uM Na-orthovanadate, 1.2 mM DTT, 50 ug/ml PEG 20000, 1 uM [γ-$^{33}$P] ATP (approx. 5 x $10^5$ cpm per well)
Reaction cocktail- 20 ul assay buffer, 5 ul ATP, 5 ul test compound (in 10% DMSO), 10 ul substrate/10 ul enzyme = 100 ng (pre-mixed).

The reaction cocktails are incubated at 30˚C for 80 minutes and terminated with addition of 50 ul 2% $H_3PO_4$. The plates are aspirated and washed two times with 200 ul $H_2O$ or 200 ul NaCl. Incorporation of radiolabelled substrate is determined with a microplate scintillation counter such as described in the Technical Note above.

**Example 6t Kinase Assay Protocol - Abl T315I**

**Upstate catalog # 14-522**

**Stock Solutions:**

1. 5X Reaction Buffer: 40mM MOPS, pH 7.0, 1mM EDTA.
2. Enzyme Dilution Buffer (EDB): 20mM MOPS, pH 7.0, 1mM EDTA, 0.01% Brij-35, 5% glycerol, 0.1% β-mercaptoethanol, 1mg/ml BSA.
3. Abl, (T315I, human) active: Dilute to 4-40ng/μl with EDB. Use 2.5μl per assay point.
4. Abltide (EAIYAAPFAKKK), (Catalog # 12-493): Prepare a 500μM stock. Use 2.5μl per assay point for a final assay concentration of 50μM per assay point.
5. [$\gamma$-$^{32}$P]ATP: Stock 1mCi/100μl (3000Ci/mmol, obtained from PerkinElmer, Cat. # BLU002A). Make 10μl aliquots (100μCi/vial). Before starting the assay, dilute an aliquot to 1μCi/μl with 90μl of 75mM $MgCl_2$ and 500μM cold ATP in 20mM MOPS, pH 7.2, 25mM β-glycerol phosphate, 5mM EGTA, 1mM sodium orthovanadate, 1mM dithiothreitol.

**Assay Protocol:**

1. Add 5μl of 5X Reaction Buffer per assay.
2. Add 2.5μl of Abltide (50μM).
3. Add 2.5μl of Abl **(T315I, human), active** (10-100ng).
4. Add 5μl sterile, distilled water.
5. Add 10μl of the diluted [$\gamma$-$^{32}$P]ATP solution.
6. Incubate for 10 minutes at 30°C.
7. Transfer a 20μl aliquot onto the center of a 2cm x 2cm P81 paper.
8. Wash the assay squares three times for 5 minutes with 0.75% phosphoric acid.
9. Wash the assay squares once for 5 minutes with acetone.
10. Transfer the assay squares to vial and add 5ml scintillation cocktail.
11. Read in scintillation counter. Compare cpm of enzyme samples to cpm of control samples that contain background control.

**Equivalents**

It should be understood that the detailed description and the specific examples above, while indicating preferred embodiments of the invention, are given by way of illustration only, since various changes and modifications within the spirit and scope of the invention will become apparent to those skilled in the art from this detailed description.
The references cited hereinabove are all incorporated herein by reference.
In view of the above, it will be appreciated that the invention also encompasses the following items:

1. A method of treating a disease or disorder listed in Table I, comprising administering to a patient with said disease or disorder a therapeutically effective amount of a compound, or isomeric, prodrug, tautomeric, pharmaceutically acceptable salt, N-oxide, or stereoisomeric form thereof, having the structure of Formula I:

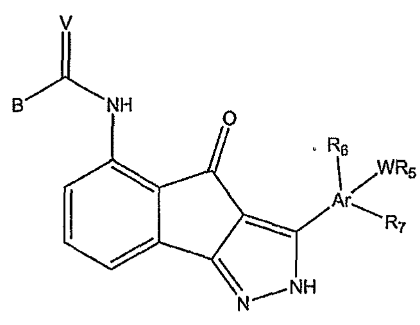

wherein

B represents $M_nR_8$;
Ar represents an aryl or heteroaryl ring;
V represents O, S, or N-CN;
W represents O, S, $S(O_2)$ C(=O), C(=S), $CH_2$, or NR";
R' represents, independently for each occurrence, H, lower alkyl, or a metal counterion;
R" represents, independently for each occurrence, H or lower alkyl;
$R_5$ represents H, P(=O)(OR')2, $M_nJK$, or $M_nQ$;
$R_6$ represents H, OH, or $M_nQ$;
$R_7$ represents H, halogen, hydroxyl, lower alkyl, or lower alkoxyl,;
$R_8$ represents substituted or unsubstituted alkyl, alkenyl, alkynyl, alkoxy, aryl, heteroaryl, cycloalkyl, heterocyclyl, or amine;
J represents C(=O), C(=S), or $SO_2$;
K represents OR', $N(R")_2$, or $N(R')SO_2R"$;
M, independently for each occurrence, represents a substituted or unsubstituted methylene group (including C (=O) and C(=S)), NR", O, S, S(O), or $S(O_2)$;
n represents an integer from 1-7 when present in B, from 0-6 when present in $R_5$, and from 1-3 when present in $R_6$; and
Q represents a substituted or unsubstituted: nitrogen-containing heteroaryl ring, secondary amino substituent, tertiary amino substituent, or nitrogen-containing heterocycle;

with the proviso that said compound is not compound A37.

2. A method of treating a disease or disorder listed in Table I, comprising administering to a patient with said disease or disorder a therapeutically effective amount of a compound, or isomeric, prodrug, tautomeric, pharmaceutically acceptable salt, N-oxide, or stereoisomeric form thereof, having the structure of Formula Ia:

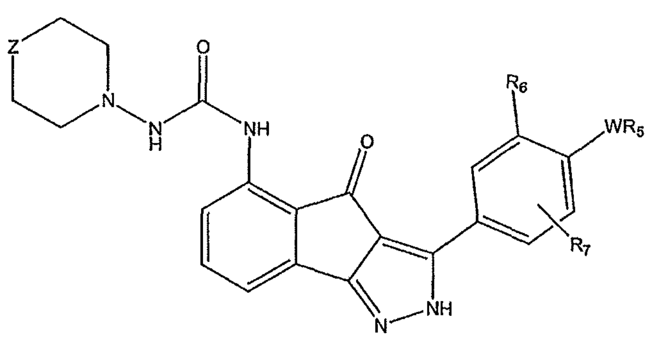

wherein

W and Z, independently, represent O or NR";
R' represents, independently for each occurrence, H, lower alkyl, or a metal counterion;
R" represents, independently for each occurrence, H or lower alkyl;
$R_5$ represents H, $P(=O)(OR')_2$,or $M_nQ$;
$R_6$ represents H, OH, or $M_nQ$;
$R_7$, independently for each occurrence, represents hydrogen, halogen, lower alkyl, or lower alkoxyl;
M, independently for each occurrence, represents a substituted or unsubstituted methylene group (including C (=S) and C(=O)), NR", O, S, S(O), or $S(O_2)$;
n represents an integer from 1-5; and
Q represents a nitrogen-containing heteroaryl ring, a tertiary amino substituent, or a substituted or unsubstituted nitrogen-containing heterocycle;
with the proviso that said compound is not compound A37.

3. The method of item 1, wherein

B represents $M_nR_8$;
Ar represents an aryl or heteroaryl ring;
V represents O, S, or N-CN;
W represents C(=O), C(=S), $SO_2$, or $CH_2$;
R' represents, independently for each occurrence, H, lower alkyl, or a metal counterion;
R" represents, independently for each occurrence, H or lower alkyl;
$R_5$ represents H, P(=O)(OR')$_2$, $M_nJK$, or $M_nQ$;
$R_6$ represents H, OH, or $M_nQ$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$;
K represents OR', N(R")$_2$, or N(R')$SO_2$R";
M, independently for each occurrence, represents a substituted or unsubstituted methylene group (including C (=S) and C(=O)), NR", O, S, S(O), or S($O_2$);
n represents an integer from 1-4 when present in B, from 0-6 when present in $R_5$, and from 1-3 when present in $R_6$; and
Q represents a substituted or unsubstituted: nitrogen-containing heteroaryl ring, secondary amino substituent, tertiary amino substituent, or nitrogen-containing heterocycle.

4. The method of item 1, wherein

B represents $M_nR_8$;
Ar represents an aryl or heteroaryl ring;
V represents O, S, or N-CN;
W represents O, S, S($O_2$), C(=O), C(=S), $CH_2$, or NR";
R' represents, independently for each occurrence, H, lower alkyl, a metal counterion, or alkaline earth metal counterion;
R" represents, independently for each occurrence, H or lower alkyl;
$R_5$ represents H, P(=O)(OR')$_2$, $M_nJK$, or $M_nQ$;
$R_6$ represents H, OH, or $M_nQ$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$;
K represents OR', N(R")$_2$, or N(R')$SO_2$R";
M, independently for each occurrence, represents a substituted or unsubstituted methylene group (including C (=S) and C(=O)), NR", O, S, S(O), or S($O_2$)
n represents an integer from 1-4 when present in B, from 0-6 when present in $R_5$, and from 1-3 when present in $R_6$; and
Q represents a substituted or unsubstituted: nitrogen-containing heteroaryl ring or secondary amino substituent.

5. The method of item 1, wherein

B represents $M_nR_8$;
Ar represents an aryl or heteroaryl ring;
V represents O, S, or N-CN;
W represents O, S, S($O_2$) C(=O), C(=S), $CH_2$, or NR";
R' represents, independently for each occurrence, H, lower alkyl, or a metal counterion;
R" represents, independently for each occurrence, H or lower alkyl;
$R_5$ represents $M_nJK$;
$R_6$ represents H, OH, or $M_nQ$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine.
J represents C(=O), C(=S), or $SO_2$;
K represents OR', N(R")$_2$, or N(R')$SO_2$R";
M, independently for each occurrence, represents a substituted or unsubstituted methylene group (including C(=S) and C(=O)), NR", O, S, S(O), or $S(O_2)$;
n represents an integer from 1-4 when present in B, from 0-6 when present in $R_5$ and from 1-3 when present in $R_6$; and
Q represents a substituted or unsubstituted: nitrogen-containing heteroaryl ring, secondary amino substituent, tertiary amino substituent, or nitrogen-containing heterocycle.

6. The method any of items 1-3, wherein $R_5$ represents $M_nQ$ and Q represents a substituted or unsubstituted: nitrogen-containing heteroaryl ring, tertiary amino substituent, or nitrogen-containing heterocycle.

7. The method of item 6, wherein Q represents a substituted or unsubstituted tertiary amino group.

8. The method of item 6, wherein Q represents a substituted or unsubstituted nitrogen-containing heterocycle.

9. The method of any of items 1, 3 and 4, wherein $R_5$ represents $M_nQ$ and Q represents a substituted or unsubstituted secondary amino group.

10. The method of any of items 1-5, wherein $R_5$ represents $M_nQ$ and Q is a substituted or unsubstituted-nitrogen-containing heteroaryl ring.

11. The method of any of items 1 and 3-5, wherein $R_8$ represents substituted or unsubstituted morpholino, piperazinyl, or cyclohexyl.

12. The method of any of items 1-5, wherein R" represents H.

13. The method of any of items 1 and 3-5, wherein W represents $CH_2$.

14. The method of any of items 1-5, wherein M when attached to Q is $CH_2$, $S(O_2)$, C(=S), or C(=O).

15. The method of item 14, wherein M, when attached to Q, is $CH_2$.

16. The method of item 6, wherein V is O, $M_n$ in B represents NH, and $R_8$ has the structure:

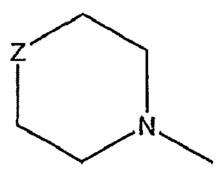

where Z represents O or NR".

17. The method of item 16, wherein Ar represents a phenyl ring and $F_6$ and $R_7$ represent H for all occurrences ring.

18. The method of any of items 1-5, wherein substituents include, independently for each occurrence, alkyl, oxo, acyl amino, hydroxyl, carbonyl, sulfonyl, ester, amide, $N(R")_2$, hydroxy alkyl, alkoxy alkyl, aryl, heterocyclyl, cycloalkyl, or oligo(ethylene glycol).

19. The method of item 1, wherein the compound is selected from A47, A49, A51, and A82.

20. The method of item 2, wherein $R_6$ represents H and $R_7$ represents a methyl or methoxy substituent ortho to W.

21. The method of item 2 or 20, wherein Q in Formula Ia represents a nitrogen-containing heteroaryl ring, a tertiary amino substituent, or a substituted or unsubstituted nitrogen-containing heterocycle.

22. A method of treating a disease or disorder listed in Table I, comprising administering to a patient with said disease or disorder a therapeutically effective amount of a compound, or a prodrug, isomeric, tautomeric, pharmaceutically acceptable salt, N-oxide, or stereoisomeric form thereof, having a structure of Formula II:

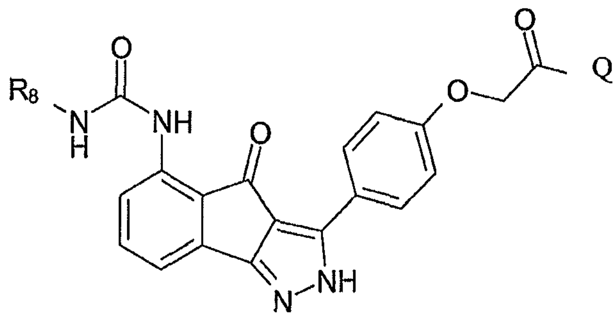

wherein

$R_8$ represents a substituted or unsubstituted heterocycle; and
Q represents a substituted or unsubstituted: nitrogen-containing heteroaryl ring, tertiary amino substituent, or nitrogen-containing heterocycle;
with the proviso that said compound is not compound A37.

23. The method of item 22, wherein $R_8$ represents a substituted or unsubstituted morpholino or piperazinyl ring.

24. The method of item 22, wherein Q represents substituted or unsubstituted: piperazine, morpholine, piperidine, pyridine, pyrrole, oxazole, isoxazole, imidazole, or pyrazole.

25. The method of item 2, wherein the compound is selected from A34, A36, A44, A46, A76, A77, A78, A79, A80, and A81.

26. A method of treating a disease or disorder listed in Table I, comprising administering to a patient with said disease or disorder a therapeutically effective amount of a compound, or a prodrug, isomeric, tautomeric, pharmaceutically acceptable salt, N-oxide, or stereoisomeric form thereof, having a structure of Formula III:

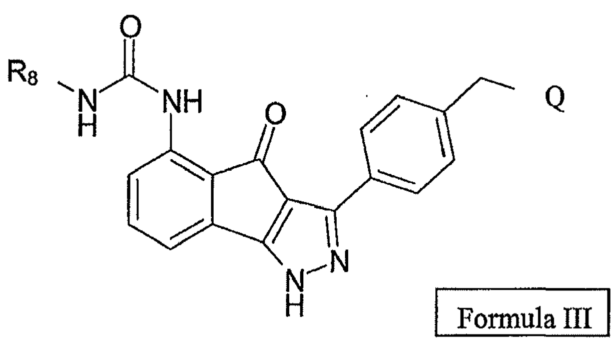

Formula III

wherein

$R_8$ represents a substituted or unsubstituted heterocycle;
Q represents a substituted or unsubstituted secondary amino substituent, tertiary amino substituent, or substituted or unsubstituted nitrogen-containing heterocycle;

27. The method of item 26, wherein $R_8$ represents a morpholino or piperazine ring.

28. The method of item 26, wherein Q represents pyrrolidine.

29. The method of item 26, wherein the compound is selected from: A47, A49, A51, A82, B1, B2, B3, B4, B5, B6, B7, B8, B9, B10, B11, B12, B13, B14, B15, B16, B17, B18, B19, B20 and C2.

30. The method of item 26, wherein the compound is B16.

31. The method of any of items 22-24, wherein substituents are selected from independently for each occurrence, alkyl, oxo, hydroxyl, alkoxy, hydroxy-alkoxy, carbonyl, sulfonyl, ester, amide, $N(R'')_2$, alkyl halide, acyl amino, or substituted or unsubstituted aryl, heteroaryl, heterocyclyl, cycloalkyl, and oligo(ethylene glycol).

32. A pharmaceutical composition for treating at least one disease or disorder listed in Table I, comprising a pharmaceutically acceptable excipient and a compound as recited in any one of items 1-5, 22, 26 and 30.

33. The use of a compound as recited in any of items 1-5, 22, 26 and 30, for the manufacture of a medicament for treating at least one disease or disorder listed in Table I.

34. The method of any of items 1-5, 22, 26, and 30, wherein said disease is: CML, ALL, GIST, inflammation, Alzheimers or Parkinson's disease.

35. A packaged pharmaceutical comprising a pharmaceutical composition of a compound as recited in any of items 1-5, 22, 26 and 30, or an isomeric, prodrug, tautomeric, pharmaceutically acceptable salt, N-oxide, or stereoisomeric form thereof, wherein said packaged pharmaceutical further comprises instructions to administer an effective amount of the pharmaceutical composition to an individual suffering from at least one disease or disorder in Table I.

36. A method of killing or inhibiting the growth of a cell from a cancer or a tumor resistant to a therapeutic agent, comprising exposing said cell to an effective amount of compound A37 or of a compound as recited in any of items 1-5, 22, 26 and 30, or an isomeric, prodrug, tautomeric, pharmaceutically acceptable salt, N-oxide, or stereoisomeric form thereof.

37. The method of item 36, wherein said resistance of the cancer or tumor is mediated by a kinase, or the proliferation and/or progression of said cancer or tumor is dependent on increased activity of said kinase, and wherein said kinase is: GSK3β, CK1, MEK1, MKK6, JNK (*e.g.*, 1a1, 2a2, or 3, etc.), AMPK, Rsk (*e.g.*, Rsk1 - Rsk4, RskB), c-Abl, Bcr-Ab1, Bcr-Ab1 T315I, c-Src and the related v-Src, c-Yes, v-Yes, Fyn, Lyn, Lck, Blk, Hck, c-Fgr, v-Fgr, p56Lck, TkI, Csk, Ctk or Yrk, Fes (*e.g.* c-fes/fps, v- fes/fps, p94-c-fes-related protein, and Fer), or c-Kit.

38. The method of item 36, wherein said compound is **A37** or **B16,** or a pharmaceutically acceptable salt, isomer or prodrug thereof.

39. The method of any one of items 36 - 38, wherein the resistance of said tumor cell to said therapeutic agent is mediated by multidrug resistance.

40. The method of item 39, wherein the resistance of said tumor cell is mediated through an ATP-binding cassette (ABC) transporter.

41. The method of item 40, wherein the ATP-binding cassette transporter is P-glycoprotein.

42. The method of item 41, wherein the therapeutic agent is selected from: vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puxomycin), toxic peptides (valinomycin), topoisomerase I inhibitors (topotecan), DNA intercalators (ethidium bromide) and anti-mitotics.

43. The method of any of items 36 - 38, wherein the resistance of said tumor cell to a therapeutic agent is mediated through tubulin.

44. The method of item 43, wherein the therapeutic agent is selected from: taxanes (paclitaxel, docetaxel and taxol derivatives), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine and nocodazole.

45. The method of any of items 36 - 38, wherein the resistance of said tumor cell to a therapeutic agent is mediated through topoisomerase I.

46. The method of item 45, wherein the therapeutic agent is selected from: camptothecin, 9-nitrocamptothecin (Orethecin, rubitecan), 9-aminocamptothecin (IDEC-13'), exatecan (DX-8951f), lurtotecan (GI-147211C), BAY

38-3441, the homocamptothecins such as diflomotecan (BN-80915) and BN-80927, topotecan (Hycamptin), NB-506, J107088, pyrazolo [1,5-a] indole derivatives, such as GS-5, lamellarin D and irinotecan (Camptosar, CPT-11).

47. The method of any of item 36 - 38, wherein said tumor cell is resistant to paclitaxel, docetaxol, adriamycin, camptothecin, Mitoxanthrone, platinum-based compound, or GLEEVEC®.

48. The method of item 47, wherein said platinum-based compound is carboplatin, cisplatin, oxaliplatin, iproplatin, tetraplatin, lobaplatin, DCP, PLD-147, JM118, JM335, or satraplatin.

49. The method of any of items 36 - 38, wherein said tumor cell comprises or is derived from a solid tumor.

50. The method of item 49, wherein said solid tumor is: breast cancer, cervical cancer, colorectal cancer, peritoneal cancer, ovarian cancer, bronchial cancer, small cell lung cancer, non-small cell lung cancer, gastric, prostate, head and neck cancer, or metastases thereof.

51. The method of any of items 36 - 38, wherein said tumor cell comprises or is derived from blood cells, including cells of leukemia, lymphoma, Hodgkin's disease, or non-Hodgkin's lymphoma.

52. The method of item 51, wherein said leukemia is CML or ALL.

53. The method of any of items 36 - 38, wherein said tumor cell is a sarcoma cell, preferably a GIST cell.

54. The method of any of items 36 - 38, wherein the resistance of said tumor cell is mediated by one or more mutations of a kinase.

55. The method of item 54, wherein said kinase is c-kit or bcr-abl.

56. The method of any of items 36 - 38, wherein said tumor cell comprises or is derived from a hematological tumor, such as CML or ALL.

57. The method of any of items 36 - 38, wherein said compound is administered to an individual suffering from a cancer or tumor refractory to or previously treated with said therapeutic agent.

58. The method of item 57, wherein said therapeutic agent is paclitaxel, Taxotere, Camptothecin, Cisplatin, Mitoxanthrone or GLEEVEC®.

59. The method of item 57, wherein said individual is further administered with one or more anti-emetic or anti-diarrheal agents.

60. The method of item 57, wherein said individual is further administered with one or more other anti-cancer therapeutic agents.

61. A method for treating an individual with a cancer or a tumor resistant or refractory to a therapeutic agent, comprising administering to the individual an effective amount of compound A37 or of a compound as recited in any of items 1-5, 22, 26, and 30, or an isomeric, prodrug, tautomeric, pharmaceutically acceptable salt, N-oxide, or stereoisomeric form thereof.

62. The method of item 61, wherein said compound is **B16,** or a pharmaceutically acceptable salt, isomer or prodrug thereof.

63. The method of item 61 or 62, wherein the resistance of said tumor to said therapeutic agent is mediated by multidrug resistance.

64. The method of item 61 or 62, wherein the resistance of said tumor is mediated through an ATP-binding cassette (ABC) transporter.

65. The method of item 64, wherein the ATP-binding cassette transporter is P-glycoprotein.

66. The method of item 65, wherein the therapeutic agent is selected from: vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide) and anti-mitotics.

67. The method of item 61 or 62, wherein the resistance of said tumor is mediated through tubulin.

68. The method of item 67, wherein the therapeutic agent is selected from: taxanes (paclitaxel, docetaxel and taxol derivatives), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine and nocodazole.

69. The method of item 61 or 62, wherein the resistance of said tumor is mediated through topoisomerase I.

70. The method of item 69, wherein the therapeutic agent is selected from: camptothecin, 9-nitrocamptothecin (Orethecin, rubitecan), 9-aminocamptothecin (IDEC-13'), exatecan (DX-8951f), lurtotecan (GI-147211C), BAY 38-3441, the homocamptothecins such as diflomotecan (BN-80915) and BN-80927, topotecan (Hycamptin), NB-506, J107088, pyrazolo [1,5-a] indole derivatives, such as GS-5, lamellarin D and irinotecan (Camptosar, CPT-11).

71. The method of item 61 or 62, wherein said tumor is resistant or refractory to paclitaxel, docetaxol, adriamycin, camptothecin, Mitoxanthrone, platinum-based compound, or GLEEVEC®.

72. The method of item 71, wherein said platinum-based compound is carboplatin, cisplatin, oxaliplatin, iproplatin, tetraplatin, lobaplatin, DCP, PLD-147, JM118, JM335, or satraplatin.

73. The method of item 61 or 62, wherein said tumor comprises a solid tumor.

74. The method of, item 73, wherein said solid tumor is: liver cancer, stomach cancer, colon cancer, breast cancer, pancreas cancer, prostate cancer, skin cancer, cervical cancer, ovarian cancer, testicular cancer, lung cancer, head and neck cancer, or metastases thereof.

75. The method of item 1 or 62, wherein said tumor comprises or is derived from blood cells, including cells of leukemia, lymphoma, Hodgkin's disease, or non-Hodgkin's lymphoma.

76. The method of item 75, wherein said leukemia is CML or ALL.

77. The method of item 61 or 62, wherein said tumor is a sarcoma, preferably GIST.

78. The method of item 61 or 62, wherein the resistance of said tumor is mediated by one or more mutations of said kinase.

79. The method of item 78, wherein said kinase is c-Kit or Bcr-Abl.

80. The method of item 1 or 62, wherein said tumor comprises a haematological tumor.

81. The method of item 61 or 62, wherein said compound is administered to an individual previously treated with said therapeutic agent.

82. The method of item 81, wherein said individual is further administered with one or more anti-emetic or anti-diarrheal agents.

83. The method of item 81, wherein said individual is further administered with one or more other anti-cancer therapeutic agents.

84. A packaged pharmaceutical comprising a pharmaceutical composition of compound A37 or of a compound as recited in any of item 1-5, 22, 26, and 30, or an isomeric, prodrug, tautomeric, pharmaceutically acceptable salt, N-oxide, or stereoisomeric form thereof, wherein said packaged pharmaceutical further comprises instructions to administer an effective amount of the pharmaceutical composition to an individual suffering from a disease resistant

or refractory to a therapeutic agent.

85. The packaged pharmaceuticals of item 84, further comprising an anti-emetic or anti-diarrheal therapeutic composition and/or instructions to further administer an effective amount of the anti-emetic or anti-diarrheal therapeutic composition to said individual.

86. A pharmaceutical composition for use in treating a cancer or a tumor resistant or refractory to a therapeutic agent, the pharmaceutical composition comprising a compound together with a pharmaceutically acceptable carrier, diluent or vehicle, wherein the compound is compound A37 or a compound recited in any of items 1-5, 22, 26, and 30, or an isomeric, prodrug, tautomeric, pharmaceutically acceptable salt, N-oxide, or stereoisomeric form thereof.

87. A method for treating an individual suffering from a cancer or tumor resistant or refractory to a taxane-based therapeutic agent, comprising administering to the individual an effective amount **of A37** or **B16,** or a pharmaceutically acceptable salt, isomer or prodrug thereof.

88. A method for treating an individual suffering from a cancer or tumor resistant or refractory to a platinum-based therapeutic agent, comprising administering to the individual an effective amount **of A37** or **B16,** or a pharmaceutically acceptable salt, isomer or prodrug thereof.

89. An article of manufacture comprising a pharmaceutical composition and a label which indicates that said pharmaceutical composition can be used for the treatment of an individual suffering from a cancer or tumor resistant or refractory to a chemotherapeutic agent, wherein said pharmaceutical composition comprises a compound together with a pharmaceutically acceptable carrier, diluent or vehicle, wherein the compound is compound A37 or a compound as recited in any of items 1-5, 22, 26, and 30, or an isomeric, prodrug, tautomeric, pharmaceutically acceptable salt, N-oxide, or stereoisomeric form thereof.

90. The article of item 89, further comprising packaging material, wherein said pharmaceutical composition is contained within said packaging, or wherein said label is contained in or is comprised by said packaging.

91. The article of item 89, further comprising an anti-emetic or anti-diarrheal agent, or wherein said label further indicates that an anti-emetic or anti-diarrheal agent is to be further administered with said pharmaceutical composition.

92. A method for treating an individual with a disease condition associated with a mutant kinase, comprising administering to said individual an effective amount of compound A37 or of a compound as recited in any of items 1-5, 21, 26, and 30, or an isomeric, prodrug, tautomeric, pharmaceutically acceptable salt, N-oxide, or stereoisomeric form thereof.

93. The method of item 92, wherein said disease condition is cancer or tumor.

94. The method of item 93, wherein said cancer is Chronic Myeloid Leukemia (CML) or Gastrointestinal Stromal Tumor (GIST).

95. The method of item 92, wherein said mutant kinase has an altered sequence in its catalytic domain and/or substrate / drug binding domain and/or regulatory domain.

96. The method of item 95, wherein said kinase is Bcr-Abl with one or more point mutations at residue 253, 255, 315 or 351.

97. The method of item 96, wherein said point mutations include residue 315, preferably a T315I mutation.

98. The method of item 95, wherein said kinase is c-Kit.

99. The method of item 96, wherein said individual has previously been treated for said disease condition by therapeutic agents other than said compound.

100. The method of item 99, wherein said therapeutic agents other than said compound is GLEEVEC®.

101. A packaged pharmaceutical comprising a pharmaceutical composition of compound A37 or of a compound as recited in any of items 1-5, 21, 26, and 30, or an isomeric, prodrug, tautomeric, pharmaceutically acceptable salt, N-oxide, or stereoisomeric form thereof, wherein said packaged pharmaceutical further comprises instructions to administer an effective amount of the pharmaceutical composition to an individual suffering from a disease associated with a mutant kinase.

102. The packaged pharmaceutical of item 101, wherein said mutant kinase is a mutant form of Bcr-Ab1 kinase, or a c-Kit kinase.

103. The packaged pharmaceutical of item 102, wherein said mutant form of Bcr-Abl kinase contains a T315I mutation.

104. The packaged pharmaceutical of item 101, wherein said disease is CML, preferably relapsed CML.

## Claims

**1.** A compound of Formula I:

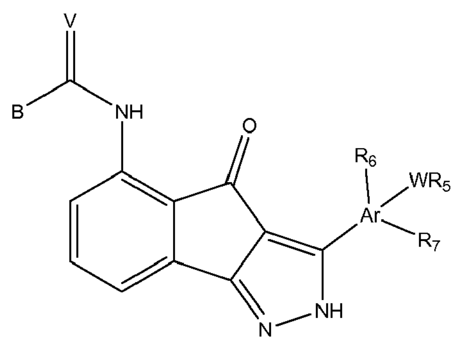

or isomeric, prodrug, tautomeric, pharmaceutically acceptable salt, N-oxide, or stereoisomeric form thereof, wherein

B represents $M_nR_8$;
Ar represents an aryl or heteroaryl ring;
V represents O, S, or N-CN;
W represents O, S, $S(O_2)$, C(=O), C(=S), $CH_2$, or NR";
R' represents, independently for each occurrence, H, lower alkyl, or a metal counterion;
R" represents, independently for each occurrence, H or lower alkyl;
$R_5$ represents H, P(=O)(OR')2, $M_nJK$, or $M_nQ$;
$R_6$ represents H, OH, or $M_nQ$;
$R_7$ represents H, halogen, hydroxyl, lower alkyl, or lower alkoxyl;
$R_8$ represents substituted or unsubstituted alkyl, alkenyl, alkynyl, alkoxy, aryl, heteroaryl, cycloalkyl, heterocyclyl, or amine;
J represents C(=O), C(=S), or $SO_2$
K represents OR', $N(R")_2$, or $N(R')SO_2R"$;
M, independently for each occurrence, represents a substituted or unsubstituted methylene group (including C (=O) and C(=S)), NR", O, S, S(O), or S($O_2$
n represents an integer from 1-7 when present in B, from 0-6 when present in $R_5$, and from 1-3 when present in $R_6$; and
Q represents a substituted or unsubstituted: nitrogen-containing heteroaryl ring, secondary amino substituent, tertiary amino substituent, or nitrogen-containing heterocycle;

for use in the treatment of a solid tumor;
with the proviso that said compound is not compound A37.

**2.** The compound of claim 1, wherein the compound has a structure of Formula Ia:

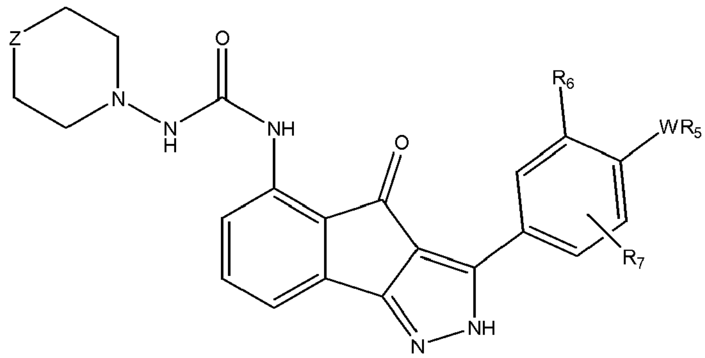

or isomeric, prodrug, tautomeric, pharmaceutically acceptable salt, N-oxide, or stereoisomeric form thereof, wherein

W and Z, independently, represent O or NR";
R' represents, independently for each occurrence, H, lower alkyl, or a metal counterion;
R" represents, independently for each occurrence, H or lower alkyl;
$R_5$ represents H, P(=O)(OR')2, or $M_nQ$;
$R_6$ represents H, OH, or $M_nQ$;
$R_7$, independently for each occurrence, represents hydrogen, halogen, lower alkyl, or lower alkoxyl;
M, independently for each occurrence, represents a substituted or unsubstituted methylene group (including C(=S) and C(=O)), NR", O, S, S(O), or $S(O_2)$;
n represents an integer from 1-5; and
Q represents a nitrogen-containing heteroaryl ring, a tertiary amino substituent, or a substituted or unsubstituted nitrogen-containing heterocycle;

optionally wherein $R_6$ represents H and $R_7$ represents a methyl or methoxy substituent ortho to W;
with the proviso that said compound is not compound **A37.**

**3.** The compound of claim 1, wherein

a) B represents $M_nR_8$;

Ar represents an aryl or heteroaryl ring;
V represents O, S, or N-CN;
W represents C(=O), C(=S), $SO_2$ or $CH_2$;
R' represents, independently for each occurrence, H, lower alkyl, or a metal counterion;
R" represents, independently for each occurrence, H or lower alkyl;
$R_5$ represents H, P(=O)(OR')2, $M_nJK$, or $M_nQ$;
$R_6$ represents H, OH, or $M_nQ$;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$
K represents OR', $N(R")_2$, or $N(R')SO_2R"$;
M, independently for each occurrence, represents a substituted or unsubstituted methylene group (including C(=S) and C(=O)), NR", O, S, S(O), or $S(O_2)$;
n represents an integer from 1-4 when present in B, from 0-6 when present in $R_5$, and from 1-3 when present in $R_6$; and
Q represents a substituted or unsubstituted: nitrogen-containing heteroaryl ring, secondary amino substituent, tertiary amino substituent, or nitrogen-containing heterocycle;

b) B represents $M_nR_8$;

Ar represents an aryl or heteroaryl ring;
V represents O, S, or N-CN;
W represents O, S, $S(O_2)$, C(=O), C(=S), $CH_2$, or NR";
R' represents, independently for each occurrence, H, lower alkyl, a metal counterion, or alkaline earth metal counterion;
R" represents, independently for each occurrence, H or lower alkyl;
$R_5$ represents H, P(=O)(OR')2, $M_n$JK, or $M_n$Q;
$R_6$ represents H, OH, or $M_n$Q;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$
K represents OR', NR", or N(R')$SO_2$R".
M, independently for each occurrence, represents a substituted or unsubstituted methylene group (including C(=S) and C(=O)), NR", O, S, S(O), or S($O_2$
n represents an integer from 1-4 when present in B, from 0-6 when present in $R_5$, and from 1-3 when present in $R_6$; and
Q represents a substituted or unsubstituted: nitrogen-containing heteroaryl ring or secondary amino substituent; or

c) B represents $M_nR_8$;

Ar represents an aryl or heteroaryl ring;
V represents O, S, or N-CN;
W represents O, S, $S(O_2)$, C(=O), C(=S), $CH_2$, or NR";
R' represents, independently for each occurrence, H, lower alkyl, or a metal counterion;
R" represents, independently for each occurrence, H or lower alkyl;
$R_5$ represents $M_n$JK;
$R_6$ represents H, OH, or $M_n$Q;
$R_7$ represents H, halogen, hydroxyl, lower alkyl or lower alkoxyl;
$R_8$ represents substituted or unsubstituted alkyl, alkenyl, alkynyl, alkoxy, aryl, heteroaryl, cyclo-alkyl, heterocyclyl, or amine;
J represents C(=O), C(=S), or $SO_2$
K represents OR', N(R")$_2$, or N(R')$SO_2$R";
M, independently for each occurrence, represents a substituted or unsubstituted methylene group (including C(=S) and C(=O)), NR", O, S, S(O), or $S(O_2)$;
n represents an integer from 1-4 when present in B, from 0-6 when present in $R_5$ and from 1-3 when present in $R_6$; and
Q represents a substituted or unsubstituted: nitrogen-containing heteroaryl ring, secondary amino substituent, tertiary amino substituent, or nitrogen-containing heterocycle.

**4.** The compound of any of claims 1-3, wherein

a) $R_5$ represents $M_n$Q and Q represents a substituted or unsubstituted secondary amino group;
b) $R_5$ represents $M_n$Q and Q is a substituted or unsubstituted nitrogen-containing heteroaryl ring;
c) $R_8$ represents substituted or unsubstituted morpholino, piperazinyl, or cyclohexyl;
d) R" represents H;
e) W represents $CH_2$;
f) M when attached to Q is $CH_2$, $S(O_2)$, C(=S), or C(=O), optionally $CH_2$; or
g) substituents include, independently for each occurrence, alkyl, oxo, acyl amino, hydroxyl, carbonyl, sulfonyl, ester, amide, (NR")$_2$, hydroxy alkyl, alkoxy alkyl, aryl, heterocyclyl, cycloalkyl, or oligo(ethylene glycol).

**5.** The compound of any of claims 1-3, wherein $R_5$ represents $M_n$Q and Q represents a substituted or unsubstituted: nitrogen-containing heteroaryl ring, tertiary amino substituent, or nitrogen-containing heterocycle; optionally wherein

a) Q represents a substituted or unsubstituted tertiary amino group;
b) Q represents a substituted or unsubstituted nitrogen-containing heterocycle; or
c) V is O, $M_n$ in B represents NH, and $R_8$ has the structure:

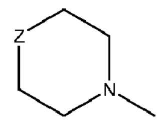

where Z represents O or NR", optionally wherein Ar represents a phenyl ring and $R_6$ and $R_7$ represent H for all occurrences ring.

**6.** A compound of Formula II:

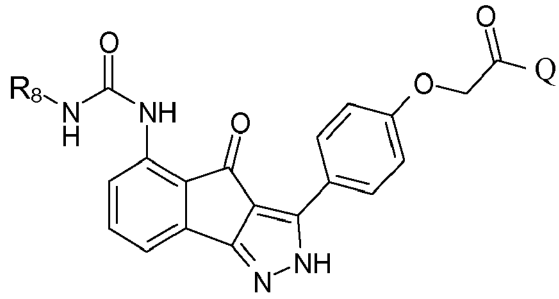

or a prodrug, isomeric, tautomeric, pharmaceutically acceptable salt, N-oxide, or stereoisomeric form thereof, wherein

$R_8$ represents a substituted or unsubstituted heterocycle; and
Q represents a substituted or unsubstituted: nitrogen-containing heteroaryl ring, tertiary amino substituent, or nitrogen-containing heterocycle, for use in the treatment of a solid tumor,

with the proviso that said compound is not compound **A37;**
optionally wherein

a) $R_8$ represents a substituted or unsubstituted morpholino or piperazinyl ring;
b) Q represents substituted or unsubstituted: piperazine, morpholine, piperidine, pyridine, pyrrole, oxazole, isoxazole, imidazole, or pyrazole; or
c) the compound is selected from A34, A36, A44, A46, A76, A77, A78, A79, A80, or A81.

**7.** A compound of Formula III:

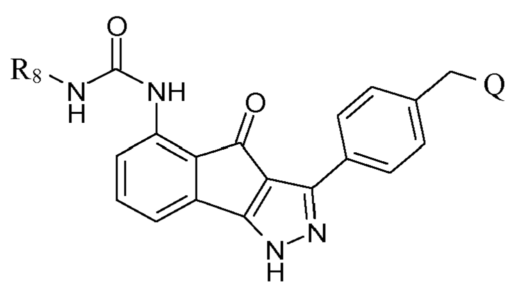

or a prodrug, isomeric, tautomeric, pharmaceutically acceptable salt, N-oxide, or stereoisomeric form thereof, wherein

$R_8$ represents a substituted or unsubstituted heterocycle;
Q represents a substituted or unsubstituted secondary amino substituent, tertiary amino substituent, or substituted or unsubstituted nitrogen-containing heterocycle,

for use in the treatment of a solid tumor.

**8.** The compound of claim 7, wherein

a) $R_8$ represents a morpholino or piperazine ring;
b) Q represents pyrrolidine; or
c) the compound is selected from A47, A49, A51, A82, B1, B2, B3, B4, B5, B6, B7, B8, B9, B10, B11, B12, B13, B14, B15, B16, B17, B18, B19, B20, or C2, optionally B 16.

**9.** The compound of claim 6, wherein substituents are selected from independently for each occurrence, alkyl, oxo, hydroxyl, alkoxy, hydroxy-alkoxy, carbonyl, sulfonyl, ester, amide, $(NR'')_2$, alkyl halide, acyl amino, or substituted or unsubstituted aryl, heteroaryl, heterocyclyl, cycloalkyl, or oligo(ethylene glycol).

**10.** A pharmaceutical composition for use in the treatment of a solid tumor, wherein

a) the pharmaceutical composition comprises a pharmaceutically acceptable excipient and a compound as recited in any one of claims 1-9; or
b) the solid tumor is resistant or refractory to a therapeutic agent, and the pharmaceutical composition comprises a compound together with a pharmaceutically acceptable carrier, diluent or vehicle, wherein the compound is compound A37 or a compound recited in any of claims 1-9, or an isomeric, prodrug, tautomeric, pharmaceutically acceptable salt, N-oxide, or stereoisomeric form thereof.

**11.** A packaged pharmaceutical comprising

a) a pharmaceutical composition of a compound as recited in any of claims 1-9, or an isomeric, prodrug, tautomeric, pharmaceutically acceptable salt, N-oxide, or stereoisomeric form thereof, wherein said packaged pharmaceutical further comprises instructions to administer an effective amount of the pharmaceutical composition to an individual suffering from a solid tumor;
b) a pharmaceutical composition of compound A37 or of a compound as recited in any of claims 1-9, or an isomeric, prodrug, tautomeric, pharmaceutically acceptable salt, N-oxide, or stereoisomeric form thereof, wherein said packaged pharmaceutical further comprises instructions to administer an effective amount of the pharmaceutical composition to an individual suffering from a solid tumor resistant or refractory to a therapeutic agent, optionally further comprising an anti-emetic or anti-diarrheal therapeutic composition and/or instructions to further administer an effective amount of the anti-emetic or anti-diarrheal therapeutic composition to said individual; or
c) a pharmaceutical composition of compound A37 or of a compound as recited in any of claims 1-9, or an isomeric, prodrug, tautomeric, pharmaceutically acceptable salt, N-oxide, or stereoisomeric form thereof, wherein said packaged pharmaceutical further comprises instructions to administer an effective amount of the pharmaceutical composition to an individual suffering from a disease associated with a mutant kinase, optionally wherein

aa) said mutant kinase is a mutant form of Bcr-Abl kinase, or a c-Kit kinase, optionally wherein said mutant form of Bcr-Abl kinase contains a T315I mutation, or
bb) said disease is CML, optionally relapsed CML.

**12.** A method of killing or inhibiting the growth of a cell from a solid tumor resistant to a therapeutic agent, comprising exposing said cell to an effective amount of compound A37 or of a compound as recited in any of claims 1-9, or an isomeric, prodrug, tautomeric, pharmaceutically acceptable salt, N-oxide, or stereoisomeric form thereof, optionally wherein

a) said resistance of the cancer or tumor is mediated by a kinase, or the proliferation and/or progression of said cancer or tumor is dependent on increased activity of said kinase, and wherein said kinase is: GSK3β, CKl, MEK1, MKK6, JNK (e.g., 1a1, 2a2, or 3, etc.), AMPK, Rsk (e.g., Rskl - Rsk4, RskB), c-Abl, Bcr-Abl, Bcr-Abl T315I, c-Src and the related v-Src, c-Yes, v-Yes, Fyn, Lyn, Lck, Blk, Hck, c-Fgr, v-Fgr, p56Lck, Tkl, Csk, Ctk or Yrk, Fes (e.g. c-fes/fps, v- fes/fps, p94-c-fes-related protein, and Fer), or c-Kit; or
b) said compound is

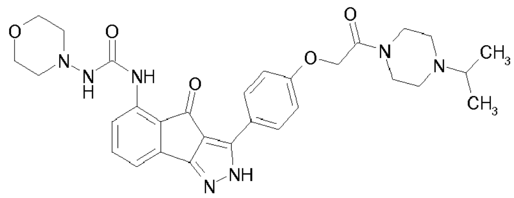

(A37)

or

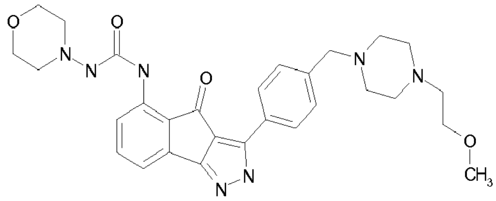

(B16),

or a pharmaceutically acceptable salt, isomer or prodrug thereof.

**13.** The compound of any one of claims 1-9 or the method of claim 12, wherein

a) the resistance of said tumor cell to said therapeutic agent is mediated by multidrug resistance,
optionally wherein the resistance of said tumor cell is mediated through an ATP-binding cassette (ABC) transporter, optionally wherein the ATP-binding cassette transporter is P-glycoprotein,
further optionally wherein the therapeutic agent is selected from: vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide) or anti-mitotics;
b) the resistance of said tumor cell to a therapeutic agent is mediated through tubulin,
optionally wherein the therapeutic agent is selected from: taxanes (paclitaxel, docetaxel and taxol derivatives), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine or nocodazole;
c) the resistance of said tumor cell to a therapeutic agent is mediated through topoisomerase I,
optionally wherein the therapeutic agent is selected from: camptothecin, 9-nitrocamptothecin (Orethecin, rubitecan), 9-aminocamptothecin (IDEC-13'), exatecan (DX-8951f), lurtotecan (GI-147211C), BAY 38-3441, the homocamptothecins such as diflomotecan (BN-80915) and BN-80927, topotecan (Hycamptin), NB-506, J107088, pyrazolo [1,5-a] indole derivatives, such as GS-5, lamellarin D or irinotecan (Camptosar, CPT-11);
d) said tumor cell is resistant to paclitaxel, docetaxol, adriamycin, camptothecin, Mitoxanthrone, platinum-based compound, or GLEEVEC®,
optionally wherein said platinum-based compound is carboplatin, cisplatin, oxaliplatin, iproplatin, tetraplatin, lobaplatin, DCP, PLD-147, JM118, JM335, or satraplatin;
e) said solid tumor is: breast cancer, cervical cancer, colorectal cancer, peritoneal cancer, ovarian cancer, bronchial cancer, small cell lung cancer, non-small cell lung cancer, gastric, prostate, head and neck cancer, or metastases thereof; or liver cancer, stomach cancer, colon cancer, pancreas cancer, skin cancer, testicular cancer, lung cancer, or metastases thereof.
f) said tumor cell is a sarcoma cell, optionally a GIST cell;
g) the resistance of said tumor cell is mediated by one or more mutations of a kinase,
optionally wherein said kinase is c-kit or bcr-abl; or
h) said compound is administered to an individual suffering from a cancer or tumor refractory to or previously treated with said therapeutic agent, optionally wherein

aa) said therapeutic agent is paclitaxel, Taxotere, Camptothecin, Cisplatin, Mitoxanthrone or GLEEVEC®,
bb) said individual is further administered with one or more anti-emetic or anti-diarrheal agents, or

cc) wherein said individual is further administered with one or more other anti-cancer therapeutic agents.

**14.** A compound of formula

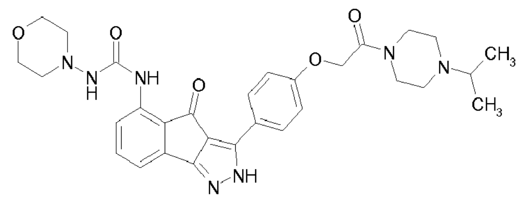

(A37),

or

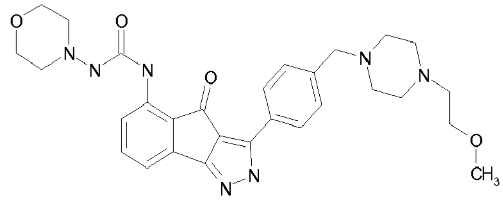

(B16),

or a pharmaceutically acceptable salt, isomer or prodrug thereof,
for use in the treatment of a solid tumor,
wherein

a) the solid tumor is resistant or refractory to a taxane-based therapeutic agent; or
b) the solid tumor is resistant or refractory to a platinum-based therapeutic agent.

**15.** An article of manufacture comprising a pharmaceutical composition and a label which indicates that said pharmaceutical composition can be used for the treatment of an individual suffering from a solid tumor resistant or refractory to a chemotherapeutic agent, wherein said pharmaceutical composition comprises a compound together with a pharmaceutically acceptable carrier, diluent or vehicle, wherein the compound is compound A37 or a compound as recited in any of claims 1-9, or an isomeric, prodrug, tautomeric, pharmaceutically acceptable salt, N-oxide, or stereoisomeric form thereof,
optionally further comprising

a) packaging material, wherein said pharmaceutical composition is contained within said packaging, or wherein said label is contained in or is comprised by said packaging; and/or
b) an anti-emetic or anti-diarrheal agent, or wherein said label further indicates that an anti-emetic or anti-diarrheal agent is to be further administered with said pharmaceutical composition.

Figure 1

(a)

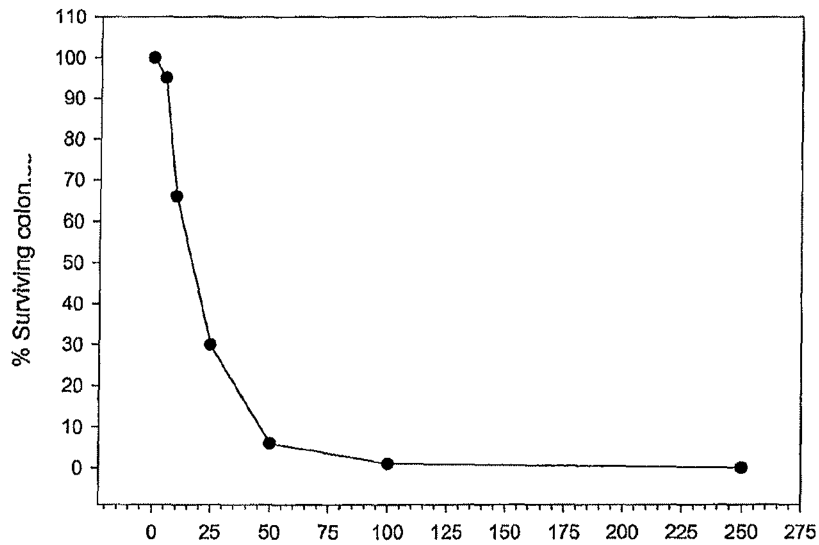
% Surviving colon...
110
100
90
80
70
60
50
40
30
20
10
0
0
25
50
75
100
125
150
175
200
225
250
275

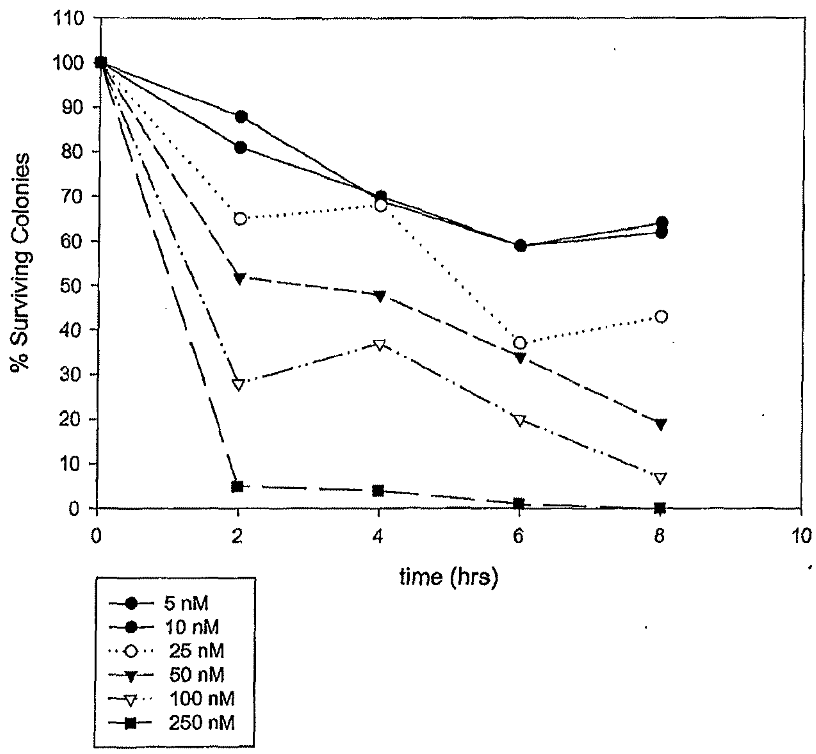
% Surviving Colonies
110
100
90
80
70
60
50
40
30
20
10
0
0
2
4
6
8
10
time (hrs)
5 nM
10 nM
25 nM
50 nM
100 nM
250 nM

(b)

Figure 2

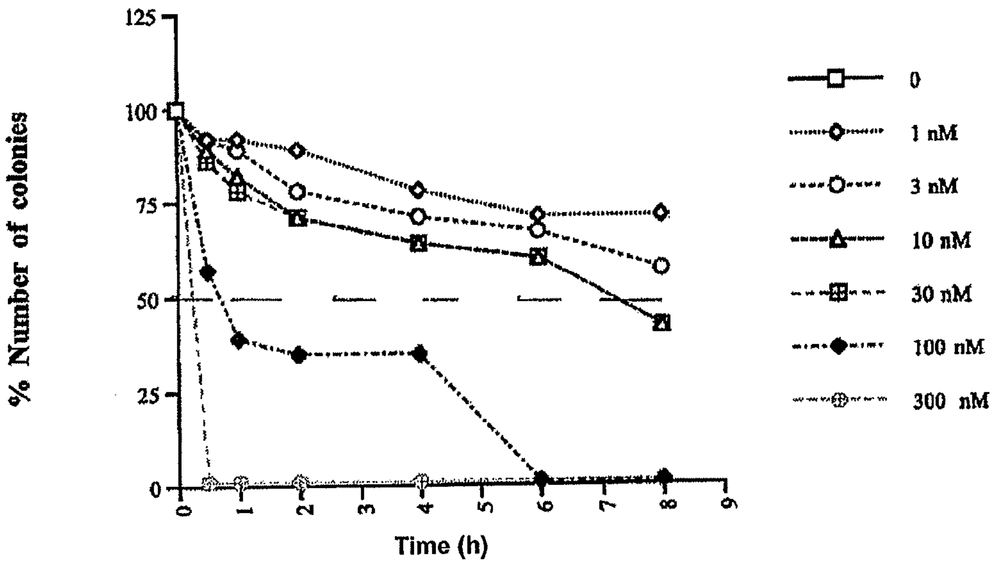
% Number of colonies
125
100
75
50
25
0
0
1
2
3
4
5
6
7
8
9
Time (h)
0
1 nM
3 nM
10 nM
30 nM
100 nM
300 nM

FIGURE 3

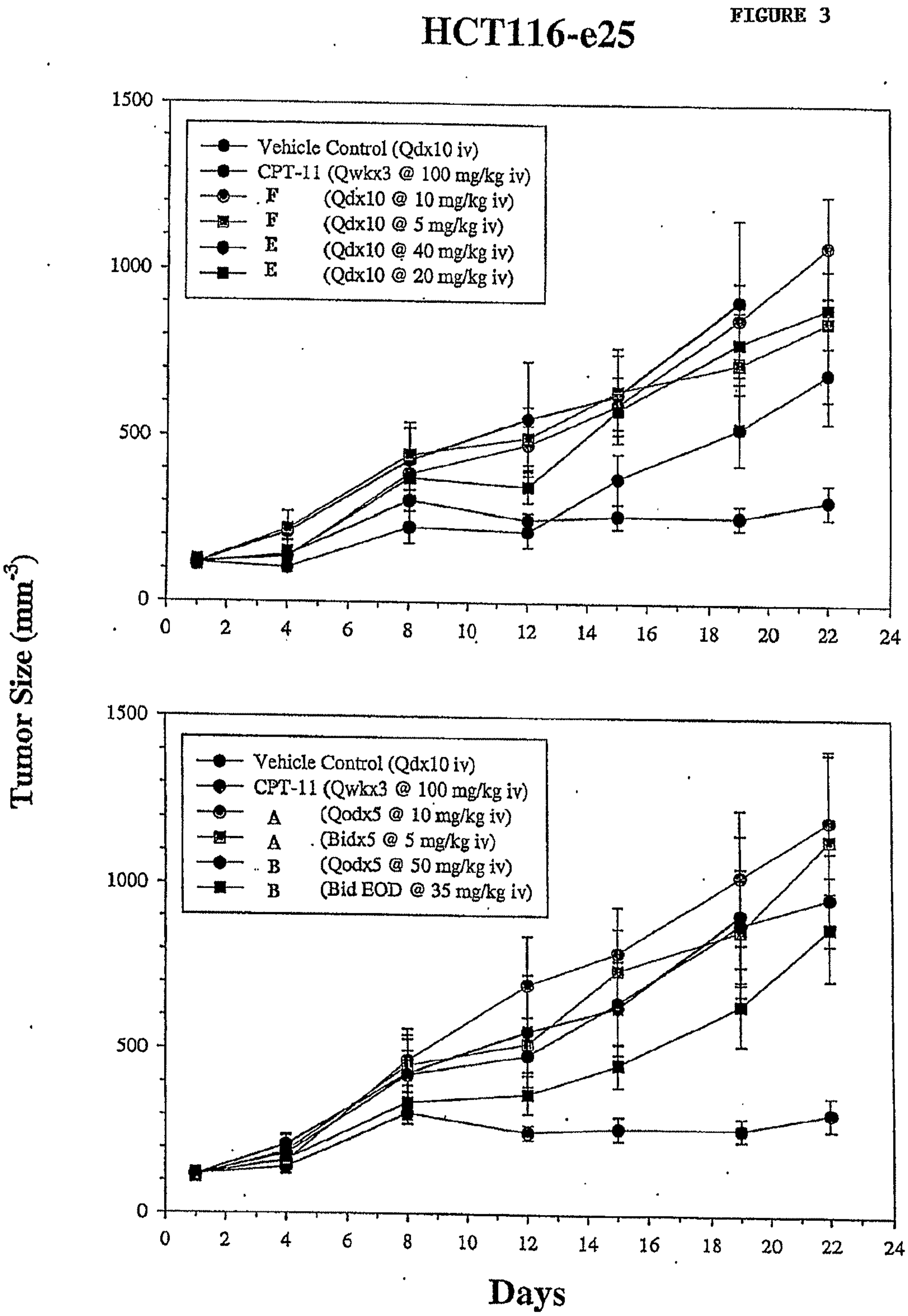
HCT116-e25
Tumor Size (mm^-3)
Days
Vehicle Control (Qdx10 iv)
CPT-11 (Qwkx3 @ 100 mg/kg iv)
F (Qdx10 @ 10 mg/kg iv)
F (Qdx10 @ 5 mg/kg iv)
E (Qdx10 @ 40 mg/kg iv)
E (Qdx10 @ 20 mg/kg iv)
Vehicle Control (Qdx10 iv)
CPT-11 (Qwkx3 @ 100 mg/kg iv)
A (Qodx5 @ 10 mg/kg iv)
A (Bidx5 @ 5 mg/kg iv)
B (Qodx5 @ 50 mg/kg iv)
B (Bid EOD @ 35 mg/kg iv)

HCT116-e26 FIGURE 3

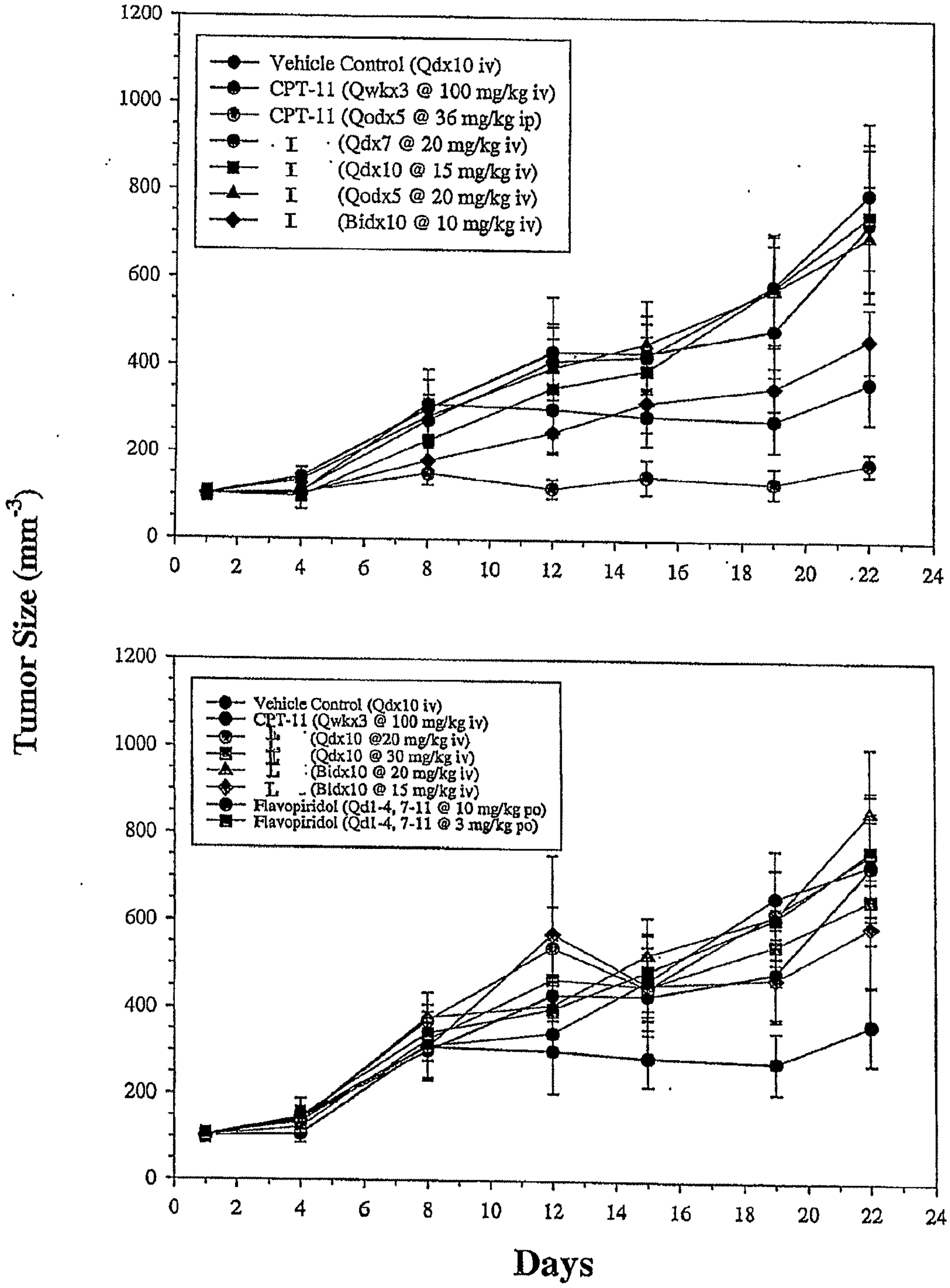

# HCT116-e28

FIGURE 3

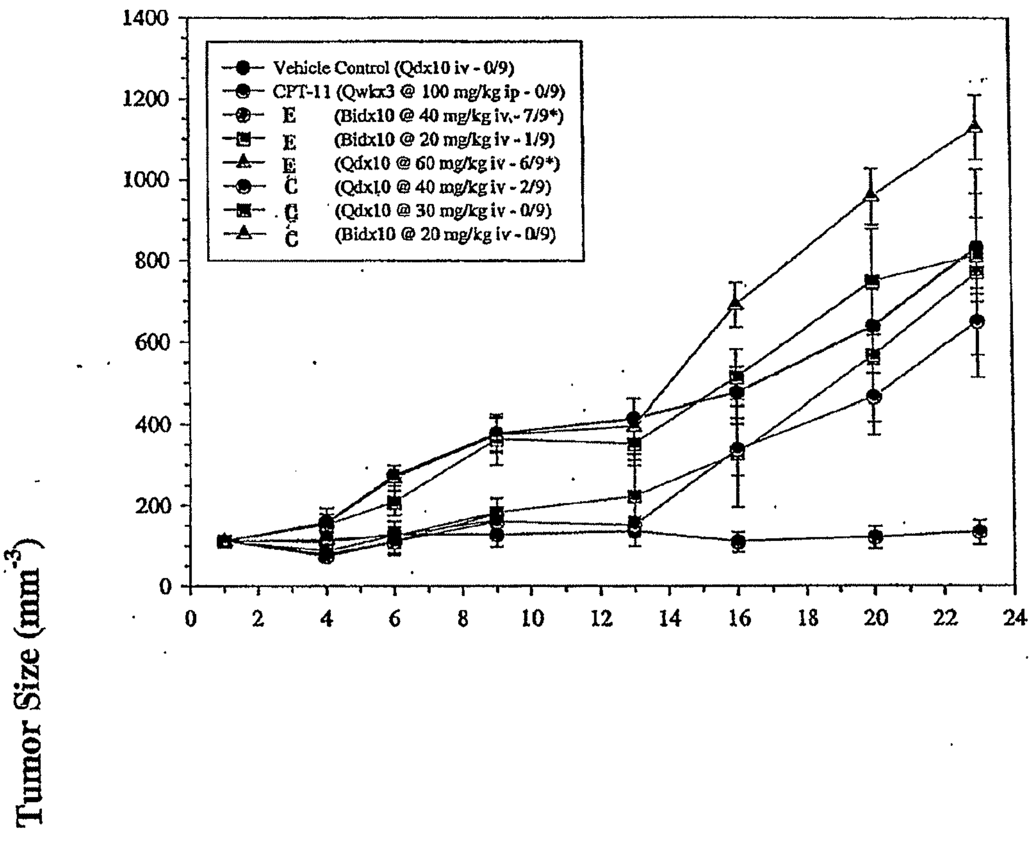

# HCT116-e31

FIGURE 3

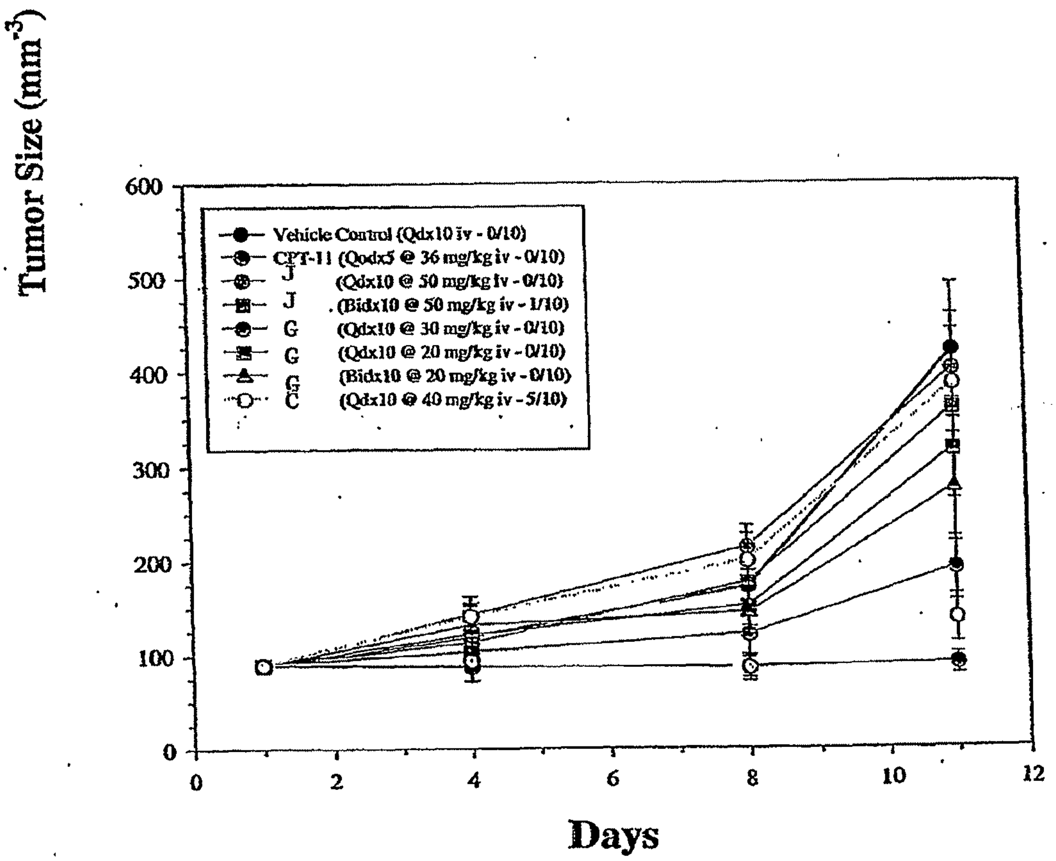

FIGURE 3

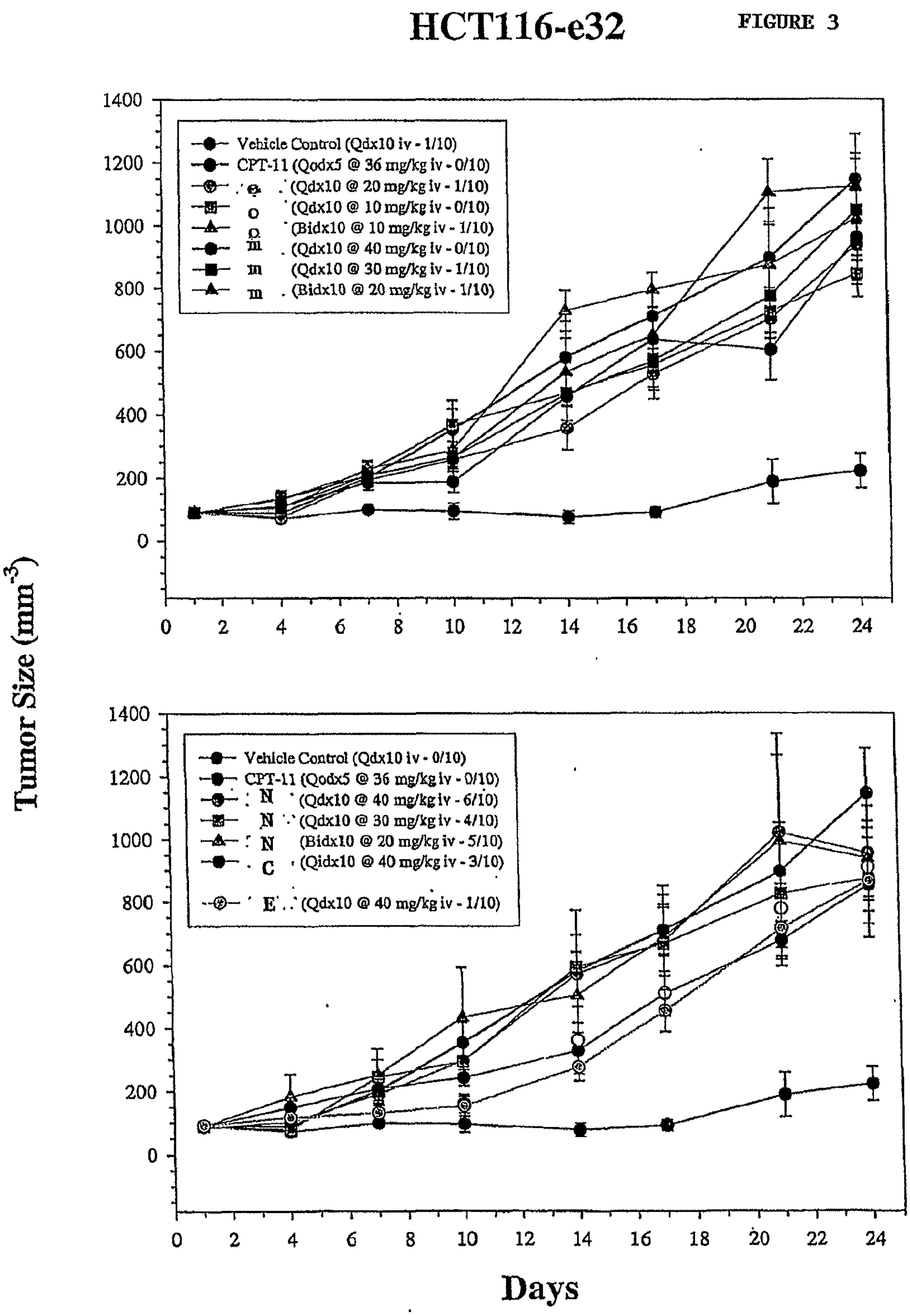
HCT116-e32
Vehicle Control (Qdx10 iv - 1/10)
CPT-11 (Qodx5 @ 36 mg/kg iv - 0/10)
(Qdx10 @ 20 mg/kg iv - 1/10)
(Qdx10 @ 10 mg/kg iv - 0/10)
(Bidx10 @ 10 mg/kg iv - 1/10)
(Qdx10 @ 40 mg/kg iv - 0/10)
(Qdx10 @ 30 mg/kg iv - 1/10)
(Bidx10 @ 20 mg/kg iv - 1/10)
Vehicle Control (Qdx10 iv - 0/10)
CPT-11 (Qodx5 @ 36 mg/kg iv - 0/10)
N (Qdx10 @ 40 mg/kg iv - 6/10)
N (Qdx10 @ 30 mg/kg iv - 4/10)
N (Bidx10 @ 20 mg/kg iv - 5/10)
C (Qidx10 @ 40 mg/kg iv - 3/10)
E (Qdx10 @ 40 mg/kg iv - 1/10)
Tumor Size (mm^-3)
Days

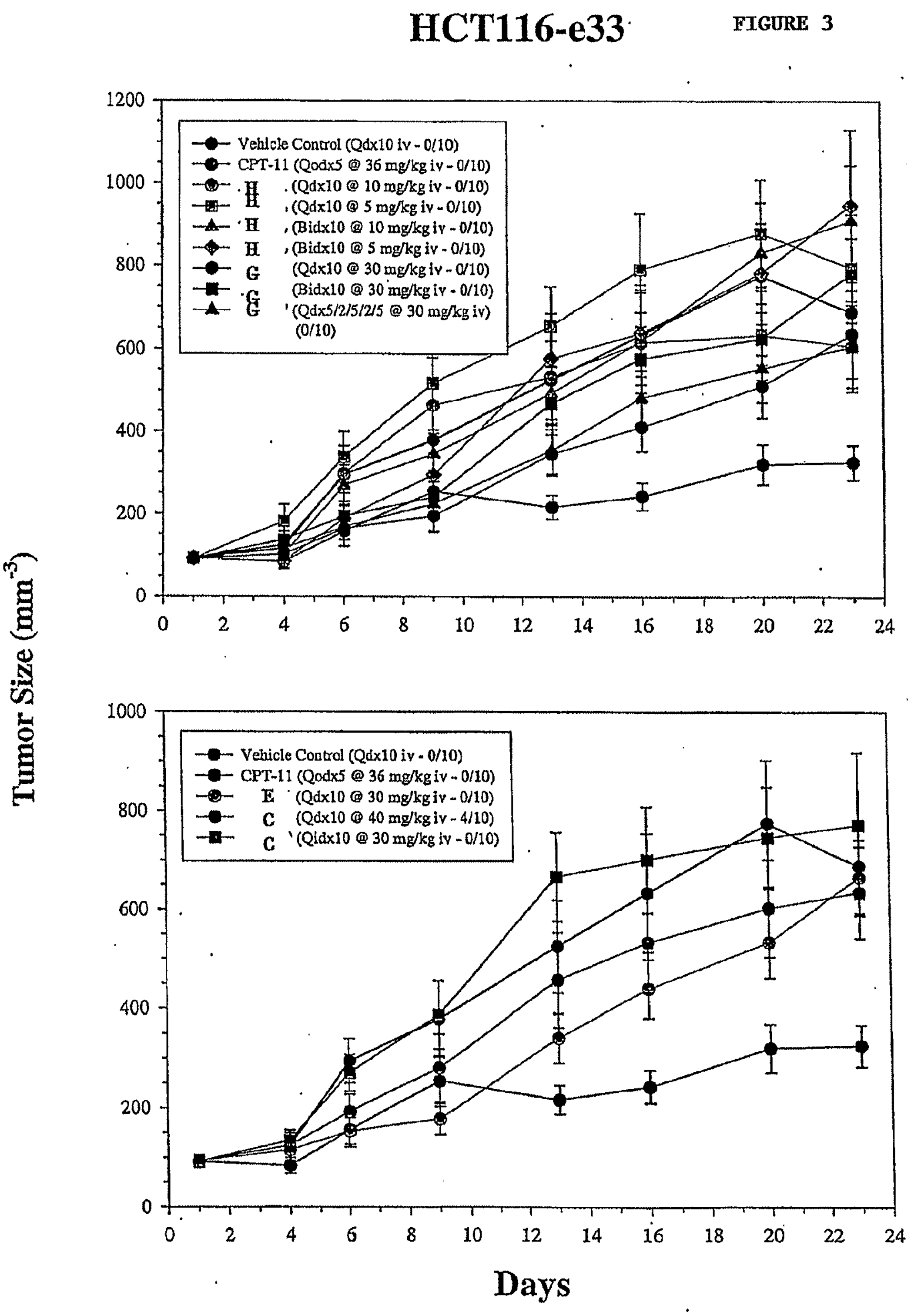
HCT116-e33
FIGURE 3
Vehicle Control (Qdx10 iv - 0/10)
CPT-11 (Qodx5 @ 36 mg/kg iv - 0/10)
H (Qdx10 @ 10 mg/kg iv - 0/10)
H (Qdx10 @ 5 mg/kg iv - 0/10)
H (Bidx10 @ 10 mg/kg iv - 0/10)
H (Bidx10 @ 5 mg/kg iv - 0/10)
G (Qdx10 @ 30 mg/kg iv - 0/10)
G (Bidx10 @ 30 mg/kg iv - 0/10)
G (Qdx5/2/5/2/5 @ 30 mg/kg iv) (0/10)
Vehicle Control (Qdx10 iv - 0/10)
CPT-11 (Qodx5 @ 36 mg/kg iv - 0/10)
E (Qdx10 @ 30 mg/kg iv - 0/10)
C (Qdx10 @ 40 mg/kg iv - 4/10)
C (Qidx10 @ 30 mg/kg iv - 0/10)
Tumor Size (mm-3)
Days

# HCT116-e34

FIGURE 3

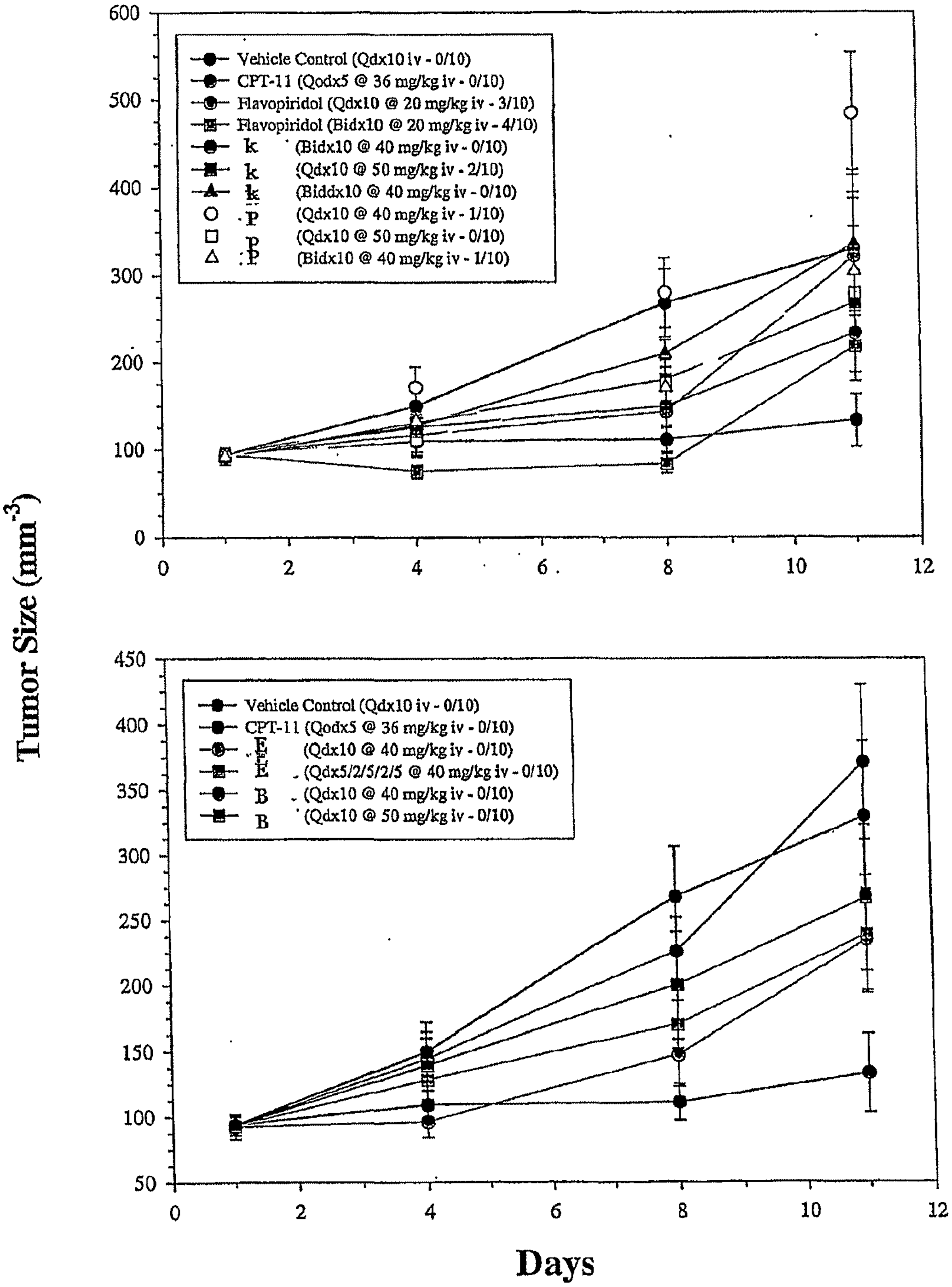

Figure 4

(a)

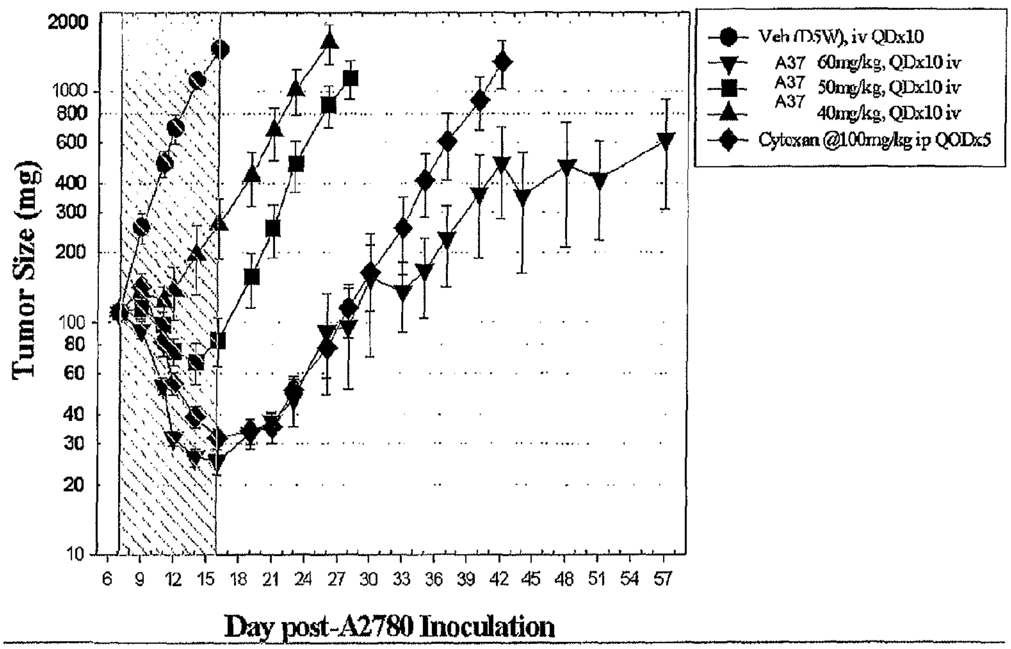


(b)

| Treatment | Dose (mg/Kg) | Max % BW loss (day) | PR | CR | Cures | Max TGI (day) | LCK (Td:2.2) |
|---|---|---|---|---|---|---|---|
| Vehicle | ----- | ------ | --- | --- | --- | ---------- | ---------- |
| Cytoxan (QODx5) | 100 (ip) | -8.5 (16) | 9 | 1 | 0 | 98.5 (19) | 3.47 |
| A37 (QDx10) | 60 (iv) | -23.6 (12) | 9 | 1 | 3 | 98.5 (19) | 3.64 |
| A37 (QDx10) | 50 (iv) | -14 (16) | 5 | 1 | 0 | 94.5 (16) | 1.73 |
| A37 (QDx10) | 40 (iv) | -9.7(16) | 1 | 0 | 0 | 82.5 (14) | 1.23 |

Figure 5

(a)

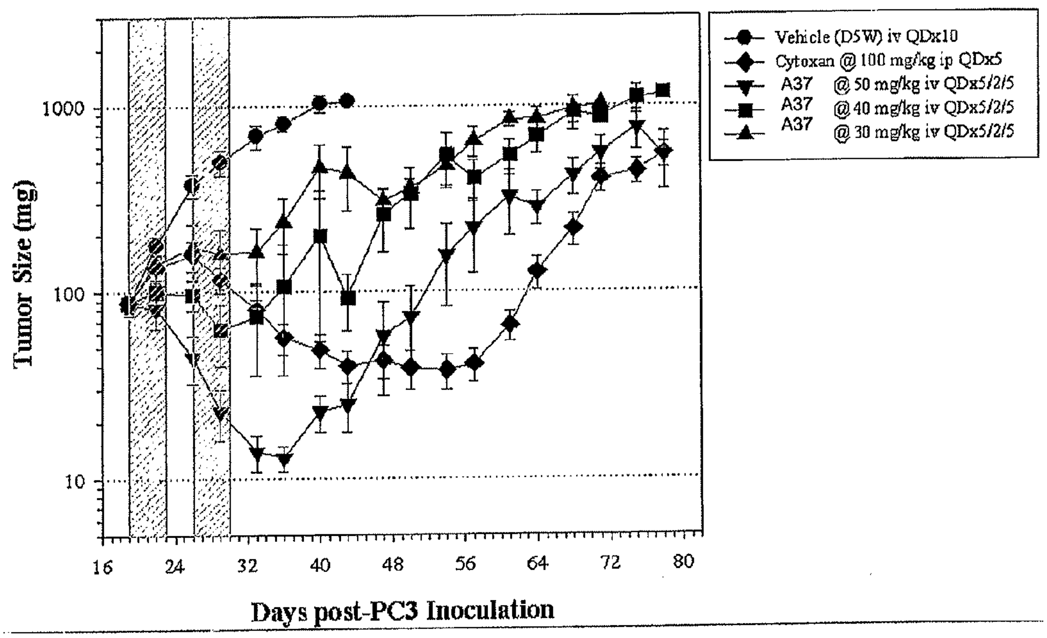


(b)

| Treatment | Dose (mg/Kg) | Max % BW loss (day) | PR | CR | Cures | Max TGI (day) | LCK (Td:2.2) |
|---|---|---|---|---|---|---|---|
| Vehicle | ----- | ------ | --- | --- | --- | ---------- | ---------- |
| Cytoxan (QDx5) | 100 (ip) | ------ | 5 | 0 | 0 | 96 (25) | 3.47 |
| A37 (QDx5/2/5) | 50 (iv) | -5.7 (15) | 8 | 0 | 1 | 98 (18) | 1.69 |
| A37 (QDx5/2/5) | 40 (iv) | ------ | 4 | 1 | 0 | 89 (15) | 1.21 |
| A37 (QDx5/2/5) | 30 (iv) | ------ | 1 | 0 | 0 | 76 (15) | 0.29 |

Figure 6

(a)

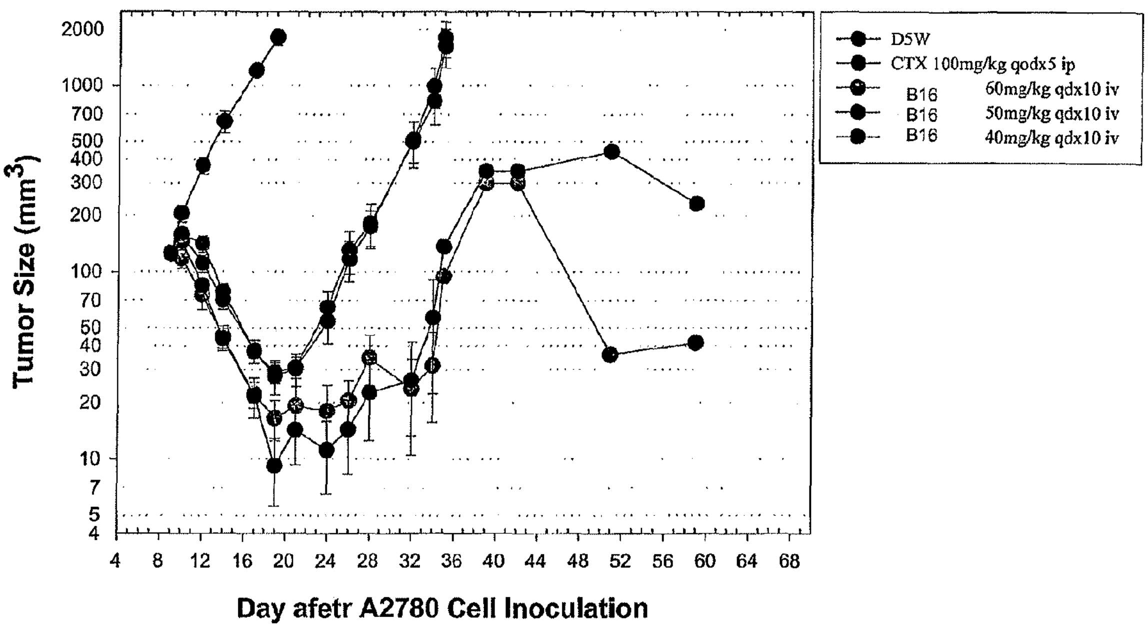


(b)

| Treatment | Dose (mg/Kg) | Toxic Deaths | PR | CR | Cures | Max TGI (day) | LCK (Td:2.2) |
|---|---|---|---|---|---|---|---|
| Vehicle | ----- | 0 | --- | --- | --- | ---------- | ---------- |
| Cytoxan (QDx5) | 100 (ip) | 0 | 3 | 7 | 0 | 99 (15) | 4.4 |
| B16 (QDx10) | 70 (iv) | 2 | 0 | 8 | 5 | 99 (15) | >4.4 |
| B16 (QDx10) | 50 (iv) | 0 | 1 | 9 | 7 | 99 (15) | >4.4 |
| B16 (QDx10) | 30 (iv) | 0 | 2 | 1 | 1 | 79 (14) | 1.7 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03013540 A1
- US 20040077663 A1
- WO 04024942 A1
- WO 041024942 A1
- EP 0147926 A
- US 5072011 A
- US 5244919 A
- US 5519155 A
- US 6503943 B
- WO 01064696 A
- US SN60546097 P
- US 5440056 A
- US 4942184 A
- US 6380405 B
- WO 03101998 A
- US 6100273 A
- US 5587673 A
- WO 0071752 A
- WO 03099210 A, Giannakakou
- WO 2000 A
- WO 9920791 A
- US 6114365 A
- US 6107305 A
- US SN10321284 A
- US 20030162797 A
- US SN10492116 A
- US SN10820453 A
- WO 3033499 A
- US SN10819899 A
- US 0410381 W
- WO 03033499 A
- WO 0147533 A2
- EP 1180105 B1
- US 20040072836 A1
- US 20030187004 A1
- WO 0170729 A1
- WO 03037891 A1
- WO 03004478 A1
- WO 02066480 A2
- WO 03020702 A2
- US 20030211040 A1
- WO 0141768 A2
- WO 03066086 A2
- US 6319955 B
- US 6150401 A
- WO 9934792 A1
- WO 0228388 A2
- WO 02069960 A2
- EP 1399211 A1
- EP 1391211 A1
- WO 0031106 A1
- WO 04035082 A2
- WO 0013015 A1
- US 20020056144 A1
- US 20040067883 A1
- WO 0205792 A2
- US 20030148395 A1
- US 20020058245 A1
- WO 9958982 A1
- US 6221850 B
- WO 9909214 A1
- US 20040097531 A1
- WO 0120003 A2
- WO 03064466 A1
- WO 03105766 A2
- WO 0071096 A2
- US 2003190688 A1
- WO 2003037322 A1
- WO 03043591 A1
- WO 03013540 A
- US 20040101850 A1
- WO 04038422 A2
- EP 1206265 B1
- US 20030207873 A1
- US 20030171389 A1
- WO 03065995 A2
- WO 9807835 A2
- WO 9816638 A1
- US 6685938 B
- US 6190912 B
- US 6600024 B
- WO 9950414 A1
- US 20020019346 A1
- WO 0247710 A2
- US 20020151497 A1
- WO 04010986 A1
- CA 2084120 AA
- DE 10237423 A1
- WO 2004037996 A2
- WO 2004032709 A2
- EP 0639979 B1
- US 20030103937 A1
- WO 03035049 A2
- WO 03028711 A2
- US 20020010203 A1
- WO 04014903 A1
- WO 02102976 A2
- WO 9718232 A3

- WO 9509365 A1
- EP 0721586 B1
- US 6066463 A
- WO 9625520 A1
- US 6107457 A

**Non-patent literature cited in the description**


- **BROXTERMAN ; GEORGOPAPADAKOU.** *Drug Resist Updat.,* 2004, vol. 7 (2), 79-87
- **HARBOUR et al.** *Cell,* 1999, vol. 98, 859-869
- **CARPENTER.** *Cell,* 1984, vol. 37, 357-358
- **WAGMAN et al.** *Curr Pharm Des.,* 2004, vol. 10 (10), 1105-37
- **WOLFEL et al.** *Science,* 1995, vol. 269, 1281-1284
- **YANG-FENG et al.** *Cytogenet. Cell Genet.,* 1985, vol. 40, 784
- **COUSSENS et al.** *Science,* 1985, vol. 230, 1132-1139
- **SEMBA et al.** *Proc. Nat. Acad. Sci.,* 1985, vol. 82, 6497-6501
- **DI FIORE et al.** *Science,* 1987, vol. 237, 178-182
- **AKIYAMA et al.** *Science,* 1986, vol. 232, 1644-1646
- **FUKUSHIGE et al.** *Biochem. Biophys. Res. Commun.,* 1986, vol. 134, 477-483
- **VAN DE VIJVER et al.** *New Eng. J. Med.,* 1988, vol. 319, 1239-1245
- **SLAMON et al.** *Science,* 1989, vol. 244, 707-712
- **CORBIN et al.** *Blood,* 2000, vol. 96, 470a
- **CORBIN et al.** *J. Biol. Chem.,* 2002, vol. 277, 32214-32219
- **SCHINDLER et al.** *Science,* 2000, vol. 289, 1938-1942
- Computer-Assisted Drug Design. **J. PHILLIP BOWEN ; MICHAEL CORY.** Encyclopedia of Pharmaceutical Technology
- **RAYNAUD et al.** *Cancer Chemother. Pharmacol.,* 1996, vol. 38, 155-162
- Remington's Pharmaceutical Sciences. Mack Publishing Company, 1990, 1445
- **MANNING et al.** The Protein Kinase Complement of the Human Genome. *Science,* 2002, vol. 298, 1912-34
- **HONG et al.** *Science,* 1998, vol. 282, 1914-1917
- **HUTTON et al.** *Nature,* 1998, vol. 393, 702-705
- **POORKAJ et al.** *Ann Neurol,* 1998, vol. 43, 815-825
- **CLARK.** *Proc. Natl. Acad. Sci. USA,* 1998, vol. 95, 13103-13107
- **SCHWAB et al.** *Neurobiol Aging,* 2000, vol. 21 (4), 503-10
- **WILLIAMSON et al.** *J. Neurochem.,* 2002, vol. 22, 10-20
- **SLACK ; BERSE.** Society for Neuroscience Abstracts. *Conference/Meeting Information: 28th Annual Meeting of the Society for Neuroscience, Part 1, Los Angeles, California, USA, Nov. 7-12, 1998 Society for Neuroscience,* 07 November 1998, vol. 24 (1-2), ISSN 0190-5295, 208
- **YATES.** Role of c-Fes in normal and neoplastic. *hematopoiesis. Stem Cells,* January 1996, vol. 14 (1), 117
- **HAGEMEIJER et al.** *Hum. Genet.,* 1982, vol. 61, 223-227
- **MINOKOSHI.** *Nature,* 2004, vol. 428 (6982), 569-74
- **HOPKINS et al.** *Biochem Soc Trans.,* 2003, vol. 31, 207-12
- **SAMBANDAM ; LOPASCHUK.** *Prog. Lipid Res.,* 2003, vol. 42 (3), 238-56
- **SHEKAN et al.** *J. Natl. Cancer. Inst.,* 1990, vol. 82, 1107-12
- **PAZDUR et al.** *Cancer Treat Res.,* 1993, vol. 19, 3-5 1
- **BISSERY et al.** *Cancer Res.,* 1991, vol. 51, 4845
- *Annu. Rev. Med.,* 1997, vol. 48, 353-374
- **MARKMAN et al.** *Yale Journal of Biology and Medicine,* 1991, vol. 64, 583
- **MCGUIRE et al.** *Ann. Intern. Med.,* 1989, vol. 111, 273
- **HOLMES et al.** *J. Nat. Cancer Inst.,* 1991, vol. 83, 1797
- **EINZIG et al.** *Proc. Am. Soc. Clin. Oncol.,* vol. 20, 46
- **FORASTIRE et al.** *Sem. Oncol.,* 1990, vol. 20, 56
- **WOO et al.** *Nature,* 1994, vol. 368, 750
- **D. G. 1. KINGSTON et al.** Synthesis and Anticancer Activity of Taxol other Derivatives. *Studies in Organic Chemistry,* vol. 26
- New Trends in Natural Products Chemistry. Elvesier, 1986, 219-235
- *Clin Cancer Res,* 1999, vol. 5, 3445-3453
- *Cytokine,* 2002, vol. 17, 234-242
- *Cancer Res,* 1996, vol. 56, 1303-1308
- *Oncogene,* 1996, vol. 13, 1359-1365
- *Oncogene,* 2000, vol. 19, 6550-6565
- *Bioessays,* 2000, vol. 22, 673-680
- *Oncogene,* 2001, vol. 20, 4995-5004
- *Jpn J Clin Oncol,* 1996, vol. 26, 1-5

- *Cancer Chemother Pharmacol,* 2000, vol. 46, 329-337
- *Leukemia,* 1997, vol. 11, 253-257
- *Cancer Res,* 1997, vol. 57, 1109-1115
- *Breast Cancer Res Treat,* 1997, vol. 42, 73-81
- *Cancer Res,* 1996, vol. 56, 3461-3467
- *J Cell Biol,* 1986, vol. 102, 1522-1531
- *Proc Natl Acad Sci USA,* 2000, vol. 97, 8658-8663
- *Cancer Res,* 2001, vol. 61, 6540-6547
- *Surgery,* vol. 130, 143-150
- *Anticancer Drugs,* 2000, vol. 11, 439-443
- *Chemotherapy,* 2000, vol. 46, 327-334
- *Int J Cancer,* 1993, vol. 54, 302-308
- *Int J Cancer,* 2001, vol. 93, 179-184
- *Anticancer Drugs,* 1997, vol. 8, 189-198
- *Br J Cancer,* 2000, vol. 83, 83-88
- *Cancer Res,* 2001, vol. 61, 4258-4265
- *J Leukoc Biol,* 1996, vol. 59, 280-286
- *Mech Dev,* 1998, vol. 73, 117-123
- *Cancer Res,* 1997, vol. 57, 2388-2393
- **PIZZOLATO ; SALTZ.** *The Lancet,* 2003, vol. 361, 2235-42
- **ULUKAN ; SWAAN.** *Drug,* 2002, vol. 62, 2039-57
- **REED.** *Cancer, Principles and Practice of Oncology,* 1993, 390-4001
- **EASTMAN.** *Cancer Cells,* 1990, vol. 2, 275-2802
- **E. K. ROWINSKY ; R. C. DONEHOWER.** *Pharmacology and Therapeutics,* 1991, vol. 52, 35-84
- **MINOTTI, A. M. ; BARLOW, S. B. ; CABRAL, F.** *J Biol Chem,* 1991, vol. 266, 3987-3994
- **HABER, M. ; BURKHART, C. A. ; REGL, D. L. ; MADAFIGLIO, J. ; NORRIS, M. D. ; HORWITZ, S. B.** *J Biol Chem.,* 1995, vol. 270, 31269-75
- **JAFFREZOU, J. P ; DURNONTET, C. ; DENY, W. B. ; DURAN, G. ; CHEN, G. ; TSUCHIYA, E. ; WILSON, L. ; JORDAN, M. A. ; SIKIC, B. 1.** *Oncology Res.,* 1995, vol. 7, 517-27
- **KAVALLARIS, M. ; KUO, D. Y. S. ; BURKHART, C. A. ; REGI, D. L. ; NORRIS, M. D. ; HABER, M. ; HORWITZ, S. B.** *J Clin. Invest.,* 1997, vol. 100, 1282-93
- **RANGANATHAN, S. ; DEXTER, D. W. ; BENETATOS, C. A. ; HUDES, G. R.** *Biochin7. Biophys. Acta,* 1998, vol. 1395, 237-245
- *Jpn J Cancer Res,* vol. 85, 290-297
- *Mol Cell Biol,* 1999, vol. 19, 2242-2250
- *Biochem Pharmacol,* 2001, vol. 62, 1469-1480
- *Cancer Res,* 1998, vol. 58, 4160-4167
- *Nature Rev Cancer,* 2002, vol. 2, 48-58
- *Cancer Res,* 1993, vol. 53, 747-754
- *J Biol Chem,* 1995, vol. 270, 31269-31275
- *Leukemia,* 1994, vol. 8, 465-475
- *Biochem Pharmacol,* 1997, vol. 53, 461-470
- **LEONARD et al.** *The Oncologist,* 2003, vol. 8, 411-424
- *Anticancer Res,* 2002, vol. 22, 2199-2203
- *J Clin Invest,* 1995, vol. 95, 2205-2214
- *Cancer Lett,* 1999, vol. 146, 195-199
- *Nat Genet,* 2001, vol. 27, 23-29
- *Cell,* 1988, vol. 53, 519-529
- *Proc Natl Acad Sci USA,* 1991, vol. 88, 7289-7293
- *Proc Nat1 Acad Sci USA,* 1992, vol. 89, 4564-4568
- *J Clin Invest,* 1997, vol. 99, 1947-1957
- The Camptothecins: Unfolding Their Anticancer Potential. Annals of the New York Academy of Science. vol. 922
- *Oncogene,* 2003, vol. 22, 7296-7304
- *Cancer Res,* 1995, vol. 55, 1339-1346
- **KAPOOR et al.** *Oncology Research,* 1995, vol. 7, 83-95
- *Oncol Res,* 1995, vol. 7, 83-95
- Ann N Y Acad Sci. 2000, vol. 922, 46-55
- **ROSS et al.** *J. Natl. Cancer Inst.,* 1999, vol. 91, 429-433
- **MIYAKE et al.** *Cancer Res.,* 1999, vol. 59, 8-13
- **LITMAN et al.** *J. Cell Sci.,* 2000, vol. 113, 2011-2021
- **DOYLE et al.** *Proc. Natl. Acad. Sci. U.S.A.,* 1998, vol. 95, 15665-15670
- **HAZLEHURST et al.** *Cancer Res.,* 1999, vol. 59, 1021-1028
- **NIELSEN et al.** *Biochem. Pharmacol.,* 2000, vol. 60, 363-370
- **BOONSTRA et al.** *Br J Cancer.,* 18 May 2004
- **SKEKAN et al.** *J. Natl. Cancer. Inst.,* 1990, vol. 82, 1107-12
- **SHEKAN et al.** *J Natl Cancer Inst,* 1990, vol. 82, 1107-112
- Handbook of Fluorescent Probes and Research Products. Molecular Probes Handbook
- **TORRANCE et al.** *Nat. Biotech.,* 2001, vol. 19, 940-945
- *J Biol Chem,* 1997, vol. 272, 17118-17124
- **VICKERS et al.** *Mol Endocrinology,* 1989, vol. 3 (1), 157-164
- *Oncogene,* 2004, vol. 23, 474-482
- *Clin Cancer Res,* 2003, vol. 9, 2778-2785
- **LUM et al.** *Drug Resist. Clin. Onc. Hemat.,* 1995, vol. 9, 319-336
- **SCHINKEL et al.** *Eur. J. Cancer,* 1995, vol. 31A, 1295-1298
- **DEININGER et al.** *Blood,* 2000, vol. 96, 3343-3356
- **SAWYERS.** *N. Engl. J. Med.,* 1999, vol. 340, 1330-1340
- **DRUKER et al.** *N. Engl. J. Med.,* 2001, vol. 344, 1038-1042

- **DRUKER et al.** *N. Engl. J. Med.,* 2001, vol. 344, 1031-1037
- **KANTARJIAN et al.** *N. Engl. J. Med.,* 2002, vol. 346, 645-652
- **SAWYERS et al.** *Blood,* 2002, vol. 99, 3530-3539
- **GORRE et al.** *Science,* 2001, vol. 293, 876-880
- **HOCHHAUS et al.** *Leukemia,* 2002, vol. 16, 2190-2196
- **HOCHHAUS et al.** *Science,* 2001, vol. 293, 2163
- **BRANFORD et al.** *Blood,* 2002, vol. 99, 3472-3475
- **HOFMANN et al.** *Blood,* 2002, vol. 99, 1860-1862
- **ROCHE-LESTIENNE et al.** *Blood,* 2002, vol. 100, 1014-1018
- **SHAH et al.** *Cancer Cell,* 2002, vol. 2, 117
- **BUBNOFF et al.** *Lancet,* 2002, vol. 359, 487-491
- **GORRE et al.** *Science,* 2001, vol. 293, 876
- Remington's Pharmaceutical Sciences. Mack Publishing Company, 1985
- Remington's Pharmaceutical Sciences. Mack Publishing Company, 1990
- *J. Biochem,* 1995, vol. 117, 741-749
- *J. Cell Sci.,* 1995, vol. 108, 2897
- *Cancer Research,* 1997, vol. 57, 3375
- *Progress in Cell Cycle Research,* vol. 5, 489-496
- **SCHWAB.** *Neurobio! Aging.,* 2000, vol. 21 (4), 503-10
- **YASOJIMA.** *Brain Res.,* 2000, vol. 865 (1), 116-20
- *Natur Rev. Drug Discov.,* vol. 2, 554-565
- **BOZYCZKO-COYNE et al.** *Curr Drug Targets CNS Neurol Disord,* February 2002, vol. 1 (1), 31-49
- **MANNING et al.** *Nat Rev Drug Discov.,* July 2003, vol. 2 (7), 554-65
- **VASILEVSKAYA et al.** *Drug Resist Updat.,* June 2003, vol. 6 (3), 147-56
- **BENNETT et al.** *Curr Opin Pharmacol.,* August 2003, vol. 3 (4), 420-5
- **VARFOLOMEEV et al.** *Cell,* 20 February 2004, vol. 116 (4), 491-7
- **LRBY.** *Nat Genet.,* 1999, vol. 21 (2), 187-90
- **SORIANO.** *Cell,* 1991, vol. 64 (4), 693-702
- *Bone,* 1999, vol. 24, 437
- *Curr. Pharm. Des.,* 2002, vol. 8, 2049
- **HAN.** *Clin Cancer Res.,* 1996, vol. 2 (8), 1397-404
- *Curr. Pharm. Des.,* 2000, vol. 6, 59
- *Expert Opin. Biol. Ther.,* 2004, vol. 4, 837
- *Biochim Biophys Acta,* 2004, vol. 1697, 53-69
- *Science,* 2002, vol. 296, 1639
- *JBC,* 1996, vol. 271, 695
- *Nature Medicine,* 2000, vol. 6, 429
- **PEREGO et al.** *Cancer Research,* 2001, vol. 61, 6034-6037
- **R.D. BAIRD ; S.B. KAYE.** Drug resistance reversal - are we getting closer?. *European Journal of Cancer,* 2003, vol. 39, 2450-61
- **P. GIANNOKAKOU et al.** *J. Biol. Chem.,* 1997, vol. 272, 17118-125
- **P. GIANNOKAKOU et al.** *Proc. Natl. Acad. Sci. U.S.A.,* 2000, vol. 97, 2904-2909
- **KELLAND L R.** An update on satraplatin: the first orally available platinum anticancer drug. *Expert Opin Investig Drugs,* 2000, vol. 9 (6), 1373-1382
- **KISHI.** *Leuk. Res.,* 1985, vol. 9, 381-390
- **GORRE et al.** *Science,* 2001, vol. 293, 876-80
- **BUBNOFF et al.** *Lancet,* 2002, vol. 359, 487-91